(19) Europäisches Patentamt
European Patent Office
Office européen des brevets

(11) EP 3 243 832 A1

(12) **EUROPEAN PATENT APPLICATION**

(43) Date of publication:
**15.11.2017 Bulletin 2017/46**

(21) Application number: **16169487.2**

(22) Date of filing: **13.05.2016**

(51) Int Cl.:
***C07K 14/705*** $^{(2006.01)}$ ***A61K 39/395*** $^{(2006.01)}$
***C07K 16/28*** $^{(2006.01)}$ ***C12N 15/62*** $^{(2006.01)}$

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **F. Hoffmann-La Roche AG**
**4070 Basel (CH)**

(72) Inventor: **The designation of the inventor has not yet been filed**

(74) Representative: **Klostermeyer-Rauber, Dörte**
**F. Hoffmann-La Roche AG**
**Corporate Law Patents (CLP)**
**Grenzacherstrasse 124**
**4070 Basel (CH)**

(54) **ANTIGEN BINDING MOLECULES COMPRISING A TNF FAMILY LIGAND TRIMER AND PD1 BINDING MOIETY**

(57) The invention relates to novel TNF family ligand trimer-containing antigen binding molecules comprising (a) at least one moiety capable of specific binding to PD1 and (b) a first and a second polypeptide that are linked to each other by a disulfide bond, characterized in that the first polypeptide comprises two ectodomains of a TNF ligand family member or fragments thereof that are connected to each other by a peptide linker and in that the second polypeptide comprises only one ectodomain of said TNF ligand family member or a fragment thereof.

EP 3 243 832 A1

SEQUENCE LISTING

<110> F. Hoffmann-La Roche AG

<120> Antigen Binding Molecules comprising a TNF family ligand trimer and PD1 binding moiety

<130> P33618-EP

<160> 170

<170> PatentIn version 3.5

<210> 1
<211> 184
<212> PRT
<213> Homo sapiens

<400> 1

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175
```

```
Gly Leu Pro Ser Pro Arg Ser Glu
            180


<210>  2
<211>  170
<212>  PRT
<213>  Homo sapiens

<400>  2

Leu Asp Leu Arg Gln Gly Met Phe Ala Gln Leu Val Ala Gln Asn Val
1               5                   10                  15


Leu Leu Ile Asp Gly Pro Leu Ser Trp Tyr Ser Asp Pro Gly Leu Ala
            20                  25                  30


Gly Val Ser Leu Thr Gly Gly Leu Ser Tyr Lys Glu Asp Thr Lys Glu
        35                  40                  45


Leu Val Val Ala Lys Ala Gly Val Tyr Tyr Val Phe Phe Gln Leu Glu
    50                  55                  60


Leu Arg Arg Val Val Ala Gly Glu Gly Ser Gly Ser Val Ser Leu Ala
65                  70                  75                  80


Leu His Leu Gln Pro Leu Arg Ser Ala Ala Gly Ala Ala Ala Leu Ala
                85                  90                  95


Leu Thr Val Asp Leu Pro Pro Ala Ser Ser Glu Ala Arg Asn Ser Ala
            100                 105                 110


Phe Gly Phe Gln Gly Arg Leu Leu His Leu Ser Ala Gly Gln Arg Leu
        115                 120                 125


Gly Val His Leu His Thr Glu Ala Arg Ala Arg His Ala Trp Gln Leu
    130                 135                 140


Thr Gln Gly Ala Thr Val Leu Gly Leu Phe Arg Val Thr Pro Glu Ile
145                 150                 155                 160


Pro Ala Gly Leu Pro Ser Pro Arg Ser Glu
                165                 170


<210>  3
<211>  175
<212>  PRT
<213>  Homo sapiens

<400>  3
```

```
Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg
    130                 135                 140

His Ala Trp Gln Leu Thr Gln Gly Ala Thr Val Leu Gly Leu Phe Arg
145                 150                 155                 160

Val Thr Pro Glu Ile Pro Ala Gly Leu Pro Ser Pro Arg Ser Glu
                165                 170                 175
```

<210> 4
<211> 203
<212> PRT
<213> Homo sapiens

<400> 4

```
Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
        35                  40                  45
```

```
Val Leu Leu Ile Asp Gly Pro Leu Ser Trp Tyr Ser Asp Pro Gly Leu
    50                  55                  60

Ala Gly Val Ser Leu Thr Gly Gly Leu Ser Tyr Lys Glu Asp Thr Lys
65                  70                  75                  80

Glu Leu Val Val Ala Lys Ala Gly Val Tyr Tyr Val Phe Phe Gln Leu
                85                  90                  95

Glu Leu Arg Arg Val Val Ala Gly Glu Gly Ser Gly Ser Val Ser Leu
            100                 105                 110

Ala Leu His Leu Gln Pro Leu Arg Ser Ala Ala Gly Ala Ala Ala Leu
        115                 120                 125

Ala Leu Thr Val Asp Leu Pro Pro Ala Ser Ser Glu Ala Arg Asn Ser
    130                 135                 140

Ala Phe Gly Phe Gln Gly Arg Leu Leu His Leu Ser Ala Gly Gln Arg
145                 150                 155                 160

Leu Gly Val His Leu His Thr Glu Ala Arg Ala Arg His Ala Trp Gln
                165                 170                 175

Leu Thr Gln Gly Ala Thr Val Leu Gly Leu Phe Arg Val Thr Pro Glu
            180                 185                 190

Ile Pro Ala Gly Leu Pro Ser Pro Arg Ser Glu
        195                 200


<210>  5
<211>  178
<212>  PRT
<213>  Homo sapiens

<400>  5

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60
```

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu

<210> 6
<211> 164
<212> PRT
<213> Homo sapiens

<400> 6

Leu Asp Leu Arg Gln Gly Met Phe Ala Gln Leu Val Ala Gln Asn Val
1               5                   10                  15

Leu Leu Ile Asp Gly Pro Leu Ser Trp Tyr Ser Asp Pro Gly Leu Ala
            20                  25                  30

Gly Val Ser Leu Thr Gly Gly Leu Ser Tyr Lys Glu Asp Thr Lys Glu
        35                  40                  45

Leu Val Val Ala Lys Ala Gly Val Tyr Tyr Val Phe Phe Gln Leu Glu
    50                  55                  60

Leu Arg Arg Val Val Ala Gly Glu Gly Ser Gly Ser Val Ser Leu Ala
65                  70                  75                  80

Leu His Leu Gln Pro Leu Arg Ser Ala Ala Gly Ala Ala Ala Leu Ala
                85                  90                  95

```
Leu Thr Val Asp Leu Pro Pro Ala Ser Ser Glu Ala Arg Asn Ser Ala
            100                 105                 110

Phe Gly Phe Gln Gly Arg Leu Leu His Leu Ser Ala Gly Gln Arg Leu
        115                 120                 125

Gly Val His Leu His Thr Glu Ala Arg Ala Arg His Ala Trp Gln Leu
    130                 135                 140

Thr Gln Gly Ala Thr Val Leu Gly Leu Phe Arg Val Thr Pro Glu Ile
145                 150                 155                 160

Pro Ala Gly Leu
```

```
<210>  7
<211>  169
<212>  PRT
<213>  Homo sapiens

<400>  7

Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg
    130                 135                 140
```

```
His Ala Trp Gln Leu Thr Gln Gly Ala Thr Val Leu Gly Leu Phe Arg
145                 150                 155                 160

Val Thr Pro Glu Ile Pro Ala Gly Leu
                165

<210>  8
<211>  197
<212>  PRT
<213>  Homo sapiens

<400>  8

Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
        35                  40                  45

Val Leu Leu Ile Asp Gly Pro Leu Ser Trp Tyr Ser Asp Pro Gly Leu
    50                  55                  60

Ala Gly Val Ser Leu Thr Gly Gly Leu Ser Tyr Lys Glu Asp Thr Lys
65                  70                  75                  80

Glu Leu Val Val Ala Lys Ala Gly Val Tyr Tyr Val Phe Phe Gln Leu
                85                  90                  95

Glu Leu Arg Arg Val Val Ala Gly Glu Gly Ser Gly Ser Val Ser Leu
            100                 105                 110

Ala Leu His Leu Gln Pro Leu Arg Ser Ala Ala Gly Ala Ala Ala Leu
        115                 120                 125

Ala Leu Thr Val Asp Leu Pro Pro Ala Ser Ser Glu Ala Arg Asn Ser
    130                 135                 140

Ala Phe Gly Phe Gln Gly Arg Leu Leu His Leu Ser Ala Gly Gln Arg
145                 150                 155                 160

Leu Gly Val His Leu His Thr Glu Ala Arg Ala Arg His Ala Trp Gln
                165                 170                 175

Leu Thr Gln Gly Ala Thr Val Leu Gly Leu Phe Arg Val Thr Pro Glu
            180                 185                 190
```

Ile Pro Ala Gly Leu
        195

<210> 9
<211> 378
<212> PRT
<213> Artificial Sequence

<220>
<223> dimeric hu 4-1BBL (71-254) connected by (G4S)2 linker

<400> 9

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Pro Ser Pro Arg Ser Glu Gly Gly Gly Gly Ser Gly Gly Gly
            180                 185                 190

Gly Ser Arg Glu Gly Pro Glu Leu Ser Pro Asp Asp Pro Ala Gly Leu

```
        195                 200                 205

Leu Asp Leu Arg Gln Gly Met Phe Ala Gln Leu Val Ala Gln Asn Val
    210                 215                 220

Leu Leu Ile Asp Gly Pro Leu Ser Trp Tyr Ser Asp Pro Gly Leu Ala
225                 230                 235                 240

Gly Val Ser Leu Thr Gly Gly Leu Ser Tyr Lys Glu Asp Thr Lys Glu
                245                 250                 255

Leu Val Val Ala Lys Ala Gly Val Tyr Tyr Val Phe Phe Gln Leu Glu
            260                 265                 270

Leu Arg Arg Val Val Ala Gly Glu Gly Ser Gly Ser Val Ser Leu Ala
        275                 280                 285

Leu His Leu Gln Pro Leu Arg Ser Ala Ala Gly Ala Ala Ala Leu Ala
    290                 295                 300

Leu Thr Val Asp Leu Pro Pro Ala Ser Ser Glu Ala Arg Asn Ser Ala
305                 310                 315                 320

Phe Gly Phe Gln Gly Arg Leu Leu His Leu Ser Ala Gly Gln Arg Leu
                325                 330                 335

Gly Val His Leu His Thr Glu Ala Arg Ala Arg His Ala Trp Gln Leu
            340                 345                 350

Thr Gln Gly Ala Thr Val Leu Gly Leu Phe Arg Val Thr Pro Glu Ile
        355                 360                 365

Pro Ala Gly Leu Pro Ser Pro Arg Ser Glu
    370                 375


<210>  10
<211>  366
<212>  PRT
<213>  Artificial Sequence

<220>
<223>  dimeric hu 4-1BBL (71-248) connected by (G4S)2 linker

<400>  10

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30
```

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Arg Glu Gly Pro
180 185 190

Glu Leu Ser Pro Asp Asp Pro Ala Gly Leu Leu Asp Leu Arg Gln Gly
195 200 205

Met Phe Ala Gln Leu Val Ala Gln Asn Val Leu Leu Ile Asp Gly Pro
210 215 220

Leu Ser Trp Tyr Ser Asp Pro Gly Leu Ala Gly Val Ser Leu Thr Gly
225 230 235 240

Gly Leu Ser Tyr Lys Glu Asp Thr Lys Glu Leu Val Val Ala Lys Ala
245 250 255

Gly Val Tyr Tyr Val Phe Phe Gln Leu Glu Leu Arg Arg Val Val Ala
260 265 270

Gly Glu Gly Ser Gly Ser Val Ser Leu Ala Leu His Leu Gln Pro Leu

```
            275                 280                 285

Arg Ser Ala Ala Gly Ala Ala Ala Leu Ala Leu Thr Val Asp Leu Pro
    290                 295                 300

Pro Ala Ser Ser Glu Ala Arg Asn Ser Ala Phe Gly Phe Gln Gly Arg
305                 310                 315                 320

Leu Leu His Leu Ser Ala Gly Gln Arg Leu Gly Val His Leu His Thr
                325                 330                 335

Glu Ala Arg Ala Arg His Ala Trp Gln Leu Thr Gln Gly Ala Thr Val
            340                 345                 350

Leu Gly Leu Phe Arg Val Thr Pro Glu Ile Pro Ala Gly Leu
        355                 360                 365


<210>  11
<211>  360
<212>  PRT
<213>  Artificial Sequence

<220>
<223>  dimeric hu 4-1BBL (80-254) connected by (G4S)2 linker

<400>  11

Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125
```

Ala Gly Gln Arg Leu Gly Val His Leu His Thr Glu Ala Arg Ala Arg
130 135 140

His Ala Trp Gln Leu Thr Gln Gly Ala Thr Val Leu Gly Leu Phe Arg
145 150 155 160

Val Thr Pro Glu Ile Pro Ala Gly Leu Pro Ser Pro Arg Ser Glu Gly
165 170 175

Gly Gly Gly Ser Gly Gly Gly Gly Ser Asp Pro Ala Gly Leu Leu Asp
180 185 190

Leu Arg Gln Gly Met Phe Ala Gln Leu Val Ala Gln Asn Val Leu Leu
195 200 205

Ile Asp Gly Pro Leu Ser Trp Tyr Ser Asp Pro Gly Leu Ala Gly Val
210 215 220

Ser Leu Thr Gly Gly Leu Ser Tyr Lys Glu Asp Thr Lys Glu Leu Val
225 230 235 240

Val Ala Lys Ala Gly Val Tyr Tyr Val Phe Phe Gln Leu Glu Leu Arg
245 250 255

Arg Val Val Ala Gly Glu Gly Ser Gly Ser Val Ser Leu Ala Leu His
260 265 270

Leu Gln Pro Leu Arg Ser Ala Ala Gly Ala Ala Ala Leu Ala Leu Thr
275 280 285

Val Asp Leu Pro Pro Ala Ser Ser Glu Ala Arg Asn Ser Ala Phe Gly
290 295 300

Phe Gln Gly Arg Leu Leu His Leu Ser Ala Gly Gln Arg Leu Gly Val
305 310 315 320

His Leu His Thr Glu Ala Arg Ala Arg His Ala Trp Gln Leu Thr Gln
325 330 335

Gly Ala Thr Val Leu Gly Leu Phe Arg Val Thr Pro Glu Ile Pro Ala
340 345 350

Gly Leu Pro Ser Pro Arg Ser Glu
355 360

<210> 12

<211> 416
<212> PRT
<213> Artificial Sequence

<220>
<223> dimeric hu 4-1BBL (52-254) connected by (G4S)2 linker

<400> 12

```
Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
        35                  40                  45

Val Leu Leu Ile Asp Gly Pro Leu Ser Trp Tyr Ser Asp Pro Gly Leu
    50                  55                  60

Ala Gly Val Ser Leu Thr Gly Gly Leu Ser Tyr Lys Glu Asp Thr Lys
65                  70                  75                  80

Glu Leu Val Val Ala Lys Ala Gly Val Tyr Tyr Val Phe Phe Gln Leu
                85                  90                  95

Glu Leu Arg Arg Val Val Ala Gly Glu Gly Ser Gly Ser Val Ser Leu
            100                 105                 110

Ala Leu His Leu Gln Pro Leu Arg Ser Ala Ala Gly Ala Ala Ala Leu
        115                 120                 125

Ala Leu Thr Val Asp Leu Pro Pro Ala Ser Ser Glu Ala Arg Asn Ser
    130                 135                 140

Ala Phe Gly Phe Gln Gly Arg Leu Leu His Leu Ser Ala Gly Gln Arg
145                 150                 155                 160

Leu Gly Val His Leu His Thr Glu Ala Arg Ala Arg His Ala Trp Gln
                165                 170                 175

Leu Thr Gln Gly Ala Thr Val Leu Gly Leu Phe Arg Val Thr Pro Glu
            180                 185                 190

Ile Pro Ala Gly Leu Pro Ser Pro Arg Ser Glu Gly Gly Gly Gly Ser
        195                 200                 205

Gly Gly Gly Gly Ser Pro Trp Ala Val Ser Gly Ala Arg Ala Ser Pro
    210                 215                 220
```

Gly Ser Ala Ala Ser Pro Arg Leu Arg Glu Gly Pro Glu Leu Ser Pro
225                 230                 235                 240

Asp Asp Pro Ala Gly Leu Leu Asp Leu Arg Gln Gly Met Phe Ala Gln
                245                 250                 255

Leu Val Ala Gln Asn Val Leu Leu Ile Asp Gly Pro Leu Ser Trp Tyr
            260                 265                 270

Ser Asp Pro Gly Leu Ala Gly Val Ser Leu Thr Gly Gly Leu Ser Tyr
        275                 280                 285

Lys Glu Asp Thr Lys Glu Leu Val Val Ala Lys Ala Gly Val Tyr Tyr
    290                 295                 300

Val Phe Phe Gln Leu Glu Leu Arg Arg Val Val Ala Gly Glu Gly Ser
305                 310                 315                 320

Gly Ser Val Ser Leu Ala Leu His Leu Gln Pro Leu Arg Ser Ala Ala
                325                 330                 335

Gly Ala Ala Ala Leu Ala Leu Thr Val Asp Leu Pro Pro Ala Ser Ser
            340                 345                 350

Glu Ala Arg Asn Ser Ala Phe Gly Phe Gln Gly Arg Leu Leu His Leu
        355                 360                 365

Ser Ala Gly Gln Arg Leu Gly Val His Leu His Thr Glu Ala Arg Ala
    370                 375                 380

Arg His Ala Trp Gln Leu Thr Gln Gly Ala Thr Val Leu Gly Leu Phe
385                 390                 395                 400

Arg Val Thr Pro Glu Ile Pro Ala Gly Leu Pro Ser Pro Arg Ser Glu
                405                 410                 415

<210>  13
<211>  7
<212>  PRT
<213>  Mus Musculus

<400>  13

Gly Phe Ser Phe Ser Ser Tyr
1               5

<210>  14
<211>  3

<212> PRT
<213> Mus Musculus

<400> 14

Gly Gly Arg
1

<210> 15
<211> 9
<212> PRT
<213> Mus Musculus

<400> 15

Thr Gly Arg Val Tyr Phe Ala Leu Asp
1 5

<210> 16
<211> 11
<212> PRT
<213> Mus Musculus

<400> 16

Ser Glu Ser Val Asp Thr Ser Asp Asn Ser Phe
1 5 10

<210> 17
<211> 3
<212> PRT
<213> Mus Musculus

<400> 17

Arg Ser Ser
1

<210> 18
<211> 6
<212> PRT
<213> Mus Musculus

<400> 18

Asn Tyr Asp Val Pro Trp
1 5

<210> 19
<211> 120
<212> PRT
<213> Mus Musculus

<400> 19

Glu Val Ile Leu Val Glu Ser Gly Gly Gly Leu Val Lys Pro Gly Gly
1 5 10 15

Ser Leu Lys Leu Ser Cys Ala Ala Ser Gly Phe Ser Phe Ser Ser Tyr
            20                  25                  30

Thr Met Ser Trp Val Arg Gln Thr Pro Glu Lys Arg Leu Asp Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Glu Met Ser Ser Leu Met Ser Glu Asp Thr Ala Leu Tyr Tyr Cys
                85                  90                  95

Val Leu Leu Thr Gly Arg Val Tyr Phe Ala Leu Asp Ser Trp Gly Gln
            100                 105                 110

Gly Thr Ser Val Thr Val Ser Ser
        115                 120

<210> 20
<211> 111
<212> PRT
<213> Mus Musculus

<400> 20

Lys Ile Val Leu Thr Gln Ser Pro Ala Ser Leu Pro Val Ser Leu Gly
1               5                   10                  15

Gln Arg Ala Thr Ile Ser Cys Arg Ala Ser Glu Ser Val Asp Thr Ser
            20                  25                  30

Asp Asn Ser Phe Ile His Trp Tyr Gln Gln Arg Pro Gly Gln Ser Pro
        35                  40                  45

Lys Leu Leu Ile Tyr Arg Ser Ser Thr Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Val Ala Thr Tyr Tyr Cys Gln Gln Asn Tyr
                85                  90                  95

Asp Val Pro Trp Thr Phe Gly Gly Gly Thr Lys Leu Glu Ile Lys
            100                 105                 110

<210> 21
<211> 120
<212> PRT
<213> Artificial Sequence

<220>
<223> humanized variant -heavy chain variable domain VH of PD1-0103-0312, -0313, -0314 and -0315

<400> 21

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Ser Phe Ser Ser Tyr
20 25 30

Thr Met Ser Trp Val Arg Gln Ala Pro Gly Lys Gly Leu Glu Trp Val
35 40 45

Ala Thr Ile Ser Gly Gly Gly Arg Asp Ile Tyr Tyr Pro Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Val Leu Leu Thr Gly Arg Val Tyr Phe Ala Leu Asp Ser Trp Gly Gln
100 105 110

Gly Thr Leu Val Thr Val Ser Ser
115 120

<210> 22
<211> 111
<212> PRT
<213> Artificial Sequence

<220>
<223> humanized variant -light chain variable domain VL of PD1-0103-0312

<400> 22

Asp Ile Val Met Thr Gln Ser Pro Asp Ser Leu Ala Val Ser Leu Gly
1 5 10 15

Glu Arg Ala Thr Ile Asn Cys Lys Ala Ser Glu Ser Val Asp Thr Ser
20 25 30

```
Asp Asn Ser Phe Ile His Trp Tyr Gln Gln Lys Pro Gly Gln Ser Pro
        35                  40                  45

Lys Leu Leu Ile Tyr Arg Ser Ser Thr Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Asn Tyr
                85                  90                  95

Asp Val Pro Trp Thr Phe Gly Gln Gly Thr Lys Val Glu Ile Lys
            100                 105                 110


<210>  23
<211>  111
<212>  PRT
<213>  Artificial Sequence

<220>
<223>  humanized variant -light chain variable domain VL of
       PD1-0103-0313

<400>  23

Asp Val Val Met Thr Gln Ser Pro Leu Ser Leu Pro Val Thr Leu Gly
1               5                   10                  15

Gln Pro Ala Ser Ile Ser Cys Arg Ala Ser Glu Ser Val Asp Thr Ser
            20                  25                  30

Asp Asn Ser Phe Ile His Trp Tyr Gln Gln Arg Pro Gly Gln Ser Pro
        35                  40                  45

Arg Leu Leu Ile Tyr Arg Ser Ser Thr Leu Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Val Tyr Tyr Cys Gln Gln Asn Tyr
                85                  90                  95

Asp Val Pro Trp Thr Phe Gly Gln Gly Thr Lys Val Glu Ile Lys
            100                 105                 110


<210>  24
<211>  111
<212>  PRT
<213>  Artificial Sequence
```

<220>
<223> humanized variant -light chain variable domain VL of PD1-0103-0314

<400> 24

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Asp Thr Ser
            20                  25                  30

Asp Asn Ser Phe Ile His Trp Tyr Gln Gln Lys Pro Gly Gln Ser Pro
        35                  40                  45

Arg Leu Leu Ile Tyr Arg Ser Ser Thr Leu Glu Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Asn Tyr
                85                  90                  95

Asp Val Pro Trp Thr Phe Gly Gln Gly Thr Lys Val Glu Ile Lys
            100                 105                 110

<210> 25
<211> 111
<212> PRT
<213> Artificial Sequence

<220>
<223> humanized variant -light chain variable domain VL of PD1-0103-0315

<400> 25

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Asp Thr Ser
            20                  25                  30

Asp Asn Ser Phe Ile His Trp Tyr Gln Gln Lys Pro Gly Gln Ser Pro
        35                  40                  45

Arg Leu Leu Ile Tyr Arg Ser Ser Thr Leu Glu Ser Gly Ile Pro Ala
    50                  55                  60

Arg Phe Ser Gly Ser Gly Ser Gly Thr Asp Phe Thr Leu Thr Ile Ser

65 70 75 80

Ser Leu Glu Pro Glu Asp Phe Ala Val Tyr Tyr Cys Gln Gln Asn Tyr
85 90 95

Asp Val Pro Trp Thr Phe Gly Gln Gly Thr Lys Val Glu Ile Lys
100 105 110

<210> 26
<211> 8
<212> PRT
<213> Artificial Sequence

<220>
<223> heavy chain HVR-H1, PD1-0098

<400> 26

Gly Tyr Ser Ile Thr Ser Asp Tyr
1 5

<210> 27
<211> 3
<212> PRT
<213> Artificial Sequence

<220>
<223> heavy chain HVR-H2, PD1-0098

<400> 27

Tyr Ser Gly
1

<210> 28
<211> 9
<212> PRT
<213> Artificial Sequence

<220>
<223> heavy chain HVR-H3, PD1-0098

<400> 28

His Gly Ser Ala Pro Trp Tyr Phe Asp
1 5

<210> 29
<211> 12
<212> PRT
<213> Artificial Sequence

<220>
<223> light chain HVR-L1, PD1-0098

<400> 29

Ser Gln Asn Ile Val His Ser Asp Gly Asn Thr Tyr
1               5                   10

<210>  30
<211>  3
<212>  PRT
<213>  Artificial Sequence

<220>
<223>  light chain HVR-L2, PD1-0098

<400>  30

Lys Val Ser
1

<210>  31
<211>  6
<212>  PRT
<213>  Artificial Sequence

<220>
<223>  light chain HVR-L3, PD1-0098

<400>  31

Gly Ser His Phe Pro Leu
1               5

<210>  32
<211>  120
<212>  PRT
<213>  Artificial Sequence

<220>
<223>  heavy chain variable domain VH, PD1-0098

<400>  32

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asp Lys Leu Glu Trp
        35                  40                  45

Leu Gly Tyr Ile Thr Tyr Ser Gly Phe Thr Asn Tyr Asn Pro Ser Leu
    50                  55                  60

Lys Ser Arg Ile Ser Ile Ser Arg Asp Thr Ser Lys Asn Gln Phe Phe
65                  70                  75                  80

Leu Gln Leu Asn Ser Val Ala Thr Glu Asp Thr Ala Thr Tyr Tyr Cys

85 90 95

Ala Arg Trp His Gly Ser Ala Pro Trp Tyr Phe Asp Tyr Trp Gly Arg
100 105 110

Gly Thr Thr Leu Thr Val Ser Ser
115 120

<210> 33
<211> 112
<212> PRT
<213> Artificial Sequence

<220>
<223> light chain variable domain VL, PD1-0098

<400> 33

Asp Val Leu Met Thr Gln Thr Pro Leu Ser Leu Pro Val Ser Leu Gly
1 5 10 15

Asp Gln Ala Ser Ile Ser Cys Arg Ser Ser Gln Asn Ile Val His Ser
20 25 30

Asp Gly Asn Thr Tyr Leu Glu Trp Tyr Leu Gln Lys Pro Gly Gln Ser
35 40 45

Pro Asn Leu Leu Ile Tyr Lys Val Ser Arg Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Leu Gly Val Tyr Tyr Cys Phe Gln Gly
85 90 95

Ser His Phe Pro Leu Thr Phe Gly Ala Gly Thr Lys Leu Glu Leu Lys
100 105 110

<210> 34
<211> 8
<212> PRT
<213> Artificial Sequence

<220>
<223> heavy chain HVR-H1, PD1-0050

<400> 34

Gly Tyr Ser Ile Thr Ser Asp Tyr
1 5

<210> 35
<211> 3
<212> PRT
<213> Artificial Sequence

<220>
<223> heavy chain HVR-H2, PD1-0050

<400> 35

Tyr Thr Gly
1

<210> 36
<211> 9
<212> PRT
<213> Artificial Sequence

<220>
<223> heavy chain HVR-H3, PD1-0050

<400> 36

Met Asp Tyr Tyr Gly Ser Thr Leu Asp
1 5

<210> 37
<211> 11
<212> PRT
<213> Artificial Sequence

<220>
<223> light chain HVR-L1, PD1-0050

<400> 37

Ser Glu Ser Val Asp Arg Tyr Gly Asn Ser Phe
1 5 10

<210> 38
<211> 3
<212> PRT
<213> Artificial Sequence

<220>
<223> light chain HVR-L2, PD1-0050

<400> 38

Arg Ala Ser
1

<210> 39
<211> 6
<212> PRT
<213> Artificial Sequence

<220>
<223> light chain HVR-L3, PD1-0050

<400> 39

Asn Asn Glu Asp Pro Tyr
1               5

<210> 40
<211> 120
<212> PRT
<213> Artificial Sequence

<220>
<223> heavy chain variable domain VH, PD1-0050

<400> 40

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Thr Tyr Thr Gly Arg Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Glu Met Asp Tyr Tyr Gly Ser Thr Leu Asp Tyr Trp Gly Gln
            100                 105                 110

Gly Thr Thr Leu Thr Val Ser Ser
        115                 120

<210> 41
<211> 111
<212> PRT
<213> Artificial Sequence

<220>
<223> light chain variable domain VL, PD1-0050

<400> 41

Lys Ile Val Leu Thr Gln Ser Pro Ala Ser Leu Ala Val Ser Leu Arg
1               5                   10                  15

Gln Arg Ala Thr Ile Ser Cys Arg Ala Ser Glu Ser Val Asp Arg Tyr
20 25 30

Gly Asn Ser Phe Ile His Trp Tyr Gln Gln Lys Pro Gly Gln Pro Pro
35 40 45

Lys Val Leu Ile Tyr Arg Ala Ser Asn Leu Glu Ser Gly Phe Pro Ala
50 55 60

Arg Phe Ser Gly Ser Gly Ser Arg Thr Asp Phe Thr Leu Thr Ile Asp
65 70 75 80

Pro Val Glu Ala Asp Asp Ala Ala Thr Tyr Tyr Cys Gln Gln Asn Asn
85 90 95

Glu Asp Pro Tyr Thr Phe Gly Ser Gly Thr Lys Leu Glu Ile Lys
100 105 110

<210> 42
<211> 7
<212> PRT
<213> Artificial Sequence

<220>
<223> heavy chain HVR-H1, PD1-0069

<400> 42

Gly Tyr Thr Phe Thr Asp Tyr
1 5

<210> 43
<211> 3
<212> PRT
<213> Artificial Sequence

<220>
<223> heavy chain HVR-H2, PD1-0069

<400> 43

Tyr Ser Gly
1

<210> 44
<211> 7
<212> PRT
<213> Artificial Sequence

<220>
<223> heavy chain HVR-H3, PD1-0069

<400> 44

Gly Ile Thr Thr Gly Phe Ala

1 5

<210> 45
<211> 11
<212> PRT
<213> Artificial Sequence

<220>
<223> light chain HVR-L1, PD1-0069

<400> 45

Ser Lys Gly Val Ser Thr Ser Ser Tyr Ser Phe
1 5 10

<210> 46
<211> 3
<212> PRT
<213> Artificial Sequence

<220>
<223> light chain HVR-L2, PD1-0069

<400> 46

Tyr Ala Ser
1

<210> 47
<211> 6
<212> PRT
<213> Artificial Sequence

<220>
<223> light chain HVR-L3, PD1-0069

<400> 47

Ser Arg Glu Phe Pro Trp
1 5

<210> 48
<211> 118
<212> PRT
<213> Artificial Sequence

<220>
<223> heavy chain variable domain VH, PD1-0069

<400> 48

Gln Val Gln Leu Gln Gln Ser Gly Pro Glu Leu Val Arg Pro Gly Val
1 5 10 15

Ser Val Lys Ile Ser Cys Lys Gly Ser Gly Tyr Thr Phe Thr Asp Tyr
20 25 30

```
Ala Met His Trp Val Lys Gln Ser His Ala Arg Thr Leu Glu Trp Ile
        35                  40                  45

Gly Val Ile Ser Thr Tyr Ser Gly Asp Thr Asn Tyr Asn Gln Lys Phe
    50                  55                  60

Lys Asp Lys Ala Thr Met Thr Val Asp Lys Ser Ser Ser Thr Ala Tyr
65                  70                  75                  80

Leu Glu Leu Ala Arg Met Thr Ser Glu Asp Ser Ala Ile Tyr Tyr Cys
                85                  90                  95

Ala Arg Leu Gly Ile Thr Thr Gly Phe Ala Tyr Trp Gly Gln Gly Thr
            100                 105                 110

Leu Val Thr Val Ser Ala
        115


<210>  49
<211>  111
<212>  PRT
<213>  Artificial Sequence

<220>
<223>  light chain variable domain VL, PD1-0069

<400>  49

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Gly Val Ser Thr Ser
            20                  25                  30

Ser Tyr Ser Phe Met His Trp Tyr Gln Gln Lys Pro Arg Gln Pro Pro
        35                  40                  45

Lys Leu Leu Ile Lys Tyr Ala Ser Tyr Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys His His Ser Arg
                85                  90                  95

Glu Phe Pro Trp Thr Phe Gly Gly Gly Thr Lys Leu Glu Ile Lys
            100                 105                 110


<210>  50
```

<211>  7
<212>  PRT
<213>  Artificial Sequence

<220>
<223>  heavy chain HVR-H1, PD1-0073

<400>  50

Gly Phe Thr Phe Ser Asn Tyr
1               5

<210>  51
<211>  3
<212>  PRT
<213>  Artificial Sequence

<220>
<223>  heavy chain HVR-H2, PD1-0073

<400>  51

Gly Gly Arg
1

<210>  52
<211>  5
<212>  PRT
<213>  Artificial Sequence

<220>
<223>  heavy chain HVR-H3, PD1-0073

<400>  52

Tyr Tyr Gly Ile Asp
1               5

<210>  53
<211>  7
<212>  PRT
<213>  Artificial Sequence

<220>
<223>  light chain HVR-L1, PD1-0073

<400>  53

Ser Gln Asp Val Thr Thr Ala
1               5

<210>  54
<211>  3
<212>  PRT
<213>  Artificial Sequence

<220>
<223>  light chain HVR-L2, PD1-0073

<400> 54

Trp Ala Ser
1

<210> 55
<211> 6
<212> PRT
<213> Artificial Sequence

<220>
<223> light chain HVR-L3, PD1-0073

<400> 55

His Tyr Ser Ile Pro Trp
1 5

<210> 56
<211> 116
<212> PRT
<213> Artificial Sequence

<220>
<223> heavy chain variable domain VH, PD1-0073

<400> 56

Glu Val Lys Leu Val Glu Ser Gly Gly Gly Leu Val Lys Pro Gly Gly
1 5 10 15

Ser Leu Lys Leu Ser Cys Ala Ala Ser Gly Phe Thr Phe Ser Asn Tyr
20 25 30

Gly Met Ser Trp Ile Arg Gln Thr Pro Glu Lys Gly Leu Glu Trp Val
35 40 45

Ala Thr Ile Ser Gly Gly Gly Arg Asp Thr Tyr Tyr Pro Asp Ser Val
50 55 60

Lys Gly Arg Phe Thr Ile Ser Arg Asp Asn Val Lys Asn Asn Leu Tyr
65 70 75 80

Leu Gln Met Ser Ser Leu Arg Ser Glu Asp Thr Ala Phe Tyr Tyr Cys
85 90 95

Ala Ser Tyr Tyr Tyr Gly Ile Asp Tyr Trp Gly Gln Gly Thr Ser Val
100 105 110

Thr Val Ser Ser
115

<210> 57

<211> 107
<212> PRT
<213> Artificial Sequence

<220>
<223> light chain variable domain VL, PD1-0073

<400> 57

Asp Ile Val Met Thr Gln Pro His Lys Phe Met Ser Thr Ser Val Gly
1 5 10 15

Asp Arg Val Arg Ile Thr Cys Lys Ala Ser Gln Asp Val Thr Thr Ala
20 25 30

Val Ala Trp Tyr Gln Gln Lys Pro Gly Gln Ser Pro Lys Leu Leu Ile
35 40 45

Tyr Trp Ala Ser Thr Arg His Thr Gly Val Pro Asp Arg Phe Thr Gly
50 55 60

Ser Gly Ser Gly Thr Glu Phe Thr Leu Thr Ile Ser Ser Val Gln Ala
65 70 75 80

Glu Asp Leu Ala Leu Tyr Tyr Cys Gln Gln His Tyr Ser Ile Pro Trp
85 90 95

Thr Phe Gly Gly Gly Thr Lys Leu Glu Ile Lys
100 105

<210> 58
<211> 7
<212> PRT
<213> Artificial Sequence

<220>
<223> heavy chain HVR-H1, PD1-0078

<400> 58

Gly Tyr Thr Phe Thr Ser Thr
1 5

<210> 59
<211> 3
<212> PRT
<213> Artificial Sequence

<220>
<223> heavy chain HVR-H2, PD1-0078

<400> 59

Ser Asp Ser
1

<210> 60
<211> 3
<212> PRT
<213> Artificial Sequence

<220>
<223> heavy chain HVR-H3, PD1-0078

<400> 60

Pro Phe Asp
1

<210> 61
<211> 7
<212> PRT
<213> Artificial Sequence

<220>
<223> light chain HVR-L1, PD1-0078

<400> 61

Ser Gln Asp Val Ser Thr Ala
1 5

<210> 62
<211> 3
<212> PRT
<213> Artificial Sequence

<220>
<223> light chain HVR-L2, PD1-0078

<400> 62

Ser Ala Ser
1

<210> 63
<211> 6
<212> PRT
<213> Artificial Sequence

<220>
<223> light chain HVR-L3, PD1-0078

<400> 63

His Tyr Ser His Pro Phe
1 5

<210> 64
<211> 114
<212> PRT
<213> Artificial Sequence

<220>
<223>  heavy chain variable domain VH, PD1-0078

<400>  64

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Thr
            20                  25                  30

Trp Met His Trp Val Lys Gln Arg Pro Gly Gln Gly Leu Glu Trp Ile
        35                  40                  45

Gly Ala Ile Asp Pro Ser Asp Ser Tyr Thr Thr Tyr Asn Gln Lys Phe
    50                  55                  60

Lys Gly Lys Ala Thr Leu Thr Val Asp Thr Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Thr Arg Ser Pro Phe Asp Tyr Trp Gly Gln Gly Thr Thr Leu Thr Val
            100                 105                 110

Ser Ser

<210>  65
<211>  107
<212>  PRT
<213>  Artificial Sequence

<220>
<223>  light chain variable domain VL, PD1-0078

<400>  65

Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Ala Ile Ser Ser Val Gln Ala
65                  70                  75                  80

Glu Asp Leu Ala Val Tyr Tyr Cys Gln Gln His Tyr Ser His Pro Phe
                85                  90                  95

Thr Phe Gly Ser Gly Thr Lys Leu Glu Ile Lys
            100                 105

<210> 66
<211> 8
<212> PRT
<213> Artificial Sequence

<220>
<223> heavy chain HVR-H1, PD1-0102

<400> 66

Gly Tyr Ser Ile Thr Ser Gly Tyr
1               5

<210> 67
<211> 3
<212> PRT
<213> Artificial Sequence

<220>
<223> heavy chain HVR-H2, PD1-0102

<400> 67

Ser Ser Gly
1

<210> 68
<211> 6
<212> PRT
<213> Artificial Sequence

<220>
<223> heavy chain HVR-H3, PD1-0102

<400> 68

Arg Asn Trp Tyr Phe Asp
1               5

<210> 69
<211> 13
<212> PRT
<213> Artificial Sequence

<220>
<223> light chain HVR-L1, PD1-0102

<400> 69

Ser Gln Ser Leu Leu Asn Ser Gly Thr Gln Lys Asn Tyr
1               5                   10

<210>  70
<211>  3
<212>  PRT
<213>  Artificial Sequence

<220>
<223>  light chain HVR-L2, PD1-0102

<400>  70

Trp Ala Ser
1

<210>  71
<211>  6
<212>  PRT
<213>  Artificial Sequence

<220>
<223>  light chain HVR-L3, PD1-0102

<400>  71

Asp Tyr Thr Phe Pro Leu
1               5

<210>  72
<211>  117
<212>  PRT
<213>  Artificial Sequence

<220>
<223>  heavy chain variable domain VH, PD1-0102

<400>  72

Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Gly
            20                  25                  30

Tyr Ser Trp His Trp Ile Arg Gln Phe Pro Gly Asn Lys Leu Glu Trp
        35                  40                  45

Met Gly Phe Ile His Ser Ser Gly Asp Thr Asn Tyr Asn Pro Ser Leu
    50                  55                  60

Lys Ser Arg Ile Ser Phe Thr Arg Asp Thr Ser Lys Asn Gln Phe Phe
65                  70                  75                  80

Leu Gln Leu Ser Ser Leu Thr Asp Glu Asp Thr Ala Thr Tyr Tyr Cys
85 90 95

Ala Thr Tyr Arg Asn Trp Tyr Phe Asp Val Trp Gly Ala Gly Thr Thr
100 105 110

Val Thr Val Ser Ser
115

<210> 73
<211> 113
<212> PRT
<213> Artificial Sequence

<220>
<223> light chain variable domain VL, PD1-0102

<400> 73

Asp Ile Val Met Thr Gln Ser Pro Ser Ser Leu Thr Val Thr Ala Gly
1 5 10 15

Glu Lys Val Thr Met Arg Cys Lys Ser Ser Gln Ser Leu Leu Asn Ser
20 25 30

Gly Thr Gln Lys Asn Tyr Leu Thr Trp Tyr Gln Gln Lys Pro Gly Gln
35 40 45

Pro Pro Lys Leu Leu Ile Tyr Trp Ala Ser Thr Arg Glu Ser Gly Val
50 55 60

Pro Asn Arg Phe Thr Gly Ser Gly Ser Gly Thr Asp Phe Thr Leu Thr
65 70 75 80

Ile Ser Ser Val Gln Ala Glu Asp Leu Ser Val Tyr Tyr Cys Gln Ser
85 90 95

Asp Tyr Thr Phe Pro Leu Thr Phe Gly Gly Gly Thr Lys Leu Glu Leu
100 105 110

Lys

<210> 74
<211> 722
<212> PRT
<213> Artificial Sequence

<220>
<223> Dimeric hu 4-1BBL (71-254) - CL* Fc knob chain

<400> 74

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Pro Ser Pro Arg Ser Glu Gly Gly Gly Gly Ser Gly Gly Gly
            180                 185                 190

Gly Ser Arg Glu Gly Pro Glu Leu Ser Pro Asp Asp Pro Ala Gly Leu
        195                 200                 205

Leu Asp Leu Arg Gln Gly Met Phe Ala Gln Leu Val Ala Gln Asn Val
    210                 215                 220

Leu Leu Ile Asp Gly Pro Leu Ser Trp Tyr Ser Asp Pro Gly Leu Ala
225                 230                 235                 240

Gly Val Ser Leu Thr Gly Gly Leu Ser Tyr Lys Glu Asp Thr Lys Glu
                245                 250                 255
```

Leu Val Val Ala Lys Ala Gly Val Tyr Tyr Val Phe Phe Gln Leu Glu
260 265 270

Leu Arg Arg Val Val Ala Gly Glu Gly Ser Gly Ser Val Ser Leu Ala
275 280 285

Leu His Leu Gln Pro Leu Arg Ser Ala Ala Gly Ala Ala Ala Leu Ala
290 295 300

Leu Thr Val Asp Leu Pro Pro Ala Ser Ser Glu Ala Arg Asn Ser Ala
305 310 315 320

Phe Gly Phe Gln Gly Arg Leu Leu His Leu Ser Ala Gly Gln Arg Leu
325 330 335

Gly Val His Leu His Thr Glu Ala Arg Ala Arg His Ala Trp Gln Leu
340 345 350

Thr Gln Gly Ala Thr Val Leu Gly Leu Phe Arg Val Thr Pro Glu Ile
355 360 365

Pro Ala Gly Leu Pro Ser Pro Arg Ser Glu Gly Gly Gly Gly Ser Gly
370 375 380

Gly Gly Gly Ser Arg Thr Val Ala Ala Pro Ser Val Phe Ile Phe Pro
385 390 395 400

Pro Ser Asp Arg Lys Leu Lys Ser Gly Thr Ala Ser Val Val Cys Leu
405 410 415

Leu Asn Asn Phe Tyr Pro Arg Glu Ala Lys Val Gln Trp Lys Val Asp
420 425 430

Asn Ala Leu Gln Ser Gly Asn Ser Gln Glu Ser Val Thr Glu Gln Asp
435 440 445

Ser Lys Asp Ser Thr Tyr Ser Leu Ser Ser Thr Leu Thr Leu Ser Lys
450 455 460

Ala Asp Tyr Glu Lys His Lys Val Tyr Ala Cys Glu Val Thr His Gln
465 470 475 480

Gly Leu Ser Ser Pro Val Thr Lys Ser Phe Asn Arg Gly Glu Cys Asp
485 490 495

Lys Thr His Thr Cys Pro Pro Cys Pro Ala Pro Glu Ala Ala Gly Gly

500 505 510

Pro Ser Val Phe Leu Phe Pro Pro Lys Pro Lys Asp Thr Leu Met Ile
515 520 525

Ser Arg Thr Pro Glu Val Thr Cys Val Val Val Asp Val Ser His Glu
530 535 540

Asp Pro Glu Val Lys Phe Asn Trp Tyr Val Asp Gly Val Glu Val His
545 550 555 560

Asn Ala Lys Thr Lys Pro Arg Glu Glu Gln Tyr Asn Ser Thr Tyr Arg
565 570 575

Val Val Ser Val Leu Thr Val Leu His Gln Asp Trp Leu Asn Gly Lys
580 585 590

Glu Tyr Lys Cys Lys Val Ser Asn Lys Ala Leu Gly Ala Pro Ile Glu
595 600 605

Lys Thr Ile Ser Lys Ala Lys Gly Gln Pro Arg Glu Pro Gln Val Tyr
610 615 620

Thr Leu Pro Pro Cys Arg Asp Glu Leu Thr Lys Asn Gln Val Ser Leu
625 630 635 640

Trp Cys Leu Val Lys Gly Phe Tyr Pro Ser Asp Ile Ala Val Glu Trp
645 650 655

Glu Ser Asn Gly Gln Pro Glu Asn Asn Tyr Lys Thr Thr Pro Pro Val
660 665 670

Leu Asp Ser Asp Gly Ser Phe Phe Leu Tyr Ser Lys Leu Thr Val Asp
675 680 685

Lys Ser Arg Trp Gln Gln Gly Asn Val Phe Ser Cys Ser Val Met His
690 695 700

Glu Ala Leu His Asn His Tyr Thr Gln Lys Ser Leu Ser Leu Ser Pro
705 710 715 720

Gly Lys

<210> 75
<211> 297
<212> PRT
<213> Artificial Sequence

<220>
<223>  Monomeric hu 4-1BBL (71-254) - CH1*

<400>  75

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Pro Ser Pro Arg Ser Glu Gly Gly Gly Gly Ser Gly Gly Gly
            180                 185                 190

Gly Ser Ala Ser Thr Lys Gly Pro Ser Val Phe Pro Leu Ala Pro Ser
        195                 200                 205

Ser Lys Ser Thr Ser Gly Gly Thr Ala Ala Leu Gly Cys Leu Val Glu
    210                 215                 220

Asp Tyr Phe Pro Glu Pro Val Thr Val Ser Trp Asn Ser Gly Ala Leu

225 230 235 240

Thr Ser Gly Val His Thr Phe Pro Ala Val Leu Gln Ser Ser Gly Leu
245 250 255

Tyr Ser Leu Ser Ser Val Val Thr Val Pro Ser Ser Ser Leu Gly Thr
260 265 270

Gln Thr Tyr Ile Cys Asn Val Asn His Lys Pro Ser Asn Thr Lys Val
275 280 285

Asp Glu Lys Val Glu Pro Lys Ser Cys
290 295

<210> 76
<211> 722
<212> PRT
<213> Artificial Sequence

<220>
<223> Dimeric hu 4-1BBL (71-254) - CL Fc knob chain

<400> 76

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Pro Ser Pro Arg Ser Glu Gly Gly Gly Gly Ser Gly Gly Gly
180 185 190

Gly Ser Arg Glu Gly Pro Glu Leu Ser Pro Asp Asp Pro Ala Gly Leu
195 200 205

Leu Asp Leu Arg Gln Gly Met Phe Ala Gln Leu Val Ala Gln Asn Val
210 215 220

Leu Leu Ile Asp Gly Pro Leu Ser Trp Tyr Ser Asp Pro Gly Leu Ala
225 230 235 240

Gly Val Ser Leu Thr Gly Gly Leu Ser Tyr Lys Glu Asp Thr Lys Glu
245 250 255

Leu Val Val Ala Lys Ala Gly Val Tyr Tyr Val Phe Phe Gln Leu Glu
260 265 270

Leu Arg Arg Val Val Ala Gly Glu Gly Ser Gly Ser Val Ser Leu Ala
275 280 285

Leu His Leu Gln Pro Leu Arg Ser Ala Ala Gly Ala Ala Ala Leu Ala
290 295 300

Leu Thr Val Asp Leu Pro Pro Ala Ser Ser Glu Ala Arg Asn Ser Ala
305 310 315 320

Phe Gly Phe Gln Gly Arg Leu Leu His Leu Ser Ala Gly Gln Arg Leu
325 330 335

Gly Val His Leu His Thr Glu Ala Arg Ala Arg His Ala Trp Gln Leu
340 345 350

Thr Gln Gly Ala Thr Val Leu Gly Leu Phe Arg Val Thr Pro Glu Ile
355 360 365

Pro Ala Gly Leu Pro Ser Pro Arg Ser Glu Gly Gly Gly Gly Ser Gly
370 375 380

Gly Gly Gly Ser Arg Thr Val Ala Ala Pro Ser Val Phe Ile Phe Pro

385 390 395 400

Pro Ser Asp Glu Gln Leu Lys Ser Gly Thr Ala Ser Val Val Cys Leu
405 410 415

Leu Asn Asn Phe Tyr Pro Arg Glu Ala Lys Val Gln Trp Lys Val Asp
420 425 430

Asn Ala Leu Gln Ser Gly Asn Ser Gln Glu Ser Val Thr Glu Gln Asp
435 440 445

Ser Lys Asp Ser Thr Tyr Ser Leu Ser Ser Thr Leu Thr Leu Ser Lys
450 455 460

Ala Asp Tyr Glu Lys His Lys Val Tyr Ala Cys Glu Val Thr His Gln
465 470 475 480

Gly Leu Ser Ser Pro Val Thr Lys Ser Phe Asn Arg Gly Glu Cys Asp
485 490 495

Lys Thr His Thr Cys Pro Pro Cys Pro Ala Pro Glu Ala Ala Gly Gly
500 505 510

Pro Ser Val Phe Leu Phe Pro Pro Lys Pro Lys Asp Thr Leu Met Ile
515 520 525

Ser Arg Thr Pro Glu Val Thr Cys Val Val Val Asp Val Ser His Glu
530 535 540

Asp Pro Glu Val Lys Phe Asn Trp Tyr Val Asp Gly Val Glu Val His
545 550 555 560

Asn Ala Lys Thr Lys Pro Arg Glu Glu Gln Tyr Asn Ser Thr Tyr Arg
565 570 575

Val Val Ser Val Leu Thr Val Leu His Gln Asp Trp Leu Asn Gly Lys
580 585 590

Glu Tyr Lys Cys Lys Val Ser Asn Lys Ala Leu Gly Ala Pro Ile Glu
595 600 605

Lys Thr Ile Ser Lys Ala Lys Gly Gln Pro Arg Glu Pro Gln Val Tyr
610 615 620

Thr Leu Pro Pro Cys Arg Asp Glu Leu Thr Lys Asn Gln Val Ser Leu
625 630 635 640

```
Trp Cys Leu Val Lys Gly Phe Tyr Pro Ser Asp Ile Ala Val Glu Trp
                645                 650                 655

Glu Ser Asn Gly Gln Pro Glu Asn Asn Tyr Lys Thr Thr Pro Pro Val
            660                 665                 670

Leu Asp Ser Asp Gly Ser Phe Phe Leu Tyr Ser Lys Leu Thr Val Asp
        675                 680                 685

Lys Ser Arg Trp Gln Gln Gly Asn Val Phe Ser Cys Ser Val Met His
    690                 695                 700

Glu Ala Leu His Asn His Tyr Thr Gln Lys Ser Leu Ser Leu Ser Pro
705                 710                 715                 720

Gly Lys
```

<210>  77
<211>  297
<212>  PRT
<213>  Artificial Sequence

<220>
<223>  Monomeric hu 4-1BBL (71-254) - CH1

<400>  77

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
```

115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Pro Ser Pro Arg Ser Glu Gly Gly Gly Gly Ser Gly Gly Gly
180 185 190

Gly Ser Ala Ser Thr Lys Gly Pro Ser Val Phe Pro Leu Ala Pro Ser
195 200 205

Ser Lys Ser Thr Ser Gly Gly Thr Ala Ala Leu Gly Cys Leu Val Lys
210 215 220

Asp Tyr Phe Pro Glu Pro Val Thr Val Ser Trp Asn Ser Gly Ala Leu
225 230 235 240

Thr Ser Gly Val His Thr Phe Pro Ala Val Leu Gln Ser Ser Gly Leu
245 250 255

Tyr Ser Leu Ser Ser Val Val Thr Val Pro Ser Ser Ser Leu Gly Thr
260 265 270

Gln Thr Tyr Ile Cys Asn Val Asn His Lys Pro Ser Asn Thr Lys Val
275 280 285

Asp Lys Lys Val Glu Pro Lys Ser Cys
290 295

<210> 78
<211> 710
<212> PRT
<213> Artificial Sequence

<220>
<223> Dimeric hu 4-1BBL (71-248) - CL* Fc knob chain

<400> 78

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Arg Glu Gly Pro
180 185 190

Glu Leu Ser Pro Asp Asp Pro Ala Gly Leu Leu Asp Leu Arg Gln Gly
195 200 205

Met Phe Ala Gln Leu Val Ala Gln Asn Val Leu Leu Ile Asp Gly Pro
210 215 220

Leu Ser Trp Tyr Ser Asp Pro Gly Leu Ala Gly Val Ser Leu Thr Gly
225 230 235 240

Gly Leu Ser Tyr Lys Glu Asp Thr Lys Glu Leu Val Val Ala Lys Ala
245 250 255

Gly Val Tyr Tyr Val Phe Phe Gln Leu Glu Leu Arg Arg Val Val Ala
260 265 270

Gly Glu Gly Ser Gly Ser Val Ser Leu Ala Leu His Leu Gln Pro Leu

275 280 285

Arg Ser Ala Ala Gly Ala Ala Ala Leu Ala Leu Thr Val Asp Leu Pro
290 295 300

Pro Ala Ser Ser Glu Ala Arg Asn Ser Ala Phe Gly Phe Gln Gly Arg
305 310 315 320

Leu Leu His Leu Ser Ala Gly Gln Arg Leu Gly Val His Leu His Thr
325 330 335

Glu Ala Arg Ala Arg His Ala Trp Gln Leu Thr Gln Gly Ala Thr Val
340 345 350

Leu Gly Leu Phe Arg Val Thr Pro Glu Ile Pro Ala Gly Leu Gly Gly
355 360 365

Gly Gly Ser Gly Gly Gly Gly Ser Arg Thr Val Ala Ala Pro Ser Val
370 375 380

Phe Ile Phe Pro Pro Ser Asp Arg Lys Leu Lys Ser Gly Thr Ala Ser
385 390 395 400

Val Val Cys Leu Leu Asn Asn Phe Tyr Pro Arg Glu Ala Lys Val Gln
405 410 415

Trp Lys Val Asp Asn Ala Leu Gln Ser Gly Asn Ser Gln Glu Ser Val
420 425 430

Thr Glu Gln Asp Ser Lys Asp Ser Thr Tyr Ser Leu Ser Ser Thr Leu
435 440 445

Thr Leu Ser Lys Ala Asp Tyr Glu Lys His Lys Val Tyr Ala Cys Glu
450 455 460

Val Thr His Gln Gly Leu Ser Ser Pro Val Thr Lys Ser Phe Asn Arg
465 470 475 480

Gly Glu Cys Asp Lys Thr His Thr Cys Pro Pro Cys Pro Ala Pro Glu
485 490 495

Ala Ala Gly Gly Pro Ser Val Phe Leu Phe Pro Pro Lys Pro Lys Asp
500 505 510

Thr Leu Met Ile Ser Arg Thr Pro Glu Val Thr Cys Val Val Val Asp
515 520 525

Val Ser His Glu Asp Pro Glu Val Lys Phe Asn Trp Tyr Val Asp Gly
    530                 535                 540

Val Glu Val His Asn Ala Lys Thr Lys Pro Arg Glu Glu Gln Tyr Asn
545                 550                 555                 560

Ser Thr Tyr Arg Val Val Ser Val Leu Thr Val Leu His Gln Asp Trp
                565                 570                 575

Leu Asn Gly Lys Glu Tyr Lys Cys Lys Val Ser Asn Lys Ala Leu Gly
            580                 585                 590

Ala Pro Ile Glu Lys Thr Ile Ser Lys Ala Lys Gly Gln Pro Arg Glu
        595                 600                 605

Pro Gln Val Tyr Thr Leu Pro Pro Cys Arg Asp Glu Leu Thr Lys Asn
    610                 615                 620

Gln Val Ser Leu Trp Cys Leu Val Lys Gly Phe Tyr Pro Ser Asp Ile
625                 630                 635                 640

Ala Val Glu Trp Glu Ser Asn Gly Gln Pro Glu Asn Asn Tyr Lys Thr
                645                 650                 655

Thr Pro Pro Val Leu Asp Ser Asp Gly Ser Phe Phe Leu Tyr Ser Lys
            660                 665                 670

Leu Thr Val Asp Lys Ser Arg Trp Gln Gln Gly Asn Val Phe Ser Cys
        675                 680                 685

Ser Val Met His Glu Ala Leu His Asn His Tyr Thr Gln Lys Ser Leu
    690                 695                 700

Ser Leu Ser Pro Gly Lys
705                 710

<210>  79
<211>  291
<212>  PRT
<213>  Artificial Sequence

<220>
<223>  Monomeric hu 4-1BBL (71-248) - CH1*

<400>  79

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu

20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Ala Ser Thr Lys
180 185 190

Gly Pro Ser Val Phe Pro Leu Ala Pro Ser Ser Lys Ser Thr Ser Gly
195 200 205

Gly Thr Ala Ala Leu Gly Cys Leu Val Glu Asp Tyr Phe Pro Glu Pro
210 215 220

Val Thr Val Ser Trp Asn Ser Gly Ala Leu Thr Ser Gly Val His Thr
225 230 235 240

Phe Pro Ala Val Leu Gln Ser Ser Gly Leu Tyr Ser Leu Ser Ser Val
245 250 255

Val Thr Val Pro Ser Ser Ser Leu Gly Thr Gln Thr Tyr Ile Cys Asn
260 265 270

Val Asn His Lys Pro Ser Asn Thr Lys Val Asp Glu Lys Val Glu Pro
275 280 285

Lys Ser Cys
290

<210> 80
<211> 710
<212> PRT
<213> Artificial Sequence

<220>
<223> Dimeric hu 4-1BBL (71-248) - CL Fc knob chain

<400> 80

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Arg Glu Gly Pro

180 185 190

Glu Leu Ser Pro Asp Asp Pro Ala Gly Leu Leu Asp Leu Arg Gln Gly
195 200 205

Met Phe Ala Gln Leu Val Ala Gln Asn Val Leu Leu Ile Asp Gly Pro
210 215 220

Leu Ser Trp Tyr Ser Asp Pro Gly Leu Ala Gly Val Ser Leu Thr Gly
225 230 235 240

Gly Leu Ser Tyr Lys Glu Asp Thr Lys Glu Leu Val Val Ala Lys Ala
245 250 255

Gly Val Tyr Tyr Val Phe Phe Gln Leu Glu Leu Arg Arg Val Val Ala
260 265 270

Gly Glu Gly Ser Gly Ser Val Ser Leu Ala Leu His Leu Gln Pro Leu
275 280 285

Arg Ser Ala Ala Gly Ala Ala Ala Leu Ala Leu Thr Val Asp Leu Pro
290 295 300

Pro Ala Ser Ser Glu Ala Arg Asn Ser Ala Phe Gly Phe Gln Gly Arg
305 310 315 320

Leu Leu His Leu Ser Ala Gly Gln Arg Leu Gly Val His Leu His Thr
325 330 335

Glu Ala Arg Ala Arg His Ala Trp Gln Leu Thr Gln Gly Ala Thr Val
340 345 350

Leu Gly Leu Phe Arg Val Thr Pro Glu Ile Pro Ala Gly Leu Gly Gly
355 360 365

Gly Gly Ser Gly Gly Gly Gly Ser Arg Thr Val Ala Ala Pro Ser Val
370 375 380

Phe Ile Phe Pro Pro Ser Asp Glu Gln Leu Lys Ser Gly Thr Ala Ser
385 390 395 400

Val Val Cys Leu Leu Asn Asn Phe Tyr Pro Arg Glu Ala Lys Val Gln
405 410 415

Trp Lys Val Asp Asn Ala Leu Gln Ser Gly Asn Ser Gln Glu Ser Val
420 425 430

Thr Glu Gln Asp Ser Lys Asp Ser Thr Tyr Ser Leu Ser Ser Thr Leu
435 440 445

Thr Leu Ser Lys Ala Asp Tyr Glu Lys His Lys Val Tyr Ala Cys Glu
450 455 460

Val Thr His Gln Gly Leu Ser Ser Pro Val Thr Lys Ser Phe Asn Arg
465 470 475 480

Gly Glu Cys Asp Lys Thr His Thr Cys Pro Pro Cys Pro Ala Pro Glu
485 490 495

Ala Ala Gly Gly Pro Ser Val Phe Leu Phe Pro Pro Lys Pro Lys Asp
500 505 510

Thr Leu Met Ile Ser Arg Thr Pro Glu Val Thr Cys Val Val Val Asp
515 520 525

Val Ser His Glu Asp Pro Glu Val Lys Phe Asn Trp Tyr Val Asp Gly
530 535 540

Val Glu Val His Asn Ala Lys Thr Lys Pro Arg Glu Glu Gln Tyr Asn
545 550 555 560

Ser Thr Tyr Arg Val Val Ser Val Leu Thr Val Leu His Gln Asp Trp
565 570 575

Leu Asn Gly Lys Glu Tyr Lys Cys Lys Val Ser Asn Lys Ala Leu Gly
580 585 590

Ala Pro Ile Glu Lys Thr Ile Ser Lys Ala Lys Gly Gln Pro Arg Glu
595 600 605

Pro Gln Val Tyr Thr Leu Pro Pro Cys Arg Asp Glu Leu Thr Lys Asn
610 615 620

Gln Val Ser Leu Trp Cys Leu Val Lys Gly Phe Tyr Pro Ser Asp Ile
625 630 635 640

Ala Val Glu Trp Glu Ser Asn Gly Gln Pro Glu Asn Asn Tyr Lys Thr
645 650 655

Thr Pro Pro Val Leu Asp Ser Asp Gly Ser Phe Phe Leu Tyr Ser Lys
660 665 670

Leu Thr Val Asp Lys Ser Arg Trp Gln Gln Gly Asn Val Phe Ser Cys
675 680 685

Ser Val Met His Glu Ala Leu His Asn His Tyr Thr Gln Lys Ser Leu
690 695 700

Ser Leu Ser Pro Gly Lys
705 710

<210> 81
<211> 291
<212> PRT
<213> Artificial Sequence

<220>
<223> Monomeric hu 4-1BBL (71-248) - CH1

<400> 81

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Ala Ser Thr Lys
180 185 190

Gly Pro Ser Val Phe Pro Leu Ala Pro Ser Ser Lys Ser Thr Ser Gly
195 200 205

Gly Thr Ala Ala Leu Gly Cys Leu Val Lys Asp Tyr Phe Pro Glu Pro
210 215 220

Val Thr Val Ser Trp Asn Ser Gly Ala Leu Thr Ser Gly Val His Thr
225 230 235 240

Phe Pro Ala Val Leu Gln Ser Ser Gly Leu Tyr Ser Leu Ser Ser Val
245 250 255

Val Thr Val Pro Ser Ser Ser Leu Gly Thr Gln Thr Tyr Ile Cys Asn
260 265 270

Val Asn His Lys Pro Ser Asn Thr Lys Val Asp Lys Lys Val Glu Pro
275 280 285

Lys Ser Cys
290

<210> 82
<211> 450
<212> PRT
<213> Artificial Sequence

<220>
<223> anti-PD1 (0314) Fc hole chain

<400> 82

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Ser Phe Ser Ser Tyr
20 25 30

Thr Met Ser Trp Val Arg Gln Ala Pro Gly Lys Gly Leu Glu Trp Val
35 40 45

Ala Thr Ile Ser Gly Gly Gly Arg Asp Ile Tyr Tyr Pro Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys

85 90 95

Val Leu Leu Thr Gly Arg Val Tyr Phe Ala Leu Asp Ser Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Ala Ala Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Gly Ala Pro Ile Glu
325 330 335

Lys Thr Ile Ser Lys Ala Lys Gly Gln Pro Arg Glu Pro Gln Val Cys
340 345 350

Thr Leu Pro Pro Ser Arg Asp Glu Leu Thr Lys Asn Gln Val Ser Leu
355 360 365

Ser Cys Ala Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Val Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 83
<211> 218
<212> PRT
<213> Artificial Sequence

<220>
<223> anti-PD1 (0314) light chain

<400> 83

Glu Ile Val Leu Thr Gln Ser Pro Ala Thr Leu Ser Leu Ser Pro Gly
1 5 10 15

Glu Arg Ala Thr Leu Ser Cys Arg Ala Ser Glu Ser Val Asp Thr Ser
20 25 30

Asp Asn Ser Phe Ile His Trp Tyr Gln Gln Lys Pro Gly Gln Ser Pro
35 40 45

Arg Leu Leu Ile Tyr Arg Ser Ser Thr Leu Glu Ser Gly Ile Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Val Tyr Tyr Cys Gln Gln Asn Tyr

85 90 95

Asp Val Pro Trp Thr Phe Gly Gln Gly Thr Lys Val Glu Ile Lys Arg
100 105 110

Thr Val Ala Ala Pro Ser Val Phe Ile Phe Pro Pro Ser Asp Glu Gln
115 120 125

Leu Lys Ser Gly Thr Ala Ser Val Val Cys Leu Leu Asn Asn Phe Tyr
130 135 140

Pro Arg Glu Ala Lys Val Gln Trp Lys Val Asp Asn Ala Leu Gln Ser
145 150 155 160

Gly Asn Ser Gln Glu Ser Val Thr Glu Gln Asp Ser Lys Asp Ser Thr
165 170 175

Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205

Val Thr Lys Ser Phe Asn Arg Gly Glu Cys
210 215

<210> 84
<211> 837
<212> PRT
<213> Artificial Sequence

<220>
<223> anti-PD1(0314) Fc hole chain fused to dimeric hu 4-1BBL (71-254)

<400> 84

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Ser Phe Ser Ser Tyr
20 25 30

Thr Met Ser Trp Val Arg Gln Ala Pro Gly Lys Gly Leu Glu Trp Val
35 40 45

Ala Thr Ile Ser Gly Gly Gly Arg Asp Ile Tyr Tyr Pro Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Val Leu Leu Thr Gly Arg Val Tyr Phe Ala Leu Asp Ser Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Ala Ala Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Gly Ala Pro Ile Glu

325 330 335

Lys Thr Ile Ser Lys Ala Lys Gly Gln Pro Arg Glu Pro Gln Val Cys
340 345 350

Thr Leu Pro Pro Ser Arg Asp Glu Leu Thr Lys Asn Gln Val Ser Leu
355 360 365

Ser Cys Ala Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Val Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Gly Gly Gly Gly Ser Gly Gly Gly Gly Ser Arg Glu Gly Pro Glu
450 455 460

Leu Ser Pro Asp Asp Pro Ala Gly Leu Leu Asp Leu Arg Gln Gly Met
465 470 475 480

Phe Ala Gln Leu Val Ala Gln Asn Val Leu Leu Ile Asp Gly Pro Leu
485 490 495

Ser Trp Tyr Ser Asp Pro Gly Leu Ala Gly Val Ser Leu Thr Gly Gly
500 505 510

Leu Ser Tyr Lys Glu Asp Thr Lys Glu Leu Val Val Ala Lys Ala Gly
515 520 525

Val Tyr Tyr Val Phe Phe Gln Leu Glu Leu Arg Arg Val Val Ala Gly
530 535 540

Glu Gly Ser Gly Ser Val Ser Leu Ala Leu His Leu Gln Pro Leu Arg
545 550 555 560

Ser Ala Ala Gly Ala Ala Ala Leu Ala Leu Thr Val Asp Leu Pro Pro
565 570 575

Ala Ser Ser Glu Ala Arg Asn Ser Ala Phe Gly Phe Gln Gly Arg Leu
580 585 590

Leu His Leu Ser Ala Gly Gln Arg Leu Gly Val His Leu His Thr Glu
595 600 605

Ala Arg Ala Arg His Ala Trp Gln Leu Thr Gln Gly Ala Thr Val Leu
610 615 620

Gly Leu Phe Arg Val Thr Pro Glu Ile Pro Ala Gly Leu Pro Ser Pro
625 630 635 640

Arg Ser Glu Gly Gly Gly Gly Ser Gly Gly Gly Gly Ser Arg Glu Gly
645 650 655

Pro Glu Leu Ser Pro Asp Asp Pro Ala Gly Leu Leu Asp Leu Arg Gln
660 665 670

Gly Met Phe Ala Gln Leu Val Ala Gln Asn Val Leu Leu Ile Asp Gly
675 680 685

Pro Leu Ser Trp Tyr Ser Asp Pro Gly Leu Ala Gly Val Ser Leu Thr
690 695 700

Gly Gly Leu Ser Tyr Lys Glu Asp Thr Lys Glu Leu Val Val Ala Lys
705 710 715 720

Ala Gly Val Tyr Tyr Val Phe Phe Gln Leu Glu Leu Arg Arg Val Val
725 730 735

Ala Gly Glu Gly Ser Gly Ser Val Ser Leu Ala Leu His Leu Gln Pro
740 745 750

Leu Arg Ser Ala Ala Gly Ala Ala Ala Leu Ala Leu Thr Val Asp Leu
755 760 765

Pro Pro Ala Ser Ser Glu Ala Arg Asn Ser Ala Phe Gly Phe Gln Gly
770 775 780

Arg Leu Leu His Leu Ser Ala Gly Gln Arg Leu Gly Val His Leu His
785 790 795 800

Thr Glu Ala Arg Ala Arg His Ala Trp Gln Leu Thr Gln Gly Ala Thr
805 810 815

Val Leu Gly Leu Phe Arg Val Thr Pro Glu Ile Pro Ala Gly Leu Pro
820 825 830

Ser Pro Arg Ser Glu
        835

<210>  85
<211>  643
<212>  PRT
<213>  Artificial Sequence

<220>
<223>  anti-PD1(0314) Fc knob chain fused to monomeric hu 4-1BBL (71-254)

<400>  85

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Leu Leu Thr Gly Arg Val Tyr Phe Ala Leu Asp Ser Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190
```

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Ala Ala Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Gly Ala Pro Ile Glu
325 330 335

Lys Thr Ile Ser Lys Ala Lys Gly Gln Pro Arg Glu Pro Gln Val Tyr
340 345 350

Thr Leu Pro Pro Cys Arg Asp Glu Leu Thr Lys Asn Gln Val Ser Leu
355 360 365

Trp Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Gly Gly Gly Gly Ser Gly Gly Gly Gly Ser Arg Glu Gly Pro Glu
    450                 455                 460

Leu Ser Pro Asp Asp Pro Ala Gly Leu Leu Asp Leu Arg Gln Gly Met
465                 470                 475                 480

Phe Ala Gln Leu Val Ala Gln Asn Val Leu Leu Ile Asp Gly Pro Leu
                485                 490                 495

Ser Trp Tyr Ser Asp Pro Gly Leu Ala Gly Val Ser Leu Thr Gly Gly
            500                 505                 510

Leu Ser Tyr Lys Glu Asp Thr Lys Glu Leu Val Val Ala Lys Ala Gly
        515                 520                 525

Val Tyr Tyr Val Phe Phe Gln Leu Glu Leu Arg Arg Val Val Ala Gly
    530                 535                 540

Glu Gly Ser Gly Ser Val Ser Leu Ala Leu His Leu Gln Pro Leu Arg
545                 550                 555                 560

Ser Ala Ala Gly Ala Ala Ala Leu Ala Leu Thr Val Asp Leu Pro Pro
                565                 570                 575

Ala Ser Ser Glu Ala Arg Asn Ser Ala Phe Gly Phe Gln Gly Arg Leu
            580                 585                 590

Leu His Leu Ser Ala Gly Gln Arg Leu Gly Val His Leu His Thr Glu
        595                 600                 605

Ala Arg Ala Arg His Ala Trp Gln Leu Thr Gln Gly Ala Thr Val Leu
    610                 615                 620

Gly Leu Phe Arg Val Thr Pro Glu Ile Pro Ala Gly Leu Pro Ser Pro
625                 630                 635                 640

Arg Ser Glu

<210>  86
<211>  825
<212>  PRT
<213>  Artificial Sequence

<220>
<223>  anti-PD1(0314) Fc hole chain fused to dimeric hu 4-1BBL (71-248)

<400>  86

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Leu Leu Thr Gly Arg Val Tyr Phe Ala Leu Asp Ser Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Gly Ala Pro Ile Glu
325 330 335

Lys Thr Ile Ser Lys Ala Lys Gly Gln Pro Arg Glu Pro Gln Val Cys
340 345 350

Thr Leu Pro Pro Ser Arg Asp Glu Leu Thr Lys Asn Gln Val Ser Leu
355 360 365

Ser Cys Ala Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Val Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Gly Gly Gly Gly Ser Gly Gly Gly Gly Ser Arg Glu Gly Pro Glu
450 455 460

Leu Ser Pro Asp Asp Pro Ala Gly Leu Leu Asp Leu Arg Gln Gly Met
465 470 475 480

Phe Ala Gln Leu Val Ala Gln Asn Val Leu Leu Ile Asp Gly Pro Leu
485 490 495

Ser Trp Tyr Ser Asp Pro Gly Leu Ala Gly Val Ser Leu Thr Gly Gly

500 505 510

Leu Ser Tyr Lys Glu Asp Thr Lys Glu Leu Val Val Ala Lys Ala Gly
515 520 525

Val Tyr Tyr Val Phe Phe Gln Leu Glu Leu Arg Arg Val Val Ala Gly
530 535 540

Glu Gly Ser Gly Ser Val Ser Leu Ala Leu His Leu Gln Pro Leu Arg
545 550 555 560

Ser Ala Ala Gly Ala Ala Ala Leu Ala Leu Thr Val Asp Leu Pro Pro
565 570 575

Ala Ser Ser Glu Ala Arg Asn Ser Ala Phe Gly Phe Gln Gly Arg Leu
580 585 590

Leu His Leu Ser Ala Gly Gln Arg Leu Gly Val His Leu His Thr Glu
595 600 605

Ala Arg Ala Arg His Ala Trp Gln Leu Thr Gln Gly Ala Thr Val Leu
610 615 620

Gly Leu Phe Arg Val Thr Pro Glu Ile Pro Ala Gly Leu Gly Gly Gly
625 630 635 640

Gly Ser Gly Gly Gly Gly Ser Arg Glu Gly Pro Glu Leu Ser Pro Asp
645 650 655

Asp Pro Ala Gly Leu Leu Asp Leu Arg Gln Gly Met Phe Ala Gln Leu
660 665 670

Val Ala Gln Asn Val Leu Leu Ile Asp Gly Pro Leu Ser Trp Tyr Ser
675 680 685

Asp Pro Gly Leu Ala Gly Val Ser Leu Thr Gly Gly Leu Ser Tyr Lys
690 695 700

Glu Asp Thr Lys Glu Leu Val Val Ala Lys Ala Gly Val Tyr Tyr Val
705 710 715 720

Phe Phe Gln Leu Glu Leu Arg Arg Val Val Ala Gly Glu Gly Ser Gly
725 730 735

Ser Val Ser Leu Ala Leu His Leu Gln Pro Leu Arg Ser Ala Ala Gly
740 745 750

Ala Ala Ala Leu Ala Leu Thr Val Asp Leu Pro Pro Ala Ser Ser Glu
755 760 765

Ala Arg Asn Ser Ala Phe Gly Phe Gln Gly Arg Leu Leu His Leu Ser
770 775 780

Ala Gly Gln Arg Leu Gly Val His Leu His Thr Glu Ala Arg Ala Arg
785 790 795 800

His Ala Trp Gln Leu Thr Gln Gly Ala Thr Val Leu Gly Leu Phe Arg
805 810 815

Val Thr Pro Glu Ile Pro Ala Gly Leu
820 825

<210> 87
<211> 637
<212> PRT
<213> Artificial Sequence

<220>
<223> anti-PD1(0314) Fc knob chain fused to monomeric hu 4-1BBL (71-248)

<400> 87

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Ser Phe Ser Ser Tyr
20 25 30

Thr Met Ser Trp Val Arg Gln Ala Pro Gly Lys Gly Leu Glu Trp Val
35 40 45

Ala Thr Ile Ser Gly Gly Gly Arg Asp Ile Tyr Tyr Pro Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Val Leu Leu Thr Gly Arg Val Tyr Phe Ala Leu Asp Ser Trp Gly Gln
100 105 110

Gly Thr Leu Val Thr Val Ser Ser Ala Ser Thr Lys Gly Pro Ser Val
115 120 125

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Gly Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Cys Arg Asp Glu Leu Thr Lys Asn Gln Val Ser Leu
        355                 360                 365

Trp Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Gly Gly Gly Gly Ser Gly Gly Gly Gly Ser Arg Glu Gly Pro Glu
450 455 460

Leu Ser Pro Asp Asp Pro Ala Gly Leu Leu Asp Leu Arg Gln Gly Met
465 470 475 480

Phe Ala Gln Leu Val Ala Gln Asn Val Leu Leu Ile Asp Gly Pro Leu
485 490 495

Ser Trp Tyr Ser Asp Pro Gly Leu Ala Gly Val Ser Leu Thr Gly Gly
500 505 510

Leu Ser Tyr Lys Glu Asp Thr Lys Glu Leu Val Val Ala Lys Ala Gly
515 520 525

Val Tyr Tyr Val Phe Phe Gln Leu Glu Leu Arg Arg Val Val Ala Gly
530 535 540

Glu Gly Ser Gly Ser Val Ser Leu Ala Leu His Leu Gln Pro Leu Arg
545 550 555 560

Ser Ala Ala Gly Ala Ala Ala Leu Ala Leu Thr Val Asp Leu Pro Pro
565 570 575

Ala Ser Ser Glu Ala Arg Asn Ser Ala Phe Gly Phe Gln Gly Arg Leu
580 585 590

Leu His Leu Ser Ala Gly Gln Arg Leu Gly Val His Leu His Thr Glu
595 600 605

Ala Arg Ala Arg His Ala Trp Gln Leu Thr Gln Gly Ala Thr Val Leu
610 615 620

Gly Leu Phe Arg Val Thr Pro Glu Ile Pro Ala Gly Leu
625 630 635

<210> 88
<211> 602
<212> PRT
<213> Artificial Sequence

<220>
<223> Fc hole dimeric ligand (71-248) chain

<400> 88

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

```
His Glu Ala Leu His Asn His Tyr Thr Gln Lys Ser Leu Ser Leu Ser
    210                 215                 220

Pro Gly Gly Gly Gly Gly Ser Gly Gly Gly Gly Ser Arg Glu Gly Pro
225                 230                 235                 240

Glu Leu Ser Pro Asp Asp Pro Ala Gly Leu Leu Asp Leu Arg Gln Gly
                245                 250                 255

Met Phe Ala Gln Leu Val Ala Gln Asn Val Leu Leu Ile Asp Gly Pro
            260                 265                 270

Leu Ser Trp Tyr Ser Asp Pro Gly Leu Ala Gly Val Ser Leu Thr Gly
        275                 280                 285

Gly Leu Ser Tyr Lys Glu Asp Thr Lys Glu Leu Val Val Ala Lys Ala
    290                 295                 300

Gly Val Tyr Tyr Val Phe Phe Gln Leu Glu Leu Arg Arg Val Val Ala
305                 310                 315                 320

Gly Glu Gly Ser Gly Ser Val Ser Leu Ala Leu His Leu Gln Pro Leu
                325                 330                 335

Arg Ser Ala Ala Gly Ala Ala Ala Leu Ala Leu Thr Val Asp Leu Pro
            340                 345                 350

Pro Ala Ser Ser Glu Ala Arg Asn Ser Ala Phe Gly Phe Gln Gly Arg
        355                 360                 365

Leu Leu His Leu Ser Ala Gly Gln Arg Leu Gly Val His Leu His Thr
    370                 375                 380

Glu Ala Arg Ala Arg His Ala Trp Gln Leu Thr Gln Gly Ala Thr Val
385                 390                 395                 400

Leu Gly Leu Phe Arg Val Thr Pro Glu Ile Pro Ala Gly Leu Gly Gly
                405                 410                 415

Gly Gly Ser Gly Gly Gly Gly Ser Arg Glu Gly Pro Glu Leu Ser Pro
            420                 425                 430

Asp Asp Pro Ala Gly Leu Leu Asp Leu Arg Gln Gly Met Phe Ala Gln
        435                 440                 445

Leu Val Ala Gln Asn Val Leu Leu Ile Asp Gly Pro Leu Ser Trp Tyr
    450                 455                 460
```

```
Ser Asp Pro Gly Leu Ala Gly Val Ser Leu Thr Gly Gly Leu Ser Tyr
465                 470                 475                 480

Lys Glu Asp Thr Lys Glu Leu Val Val Ala Lys Ala Gly Val Tyr Tyr
                485                 490                 495

Val Phe Phe Gln Leu Glu Leu Arg Arg Val Val Ala Gly Glu Gly Ser
            500                 505                 510

Gly Ser Val Ser Leu Ala Leu His Leu Gln Pro Leu Arg Ser Ala Ala
        515                 520                 525

Gly Ala Ala Ala Leu Ala Leu Thr Val Asp Leu Pro Pro Ala Ser Ser
    530                 535                 540

Glu Ala Arg Asn Ser Ala Phe Gly Phe Gln Gly Arg Leu Leu His Leu
545                 550                 555                 560

Ser Ala Gly Gln Arg Leu Gly Val His Leu His Thr Glu Ala Arg Ala
                565                 570                 575

Arg His Ala Trp Gln Leu Thr Gln Gly Ala Thr Val Leu Gly Leu Phe
            580                 585                 590

Arg Val Thr Pro Glu Ile Pro Ala Gly Leu
        595                 600


<210>  89
<211>  404
<212>  PRT
<213>  Artificial Sequence

<220>
<223>  Fc hole monomeric ligand (71-248) chain

<400>  89

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60
```

```
His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Arg Glu Gly Pro Glu Leu Ser Pro Asp Asp Pro Ala Gly Leu
225                 230                 235                 240

Leu Asp Leu Arg Gln Gly Met Phe Ala Gln Leu Val Ala Gln Asn Val
                245                 250                 255

Leu Leu Ile Asp Gly Pro Leu Ser Trp Tyr Ser Asp Pro Gly Leu Ala
            260                 265                 270

Gly Val Ser Leu Thr Gly Gly Leu Ser Tyr Lys Glu Asp Thr Lys Glu
        275                 280                 285

Leu Val Val Ala Lys Ala Gly Val Tyr Tyr Val Phe Phe Gln Leu Glu
    290                 295                 300

Leu Arg Arg Val Val Ala Gly Glu Gly Ser Gly Ser Val Ser Leu Ala
305                 310                 315                 320
```

Leu His Leu Gln Pro Leu Arg Ser Ala Ala Gly Ala Ala Ala Leu Ala
325 330 335

Leu Thr Val Asp Leu Pro Pro Ala Ser Ser Glu Ala Arg Asn Ser Ala
340 345 350

Phe Gly Phe Gln Gly Arg Leu Leu His Leu Ser Ala Gly Gln Arg Leu
355 360 365

Gly Val His Leu His Thr Glu Ala Arg Ala Arg His Ala Trp Gln Leu
370 375 380

Thr Gln Gly Ala Thr Val Leu Gly Leu Phe Arg Val Thr Pro Glu Ile
385 390 395 400

Pro Ala Gly Leu

<210> 90
<211> 815
<212> PRT
<213> Artificial Sequence

<220>
<223> anti-PD1(0314) Fc knob chain fused to dimeric hu 4-1BBL (71-248)

<400> 90

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Ser Phe Ser Ser Tyr
20 25 30

Thr Met Ser Trp Val Arg Gln Ala Pro Gly Lys Gly Leu Glu Trp Val
35 40 45

Ala Thr Ile Ser Gly Gly Gly Arg Asp Ile Tyr Tyr Pro Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Val Leu Leu Thr Gly Arg Val Tyr Phe Ala Leu Asp Ser Trp Gly Gln
100 105 110

```
Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Gly Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Cys Arg Asp Glu Leu Thr Lys Asn Gln Val Ser Leu
        355                 360                 365
```

Trp Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Arg Glu Gly Pro Glu Leu Ser Pro Asp Asp Pro Ala Gly Leu Leu
450 455 460

Asp Leu Arg Gln Gly Met Phe Ala Gln Leu Val Ala Gln Asn Val Leu
465 470 475 480

Leu Ile Asp Gly Pro Leu Ser Trp Tyr Ser Asp Pro Gly Leu Ala Gly
485 490 495

Val Ser Leu Thr Gly Gly Leu Ser Tyr Lys Glu Asp Thr Lys Glu Leu
500 505 510

Val Val Ala Lys Ala Gly Val Tyr Tyr Val Phe Phe Gln Leu Glu Leu
515 520 525

Arg Arg Val Val Ala Gly Glu Gly Ser Gly Ser Val Ser Leu Ala Leu
530 535 540

His Leu Gln Pro Leu Arg Ser Ala Ala Gly Ala Ala Ala Leu Ala Leu
545 550 555 560

Thr Val Asp Leu Pro Pro Ala Ser Ser Glu Ala Arg Asn Ser Ala Phe
565 570 575

Gly Phe Gln Gly Arg Leu Leu His Leu Ser Ala Gly Gln Arg Leu Gly
580 585 590

Val His Leu His Thr Glu Ala Arg Ala Arg His Ala Trp Gln Leu Thr
595 600 605

Gln Gly Ala Thr Val Leu Gly Leu Phe Arg Val Thr Pro Glu Ile Pro

610 615 620

Ala Gly Leu Gly Gly Gly Gly Ser Gly Gly Gly Gly Ser Arg Glu Gly
625 630 635 640

Pro Glu Leu Ser Pro Asp Asp Pro Ala Gly Leu Leu Asp Leu Arg Gln
645 650 655

Gly Met Phe Ala Gln Leu Val Ala Gln Asn Val Leu Leu Ile Asp Gly
660 665 670

Pro Leu Ser Trp Tyr Ser Asp Pro Gly Leu Ala Gly Val Ser Leu Thr
675 680 685

Gly Gly Leu Ser Tyr Lys Glu Asp Thr Lys Glu Leu Val Val Ala Lys
690 695 700

Ala Gly Val Tyr Tyr Val Phe Phe Gln Leu Glu Leu Arg Arg Val Val
705 710 715 720

Ala Gly Glu Gly Ser Gly Ser Val Ser Leu Ala Leu His Leu Gln Pro
725 730 735

Leu Arg Ser Ala Ala Gly Ala Ala Ala Leu Ala Leu Thr Val Asp Leu
740 745 750

Pro Pro Ala Ser Ser Glu Ala Arg Asn Ser Ala Phe Gly Phe Gln Gly
755 760 765

Arg Leu Leu His Leu Ser Ala Gly Gln Arg Leu Gly Val His Leu His
770 775 780

Thr Glu Ala Arg Ala Arg His Ala Trp Gln Leu Thr Gln Gly Ala Thr
785 790 795 800

Val Leu Gly Leu Phe Arg Val Thr Pro Glu Ile Pro Ala Gly Leu
805 810 815

<210> 91
<211> 414
<212> PRT
<213> Artificial Sequence

<220>
<223> Fc knob chain fused to monomeric hu 4-1BBL (71-248)

<400> 91

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Gly Ala Pro Ile
100 105 110

Glu Lys Thr Ile Ser Lys Ala Lys Gly Gln Pro Arg Glu Pro Gln Val
115 120 125

Tyr Thr Leu Pro Pro Cys Arg Asp Glu Leu Thr Lys Asn Gln Val Ser
130 135 140

Leu Trp Cys Leu Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Gly Gly Gly Gly Ser Gly Gly Gly Gly Ser Arg Glu Gly Pro
225 230 235 240

Glu Leu Ser Pro Asp Asp Pro Ala Gly Leu Leu Asp Leu Arg Gln Gly
245 250 255

Met Phe Ala Gln Leu Val Ala Gln Asn Val Leu Leu Ile Asp Gly Pro

            260                 265                 270

Leu Ser Trp Tyr Ser Asp Pro Gly Leu Ala Gly Val Ser Leu Thr Gly
        275                 280                 285

Gly Leu Ser Tyr Lys Glu Asp Thr Lys Glu Leu Val Val Ala Lys Ala
    290                 295                 300

Gly Val Tyr Tyr Val Phe Phe Gln Leu Glu Leu Arg Arg Val Val Ala
305                 310                 315                 320

Gly Glu Gly Ser Gly Ser Val Ser Leu Ala Leu His Leu Gln Pro Leu
                325                 330                 335

Arg Ser Ala Ala Gly Ala Ala Ala Leu Ala Leu Thr Val Asp Leu Pro
            340                 345                 350

Pro Ala Ser Ser Glu Ala Arg Asn Ser Ala Phe Gly Phe Gln Gly Arg
        355                 360                 365

Leu Leu His Leu Ser Ala Gly Gln Arg Leu Gly Val His Leu His Thr
    370                 375                 380

Glu Ala Arg Ala Arg His Ala Trp Gln Leu Thr Gln Gly Ala Thr Val
385                 390                 395                 400

Leu Gly Leu Phe Arg Val Thr Pro Glu Ile Pro Ala Gly Leu
                405                 410

<210>  92
<211>  637
<212>  PRT
<213>  Artificial Sequence

<220>
<223>  anti-PD1(0314) Fc hole monomeric ligand (71-248) chain

<400>  92

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Val Leu Leu Thr Gly Arg Val Tyr Phe Ala Leu Asp Ser Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Ala Ala Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys

305 310 315 320

Glu Tyr Lys Cys Lys Val Ser Asn Lys Ala Leu Gly Ala Pro Ile Glu
325 330 335

Lys Thr Ile Ser Lys Ala Lys Gly Gln Pro Arg Glu Pro Gln Val Cys
340 345 350

Thr Leu Pro Pro Ser Arg Asp Glu Leu Thr Lys Asn Gln Val Ser Leu
355 360 365

Ser Cys Ala Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Val Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Gly Gly Gly Gly Ser Gly Gly Gly Gly Ser Arg Glu Gly Pro Glu
450 455 460

Leu Ser Pro Asp Asp Pro Ala Gly Leu Leu Asp Leu Arg Gln Gly Met
465 470 475 480

Phe Ala Gln Leu Val Ala Gln Asn Val Leu Leu Ile Asp Gly Pro Leu
485 490 495

Ser Trp Tyr Ser Asp Pro Gly Leu Ala Gly Val Ser Leu Thr Gly Gly
500 505 510

Leu Ser Tyr Lys Glu Asp Thr Lys Glu Leu Val Val Ala Lys Ala Gly
515 520 525

Val Tyr Tyr Val Phe Phe Gln Leu Glu Leu Arg Arg Val Val Ala Gly
530 535 540

Glu Gly Ser Gly Ser Val Ser Leu Ala Leu His Leu Gln Pro Leu Arg
545 550 555 560

Ser Ala Ala Gly Ala Ala Ala Leu Ala Leu Thr Val Asp Leu Pro Pro
565 570 575

Ala Ser Ser Glu Ala Arg Asn Ser Ala Phe Gly Phe Gln Gly Arg Leu
580 585 590

Leu His Leu Ser Ala Gly Gln Arg Leu Gly Val His Leu His Thr Glu
595 600 605

Ala Arg Ala Arg His Ala Trp Gln Leu Thr Gln Gly Ala Thr Val Leu
610 615 620

Gly Leu Phe Arg Val Thr Pro Glu Ile Pro Ala Gly Leu
625 630 635

<210> 93
<211> 602
<212> PRT
<213> Artificial Sequence

<220>
<223> Fc knob chain fused to dimeric hu 4-1BBL (71-248)

<400> 93

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Gly Ala Pro Ile
100 105 110

Glu Lys Thr Ile Ser Lys Ala Lys Gly Gln Pro Arg Glu Pro Gln Val
115 120 125

Tyr Thr Leu Pro Pro Cys Arg Asp Glu Leu Thr Lys Asn Gln Val Ser

130 135 140

Leu Trp Cys Leu Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Gly Gly Gly Gly Ser Gly Gly Gly Gly Ser Arg Glu Gly Pro
225 230 235 240

Glu Leu Ser Pro Asp Asp Pro Ala Gly Leu Leu Asp Leu Arg Gln Gly
245 250 255

Met Phe Ala Gln Leu Val Ala Gln Asn Val Leu Leu Ile Asp Gly Pro
260 265 270

Leu Ser Trp Tyr Ser Asp Pro Gly Leu Ala Gly Val Ser Leu Thr Gly
275 280 285

Gly Leu Ser Tyr Lys Glu Asp Thr Lys Glu Leu Val Val Ala Lys Ala
290 295 300

Gly Val Tyr Tyr Val Phe Phe Gln Leu Glu Leu Arg Arg Val Val Ala
305 310 315 320

Gly Glu Gly Ser Gly Ser Val Ser Leu Ala Leu His Leu Gln Pro Leu
325 330 335

Arg Ser Ala Ala Gly Ala Ala Ala Leu Ala Leu Thr Val Asp Leu Pro
340 345 350

Pro Ala Ser Ser Glu Ala Arg Asn Ser Ala Phe Gly Phe Gln Gly Arg
355 360 365

Leu Leu His Leu Ser Ala Gly Gln Arg Leu Gly Val His Leu His Thr
370 375 380

```
Glu Ala Arg Ala Arg His Ala Trp Gln Leu Thr Gln Gly Ala Thr Val
385                 390                 395                 400

Leu Gly Leu Phe Arg Val Thr Pro Glu Ile Pro Ala Gly Leu Gly Gly
                405                 410                 415

Gly Gly Ser Gly Gly Gly Gly Ser Arg Glu Gly Pro Glu Leu Ser Pro
            420                 425                 430

Asp Asp Pro Ala Gly Leu Leu Asp Leu Arg Gln Gly Met Phe Ala Gln
        435                 440                 445

Leu Val Ala Gln Asn Val Leu Leu Ile Asp Gly Pro Leu Ser Trp Tyr
    450                 455                 460

Ser Asp Pro Gly Leu Ala Gly Val Ser Leu Thr Gly Gly Leu Ser Tyr
465                 470                 475                 480

Lys Glu Asp Thr Lys Glu Leu Val Val Ala Lys Ala Gly Val Tyr Tyr
                485                 490                 495

Val Phe Phe Gln Leu Glu Leu Arg Arg Val Val Ala Gly Glu Gly Ser
            500                 505                 510

Gly Ser Val Ser Leu Ala Leu His Leu Gln Pro Leu Arg Ser Ala Ala
        515                 520                 525

Gly Ala Ala Ala Leu Ala Leu Thr Val Asp Leu Pro Pro Ala Ser Ser
    530                 535                 540

Glu Ala Arg Asn Ser Ala Phe Gly Phe Gln Gly Arg Leu Leu His Leu
545                 550                 555                 560

Ser Ala Gly Gln Arg Leu Gly Val His Leu His Thr Glu Ala Arg Ala
                565                 570                 575

Arg His Ala Trp Gln Leu Thr Gln Gly Ala Thr Val Leu Gly Leu Phe
            580                 585                 590

Arg Val Thr Pro Glu Ile Pro Ala Gly Leu
        595                 600
```

<210>  94
<211>  288
<212>  PRT
<213>  Homo sapiens

<400>  94

Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30

Asn Pro Pro Thr Phe Ser Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
260 265 270

Ser Ala Gln Pro Leu Arg Pro Glu Asp Gly His Cys Ser Trp Pro Leu
275 280 285

<210> 95
<211> 205
<212> PRT
<213> Homo sapiens

<400> 95

Met Thr Pro Pro Glu Arg Leu Phe Leu Pro Arg Val Cys Gly Thr Thr
1 5 10 15

Leu His Leu Leu Leu Leu Gly Leu Leu Leu Val Leu Leu Pro Gly Ala
20 25 30

Gln Gly Leu Pro Gly Val Gly Leu Thr Pro Ser Ala Ala Gln Thr Ala
35 40 45

Arg Gln His Pro Lys Met His Leu Ala His Ser Thr Leu Lys Pro Ala
50 55 60

Ala His Leu Ile Gly Asp Pro Ser Lys Gln Asn Ser Leu Leu Trp Arg
65 70 75 80

Ala Asn Thr Asp Arg Ala Phe Leu Gln Asp Gly Phe Ser Leu Ser Asn
85 90 95

Asn Ser Leu Leu Val Pro Thr Ser Gly Ile Tyr Phe Val Tyr Ser Gln
100 105 110

Val Val Phe Ser Gly Lys Ala Tyr Ser Pro Lys Ala Thr Ser Ser Pro
115 120 125

Leu Tyr Leu Ala His Glu Val Gln Leu Phe Ser Ser Gln Tyr Pro Phe
130 135 140

His Val Pro Leu Leu Ser Ser Gln Lys Met Val Tyr Pro Gly Leu Gln
145 150 155 160

Glu Pro Trp Leu His Ser Met Tyr His Gly Ala Ala Phe Gln Leu Thr
165 170 175

Gln Gly Asp Gln Leu Ser Thr His Thr Asp Gly Ile Pro His Leu Val
180 185 190

Leu Ser Pro Ser Thr Val Phe Phe Gly Ala Phe Ala Leu
195 200 205

<210> 96
<211> 233
<212> PRT
<213> Homo sapiens

<400> 96

Met Ser Thr Glu Ser Met Ile Arg Asp Val Glu Leu Ala Glu Glu Ala
1 5 10 15

Leu Pro Lys Lys Thr Gly Gly Pro Gln Gly Ser Arg Arg Cys Leu Phe
20 25 30

Leu Ser Leu Phe Ser Phe Leu Ile Val Ala Gly Ala Thr Thr Leu Phe
35 40 45

Cys Leu Leu His Phe Gly Val Ile Gly Pro Gln Arg Glu Glu Phe Pro
50 55 60

Arg Asp Leu Ser Leu Ile Ser Pro Leu Ala Gln Ala Val Arg Ser Ser
65 70 75 80

Ser Arg Thr Pro Ser Asp Lys Pro Val Ala His Val Val Ala Asn Pro
85 90 95

Gln Ala Glu Gly Gln Leu Gln Trp Leu Asn Arg Arg Ala Asn Ala Leu
100 105 110

Leu Ala Asn Gly Val Glu Leu Arg Asp Asn Gln Leu Val Val Pro Ser
115 120 125

Glu Gly Leu Tyr Leu Ile Tyr Ser Gln Val Leu Phe Lys Gly Gln Gly
130 135 140

Cys Pro Ser Thr His Val Leu Leu Thr His Thr Ile Ser Arg Ile Ala
145 150 155 160

Val Ser Tyr Gln Thr Lys Val Asn Leu Leu Ser Ala Ile Lys Ser Pro
165 170 175

Cys Gln Arg Glu Thr Pro Glu Gly Ala Glu Ala Lys Pro Trp Tyr Glu
180 185 190

Pro Ile Tyr Leu Gly Gly Val Phe Gln Leu Glu Lys Gly Asp Arg Leu
195 200 205

```
Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230


<210>  97
<211>  244
<212>  PRT
<213>  Homo sapiens

<400>  97

Met Gly Ala Leu Gly Leu Glu Gly Arg Gly Gly Arg Leu Gln Gly Arg
1               5                   10                  15

Gly Ser Leu Leu Leu Ala Val Ala Gly Ala Thr Ser Leu Val Thr Leu
            20                  25                  30

Leu Leu Ala Val Pro Ile Thr Val Leu Ala Val Leu Ala Leu Val Pro
        35                  40                  45

Gln Asp Gln Gly Gly Leu Val Thr Glu Thr Ala Asp Pro Gly Ala Gln
    50                  55                  60

Ala Gln Gln Gly Leu Gly Phe Gln Lys Leu Pro Glu Glu Glu Pro Glu
65                  70                  75                  80

Thr Asp Leu Ser Pro Gly Leu Pro Ala Ala His Leu Ile Gly Ala Pro
                85                  90                  95

Leu Lys Gly Gln Gly Leu Gly Trp Glu Thr Thr Lys Glu Gln Ala Phe
            100                 105                 110

Leu Thr Ser Gly Thr Gln Phe Ser Asp Ala Glu Gly Leu Ala Leu Pro
        115                 120                 125

Gln Asp Gly Leu Tyr Tyr Leu Tyr Cys Leu Val Gly Tyr Arg Gly Arg
    130                 135                 140

Ala Pro Pro Gly Gly Gly Asp Pro Gln Gly Arg Ser Val Thr Leu Arg
145                 150                 155                 160

Ser Ser Leu Tyr Arg Ala Gly Gly Ala Tyr Gly Pro Gly Thr Pro Glu
                165                 170                 175

Leu Leu Leu Glu Gly Ala Glu Thr Val Thr Pro Val Leu Asp Pro Ala
            180                 185                 190
```

```
Arg Arg Gln Gly Tyr Gly Pro Leu Trp Tyr Thr Ser Val Gly Phe Gly
        195                 200                 205

Gly Leu Val Gln Leu Arg Arg Gly Glu Arg Val Tyr Val Asn Ile Ser
    210                 215                 220

His Pro Asp Met Val Asp Phe Ala Arg Gly Lys Thr Phe Phe Gly Ala
225                 230                 235                 240

Val Met Val Gly



<210>  98
<211>  183
<212>  PRT
<213>  Homo sapiens

<400>  98

Met Glu Arg Val Gln Pro Leu Glu Glu Asn Val Gly Asn Ala Ala Arg
1               5                   10                  15

Pro Arg Phe Glu Arg Asn Lys Leu Leu Leu Val Ala Ser Val Ile Gln
            20                  25                  30

Gly Leu Gly Leu Leu Leu Cys Phe Thr Tyr Ile Cys Leu His Phe Ser
        35                  40                  45

Ala Leu Gln Val Ser His Arg Tyr Pro Arg Ile Gln Ser Ile Lys Val
    50                  55                  60

Gln Phe Thr Glu Tyr Lys Lys Glu Lys Gly Phe Ile Leu Thr Ser Gln
65                  70                  75                  80

Lys Glu Asp Glu Ile Met Lys Val Gln Asn Asn Ser Val Ile Ile Asn
                85                  90                  95

Cys Asp Gly Phe Tyr Leu Ile Ser Leu Lys Gly Tyr Phe Ser Gln Glu
            100                 105                 110

Val Asn Ile Ser Leu His Tyr Gln Lys Asp Glu Glu Pro Leu Phe Gln
        115                 120                 125

Leu Lys Lys Val Arg Ser Val Asn Ser Leu Met Val Ala Ser Leu Thr
    130                 135                 140

Tyr Lys Asp Lys Val Tyr Leu Asn Val Thr Thr Asp Asn Thr Ser Leu
145                 150                 155                 160
```

Asp Asp Phe His Val Asn Gly Gly Glu Leu Ile Leu Ile His Gln Asn
165 170 175

Pro Gly Glu Phe Cys Val Leu
180

<210> 99
<211> 261
<212> PRT
<213> Homo sapiens

<400> 99

Met Ile Glu Thr Tyr Asn Gln Thr Ser Pro Arg Ser Ala Ala Thr Gly
1 5 10 15

Leu Pro Ile Ser Met Lys Ile Phe Met Tyr Leu Leu Thr Val Phe Leu
20 25 30

Ile Thr Gln Met Ile Gly Ser Ala Leu Phe Ala Val Tyr Leu His Arg
35 40 45

Arg Leu Asp Lys Ile Glu Asp Glu Arg Asn Leu His Glu Asp Phe Val
50 55 60

Phe Met Lys Thr Ile Gln Arg Cys Asn Thr Gly Glu Arg Ser Leu Ser
65 70 75 80

Leu Leu Asn Cys Glu Glu Ile Lys Ser Gln Phe Glu Gly Phe Val Lys
85 90 95

Asp Ile Met Leu Asn Lys Glu Glu Thr Lys Lys Glu Asn Ser Phe Glu
100 105 110

Met Gln Lys Gly Asp Gln Asn Pro Gln Ile Ala Ala His Val Ile Ser
115 120 125

Glu Ala Ser Ser Lys Thr Thr Ser Val Leu Gln Trp Ala Glu Lys Gly
130 135 140

Tyr Tyr Thr Met Ser Asn Asn Leu Val Thr Leu Glu Asn Gly Lys Gln
145 150 155 160

Leu Thr Val Lys Arg Gln Gly Leu Tyr Tyr Ile Tyr Ala Gln Val Thr
165 170 175

Phe Cys Ser Asn Arg Glu Ala Ser Ser Gln Ala Pro Phe Ile Ala Ser
180 185 190

Leu Cys Leu Lys Ser Pro Gly Arg Phe Glu Arg Ile Leu Leu Arg Ala
195 200 205

Ala Asn Thr His Ser Ser Ala Lys Pro Cys Gly Gln Gln Ser Ile His
210 215 220

Leu Gly Gly Val Phe Glu Leu Gln Pro Gly Ala Ser Val Phe Val Asn
225 230 235 240

Val Thr Asp Pro Ser Gln Val Ser His Gly Thr Gly Phe Thr Ser Phe
245 250 255

Gly Leu Leu Lys Leu
260

<210> 100
<211> 281
<212> PRT
<213> Homo sapiens

<400> 100

Met Gln Gln Pro Phe Asn Tyr Pro Tyr Pro Gln Ile Tyr Trp Val Asp
1 5 10 15

Ser Ser Ala Ser Ser Pro Trp Ala Pro Pro Gly Thr Val Leu Pro Cys
20 25 30

Pro Thr Ser Val Pro Arg Arg Pro Gly Gln Arg Arg Pro Pro Pro Pro
35 40 45

Pro Pro Pro Pro Pro Leu Pro Pro Pro Pro Pro Pro Pro Pro Leu Pro
50 55 60

Pro Leu Pro Leu Pro Pro Leu Lys Lys Arg Gly Asn His Ser Thr Gly
65 70 75 80

Leu Cys Leu Leu Val Met Phe Phe Met Val Leu Val Ala Leu Val Gly
85 90 95

Leu Gly Leu Gly Met Phe Gln Leu Phe His Leu Gln Lys Glu Leu Ala
100 105 110

Glu Leu Arg Glu Ser Thr Ser Gln Met His Thr Ala Ser Ser Leu Glu
115 120 125

Lys Gln Ile Gly His Pro Ser Pro Pro Pro Glu Lys Lys Glu Leu Arg
130 135 140

Lys Val Ala His Leu Thr Gly Lys Ser Asn Ser Arg Ser Met Pro Leu
145 150 155 160

Glu Trp Glu Asp Thr Tyr Gly Ile Val Leu Leu Ser Gly Val Lys Tyr
165 170 175

Lys Lys Gly Gly Leu Val Ile Asn Glu Thr Gly Leu Tyr Phe Val Tyr
180 185 190

Ser Lys Val Tyr Phe Arg Gly Gln Ser Cys Asn Asn Leu Pro Leu Ser
195 200 205

His Lys Val Tyr Met Arg Asn Ser Lys Tyr Pro Gln Asp Leu Val Met
210 215 220

Met Glu Gly Lys Met Met Ser Tyr Cys Thr Thr Gly Gln Met Trp Ala
225 230 235 240

Arg Ser Ser Tyr Leu Gly Ala Val Phe Asn Leu Thr Ser Ala Asp His
245 250 255

Leu Tyr Val Asn Val Ser Glu Leu Ser Leu Val Asn Phe Glu Glu Ser
260 265 270

Gln Thr Phe Phe Gly Leu Tyr Lys Leu
275 280

<210> 101
<211> 193
<212> PRT
<213> Homo sapiens

<400> 101

Met Pro Glu Glu Gly Ser Gly Cys Ser Val Arg Arg Arg Pro Tyr Gly
1 5 10 15

Cys Val Leu Arg Ala Ala Leu Val Pro Leu Val Ala Gly Leu Val Ile
20 25 30

Cys Leu Val Val Cys Ile Gln Arg Phe Ala Gln Ala Gln Gln Gln Leu
35 40 45

Pro Leu Glu Ser Leu Gly Trp Asp Val Ala Glu Leu Gln Leu Asn His
50 55 60

Thr Gly Pro Gln Gln Asp Pro Arg Leu Tyr Trp Gln Gly Gly Pro Ala
65 70 75 80

```
Leu Gly Arg Ser Phe Leu His Gly Pro Glu Leu Asp Lys Gly Gln Leu
                85                  90                  95

Arg Ile His Arg Asp Gly Ile Tyr Met Val His Ile Gln Val Thr Leu
            100                 105                 110

Ala Ile Cys Ser Ser Thr Thr Ala Ser Arg His His Pro Thr Thr Leu
        115                 120                 125

Ala Val Gly Ile Cys Ser Pro Ala Ser Arg Ser Ile Ser Leu Leu Arg
    130                 135                 140

Leu Ser Phe His Gln Gly Cys Thr Ile Ala Ser Gln Arg Leu Thr Pro
145                 150                 155                 160

Leu Ala Arg Gly Asp Thr Leu Cys Thr Asn Leu Thr Gly Thr Leu Leu
                165                 170                 175

Pro Ser Arg Asn Thr Asp Glu Thr Phe Phe Gly Val Gln Trp Val Arg
            180                 185                 190

Pro



<210>  102
<211>  234
<212>  PRT
<213>  Homo sapiens

<400>  102

Met Asp Pro Gly Leu Gln Gln Ala Leu Asn Gly Met Ala Pro Pro Gly
1               5                   10                  15

Asp Thr Ala Met His Val Pro Ala Gly Ser Val Ala Ser His Leu Gly
            20                  25                  30

Thr Thr Ser Arg Ser Tyr Phe Tyr Leu Thr Thr Ala Thr Leu Ala Leu
        35                  40                  45

Cys Leu Val Phe Thr Val Ala Thr Ile Met Val Leu Val Val Gln Arg
    50                  55                  60

Thr Asp Ser Ile Pro Asn Ser Pro Asp Asn Val Pro Leu Lys Gly Gly
65                  70                  75                  80

Asn Cys Ser Glu Asp Leu Leu Cys Ile Leu Lys Arg Ala Pro Phe Lys
                85                  90                  95
```

```
Lys Ser Trp Ala Tyr Leu Gln Val Ala Lys His Leu Asn Lys Thr Lys
            100                 105                 110

Leu Ser Trp Asn Lys Asp Gly Ile Leu His Gly Val Arg Tyr Gln Asp
        115                 120                 125

Gly Asn Leu Val Ile Gln Phe Pro Gly Leu Tyr Phe Ile Ile Cys Gln
    130                 135                 140

Leu Gln Phe Leu Val Gln Cys Pro Asn Asn Ser Val Asp Leu Lys Leu
145                 150                 155                 160

Glu Leu Leu Ile Asn Lys His Ile Lys Lys Gln Ala Leu Val Thr Val
                165                 170                 175

Cys Glu Ser Gly Met Gln Thr Lys His Val Tyr Gln Asn Leu Ser Gln
            180                 185                 190

Phe Leu Leu Asp Tyr Leu Gln Val Asn Thr Thr Ile Ser Val Asn Val
        195                 200                 205

Asp Thr Phe Gln Tyr Ile Asp Thr Ser Thr Phe Pro Leu Glu Asn Val
    210                 215                 220

Leu Ser Ile Phe Leu Tyr Ser Asn Ser Asp
225                 230


<210>  103
<211>  254
<212>  PRT
<213>  Homo sapiens

<400>  103

Met Glu Tyr Ala Ser Asp Ala Ser Leu Asp Pro Glu Ala Pro Trp Pro
1               5                   10                  15

Pro Ala Pro Arg Ala Arg Ala Cys Arg Val Leu Pro Trp Ala Leu Val
            20                  25                  30

Ala Gly Leu Leu Leu Leu Leu Leu Leu Ala Ala Ala Cys Ala Val Phe
        35                  40                  45

Leu Ala Cys Pro Trp Ala Val Ser Gly Ala Arg Ala Ser Pro Gly Ser
    50                  55                  60

Ala Ala Ser Pro Arg Leu Arg Glu Gly Pro Glu Leu Ser Pro Asp Asp
65                  70                  75                  80
```

Pro Ala Gly Leu Leu Asp Leu Arg Gln Gly Met Phe Ala Gln Leu Val
85 90 95

Ala Gln Asn Val Leu Leu Ile Asp Gly Pro Leu Ser Trp Tyr Ser Asp
100 105 110

Pro Gly Leu Ala Gly Val Ser Leu Thr Gly Gly Leu Ser Tyr Lys Glu
115 120 125

Asp Thr Lys Glu Leu Val Val Ala Lys Ala Gly Val Tyr Tyr Val Phe
130 135 140

Phe Gln Leu Glu Leu Arg Arg Val Val Ala Gly Glu Gly Ser Gly Ser
145 150 155 160

Val Ser Leu Ala Leu His Leu Gln Pro Leu Arg Ser Ala Ala Gly Ala
165 170 175

Ala Ala Leu Ala Leu Thr Val Asp Leu Pro Pro Ala Ser Ser Glu Ala
180 185 190

Arg Asn Ser Ala Phe Gly Phe Gln Gly Arg Leu Leu His Leu Ser Ala
195 200 205

Gly Gln Arg Leu Gly Val His Leu His Thr Glu Ala Arg Ala Arg His
210 215 220

Ala Trp Gln Leu Thr Gln Gly Ala Thr Val Leu Gly Leu Phe Arg Val
225 230 235 240

Thr Pro Glu Ile Pro Ala Gly Leu Pro Ser Pro Arg Ser Glu
245 250

<210> 104
<211> 281
<212> PRT
<213> Homo sapiens

<400> 104

Met Ala Met Met Glu Val Gln Gly Gly Pro Ser Leu Gly Gln Thr Cys
1 5 10 15

Val Leu Ile Val Ile Phe Thr Val Leu Leu Gln Ser Leu Cys Val Ala
20 25 30

Val Thr Tyr Val Tyr Phe Thr Asn Glu Leu Lys Gln Met Gln Asp Lys
35 40 45

Tyr Ser Lys Ser Gly Ile Ala Cys Phe Leu Lys Glu Asp Asp Ser Tyr
    50                  55                  60

Trp Asp Pro Asn Asp Glu Glu Ser Met Asn Ser Pro Cys Trp Gln Val
65                  70                  75                  80

Lys Trp Gln Leu Arg Gln Leu Val Arg Lys Met Ile Leu Arg Thr Ser
                85                  90                  95

Glu Glu Thr Ile Ser Thr Val Gln Glu Lys Gln Gln Asn Ile Ser Pro
            100                 105                 110

Leu Val Arg Glu Arg Gly Pro Gln Arg Val Ala Ala His Ile Thr Gly
        115                 120                 125

Thr Arg Gly Arg Ser Asn Thr Leu Ser Ser Pro Asn Ser Lys Asn Glu
    130                 135                 140

Lys Ala Leu Gly Arg Lys Ile Asn Ser Trp Glu Ser Ser Arg Ser Gly
145                 150                 155                 160

His Ser Phe Leu Ser Asn Leu His Leu Arg Asn Gly Glu Leu Val Ile
                165                 170                 175

His Glu Lys Gly Phe Tyr Tyr Ile Tyr Ser Gln Thr Tyr Phe Arg Phe
            180                 185                 190

Gln Glu Glu Ile Lys Glu Asn Thr Lys Asn Asp Lys Gln Met Val Gln
        195                 200                 205

Tyr Ile Tyr Lys Tyr Thr Ser Tyr Pro Asp Pro Ile Leu Leu Met Lys
    210                 215                 220

Ser Ala Arg Asn Ser Cys Trp Ser Lys Asp Ala Glu Tyr Gly Leu Tyr
225                 230                 235                 240

Ser Ile Tyr Gln Gly Gly Ile Phe Glu Leu Lys Glu Asn Asp Arg Ile
                245                 250                 255

Phe Val Ser Val Thr Asn Glu His Leu Ile Asp Met Asp His Glu Ala
            260                 265                 270

Ser Phe Phe Gly Ala Phe Leu Val Gly
        275                 280

<210>  105
<211>  317

<212> PRT
<213> Homo sapiens

<400> 105

```
Met Arg Arg Ala Ser Arg Asp Tyr Thr Lys Tyr Leu Arg Gly Ser Glu
1               5                   10                  15

Glu Met Gly Gly Gly Pro Gly Ala Pro His Glu Gly Pro Leu His Ala
            20                  25                  30

Pro Pro Pro Pro Ala Pro His Gln Pro Pro Ala Ala Ser Arg Ser Met
        35                  40                  45

Phe Val Ala Leu Leu Gly Leu Gly Leu Gly Gln Val Val Cys Ser Val
    50                  55                  60

Ala Leu Phe Phe Tyr Phe Arg Ala Gln Met Asp Pro Asn Arg Ile Ser
65                  70                  75                  80

Glu Asp Gly Thr His Cys Ile Tyr Arg Ile Leu Arg Leu His Glu Asn
                85                  90                  95

Ala Asp Phe Gln Asp Thr Thr Leu Glu Ser Gln Asp Thr Lys Leu Ile
            100                 105                 110

Pro Asp Ser Cys Arg Arg Ile Lys Gln Ala Phe Gln Gly Ala Val Gln
        115                 120                 125

Lys Glu Leu Gln His Ile Val Gly Ser Gln His Ile Arg Ala Glu Lys
    130                 135                 140

Ala Met Val Asp Gly Ser Trp Leu Asp Leu Ala Lys Arg Ser Lys Leu
145                 150                 155                 160

Glu Ala Gln Pro Phe Ala His Leu Thr Ile Asn Ala Thr Asp Ile Pro
                165                 170                 175

Ser Gly Ser His Lys Val Ser Leu Ser Ser Trp Tyr His Asp Arg Gly
            180                 185                 190

Trp Ala Lys Ile Ser Asn Met Thr Phe Ser Asn Gly Lys Leu Ile Val
        195                 200                 205

Asn Gln Asp Gly Phe Tyr Tyr Leu Tyr Ala Asn Ile Cys Phe Arg His
    210                 215                 220

His Glu Thr Ser Gly Asp Leu Ala Thr Glu Tyr Leu Gln Leu Met Val
225                 230                 235                 240
```

Tyr Val Thr Lys Thr Ser Ile Lys Ile Pro Ser Ser His Thr Leu Met
245 250 255

Lys Gly Gly Ser Thr Lys Tyr Trp Ser Gly Asn Ser Glu Phe His Phe
260 265 270

Tyr Ser Ile Asn Val Gly Gly Phe Phe Lys Leu Arg Ser Gly Glu Glu
275 280 285

Ile Ser Ile Glu Val Ser Asn Pro Ser Leu Leu Asp Pro Asp Gln Asp
290 295 300

Ala Thr Tyr Phe Gly Ala Phe Lys Val Arg Asp Ile Asp
305 310 315

<210> 106
<211> 249
<212> PRT
<213> Homo sapiens

<400> 106

Met Ala Ala Arg Arg Ser Gln Arg Arg Arg Gly Arg Arg Gly Glu Pro
1 5 10 15

Gly Thr Ala Leu Leu Val Pro Leu Ala Leu Gly Leu Gly Leu Ala Leu
20 25 30

Ala Cys Leu Gly Leu Leu Leu Ala Val Val Ser Leu Gly Ser Arg Ala
35 40 45

Ser Leu Ser Ala Gln Glu Pro Ala Gln Glu Glu Leu Val Ala Glu Glu
50 55 60

Asp Gln Asp Pro Ser Glu Leu Asn Pro Gln Thr Glu Glu Ser Gln Asp
65 70 75 80

Pro Ala Pro Phe Leu Asn Arg Leu Val Arg Pro Arg Arg Ser Ala Pro
85 90 95

Lys Gly Arg Lys Thr Arg Ala Arg Arg Ala Ile Ala Ala His Tyr Glu
100 105 110

Val His Pro Arg Pro Gly Gln Asp Gly Ala Gln Ala Gly Val Asp Gly
115 120 125

Thr Val Ser Gly Trp Glu Glu Ala Arg Ile Asn Ser Ser Ser Pro Leu
130 135 140

Arg Tyr Asn Arg Gln Ile Gly Glu Phe Ile Val Thr Arg Ala Gly Leu
145 150 155 160

Tyr Tyr Leu Tyr Cys Gln Val His Phe Asp Glu Gly Lys Ala Val Tyr
165 170 175

Leu Lys Leu Asp Leu Leu Val Asp Gly Val Leu Ala Leu Arg Cys Leu
180 185 190

Glu Glu Phe Ser Ala Thr Ala Ala Ser Ser Leu Gly Pro Gln Leu Arg
195 200 205

Leu Cys Gln Val Ser Gly Leu Leu Ala Leu Arg Pro Gly Ser Ser Leu
210 215 220

Arg Ile Arg Thr Leu Pro Trp Ala His Leu Lys Ala Ala Pro Phe Leu
225 230 235 240

Thr Tyr Phe Gly Leu Phe Gln Val His
245

<210> 107
<211> 250
<212> PRT
<213> Homo sapiens

<400> 107

Met Pro Ala Ser Ser Pro Phe Leu Leu Ala Pro Lys Gly Pro Pro Gly
1 5 10 15

Asn Met Gly Gly Pro Val Arg Glu Pro Ala Leu Ser Val Ala Leu Trp
20 25 30

Leu Ser Trp Gly Ala Ala Leu Gly Ala Val Ala Cys Ala Met Ala Leu
35 40 45

Leu Thr Gln Gln Thr Glu Leu Gln Ser Leu Arg Arg Glu Val Ser Arg
50 55 60

Leu Gln Gly Thr Gly Gly Pro Ser Gln Asn Gly Glu Gly Tyr Pro Trp
65 70 75 80

Gln Ser Leu Pro Glu Gln Ser Ser Asp Ala Leu Glu Ala Trp Glu Asn
85 90 95

Gly Glu Arg Ser Arg Lys Arg Arg Ala Val Leu Thr Gln Lys Gln Lys
100 105 110

Lys Gln His Ser Val Leu His Leu Val Pro Ile Asn Ala Thr Ser Lys
115 120 125

Asp Asp Ser Asp Val Thr Glu Val Met Trp Gln Pro Ala Leu Arg Arg
130 135 140

Gly Arg Gly Leu Gln Ala Gln Gly Tyr Gly Val Arg Ile Gln Asp Ala
145 150 155 160

Gly Val Tyr Leu Leu Tyr Ser Gln Val Leu Phe Gln Asp Val Thr Phe
165 170 175

Thr Met Gly Gln Val Val Ser Arg Glu Gly Gln Gly Arg Gln Glu Thr
180 185 190

Leu Phe Arg Cys Ile Arg Ser Met Pro Ser His Pro Asp Arg Ala Tyr
195 200 205

Asn Ser Cys Tyr Ser Ala Gly Val Phe His Leu His Gln Gly Asp Ile
210 215 220

Leu Ser Val Ile Ile Pro Arg Ala Arg Ala Lys Leu Asn Leu Ser Pro
225 230 235 240

His Gly Thr Phe Leu Gly Phe Val Lys Leu
245 250

<210> 108
<211> 285
<212> PRT
<213> Homo sapiens

<400> 108

Met Asp Asp Ser Thr Glu Arg Glu Gln Ser Arg Leu Thr Ser Cys Leu
1 5 10 15

Lys Lys Arg Glu Glu Met Lys Leu Lys Glu Cys Val Ser Ile Leu Pro
20 25 30

Arg Lys Glu Ser Pro Ser Val Arg Ser Ser Lys Asp Gly Lys Leu Leu
35 40 45

Ala Ala Thr Leu Leu Leu Ala Leu Leu Ser Cys Cys Leu Thr Val Val
50 55 60

Ser Phe Tyr Gln Val Ala Ala Leu Gln Gly Asp Leu Ala Ser Leu Arg
65 70 75 80

```
Ala Glu Leu Gln Gly His His Ala Glu Lys Leu Pro Ala Gly Ala Gly
                85                  90                  95

Ala Pro Lys Ala Gly Leu Glu Glu Ala Pro Ala Val Thr Ala Gly Leu
            100                 105                 110

Lys Ile Phe Glu Pro Pro Ala Pro Gly Glu Gly Asn Ser Ser Gln Asn
        115                 120                 125

Ser Arg Asn Lys Arg Ala Val Gln Gly Pro Glu Glu Thr Val Thr Gln
    130                 135                 140

Asp Cys Leu Gln Leu Ile Ala Asp Ser Glu Thr Pro Thr Ile Gln Lys
145                 150                 155                 160

Gly Ser Tyr Thr Phe Val Pro Trp Leu Leu Ser Phe Lys Arg Gly Ser
                165                 170                 175

Ala Leu Glu Glu Lys Glu Asn Lys Ile Leu Val Lys Glu Thr Gly Tyr
            180                 185                 190

Phe Phe Ile Tyr Gly Gln Val Leu Tyr Thr Asp Lys Thr Tyr Ala Met
        195                 200                 205

Gly His Leu Ile Gln Arg Lys Lys Val His Val Phe Gly Asp Glu Leu
    210                 215                 220

Ser Leu Val Thr Leu Phe Arg Cys Ile Gln Asn Met Pro Glu Thr Leu
225                 230                 235                 240

Pro Asn Asn Ser Cys Tyr Ser Ala Gly Ile Ala Lys Leu Glu Glu Gly
                245                 250                 255

Asp Glu Leu Gln Leu Ala Ile Pro Arg Glu Asn Ala Gln Ile Ser Leu
            260                 265                 270

Asp Gly Asp Val Thr Phe Phe Gly Ala Leu Lys Leu Leu
        275                 280                 285


<210>  109
<211>  240
<212>  PRT
<213>  Homo sapiens

<400>  109

Met Glu Glu Ser Val Val Arg Pro Ser Val Phe Val Val Asp Gly Gln
1               5                   10                  15
```

Thr Asp Ile Pro Phe Thr Arg Leu Gly Arg Ser His Arg Arg Gln Ser
20 25 30

Cys Ser Val Ala Arg Val Gly Leu Gly Leu Leu Leu Leu Leu Met Gly
35 40 45

Ala Gly Leu Ala Val Gln Gly Trp Phe Leu Leu Gln Leu His Trp Arg
50 55 60

Leu Gly Glu Met Val Thr Arg Leu Pro Asp Gly Pro Ala Gly Ser Trp
65 70 75 80

Glu Gln Leu Ile Gln Glu Arg Arg Ser His Glu Val Asn Pro Ala Ala
85 90 95

His Leu Thr Gly Ala Asn Ser Ser Leu Thr Gly Ser Gly Gly Pro Leu
100 105 110

Leu Trp Glu Thr Gln Leu Gly Leu Ala Phe Leu Arg Gly Leu Ser Tyr
115 120 125

His Asp Gly Ala Leu Val Val Thr Lys Ala Gly Tyr Tyr Tyr Ile Tyr
130 135 140

Ser Lys Val Gln Leu Gly Gly Val Gly Cys Pro Leu Gly Leu Ala Ser
145 150 155 160

Thr Ile Thr His Gly Leu Tyr Lys Arg Thr Pro Arg Tyr Pro Glu Glu
165 170 175

Leu Glu Leu Leu Val Ser Gln Gln Ser Pro Cys Gly Arg Ala Thr Ser
180 185 190

Ser Ser Arg Val Trp Trp Asp Ser Ser Phe Leu Gly Gly Val Val His
195 200 205

Leu Glu Ala Gly Glu Lys Val Val Val Arg Val Leu Asp Glu Arg Leu
210 215 220

Val Arg Leu Arg Asp Gly Thr Arg Ser Tyr Phe Gly Ala Phe Met Val
225 230 235 240

<210> 110
<211> 251
<212> PRT
<213> Homo sapiens

<400> 110

Met Ala Glu Asp Leu Gly Leu Ser Phe Gly Glu Thr Ala Ser Val Glu
1 5 10 15

Met Leu Pro Glu His Gly Ser Cys Arg Pro Lys Ala Arg Ser Ser Ser
20 25 30

Ala Arg Trp Ala Leu Thr Cys Cys Leu Val Leu Leu Pro Phe Leu Ala
35 40 45

Gly Leu Thr Thr Tyr Leu Leu Val Ser Gln Leu Arg Ala Gln Gly Glu
50 55 60

Ala Cys Val Gln Phe Gln Ala Leu Lys Gly Gln Glu Phe Ala Pro Ser
65 70 75 80

His Gln Gln Val Tyr Ala Pro Leu Arg Ala Asp Gly Asp Lys Pro Arg
85 90 95

Ala His Leu Thr Val Val Arg Gln Thr Pro Thr Gln His Phe Lys Asn
100 105 110

Gln Phe Pro Ala Leu His Trp Glu His Glu Leu Gly Leu Ala Phe Thr
115 120 125

Lys Asn Arg Met Asn Tyr Thr Asn Lys Phe Leu Leu Ile Pro Glu Ser
130 135 140

Gly Asp Tyr Phe Ile Tyr Ser Gln Val Thr Phe Arg Gly Met Thr Ser
145 150 155 160

Glu Cys Ser Glu Ile Arg Gln Ala Gly Arg Pro Asn Lys Pro Asp Ser
165 170 175

Ile Thr Val Val Ile Thr Lys Val Thr Asp Ser Tyr Pro Glu Pro Thr
180 185 190

Gln Leu Leu Met Gly Thr Lys Ser Val Cys Glu Val Gly Ser Asn Trp
195 200 205

Phe Gln Pro Ile Tyr Leu Gly Ala Met Phe Ser Leu Gln Glu Gly Asp
210 215 220

Lys Leu Met Val Asn Val Ser Asp Ile Ser Leu Val Asp Tyr Thr Lys
225 230 235 240

Glu Asp Lys Thr Phe Phe Gly Ala Phe Leu Leu

245 250

<210> 111
<211> 199
<212> PRT
<213> Homo sapiens

<400> 111

Met Thr Leu His Pro Ser Pro Ile Thr Cys Glu Phe Leu Phe Ser Thr
1 5 10 15

Ala Leu Ile Ser Pro Lys Met Cys Leu Ser His Leu Glu Asn Met Pro
20 25 30

Leu Ser His Ser Arg Thr Gln Gly Ala Gln Arg Ser Ser Trp Lys Leu
35 40 45

Trp Leu Phe Cys Ser Ile Val Met Leu Leu Phe Leu Cys Ser Phe Ser
50 55 60

Trp Leu Ile Phe Ile Phe Leu Gln Leu Glu Thr Ala Lys Glu Pro Cys
65 70 75 80

Met Ala Lys Phe Gly Pro Leu Pro Ser Lys Trp Gln Met Ala Ser Ser
85 90 95

Glu Pro Pro Cys Val Asn Lys Val Ser Asp Trp Lys Leu Glu Ile Leu
100 105 110

Gln Asn Gly Leu Tyr Leu Ile Tyr Gly Gln Val Ala Pro Asn Ala Asn
115 120 125

Tyr Asn Asp Val Ala Pro Phe Glu Val Arg Leu Tyr Lys Asn Lys Asp
130 135 140

Met Ile Gln Thr Leu Thr Asn Lys Ser Lys Ile Gln Asn Val Gly Gly
145 150 155 160

Thr Tyr Glu Leu His Val Gly Asp Thr Ile Asp Leu Ile Phe Asn Ser
165 170 175

Glu His Gln Val Leu Lys Asn Asn Thr Tyr Trp Gly Ile Ile Leu Leu
180 185 190

Ala Asn Pro Gln Phe Ile Ser
195

<210> 112

<211> 391
<212> PRT
<213> Homo sapiens

<400> 112

```
Met Gly Tyr Pro Glu Val Glu Arg Arg Glu Leu Leu Pro Ala Ala Ala
1               5                   10                  15

Pro Arg Glu Arg Gly Ser Gln Gly Cys Gly Cys Gly Gly Ala Pro Ala
            20                  25                  30

Arg Ala Gly Glu Gly Asn Ser Cys Leu Leu Phe Leu Gly Phe Phe Gly
        35                  40                  45

Leu Ser Leu Ala Leu His Leu Leu Thr Leu Cys Cys Tyr Leu Glu Leu
    50                  55                  60

Arg Ser Glu Leu Arg Arg Glu Arg Gly Ala Glu Ser Arg Leu Gly Gly
65                  70                  75                  80

Ser Gly Thr Pro Gly Thr Ser Gly Thr Leu Ser Ser Leu Gly Gly Leu
                85                  90                  95

Asp Pro Asp Ser Pro Ile Thr Ser His Leu Gly Gln Pro Ser Pro Lys
            100                 105                 110

Gln Gln Pro Leu Glu Pro Gly Glu Ala Ala Leu His Ser Asp Ser Gln
        115                 120                 125

Asp Gly His Gln Met Ala Leu Leu Asn Phe Phe Phe Pro Asp Glu Lys
    130                 135                 140

Pro Tyr Ser Glu Glu Glu Ser Arg Arg Val Arg Arg Asn Lys Arg Ser
145                 150                 155                 160

Lys Ser Asn Glu Gly Ala Asp Gly Pro Val Lys Asn Lys Lys Lys Gly
                165                 170                 175

Lys Lys Ala Gly Pro Pro Gly Pro Asn Gly Pro Pro Gly Pro Pro Gly
            180                 185                 190

Pro Pro Gly Pro Gln Gly Pro Pro Gly Ile Pro Gly Ile Pro Gly Ile
        195                 200                 205

Pro Gly Thr Thr Val Met Gly Pro Pro Gly Pro Pro Gly Pro Pro Gly
    210                 215                 220

Pro Gln Gly Pro Pro Gly Leu Gln Gly Pro Ser Gly Ala Ala Asp Lys
```

225 230 235 240

Ala Gly Thr Arg Glu Asn Gln Pro Ala Val Val His Leu Gln Gly Gln
245 250 255

Gly Ser Ala Ile Gln Val Lys Asn Asp Leu Ser Gly Gly Val Leu Asn
260 265 270

Asp Trp Ser Arg Ile Thr Met Asn Pro Lys Val Phe Lys Leu His Pro
275 280 285

Arg Ser Gly Glu Leu Glu Val Leu Val Asp Gly Thr Tyr Phe Ile Tyr
290 295 300

Ser Gln Val Glu Val Tyr Tyr Ile Asn Phe Thr Asp Phe Ala Ser Tyr
305 310 315 320

Glu Val Val Val Asp Glu Lys Pro Phe Leu Gln Cys Thr Arg Ser Ile
325 330 335

Glu Thr Gly Lys Thr Asn Tyr Asn Thr Cys Tyr Thr Ala Gly Val Cys
340 345 350

Leu Leu Lys Ala Arg Gln Lys Ile Ala Val Lys Met Val His Ala Asp
355 360 365

Ile Ser Ile Asn Met Ser Lys His Thr Thr Phe Phe Gly Ala Ile Arg
370 375 380

Leu Gly Glu Ala Pro Ala Ser
385 390

<210> 113
<211> 205
<212> PRT
<213> Homo sapiens

<400> 113

Ala Cys Pro Trp Ala Val Ser Gly Ala Arg Ala Ser Pro Gly Ser Ala
1 5 10 15

Ala Ser Pro Arg Leu Arg Glu Gly Pro Glu Leu Ser Pro Asp Asp Pro
20 25 30

Ala Gly Leu Leu Asp Leu Arg Gln Gly Met Phe Ala Gln Leu Val Ala
35 40 45

Gln Asn Val Leu Leu Ile Asp Gly Pro Leu Ser Trp Tyr Ser Asp Pro

```
    50                  55                  60

Gly Leu Ala Gly Val Ser Leu Thr Gly Gly Leu Ser Tyr Lys Glu Asp
65                  70                  75                  80

Thr Lys Glu Leu Val Val Ala Lys Ala Gly Val Tyr Tyr Val Phe Phe
                85                  90                  95

Gln Leu Glu Leu Arg Arg Val Val Ala Gly Glu Gly Ser Gly Ser Val
            100                 105                 110

Ser Leu Ala Leu His Leu Gln Pro Leu Arg Ser Ala Ala Gly Ala Ala
        115                 120                 125

Ala Leu Ala Leu Thr Val Asp Leu Pro Pro Ala Ser Ser Glu Ala Arg
    130                 135                 140

Asn Ser Ala Phe Gly Phe Gln Gly Arg Leu Leu His Leu Ser Ala Gly
145                 150                 155                 160

Gln Arg Leu Gly Val His Leu His Thr Glu Ala Arg Ala Arg His Ala
                165                 170                 175

Trp Gln Leu Thr Gln Gly Ala Thr Val Leu Gly Leu Phe Arg Val Thr
            180                 185                 190

Pro Glu Ile Pro Ala Gly Leu Pro Ser Pro Arg Ser Glu
        195                 200                 205


<210>  114
<211>  133
<212>  PRT
<213>  Homo sapiens

<400>  114

Gln Val Ser His Arg Tyr Pro Arg Ile Gln Ser Ile Lys Val Gln Phe
1               5                   10                  15

Thr Glu Tyr Lys Lys Glu Lys Gly Phe Ile Leu Thr Ser Gln Lys Glu
            20                  25                  30

Asp Glu Ile Met Lys Val Gln Asn Asn Ser Val Ile Ile Asn Cys Asp
        35                  40                  45

Gly Phe Tyr Leu Ile Ser Leu Lys Gly Tyr Phe Ser Gln Glu Val Asn
    50                  55                  60

Ile Ser Leu His Tyr Gln Lys Asp Glu Glu Pro Leu Phe Gln Leu Lys
```

```
65                  70                  75                  80

Lys Val Arg Ser Val Asn Ser Leu Met Val Ala Ser Leu Thr Tyr Lys
                85                  90                  95

Asp Lys Val Tyr Leu Asn Val Thr Thr Asp Asn Thr Ser Leu Asp Asp
            100                 105                 110

Phe His Val Asn Gly Gly Glu Leu Ile Leu Ile His Gln Asn Pro Gly
        115                 120                 125

Glu Phe Cys Val Leu
    130


<210>  115
<211>  132
<212>  PRT
<213>  Homo sapiens

<400>  115

Val Ser His Arg Tyr Pro Arg Ile Gln Ser Ile Lys Val Gln Phe Thr
1               5                   10                  15

Glu Tyr Lys Lys Glu Lys Gly Phe Ile Leu Thr Ser Gln Lys Glu Asp
            20                  25                  30

Glu Ile Met Lys Val Gln Asn Asn Ser Val Ile Ile Asn Cys Asp Gly
        35                  40                  45

Phe Tyr Leu Ile Ser Leu Lys Gly Tyr Phe Ser Gln Glu Val Asn Ile
    50                  55                  60

Ser Leu His Tyr Gln Lys Asp Glu Glu Pro Leu Phe Gln Leu Lys Lys
65                  70                  75                  80

Val Arg Ser Val Asn Ser Leu Met Val Ala Ser Leu Thr Tyr Lys Asp
                85                  90                  95

Lys Val Tyr Leu Asn Val Thr Thr Asp Asn Thr Ser Leu Asp Asp Phe
            100                 105                 110

His Val Asn Gly Gly Glu Leu Ile Leu Ile His Gln Asn Pro Gly Glu
        115                 120                 125

Phe Cys Val Leu
    130


<210>  116
```

<211> 5
<212> PRT
<213> Artificial Sequence

<220>
<223> Peptide linker G4S

<400> 116

Gly Gly Gly Gly Ser
1 5

<210> 117
<211> 10
<212> PRT
<213> Artificial Sequence

<220>
<223> Peptide linker (G4S)2

<400> 117

Gly Gly Gly Gly Ser Gly Gly Gly Gly Ser
1 5 10

<210> 118
<211> 10
<212> PRT
<213> Artificial Sequence

<220>
<223> Peptide linker (SG4)2

<400> 118

Ser Gly Gly Gly Gly Ser Gly Gly Gly Gly
1 5 10

<210> 119
<211> 15
<212> PRT
<213> Artificial Sequence

<220>
<223> Peptide linker (G4S)3

<400> 119

Gly Gly Gly Gly Ser Gly Gly Gly Gly Ser Gly Gly Gly Gly Ser
1 5 10 15

<210> 120
<211> 14
<212> PRT
<213> Artificial Sequence

<220>
<223> Peptide linker G4(SG4)2

<400>  120

Gly Gly Gly Gly Ser Gly Gly Gly Gly Ser Gly Gly Gly Gly
1               5                   10

<210>  121
<211>  20
<212>  PRT
<213>  Artificial Sequence

<220>
<223>  Peptide linker (G4S)4

<400>  121

Gly Gly Gly Gly Ser Gly Gly Gly Gly Ser Gly Gly Gly Gly Ser Gly
1               5                   10                  15

Gly Gly Gly Ser
            20

<210>  122
<211>  10
<212>  PRT
<213>  Artificial Sequence

<220>
<223>  Peptide linker GSPGSSSSGS

<400>  122

Gly Ser Pro Gly Ser Ser Ser Ser Gly Ser
1               5                   10

<210>  123
<211>  8
<212>  PRT
<213>  Artificial Sequence

<220>
<223>  Peptide linker GSGSGSGS

<400>  123

Gly Ser Gly Ser Gly Ser Gly Ser
1               5

<210>  124
<211>  8
<212>  PRT
<213>  Artificial Sequence

<220>
<223>  Peptide linker GSGSGNGS

<400>  124

Gly Ser Gly Ser Gly Asn Gly Ser

1 5

<210> 125
<211> 8
<212> PRT
<213> Artificial Sequence

<220>
<223> Peptide linker GGSGSGSG

<400> 125

Gly Gly Ser Gly Ser Gly Ser Gly
1 5

<210> 126
<211> 6
<212> PRT
<213> Artificial Sequence

<220>
<223> Peptide linker GGSGSG

<400> 126

Gly Gly Ser Gly Ser Gly
1 5

<210> 127
<211> 4
<212> PRT
<213> Artificial Sequence

<220>
<223> Peptide linker GGSG

<400> 127

Gly Gly Ser Gly
1

<210> 128
<211> 8
<212> PRT
<213> Artificial Sequence

<220>
<223> GGSGNGSG

<400> 128

Gly Gly Ser Gly Asn Gly Ser Gly
1 5

<210> 129
<211> 8
<212> PRT
<213> Artificial Sequence

<220>
<223> Peptide inker GGNGSGSG

<400> 129

Gly Gly Asn Gly Ser Gly Ser Gly
1 5

<210> 130
<211> 6
<212> PRT
<213> Artificial Sequence

<220>
<223> Peptide linker GGNGSG

<400> 130

Gly Gly Asn Gly Ser Gly
1 5

<210> 131
<211> 2166
<212> DNA
<213> Artificial Sequence

<220>
<223> nucleotide sequence of dimeric hu 4-1BBL (71-254) - CL* Fc knob chain

<400> 131
agagagggcc ctgagctgag ccccgatgat cctgctggac tgctggacct gcggcagggc 60

atgtttgctc agctggtggc ccagaacgtg ctgctgatcg atggcccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agaagcgctg ctggcgctgc agctctggca ctgacagtgg atctgcctcc tgccagctcc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg cctgccttct 540

ccaagaagcg aaggcggagg cggatctggc ggcggaggat ctagagaggg acccgaactg 600

tcccctgacg atccagccgg gctgctggat ctgagacagg gaatgttcgc ccagctggtg 660

gctcagaatg tgctgctgat tgacggacct ctgagctggt actccgaccc agggctggca 720

ggggtgtccc tgactggggg actgtcctac aaagaagata caaaagaact ggtggtggct 780

aaagctgggg tgtactatgt gttttttcag ctggaactga ggcgggtggt ggctggggag 840

ggctcaggat ctgtgtccct ggctctgcat ctgcagccac tgcgctctgc tgctggcgca 900

gctgcactgg ctctgactgt ggacctgcca ccagcctcta gcgaggccag aaacagcgcc 960

ttcgggttcc aaggacgcct gctgcatctg agcgccggac agcgcctggg agtgcatctg 1020

catactgaag ccagagcccg gcatgcttgg cagctgactc aggggggcaac tgtgctggga 1080

ctgtttcgcg tgacacctga gatccctgcc ggactgccaa gccctagatc agaagggggc 1140

ggaggttccg gagggggagg atctcgtacg gtggctgcac catctgtctt tatcttccca 1200

cccagcgacc ggaagctgaa gtctggcaca gccagcgtcg tgtgcctgct gaataacttc 1260

tacccccgcg aggccaaggt gcagtggaag gtggacaatg ccctgcagag cggcaacagc 1320

caggaaagcg tgaccgagca ggacagcaag gactccacct acagcctgag cagcaccctg 1380

accctgagca aggccgacta cgagaagcac aaggtgtacg cctgcgaagt gacccaccag 1440

ggcctgtcta gccccgtgac caagagcttc aaccggggcg agtgcgacaa gacccacacc 1500

tgtcctccat gccctgcccc tgaagctgct ggcggcccta gcgtgttcct gttcccccca 1560

aagcccaagg acaccctgat gatcagccgg acccctgaag tgacctgcgt ggtggtggat 1620

gtgtcccacg aggaccctga agtgaagttc aattggtacg tggacggcgt ggaagtgcac 1680

aatgccaaga ccaagccgcg ggaggagcag tacaacagca cgtaccgtgt ggtcagcgtc 1740

ctcaccgtcc tgcaccagga ctggctgaat ggcaaggagt acaagtgcaa ggtctccaac 1800

aaagccctcg gcgcccccat cgagaaaacc atctccaaag ccaaagggca gccccgagaa 1860

ccacaggtgt acaccctgcc cccatgccgg gatgagctga ccaagaacca ggtcagcctg 1920

tggtgcctgg tcaaaggctt ctatcccagc gacatcgccg tggagtggga gagcaatggg 1980

cagccggaga acaactacaa gaccacgcct cccgtgctgg actccgacgg ctccttcttc 2040

ctctacagca agctcaccgt ggacaagagc aggtggcagc aggggaacgt cttctcatgc 2100

tccgtgatgc atgaggctct gcacaaccac tacacgcaga agagcctctc cctgtctccg 2160

ggtaaa 2166

<210> 132
<211> 891
<212> DNA
<213> Artificial Sequence

<220>
<223> nucleotide sequence of monomeric hu 4-1BBL (71-254) - CH1*

<400> 132
agagagggcc ctgagctgag ccccgatgat cctgctggac tgctggacct gcggcagggc 60

atgtttgctc agctggtggc ccagaacgtg ctgctgatcg atggccccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agaagcgctg ctggcgctgc agctctggct ctgacagtgg atctgcctcc tgccagctcc 360

gaggcccgga atagcgcatt tgggtttcaa ggccggctgc tgcacctgtc tgccggccag 420

agactgggag tgcatctgca cacagaggcc agagccaggc acgcctggca gctgacacag 480

ggcgctacag tgctgggcct gttcagagtg acccccgaga ttcctgccgg cctgcctagc 540

cctagatctg aaggcggcgg aggttccgga ggcggaggat ctgctagcac aaagggcccc 600

agcgtgttcc ctctggcccc tagcagcaag agcacatctg gcggaacagc cgccctgggc 660

tgcctggtgg aagattactt ccccgagccc gtgaccgtgt cctggaattc tggcgccctg 720

acaagcggcg tgcacacctt tccagccgtg ctgcagagca gcggcctgta ctctctgagc 780

agcgtcgtga cagtgcccag cagctctctg ggcacccaga cctacatctg caacgtgaac 840

cacaagccca gcaacaccaa ggtggacgag aaggtggaac ccaagtcctg c 891

<210> 133
<211> 1350
<212> DNA
<213> Artificial Sequence

<220>
<223> nucleotide sequence of anti-PD1(0314) Fc hole chain

<400> 133
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccctc cgtgttcccc ctggccccca gcagcaagag caccagcggc 420

ggcacagccg ctctgggctg cctggtcaag gactacttcc ccgagcccgt gaccgtgtcc 480

tggaacagcg gagccctgac ctccggcgtg cacaccttcc ccgccgtgct gcagagttct 540

ggcctgtata gcctgagcag cgtggtcacc gtgccttcta gcagcctggg cacccagacc 600

tacatctgca acgtgaacca caagcccagc aacaccaagg tggacaagaa ggtggagccc 660

aagagctgcg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

```
gagtacaagt gcaaggtctc caacaaagcc ctcggcgccc ccatcgagaa aaccatctcc     1020

aaagccaaag ggcagccccg agaaccacag gtgtgcaccc tgcccccatc ccgggatgag     1080

ctgaccaaga accaggtcag cctctcgtgc gcagtcaaag gcttctatcc cagcgacatc     1140

gccgtggagt gggagagcaa tgggcagccg gagaacaact acaagaccac gcctcccgtg     1200

ctggactccg acggctcctt cttcctcgtg agcaagctca ccgtggacaa gagcaggtgg     1260

cagcagggga acgtcttctc atgctccgtg atgcatgagg ctctgcacaa ccactacacg     1320

cagaagagcc tctccctgtc tccgggtaaa                                      1350
```

<210> 134
<211> 654
<212> DNA
<213> Artificial Sequence

<220>
<223> nucleotide sequence of anti-PD1 (0314) light chain

<400> 134

```
gagatcgtgc tgacccagag ccccgccaca ctctccctgt ctcctggcga aagggctacc       60

ctttcatgca gagcaagcga gtccgtggac acttctgata acagtttcat tcactggtat      120

cagcagaagc cagggcagag cccccgcctg ctcatctatc ggtcctctac actggagtca      180

gggattcctg ccaggtttag cggctccggt tctgggaccg acttcacttt gaccatcagt      240

agcctggaac cagaggattt tgctgtgtac tattgtcaac agaattacga cgtcccctgg      300

acattcggcc agggaaccaa agtggagata aagcgtacgg tggctgcacc atctgtcttc      360

atcttcccgc catctgatga gcagttgaaa tctggaactg cctctgttgt gtgcctgctg      420

aataacttct atcccagaga ggccaaagta cagtggaagg tggataacgc cctccaatcg      480

ggtaactccc aggagagtgt cacagagcag gacagcaagg acagcaccta cagcctcagc      540

agcaccctga cgctgagcaa agcagactac gagaaacaca aagtctacgc ctgcgaagtc      600

acccatcagg gcctgagctc gcccgtcaca aagagcttca acaggggaga gtgt            654
```

<210> 135
<211> 2166
<212> DNA
<213> Artificial Sequence

<220>
<223> nucleotide sequence of dimeric hu 4-1BBL (71-254) - CL Fc knob chain

<400> 135

```
agagagggcc ctgagctgag ccccgatgat cctgctggac tgctggacct gcggcagggc       60

atgtttgctc agctggtggc ccagaacgtg ctgctgatcg atggccccct gtcctggtac      120

agcgatcctg gactggctgg cgtgtcactg acaggcggcc tgagctacaa agaggacacc      180
```

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agaagcgctg ctggcgctgc agctctggca ctgacagtgg atctgcctcc tgccagctcc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg cctgccttct 540

ccaagaagcg aaggcggagg cggatctggc ggcggaggat ctagagaggg acccgaactg 600

tcccctgacg atccagccgg gctgctggat ctgagacagg gaatgttcgc ccagctggtg 660

gctcagaatg tgctgctgat tgacggacct ctgagctggt actccgaccc agggctggca 720

ggggtgtccc tgactggggg actgtcctac aaagaagata caaaagaact ggtggtggct 780

aaagctgggg tgtactatgt gttttttcag ctggaactga ggcgggtggt ggctggggag 840

ggctcaggat ctgtgtccct ggctctgcat ctgcagccac tgcgctctgc tgctggcgca 900

gctgcactgg ctctgactgt ggacctgcca ccagcctcta gcgaggccag aaacagcgcc 960

ttcgggttcc aaggacgcct gctgcatctg agcgccggac agcgcctggg agtgcatctg 1020

catactgaag ccagagcccg gcatgcttgg cagctgactc agggggcaac tgtgctggga 1080

ctgtttcgcg tgacacctga gatccctgcc ggactgccaa gccctagatc agaaggggc 1140

ggaggttccg gagggggagg atctcgtacg gtggccgctc cctccgtgtt tatctttccc 1200

ccatccgatg aacagctgaa aagcggcacc gcctccgtcg tgtgtctgct gaacaatttt 1260

taccctaggg aagctaaagt gcagtggaaa gtggataacg cactgcagtc cggcaactcc 1320

caggaatctg tgacagaaca ggactccaag gacagcacct actccctgtc ctccaccctg 1380

acactgtcta aggctgatta tgagaaacac aaagtctacg cctgcgaagt cacccatcag 1440

ggcctgagct cgcccgtcac aaagagcttc aacaggggag agtgtgacaa gacccacacc 1500

tgtccccctt gtcctgcccc tgaagctgct ggcggccctt ctgtgttcct gttcccccca 1560

aagcccaagg acaccctgat gatcagccgg acccccgaag tgacctgcgt ggtggtggat 1620

gtgtcccacg aggaccctga agtgaagttc aattggtacg tggacggcgt ggaagtgcac 1680

aatgccaaga ccaagccgcg ggaggagcag tacaacagca cgtaccgtgt ggtcagcgtc 1740

ctcaccgtcc tgcaccagga ctggctgaat ggcaaggagt acaagtgcaa ggtctccaac 1800

aaagccctcg gcgcccccat cgagaaaacc atctccaaag ccaaagggca gccccgagaa 1860

ccacaggtgt acaccctgcc cccatgccgg gatgagctga ccaagaacca ggtcagcctg 1920

tggtgcctgg tcaaaggctt ctatcccagc gacatcgccg tggagtggga gagcaatggg 1980

cagccggaga acaactacaa gaccacgcct cccgtgctgg actccgacgg ctccttcttc 2040

ctctacagca agctcaccgt ggacaagagc aggtggcagc aggggaacgt cttctcatgc 2100

tccgtgatgc atgaggctct gcacaaccac tacacgcaga agagcctctc cctgtctccg 2160

ggtaaa 2166

<210> 136
<211> 891
<212> DNA
<213> Artificial Sequence

<220>
<223> nucleotide sequence of monomeric hu 4-1BBL (71-254) - CH1

<400> 136
agagagggcc ctgagctgag ccccgatgat cctgctggac tgctggacct gcggcagggc 60

atgtttgctc agctggtggc ccagaacgtg ctgctgatcg atggcccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agaagcgctg ctggcgctgc agctctggct ctgacagtgg atctgcctcc tgccagctcc 360

gaggcccgga atagcgcatt tgggtttcaa ggccggctgc tgcacctgtc tgccggccag 420

agactgggag tgcatctgca cacagaggcc agagccaggc acgcctggca gctgacacag 480

ggcgctacag tgctgggcct gttcagagtg acccccgaga ttcctgccgg cctgcctagc 540

cctagatctg aaggcggcgg aggttccgga ggcggaggat ctgctagcac caaaggccct 600

tccgtgtttc ctctggctcc tagctccaag tccacctctg gaggcaccgc tgctctcgga 660

tgcctcgtga aggattattt tcctgagcct gtgacagtgt cctggaatag cggagcactg 720

acctctggag tgcatacttt ccccgctgtg ctgcagtcct ctggactgta cagcctgagc 780

agcgtggtga cagtgcccag cagcagcctg ggcacccaga cctacatctg caacgtgaac 840

cacaagccca gcaacaccaa ggtggacaag aaggtggaac ccaagtcttg t 891

<210> 137
<211> 2511
<212> DNA
<213> Artificial Sequence

<220>
<223> nucleotide sequence of anti-PD1(0314) Fc hole chain fused to dimeric hu 4-1BBL (71-254)

<400> 137
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240
ttgcagatga acagcctgag agctgaggat acagcagttt actactgtgt gctcctgacc 300
ggccgcgtct attttgccct tgactcctgg ggacaaggca ctctggtgac cgtatctagt 360
gctagcacca agggcccctc cgtgttcccc ctggccccca gcagcaagag caccagcggc 420
ggcacagccg ctctgggctg cctggtcaag gactacttcc ccgagcccgt gaccgtgtcc 480
tggaacagcg gagccctgac ctccggcgtg cacaccttcc ccgccgtgct gcagagttct 540
ggcctgtata gcctgagcag cgtggtcacc gtgccttcta gcagcctggg cacccagacc 600
tacatctgca acgtgaacca caagcccagc aacaccaagg tggacaagaa ggtggagccc 660
aagagctgcg acaaaactca cacatgccca ccgtgcccag cacctgaagc tgcaggggga 720
ccgtcagtct tcctcttccc cccaaaaccc aaggacaccc tcatgatctc ccggacccct 780
gaggtcacat gcgtggtggt ggacgtgagc cacgaagacc ctgaggtcaa gttcaactgg 840
tacgtggacg gcgtggaggt gcataatgcc aagacaaagc cgcgggagga gcagtacaac 900
agcacgtacc gtgtggtcag cgtcctcacc gtcctgcacc aggactggct gaatggcaag 960
gagtacaagt gcaaggtctc caacaaagcc ctcggcgccc ccatcgagaa aaccatctcc 1020
aaagccaaag ggcagccccg agaaccacag gtgtgcaccc tgcccccatc ccgggatgag 1080
ctgaccaaga accaggtcag cctctcgtgc gcagtcaaag gcttctatcc cagcgacatc 1140
gccgtggagt gggagagcaa tgggcagccg gagaacaact acaagaccac gcctcccgtg 1200
ctggactccg acggctcctt cttcctcgtg agcaagctca ccgtggacaa gagcaggtgg 1260
cagcagggga acgtcttctc atgctccgtg atgcatgagg ctctgcacaa ccactacacg 1320
cagaagagcc tctccctgtc tccgggtgga ggcggcggaa gcggaggagg aggatccaga 1380
gagggccctg agctgagccc cgatgatcct gctggactgc tggacctgcg gcagggcatg 1440
tttgctcagc tggtggccca gaacgtgctg ctgatcgatg gccccctgtc ctggtacagc 1500
gatcctggac tggctggcgt gtcactgaca ggcggcctga gctacaaaga ggacaccaaa 1560
gaactggtgg tggccaaggc cggcgtgtac tacgtgttct ttcagctgga actgcggaga 1620
gtggtggccg gcgaaggatc tggctctgtg tctctggccc tgcatctgca gcctctgaga 1680
agcgctgctg gcgctgcagc tctggcactg acagtggatc tgcctcctgc cagctccgag 1740
gcccggaata gcgcatttgg gtttcaaggc aggctgctgc acctgtctgc cggccagagg 1800
ctgggagtgc atctgcacac agaggccagg gctagacacg cctggcagct gacacagggc 1860
gctacagtgc tgggcctgtt cagagtgacc cccgagattc cagccggcct gccttctcca 1920
agaagcgaag gcggaggcgg atctggcggc ggaggatcta gagagggacc cgaactgtcc 1980
cctgacgatc cagccgggct gctggatctg agacagggaa tgttcgccca gctggtggct 2040
cagaatgtgc tgctgattga cggacctctg agctggtact ccgacccagg gctggcaggg 2100

gtgtccctga ctgggggact gtcctacaaa gaagatacaa aagaactggt ggtggctaaa 2160

gctggggtgt actatgtgtt ttttcagctg gaactgaggc gggtggtggc tggggagggc 2220

tcaggatctg tgtccctggc tctgcatctg cagccactgc gctctgctgc tggcgcagct 2280

gcactggctc tgactgtgga cctgccacca gcctctagcg aggccagaaa cagcgccttc 2340

gggttccaag gacgcctgct gcatctgagc gccggacagc gcctgggagt gcatctgcat 2400

actgaagcca gagcccggca tgcttggcag ctgactcagg gggcaactgt gctgggactg 2460

tttcgcgtga cacctgagat ccctgccgga ctgccaagcc ctagatcaga a 2511

<210> 138
<211> 1929
<212> DNA
<213> Artificial Sequence

<220>
<223> nucleotide sequence of anti-PD1(0314) Fc knob chain fused to monomeric hu 4-1BBL (71-254)

<400> 138
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtacaccc tgccccctg cagagatgag 1080

ctgaccaaga accaggtgtc cctgtggtgt ctggtcaagg gcttctaccc cagcgatatc 1140

gccgtggagt gggagagcaa cggccagcct gagaacaact acaagaccac ccccccctgtg 1200

ctggacagcg acggcagctt cttcctgtac tccaaactga ccgtggacaa gagccggtgg 1260

cagcagggca acgtgttcag ctgcagcgtg atgcacgagg ccctgcacaa ccactacacc 1320

cagaagtccc tgagcctgag ccccggcgga ggcggcggaa gcggaggagg aggatccaga 1380

gagggccctg agctgagccc cgatgatcct gctggactgc tggacctgcg gcagggcatg 1440

tttgctcagc tggtggccca gaacgtgctg ctgatcgatg gcccccctgtc ctggtacagc 1500

gatcctggac tggctggcgt gtcactgaca ggcggcctga gctacaaaga ggacaccaaa 1560

gaactggtgg tggccaaggc cggcgtgtac tacgtgttct ttcagctgga actgcggaga 1620

gtggtggccg gcgaaggatc tggctctgtg tctctggccc tgcatctgca gcctctgaga 1680

agcgctgctg gcgctgcagc tctggcactg acagtggatc tgcctcctgc cagctccgag 1740

gcccggaata gcgcatttgg gtttcaaggc aggctgctgc acctgtctgc cggccagagg 1800

ctgggagtgc atctgcacac agaggccagg gctagacacg cctggcagct gacacagggc 1860

gctacagtgc tgggcctgtt cagagtgacc cccgagattc cagccggcct gccttctcca 1920

agaagcgaa 1929

<210> 139
<211> 2130
<212> DNA
<213> Artificial Sequence

<220>
<223> nucleotide sequence of dimeric hu 4-1BBL (71-248) - CL* Fc knob chain

<400> 139
agagagggcc ctgagctgag ccccgatgat cctgctggac tgctggacct gcggcagggc 60

atgtttgctc agctggtggc ccagaacgtg ctgctgatcg atggcccccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agatctgctg ctggcgccgc tgctctggca ctgacagtgg atctgcctcc tgccagcagc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg actgggaggc 540

ggcggatctg gcggcggagg atctagagaa ggacccgagc tgtcccctga cgatccagcc 600

gggctgctgg atctgagaca gggaatgttc gcccagctgg tggctcagaa tgtgctgctg 660

attgacggac ctctgagctg gtactccgac ccagggctgg caggggtgtc cctgactggg 720

ggactgtcct acaaagaaga tacaaaagaa ctggtggtgg ctaaagctgg ggtgtactat 780

gtgtttttc agctggaact gaggcgggtg gtggctgggg agggctcagg atctgtgtcc 840

ctggctctgc atctgcagcc actgcgctct gcagcagggg ctgcagcact ggccctgact 900

gtggacctgc ccccagcttc ttccgaggcc agaaacagcg ccttcgggtt ccaaggacgc 960

ctgctgcatc tgagcgccgg acagcgcctg ggagtgcatc tgcatactga agccagagcc 1020

cggcatgctt ggcagctgac tcaggggggca actgtgctgg gactgtttcg cgtgacacct 1080

gagatccccg ctggactggg cggaggcggt tccggagggg gaggatctcg tacggtggct 1140

gcaccatctg tctttatctt cccacccagc gaccggaagc tgaagtctgg cacagccagc 1200

gtcgtgtgcc tgctgaataa cttctacccc cgcgaggcca aggtgcagtg gaaggtggac 1260

aatgccctgc agagcggcaa cagccaggaa agcgtgaccg agcaggacag caaggactcc 1320

acctacagcc tgagcagcac cctgaccctg agcaaggccg actacgagaa gcacaaggtg 1380

tacgcctgcg aagtgaccca ccagggcctg tctagccccg tgaccaagag cttcaaccgg 1440

ggcgagtgcg acaagaccca cacctgtcct ccatgccctg cccctgaagc tgctggcggc 1500

cctagcgtgt tcctgttccc cccaaagccc aaggacaccc tgatgatcag ccggacccct 1560

gaagtgacct gcgtggtggt ggatgtgtcc cacgaggacc ctgaagtgaa gttcaattgg 1620

tacgtggacg gcgtggaagt gcacaatgcc aagaccaagc cgcgggagga gcagtacaac 1680

agcacgtacc gtgtggtcag cgtcctcacc gtcctgcacc aggactggct gaatggcaag 1740

gagtacaagt gcaaggtctc caacaaagcc ctcggcgccc ccatcgagaa aaccatctcc 1800

aaagccaaag ggcagccccg agaaccacag gtgtacaccc tgcccccatg ccgggatgag 1860

ctgaccaaga accaggtcag cctgtggtgc ctggtcaaag gcttctatcc cagcgacatc 1920

gccgtggagt gggagagcaa tgggcagccg gagaacaact acaagaccac gcctcccgtg 1980

ctggactccg acggctcctt cttcctctac agcaagctca ccgtggacaa gagcaggtgg 2040

cagcagggga acgtcttctc atgctccgtg atgcatgagg ctctgcacaa ccactacacg 2100

cagaagagcc tctccctgtc tccgggtaaa 2130

<210> 140
<211> 873
<212> DNA
<213> Artificial Sequence

<220>
<223> nucleotide sequence of monomeric hu 4-1BBL (71-248) - CH1*

<400> 140
agagagggcc ctgagctgag ccccgatgat cctgctggac tgctggacct gcggcagggc 60

atgtttgctc agctggtggc ccagaacgtg ctgctgatcg atggcccccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agatctgctg ctggcgccgc tgctctggca ctgacagtgg atctgcctcc tgccagcagc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg actgggaggc 540

ggaggttccg gaggcggagg atctgctagc acaaagggcc ccagcgtgtt ccctctggcc 600

cctagcagca agagcacatc tggcggaaca gccgccctgg gctgcctggt ggaagattac 660

ttccccgagc ccgtgaccgt gtcctggaat tctggcgccc tgacaagcgg cgtgcacacc 720

tttccagccg tgctgcagag cagcggcctg tactctctga gcagcgtcgt gacagtgccc 780

agcagctctc tgggcaccca gacctacatc tgcaacgtga accacaagcc cagcaacacc 840

aaggtggacg agaaggtgga acccaagtcc tgc 873

<210> 141
<211> 2130
<212> DNA
<213> Artificial Sequence

<220>
<223> nucleotide sequence of dimeric hu 4-1BBL (71-248) - CL Fc knob chain

<400> 141
agagagggcc ctgagctgag ccccgatgat cctgctggac tgctggacct gcggcagggc 60

atgtttgctc agctggtggc ccagaacgtg ctgctgatcg atggccccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agatctgctg ctggcgccgc tgctctggca ctgacagtgg atctgcctcc tgccagcagc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg actgggaggc 540

ggcggatctg gcggcggagg atctagagaa ggacccgagc tgtcccctga cgatccagcc 600

gggctgctgg atctgagaca gggaatgttc gcccagctgg tggctcagaa tgtgctgctg 660

attgacggac ctctgagctg gtactccgac ccagggctgg caggggtgtc cctgactggg 720

ggactgtcct acaaagaaga tacaaaagaa ctggtggtgg ctaaagctgg ggtgtactat 780

gtgtttttc agctggaact gaggcgggtg gtggctgggg agggctcagg atctgtgtcc 840

ctggctctgc atctgcagcc actgcgctct gcagcagggg ctgcagcact ggccctgact 900

gtggacctgc cccagcttc ttccgaggcc agaaacagcg ccttcgggtt ccaaggacgc 960

ctgctgcatc tgagcgccgg acagcgcctg ggagtgcatc tgcatactga agccagagcc 1020

cggcatgctt ggcagctgac tcagggggca actgtgctgg gactgtttcg cgtgacacct 1080

gagatccccg ctggactggg cggaggcggt tccggagggg gaggatctcg tacggtggcc 1140

gctccctccg tgtttatctt tcccccatcc gatgaacagc tgaaaagcgg caccgcctcc 1200

gtcgtgtgtc tgctgaacaa tttttaccct agggaagcta aagtgcagtg gaaagtggat 1260

aacgcactgc agtccggcaa ctcccaggaa tctgtgacag aacaggactc caaggacagc 1320

acctactccc tgtcctccac cctgacactg tctaaggctg attatgagaa acacaaagtc 1380

tacgcctgcg aagtcaccca tcagggcctg agctcgcccg tcacaaagag cttcaacagg 1440

ggagagtgtg acaagaccca cacctgtccc ccttgtcctg cccctgaagc tgctggcggc 1500

ccttctgtgt tcctgttccc cccaaagccc aaggacaccc tgatgatcag ccggaccccc 1560

gaagtgacct gcgtggtggt ggatgtgtcc cacgaggacc ctgaagtgaa gttcaattgg 1620

tacgtggacg gcgtggaagt gcacaatgcc aagaccaagc cgcgggagga gcagtacaac 1680

agcacgtacc gtgtggtcag cgtcctcacc gtcctgcacc aggactggct gaatggcaag 1740

gagtacaagt gcaaggtctc caacaaagcc ctcggcgccc ccatcgagaa aaccatctcc 1800

aaagccaaag ggcagccccg agaaccacag gtgtacaccc tgcccccatg ccgggatgag 1860

ctgaccaaga accaggtcag cctgtggtgc ctggtcaaag gcttctatcc cagcgacatc 1920

gccgtggagt gggagagcaa tgggcagccg gagaacaact acaagaccac gcctcccgtg 1980

ctggactccg acggctcctt cttcctctac agcaagctca ccgtggacaa gagcaggtgg 2040

cagcagggga acgtcttctc atgctccgtg atgcatgagg ctctgcacaa ccactacacg 2100

cagaagagcc tctccctgtc tccgggtaaa 2130

<210> 142
<211> 873
<212> DNA
<213> Artificial Sequence

<220>
<223> nucleotide sequence of monomeric hu 4-1BBL (71-248) - CH1

<400> 142
agagagggcc ctgagctgag ccccgatgat cctgctggac tgctggacct gcggcagggc 60

atgtttgctc agctggtggc ccagaacgtg ctgctgatcg atggccccct gtcctggtac 120

agcgatcctg gactggctgg cgtgtcactg acaggcggcc tgagctacaa agaggacacc 180

aaagaactgg tggtggccaa ggccggcgtg tactacgtgt tctttcagct ggaactgcgg 240

agagtggtgg ccggcgaagg atctggctct gtgtctctgg ccctgcatct gcagcctctg 300

agatctgctg ctggcgccgc tgctctggca ctgacagtgg atctgcctcc tgccagcagc 360

gaggcccgga atagcgcatt tgggtttcaa ggcaggctgc tgcacctgtc tgccggccag 420

aggctgggag tgcatctgca cacagaggcc agggctagac acgcctggca gctgacacag 480

ggcgctacag tgctgggcct gttcagagtg acccccgaga ttccagccgg actgggaggc 540

ggaggttccg gaggcggagg atctgctagc accaaaggcc cttccgtgtt tcctctggct 600

cctagctcca agtccacctc tggaggcacc gctgctctcg gatgcctcgt gaaggattat 660

tttcctgagc ctgtgacagt gtcctggaat agcggagcac tgacctctgg agtgcatact 720

ttccccgctg tgctgcagtc ctctggactg tacagcctga gcagcgtggt gacagtgccc 780

agcagcagcc tgggcaccca gacctacatc tgcaacgtga accacaagcc cagcaacacc 840

aaggtggaca agaaggtgga acccaagtct tgt 873

<210> 143
<211> 2475
<212> DNA
<213> Artificial Sequence

<220>
<223> nucleotide sequence of anti-PD1(0314) Fc hole chain fused to dimeric hu 4-1BBL (71-248)

<400> 143
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccctc cgtgttcccc ctggccccca gcagcaagag caccagcggc 420

ggcacagccg ctctgggctg cctggtcaag gactacttcc ccgagcccgt gaccgtgtcc 480

tggaacagcg gagccctgac ctccggcgtg cacaccttcc ccgccgtgct gcagagttct 540

ggcctgtata gcctgagcag cgtggtcacc gtgccttcta gcagcctggg cacccagacc 600

tacatctgca acgtgaacca caagcccagc aacaccaagg tggacaagaa ggtggagccc 660

aagagctgcg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtgcaccc tgcccccatc ccgggatgag 1080

ctgaccaaga accaggtcag cctctcgtgc gcagtcaaag gcttctatcc cagcgacatc 1140

gccgtggagt gggagagcaa tgggcagccg gagaacaact acaagaccac gcctcccgtg 1200

ctggactccg acggctcctt cttcctcgtg agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtgga ggcggcggaa gcggaggagg aggatccaga 1380

gagggccctg agctgagccc tgatgatcct gccggactgc tggacctgcg gcagggaatg 1440

tttgcccagc tggtggccca gaacgtgctg ctgatcgatg gccccctgtc ctggtacagc 1500

gatcctggac tggctggcgt gtcactgaca ggcggcctga gctacaaaga ggacaccaaa 1560

gaactggtgg tggccaaggc cggcgtgtac tacgtgttct ttcagctgga actgcggaga 1620

gtggtggccg gcgaaggatc tggctctgtg tctctggccc tgcatctgca gcctctgaga 1680

tctgctgctg gcgccgctgc tctggcactg acagtggatc tgcctcctgc cagcagcgag 1740

gcccggaata gcgcatttgg gtttcaaggc aggctgctgc acctgtctgc cggccagagg 1800

ctgggagtgc atctgcacac agaggccagg gctagacacg cctggcagct gacacagggc 1860

gctacagtgc tgggcctgtt cagagtgacc cccgagattc cagcaggcct gggaggcggc 1920

ggatctggcg gcggaggatc tagagaagga cccgagctgt cccccgacga tcccgctggg 1980

ctgctggatc tgagacaggg catgttcgct cagctggtgg ctcagaatgt gctgctgatt 2040

gacggacctc tgagctggta ctccgaccca gggctggcag gggtgtccct gactggggga 2100

ctgtcctaca aagaagatac aaaagaactg gtggtggcta aagctggggt gtactatgtg 2160

ttttttcagc tggaactgag gcgggtggtg gctggggagg gctcaggatc tgtgtccctg 2220

gctctgcatc tgcagccact gcgctctgca gcaggggctg cagcactggc cctgactgtg 2280

gacctgcccc cagcttcttc cgaggccaga aacagcgcct tcgggttcca aggacgcctg 2340

ctgcatctga gcgccggaca gcgcctggga gtgcatctgc atactgaagc cagagcccgg 2400

catgcttggc agctgactca gggggcaact gtgctgggac tgtttcgcgt gacacctgag 2460

atcccagccg ggctc 2475

<210> 144
<211> 1911
<212> DNA
<213> Artificial Sequence

<220>
<223> nucleotide sequence anti-PD1(0314) Fc knob chain fused to monomeric hu 4-1BBL (71-248)

<400> 144

gaagtgcagc tgctcgaaag cggcgggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtacaccc tgccccctg cagagatgag 1080

ctgaccaaga accaggtgtc cctgtggtgt ctggtcaagg gcttctaccc cagcgatatc 1140

gccgtggagt gggagagcaa cggccagcct gagaacaact acaagaccac cccccctgtg 1200

ctggacagcg acggcagctt cttcctgtac tccaaactga ccgtggacaa gagccggtgg 1260

cagcagggca acgtgttcag ctgcagcgtg atgcacgagg ccctgcacaa ccactacacc 1320

cagaagtccc tgagcctgag ccccggcgga ggcggcggaa gcggaggagg aggatccaga 1380

gagggccctg agctgagccc tgatgatcct gccggactgc tggacctgcg gcagggaatg 1440

tttgcccagc tggtggccca gaacgtgctg ctgatcgatg gccccctgtc ctggtacagc 1500

gatcctggac tggctggcgt gtcactgaca ggcggcctga gctacaaaga ggacaccaaa 1560

gaactggtgg tggccaaggc cggcgtgtac tacgtgttct ttcagctgga actgcggaga 1620

gtggtggccg gcgaaggatc tggctctgtg tctctggccc tgcatctgca gcctctgaga 1680

tctgctgctg gcgccgctgc tctggcactg acagtggatc tgcctcctgc cagcagcgag 1740

gcccggaata gcgcatttgg gtttcaaggc aggctgctgc acctgtctgc cggccagagg 1800

ctgggagtgc atctgcacac agaggccagg gctagacacg cctggcagct gacacagggc 1860

gctacagtgc tgggcctgtt cagagtgacc cccgagattc ctgccgggct c 1911

<210> 145
<211> 1806
<212> DNA
<213> Artificial Sequence

<220>
<223> nucleotide sequence of Fc hole dimeric ligand (71-248) chain

<400> 145
gacaaaactc acacatgccc accgtgccca gcacctgaag ctgcaggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcggcgcc cccatcgaga aaaccatctc caaagccaaa 360

gggcagcccc gagaaccaca ggtgtgcacc ctgcccccat cccgggatga gctgaccaag 420

aaccaggtca gcctctcgtg cgcagtcaaa ggcttctatc ccagcgacat cgccgtggag 480

tgggagagca atgggcagcc ggagaacaac tacaagacca cgcctcccgt gctggactcc 540

gacggctcct tcttcctcgt gagcaagctc accgtggaca agagcaggtg gcagcagggg 600

aacgtcttct catgctccgt gatgcatgag gctctgcaca accactacac gcagaagagc 660

ctctccctgt ctccgggtgg aggcggcgga agcggaggag gaggatccag agagggccct 720

gagctgagcc ctgatgatcc tgccggactg ctggacctgc ggcagggaat gtttgcccag 780

ctggtggccc agaacgtgct gctgatcgat ggccccctgt cctggtacag cgatcctgga 840

ctggctggcg tgtcactgac aggcggcctg agctacaaag aggacaccaa agaactggtg 900

gtggccaagg ccggcgtgta ctacgtgttc tttcagctgg aactgcggag agtggtggcc 960

ggcgaaggat ctggctctgt gtctctggcc ctgcatctgc agcctctgag atctgctgct 1020

ggcgccgctg ctctggcact gacagtggat ctgcctcctg ccagcagcga ggcccggaat 1080

agcgcatttg ggtttcaagg caggctgctg cacctgtctg ccggccagag gctgggagtg 1140

catctgcaca cagaggccag ggctagacac gcctggcagc tgacacaggg cgctacagtg 1200

ctgggcctgt tcagagtgac ccccgagatt ccagcaggcc tgggaggcgg cggatctggc 1260

ggcggaggat ctagagaagg acccgagctg tcccccgacg atcccgctgg gctgctggat 1320

ctgagacagg gcatgttcgc tcagctggtg gctcagaatg tgctgctgat tgacggacct 1380

ctgagctggt actccgaccc aggctggca ggggtgtccc tgactggggg actgtcctac 1440

aaagaagata caaaagaact ggtggtggct aaagctgggg tgtactatgt gtttttttcag 1500

ctggaactga ggcgggtggt ggctggggag ggctcaggat ctgtgtccct ggctctgcat 1560

ctgcagccac tgcgctctgc agcaggggct gcagcactgg ccctgactgt ggacctgccc 1620

ccagcttctt ccgaggccag aaacagcgcc ttcgggttcc aaggacgcct gctgcatctg 1680

agcgccggac agcgcctggg agtgcatctg catactgaag ccagagcccg gcatgcttgg 1740

cagctgactc agggggcaac tgtgctggga ctgtttcgcg tgacacctga gatcccagcc 1800

gggctc 1806

<210> 146
<211> 1212
<212> DNA
<213> Artificial Sequence

<220>
<223> nucleotide sequence of Fc hole monomeric ligand (71-248) chain

<400> 146
gacaaaactc acacatgccc accgtgccca gcacctgaag ctgcaggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcggcgcc cccatcgaga aaaccatctc caaagccaaa 360

gggcagcccc gagaaccaca ggtgtgcacc ctgcccccat cccgggatga gctgaccaag 420

aaccaggtca gcctctcgtg cgcagtcaaa ggcttctatc ccagcgacat cgccgtggag 480

tgggagagca atgggcagcc ggagaacaac tacaagacca cgcctcccgt gctggactcc 540

gacggctcct tcttcctcgt gagcaagctc accgtggaca agagcaggtg gcagcagggg 600

aacgtcttct catgctccgt gatgcatgag gctctgcaca accactacac gcagaagagc 660

ctctccctgt ctccgggtag agagggccct gagctgagcc ctgatgatcc tgccggactg 720

ctggacctgc ggcagggaat gtttgcccag ctggtggccc agaacgtgct gctgatcgat 780

ggccccctgt cctggtacag cgatcctgga ctggctggcg tgtcactgac aggcggcctg 840

agctacaaag aggacaccaa agaactggtg gtggccaagg ccggcgtgta ctacgtgttc 900

tttcagctgg aactgcggag agtggtggcc ggcgaaggat ctggctctgt gtctctggcc 960

ctgcatctgc agcctctgag atctgctgct ggcgccgctg ctctggcact gacagtggat 1020

ctgcctcctg ccagcagcga ggcccggaat agcgcatttg ggtttcaagg caggctgctg 1080

cacctgtctg ccggccagag gctgggagtg catctgcaca cagaggccag ggctagacac 1140

gcctggcagc tgacacaggg cgctacagtg ctgggcctgt tcagagtgac ccccgagatt 1200

cctgccgggc tc 1212

<210> 147
<211> 2445
<212> DNA
<213> Artificial Sequence

<220>
<223> nucleotide sequence of anti-PD1(0314) Fc knob chain fused to dimeric hu 4-1BBL (71-248)

<400> 147
gaagtgcagc tgctcgaaag cggcgggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtacaccc tgccccctg cagagatgag 1080

ctgaccaaga accaggtgtc cctgtggtgt ctggtcaagg gcttctaccc cagcgatatc 1140

gccgtggagt gggagagcaa cggccagcct gagaacaact acaagaccac cccccctgtg 1200

ctggacagcg acggcagctt cttcctgtac tccaaactga ccgtggacaa gagccggtgg 1260

cagcagggca acgtgttcag ctgcagcgtg atgcacgagg ccctgcacaa ccactacacc 1320

cagaagtccc tgagcctgag ccccggcaga gagggccctg agctgagccc tgatgatcct 1380

gccggactgc tggacctgcg gcagggaatg tttgcccagc tggtggccca gaacgtgctg 1440

ctgatcgatg gccccctgtc ctggtacagc gatcctggac tggctggcgt gtcactgaca 1500

ggcggcctga gctacaaaga ggacaccaaa gaactggtgg tggccaaggc cggcgtgtac 1560

tacgtgttct ttcagctgga actgcggaga gtggtggccg gcgaaggatc tggctctgtg 1620

tctctggccc tgcatctgca gcctctgaga tctgctgctg gcgccgctgc tctggcactg 1680

acagtggatc tgcctcctgc cagcagcgag gcccggaata gcgcatttgg gtttcaaggc 1740

aggctgctgc acctgtctgc cggccagagg ctgggagtgc atctgcacac agaggccagg 1800

gctagacacg cctggcagct gacacagggc gctacagtgc tgggcctgtt cagagtgacc 1860

cccgagattc cagcaggcct gggaggcggc ggatctggcg gcggaggatc tagagaagga 1920

cccgagctgt cccccgacga tcccgctggg ctgctggatc tgagacaggg catgttcgct 1980

cagctggtgg ctcagaatgt gctgctgatt gacggacctc tgagctggta ctccgaccca 2040

gggctggcag ggtgtccct gactggggga ctgtcctaca aagaagatac aaaagaactg 2100

gtggtggcta aagctggggt gtactatgtg ttttttcagc tggaactgag gcgggtggtg 2160

gctggggagg gctcaggatc tgtgtccctg gctctgcatc tgcagccact gcgctctgca 2220

gcaggggctg cagcactggc cctgactgtg gacctgcccc cagcttcttc cgaggccaga 2280

aacagcgcct tcgggttcca aggacgcctg ctgcatctga gcgccggaca gcgcctggga 2340

gtgcatctgc atactgaagc cagagcccgg catgcttggc agctgactca gggggcaact 2400

gtgctgggac tgtttcgcgt gacacctgag atcccagccg ggctc 2445

<210> 148
<211> 1242
<212> DNA
<213> Artificial Sequence

<220>
<223> nucleotide sequence of Fc knob chain fused to monomeric hu 4-1BBL (71-248)

<400> 148
gacaaaactc acacatgccc accgtgccca gcacctgaag ctgcaggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcggcgcc cccatcgaga aaaccatctc caaagccaaa 360

gggcagcccc gagaaccaca ggtgtacacc ctgcccccct gcagagatga gctgaccaag 420

aaccaggtgt ccctgtggtg tctggtcaag ggcttctacc ccagcgatat cgccgtggag 480

tgggagagca acggccagcc tgagaacaac tacaagacca cccccccctgt gctggacagc 540

gacggcagct tcttcctgta ctccaaactg accgtggaca agagccggtg gcagcagggc 600

aacgtgttca gctgcagcgt gatgcacgag gccctgcaca accactacac ccagaagtcc 660

ctgagcctga gccccggcgg aggcggcgga agcggaggag gaggatccag agagggccct 720

gagctgagcc ctgatgatcc tgccggactg ctggacctgc ggcagggaat gtttgcccag 780

ctggtggccc agaacgtgct gctgatcgat ggccccctgt cctggtacag cgatcctgga 840

ctggctggcg tgtcactgac aggcggcctg agctacaaag aggacaccaa agaactggtg 900

gtggccaagg ccggcgtgta ctacgtgttc tttcagctgg aactgcggag agtggtggcc 960

ggcgaaggat ctggctctgt gtctctggcc ctgcatctgc agcctctgag atctgctgct 1020

ggcgccgctg ctctggcact gacagtggat ctgcctcctg ccagcagcga ggcccggaat 1080

agcgcatttg ggtttcaagg caggctgctg cacctgtctg ccggccagag gctgggagtg 1140

catctgcaca cagaggccag ggctagacac gcctggcagc tgacacaggg cgctacagtg 1200

ctgggcctgt tcagagtgac ccccgagatt cctgccgggc tc 1242

<210> 149
<211> 1911
<212> DNA
<213> Artificial Sequence

<220>
<223> nucleotide sequence of anti-PD1(0314) Fc hole monomeric ligand (71-248) chain

<400> 149
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccctc cgtgttcccc ctggccccca gcagcaagag caccagcggc 420

ggcacagccg ctctgggctg cctggtcaag gactacttcc ccgagcccgt gaccgtgtcc 480

tggaacagcg gagccctgac ctccggcgtg cacaccttcc ccgccgtgct gcagagttct 540

ggcctgtata gcctgagcag cgtggtcacc gtgccttcta gcagcctggg cacccagacc 600

tacatctgca acgtgaacca caagcccagc aacaccaagg tggacaagaa ggtggagccc 660

aagagctgcg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtgcaccc tgcccccatc ccgggatgag 1080

ctgaccaaga accaggtcag cctctcgtgc gcagtcaaag gcttctatcc cagcgacatc 1140

gccgtggagt gggagagcaa tgggcagccg gagaacaact acaagaccac gcctcccgtg 1200

ctggactccg acggctcctt cttcctcgtg agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtgga ggcggcggaa gcggaggagg aggatccaga 1380

gagggccctg agctgagccc tgatgatcct gccggactgc tggacctgcg gcagggaatg 1440

tttgcccagc tggtggccca gaacgtgctg ctgatcgatg gccccctgtc ctggtacagc 1500

gatcctggac tggctggcgt gtcactgaca ggcggcctga gctacaaaga ggacaccaaa 1560

gaactggtgg tggccaaggc cggcgtgtac tacgtgttct ttcagctgga actgcggaga 1620

gtggtggccg gcgaaggatc tggctctgtg tctctggccc tgcatctgca gcctctgaga 1680

tctgctgctg gcgccgctgc tctggcactg acagtggatc tgcctcctgc cagcagcgag 1740

gcccggaata gcgcatttgg gtttcaaggc aggctgctgc acctgtctgc cggccagagg 1800

ctgggagtgc atctgcacac agaggccagg gctagacacg cctggcagct gacacagggc 1860

gctacagtgc tgggcctgtt cagagtgacc cccgagattc ctgccgggct c 1911

<210> 150
<211> 1806
<212> DNA
<213> Artificial Sequence

<220>
<223> nucleotide sequence of Fc knob chain fused to dimeric hu 4-1BBL (71-248)

<400> 150
gacaaaactc acacatgccc accgtgccca gcacctgaag ctgcaggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcggcgcc cccatcgaga aaaccatctc caaagccaaa 360

gggcagcccc gagaaccaca ggtgtacacc ctgcccccct gcagagatga gctgaccaag 420

aaccaggtgt ccctgtggtg tctggtcaag ggcttctacc ccagcgatat cgccgtggag 480

tgggagagca acggccagcc tgagaacaac tacaagacca ccccccctgt gctggacagc 540

gacggcagct tcttcctgta ctccaaactg accgtggaca agagccggtg gcagcagggc 600

aacgtgttca gctgcagcgt gatgcacgag gccctgcaca accactacac ccagaagtcc 660

ctgagcctga gccccggcgg aggcggcgga agcggaggag gaggatccag agagggccct 720

gagctgagcc ctgatgatcc tgccggactg ctggacctgc ggcagggaat gtttgcccag 780

ctggtggccc agaacgtgct gctgatcgat ggccccctgt cctggtacag cgatcctgga 840

ctggctggcg tgtcactgac aggcggcctg agctacaaag aggacaccaa agaactggtg 900

gtggccaagg ccggcgtgta ctacgtgttc tttcagctgg aactgcggag agtggtggcc 960

ggcgaaggat ctggctctgt gtctctggcc ctgcatctgc agcctctgag atctgctgct 1020

ggcgccgctg ctctggcact gacagtggat ctgcctcctg ccagcagcga ggcccggaat 1080

agcgcatttg ggtttcaagg caggctgctg cacctgtctg ccggccagag gctgggagtg 1140

catctgcaca cagaggccag ggctagacac gcctggcagc tgacacaggg cgctacagtg 1200

ctgggcctgt tcagagtgac ccccgagatt ccagcaggcc tgggaggcgg cggatctggc 1260

ggcggaggat ctagagaagg acccgagctg tcccccgacg atcccgctgg gctgctggat 1320

ctgagacagg gcatgttcgc tcagctggtg gctcagaatg tgctgctgat tgacggacct 1380

ctgagctggt actccgaccc agggctggca ggggtgtccc tgactggggg actgtcctac 1440

aaagaagata caaaagaact ggtggtggct aaagctgggg tgtactatgt gtttttttcag 1500

ctggaactga ggcgggtggt ggctggggag ggctcaggat ctgtgtccct ggctctgcat 1560

ctgcagccac tgcgctctgc agcaggggct gcagcactgg ccctgactgt ggacctgccc 1620

ccagcttctt ccgaggccag aaacagcgcc ttcgggttcc aaggacgcct gctgcatctg 1680

agcgccggac agcgcctggg agtgcatctg catactgaag ccagagcccg gcatgcttgg 1740

cagctgactc aggggggcaac tgtgctggga ctgtttcgcg tgacacctga gatcccagcc 1800

gggctc 1806

<210> 151
<211> 1335
<212> DNA
<213> Artificial Sequence

<220>
<223> nucleotide sequence of DP47 Fc hole chain

<400> 151
gaggtgcaat tgttggagtc tgggggaggc ttggtacagc ctggggggtc cctgagactc 60

tcctgtgcag cctccggatt cacctttagc agttatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcagatga acagcctgag agccgaggac acggccgtat attactgtgc gaaaggcagc 300

```
ggatttgact actggggcca aggaaccctg gtcaccgtct cgagtgctag caccaagggc      360

ccatcggtct tccccctggc accctcctcc aagagcacct ctgggggcac agcggccctg      420

ggctgcctgg tcaaggacta cttccccgaa ccggtgacgg tgtcgtggaa ctcaggcgcc      480

ctgaccagcg gcgtgcacac cttcccggct gtcctacagt cctcaggact ctactccctc      540

agcagcgtgg tgaccgtgcc ctccagcagc ttgggcaccc agacctacat ctgcaacgtg      600

aatcacaagc ccagcaacac caaggtggac aagaaagttg agcccaaatc ttgtgacaaa      660

actcacacat gcccaccgtg cccagcacct gaagctgcag gggggaccgtc agtcttcctc      720

ttccccccaa aacccaagga caccctcatg atctcccgga cccctgaggt cacatgcgtg      780

gtggtggacg tgagccacga agaccctgag gtcaagttca actggtacgt ggacggcgtg      840

gaggtgcata atgccaagac aaagccgcgg gaggagcagt acaacagcac gtaccgtgtg      900

gtcagcgtcc tcaccgtcct gcaccaggac tggctgaatg gcaaggagta caagtgcaag      960

gtctccaaca aagccctcgg cgcccccatc gagaaaacca tctccaaagc caaagggcag     1020

ccccgagaac cacaggtgtg caccctgccc ccatcccggg atgagctgac caagaaccag     1080

gtcagcctct cgtgcgcagt caaaggcttc tatcccagcg acatcgccgt ggagtgggag     1140

agcaatgggc agccggagaa caactacaag accacgcctc ccgtgctgga ctccgacggc     1200

tccttcttcc tcgtgagcaa gctcaccgtg gacaagagca ggtggcagca ggggaacgtc     1260

ttctcatgct ccgtgatgca tgaggctctg cacaaccact acacgcagaa gagcctctcc     1320

ctgtctccgg gtaaa                                                      1335
```

```
<210>  152
<211>  645
<212>  DNA
<213>  Artificial Sequence

<220>
<223>  nucleotide sequence of DP47 light chain

<400>  152
gaaatcgtgt taacgcagtc tccaggcacc ctgtctttgt ctccagggga aagagccacc       60

ctctcttgca gggccagtca gagtgttagc agcagctact tagcctggta ccagcagaaa      120

cctggccagg ctcccaggct cctcatctat ggagcatcca gcagggccac tggcatccca      180

gacaggttca gtggcagtgg atccgggaca gacttcactc tcaccatcag cagactggag      240

cctgaagatt ttgcagtgta ttactgtcag cagtatggta gctcaccgct gacgttcggc      300

caggggacca aagtggaaat caaacgtacg gtggctgcac catctgtctt catcttcccg      360

ccatctgatg agcagttgaa atctggaact gcctctgttg tgtgcctgct gaataacttc      420

tatcccagag aggccaaagt acagtggaag gtggataacg ccctccaatc gggtaactcc      480
```

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgt 645

<210> 153
<211> 445
<212> PRT
<213> Artificial Sequence

<220>
<223> DP47 Fc hole chain

<400> 153

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190
```

```
Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Gly Ala Pro Ile Glu Lys Thr Ile Ser Lys
                325                 330                 335

Ala Lys Gly Gln Pro Arg Glu Pro Gln Val Cys Thr Leu Pro Pro Ser
            340                 345                 350

Arg Asp Glu Leu Thr Lys Asn Gln Val Ser Leu Ser Cys Ala Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Val Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
```

435 440 445

<210> 154
<211> 215
<212> PRT
<213> Artificial Sequence

<220>
<223> DP47 light chain

<400> 154

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Leu Thr Phe Gly Gln Gly Thr Lys Val Glu Ile Lys Arg Thr Val Ala
100 105 110

Ala Pro Ser Val Phe Ile Phe Pro Pro Ser Asp Glu Gln Leu Lys Ser
115 120 125

Gly Thr Ala Ser Val Val Cys Leu Leu Asn Asn Phe Tyr Pro Arg Glu
130 135 140

Ala Lys Val Gln Trp Lys Val Asp Asn Ala Leu Gln Ser Gly Asn Ser
145 150 155 160

Gln Glu Ser Val Thr Glu Gln Asp Ser Lys Asp Ser Thr Tyr Ser Leu
165 170 175

Ser Ser Thr Leu Thr Leu Ser Lys Ala Asp Tyr Glu Lys His Lys Val
180 185 190

Tyr Ala Cys Glu Val Thr His Gln Gly Leu Ser Ser Pro Val Thr Lys
195 200 205

```
Ser Phe Asn Arg Gly Glu Cys
    210                 215


<210>  155
<211>  2496
<212>  DNA
<213>  Artificial Sequence

<220>
<223>  nucleotide sequence of DP47 Fc hole chain fused to dimeric hu
       4-1BBL (71-254)

<400>  155
gaggtgcaat tgttggagtc tgggggaggc ttggtacagc ctggggggtc cctgagactc      60

tcctgtgcag cctccggatt cacctttagc agttatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcagatga acagcctgag agccgaggac acggccgtat attactgtgc gaaaggcagc     300

ggatttgact actggggcca aggaaccctg gtcaccgtct cgagtgctag caccaagggc     360

ccctccgtgt tccccctggc ccccagcagc aagagcacca gcggcggcac agccgctctg     420

ggctgcctgg tcaaggacta cttccccgag cccgtgaccg tgtcctggaa cagcggagcc     480

ctgacctccg gcgtgcacac cttccccgcc gtgctgcaga gttctggcct gtatagcctg     540

agcagcgtgg tcaccgtgcc ttctagcagc ctgggcaccc agacctacat ctgcaacgtg     600

aaccacaagc ccagcaacac caaggtggac aagaaggtgg agcccaagag ctgcgacaaa     660

actcacacat gcccaccgtg cccagcacct gaagctgcag gggggaccgtc agtcttcctc     720

ttccccccaa aacccaagga caccctcatg atctcccgga cccctgaggt cacatgcgtg     780

gtggtggacg tgagccacga agaccctgag gtcaagttca actggtacgt ggacggcgtg     840

gaggtgcata atgccaagac aaagccgcgg gaggagcagt acaacagcac gtaccgtgtg     900

gtcagcgtcc tcaccgtcct gcaccaggac tggctgaatg gcaaggagta caagtgcaag     960

gtctccaaca aagccctcgg cgcccccatc gagaaaacca tctccaaagc caaagggcag    1020

ccccgagaac cacaggtgtg caccctgccc ccatcccggg atgagctgac caagaaccag    1080

gtcagcctct cgtgcgcagt caaaggcttc tatcccagcg acatcgccgt ggagtgggag    1140

agcaatgggc agccggagaa caactacaag accacgcctc ccgtgctgga ctccgacggc    1200

tccttcttcc tcgtgagcaa gctcaccgtg gacaagagca ggtggcagca ggggaacgtc    1260

ttctcatgct ccgtgatgca tgaggctctg cacaaccact acacgcagaa gagcctctcc    1320

ctgtctccgg gtggaggcgg cggaagcgga ggaggaggat ccagagaggg ccctgagctg    1380

agccccgatg atcctgctgg actgctggac ctgcggcagg gcatgtttgc tcagctggtg    1440
```

gcccagaacg tgctgctgat cgatggcccc ctgtcctggt acagcgatcc tggactggct 1500

ggcgtgtcac tgacaggcgg cctgagctac aaagaggaca ccaaagaact ggtggtggcc 1560

aaggccggcg tgtactacgt gttctttcag ctggaactgc ggagagtggt ggccggcgaa 1620

ggatctggct ctgtgtctct ggccctgcat ctgcagcctc tgagaagcgc tgctggcgct 1680

gcagctctgg cactgacagt ggatctgcct cctgccagct ccgaggcccg gaatagcgca 1740

tttgggtttc aaggcaggct gctgcacctg tctgccggcc agaggctggg agtgcatctg 1800

cacacagagg ccagggctag acacgcctgg cagctgacac agggcgctac agtgctgggc 1860

ctgttcagag tgacccccga gattccagcc ggcctgcctt ctccaagaag cgaaggcgga 1920

ggcggatctg gcggcggagg atctagagag ggacccgaac tgtcccctga cgatccagcc 1980

gggctgctgg atctgagaca gggaatgttc gcccagctgg tggctcagaa tgtgctgctg 2040

attgacggac ctctgagctg gtactccgac ccagggctgg caggggtgtc cctgactggg 2100

ggactgtcct acaaagaaga tacaaaagaa ctggtggtgg ctaaagctgg ggtgtactat 2160

gtgtttttc agctggaact gaggcgggtg gtggctgggg agggctcagg atctgtgtcc 2220

ctggctctgc atctgcagcc actgcgctct gctgctggcg cagctgcact ggctctgact 2280

gtggacctgc caccagcctc tagcgaggcc agaaacagcg ccttcgggtt ccaaggacgc 2340

ctgctgcatc tgagcgccgg acagcgcctg ggagtgcatc tgcatactga agccagagcc 2400

cggcatgctt ggcagctgac tcagggggca actgtgctgg gactgtttcg cgtgacacct 2460

gagatccctg ccggactgcc aagccctaga tcagaa 2496

<210> 156
<211> 1914
<212> DNA
<213> Artificial Sequence

<220>
<223> nucleotide sequence of DP47 Fc knob chain fused to monomeric hu 4-1BBL (71-254)

<400> 156
gaggtgcaat tgttggagtc tgggggaggc ttggtacagc ctggggggtc cctgagactc 60

tcctgtgcag cctccggatt cacctttagc agttatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcagatga acagcctgag agccgaggac acggccgtat attactgtgc gaaaggcagc 300

ggatttgact actggggcca aggaaccctg gtcaccgtct cgagtgctag caccaagggc 360

ccatcggtct tccccctggc accctcctcc aagagcacct ctgggggcac agcggccctg 420

ggctgcctgg tcaaggacta cttccccgaa ccggtgacgg tgtcgtggaa ctcaggcgcc 480

ctgaccagcg gcgtgcacac cttcccggct gtcctacagt cctcaggact ctactccctc 540

agcagcgtgg tgaccgtgcc ctccagcagc ttgggcaccc agacctacat ctgcaacgtg 600

aatcacaagc ccagcaacac caaggtggac aagaaagttg agcccaaatc ttgtgacaaa 660

actcacacat gcccaccgtg cccagcacct gaagctgcag ggggaccgtc agtcttcctc 720

ttccccccaa aacccaagga caccctcatg atctcccgga cccctgaggt cacatgcgtg 780

gtggtggacg tgagccacga agaccctgag gtcaagttca actggtacgt ggacggcgtg 840

gaggtgcata atgccaagac aaagccgcgg gaggagcagt acaacagcac gtaccgtgtg 900

gtcagcgtcc tcaccgtcct gcaccaggac tggctgaatg gcaaggagta caagtgcaag 960

gtctccaaca aagccctcgg cgcccccatc gagaaaacca tctccaaagc caaagggcag 1020

ccccgagaac cacaggtgta caccctgccc ccctgcagag atgagctgac caagaaccag 1080

gtgtccctgt ggtgtctggt caagggcttc taccccagcg atatcgccgt ggagtgggag 1140

agcaacggcc agcctgagaa caactacaag accacccccc ctgtgctgga cagcgacggc 1200

agcttcttcc tgtactccaa actgaccgtg gacaagagcc ggtggcagca gggcaacgtg 1260

ttcagctgca gcgtgatgca cgaggccctg cacaaccact acacccagaa gtccctgagc 1320

ctgagccccg gcggaggcgg cggaagcgga ggaggaggat ccagagaggg ccctgagctg 1380

agccccgatg atcctgctgg actgctggac ctgcggcagg gcatgtttgc tcagctggtg 1440

gcccagaacg tgctgctgat cgatggcccc ctgtcctggt acagcgatcc tggactggct 1500

ggcgtgtcac tgacaggcgg cctgagctac aaagaggaca ccaaagaact ggtggtggcc 1560

aaggccggcg tgtactacgt gttctttcag ctggaactgc ggagagtggt ggccggcgaa 1620

ggatctggct ctgtgtctct ggccctgcat ctgcagcctc tgagaagcgc tgctggcgct 1680

gcagctctgg cactgacagt ggatctgcct cctgccagct ccgaggcccg gaatagcgca 1740

tttgggtttc aaggcaggct gctgcacctg tctgccggcc agaggctggg agtgcatctg 1800

cacacagagg ccagggctag acacgcctgg cagctgacac agggcgctac agtgctgggc 1860

ctgttcagag tgacccccga gattccagcc ggcctgcctt ctccaagaag cgaa 1914

<210> 157
<211> 832
<212> PRT
<213> Artificial Sequence

<220>
<223> DP47 Fc hole chain fused to dimeric hu 4-1BBL (71-254)

<400> 157

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Cys Thr Leu Pro Pro Ser
340 345 350

Arg Asp Glu Leu Thr Lys Asn Gln Val Ser Leu Ser Cys Ala Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Val Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Gly Gly Gly Gly
435 440 445

Ser Gly Gly Gly Gly Ser Arg Glu Gly Pro Glu Leu Ser Pro Asp Asp
450 455 460

Pro Ala Gly Leu Leu Asp Leu Arg Gln Gly Met Phe Ala Gln Leu Val
465 470 475 480

Ala Gln Asn Val Leu Leu Ile Asp Gly Pro Leu Ser Trp Tyr Ser Asp
485 490 495

Pro Gly Leu Ala Gly Val Ser Leu Thr Gly Gly Leu Ser Tyr Lys Glu
500 505 510

Asp Thr Lys Glu Leu Val Val Ala Lys Ala Gly Val Tyr Tyr Val Phe

515 520 525

Phe Gln Leu Glu Leu Arg Arg Val Val Ala Gly Glu Gly Ser Gly Ser
530 535 540

Val Ser Leu Ala Leu His Leu Gln Pro Leu Arg Ser Ala Ala Gly Ala
545 550 555 560

Ala Ala Leu Ala Leu Thr Val Asp Leu Pro Pro Ala Ser Ser Glu Ala
565 570 575

Arg Asn Ser Ala Phe Gly Phe Gln Gly Arg Leu Leu His Leu Ser Ala
580 585 590

Gly Gln Arg Leu Gly Val His Leu His Thr Glu Ala Arg Ala Arg His
595 600 605

Ala Trp Gln Leu Thr Gln Gly Ala Thr Val Leu Gly Leu Phe Arg Val
610 615 620

Thr Pro Glu Ile Pro Ala Gly Leu Pro Ser Pro Arg Ser Glu Gly Gly
625 630 635 640

Gly Gly Ser Gly Gly Gly Gly Ser Arg Glu Gly Pro Glu Leu Ser Pro
645 650 655

Asp Asp Pro Ala Gly Leu Leu Asp Leu Arg Gln Gly Met Phe Ala Gln
660 665 670

Leu Val Ala Gln Asn Val Leu Leu Ile Asp Gly Pro Leu Ser Trp Tyr
675 680 685

Ser Asp Pro Gly Leu Ala Gly Val Ser Leu Thr Gly Gly Leu Ser Tyr
690 695 700

Lys Glu Asp Thr Lys Glu Leu Val Val Ala Lys Ala Gly Val Tyr Tyr
705 710 715 720

Val Phe Phe Gln Leu Glu Leu Arg Arg Val Val Ala Gly Glu Gly Ser
725 730 735

Gly Ser Val Ser Leu Ala Leu His Leu Gln Pro Leu Arg Ser Ala Ala
740 745 750

Gly Ala Ala Ala Leu Ala Leu Thr Val Asp Leu Pro Pro Ala Ser Ser
755 760 765

Glu Ala Arg Asn Ser Ala Phe Gly Phe Gln Gly Arg Leu Leu His Leu
770 775 780

Ser Ala Gly Gln Arg Leu Gly Val His Leu His Thr Glu Ala Arg Ala
785 790 795 800

Arg His Ala Trp Gln Leu Thr Gln Gly Ala Thr Val Leu Gly Leu Phe
805 810 815

Arg Val Thr Pro Glu Ile Pro Ala Gly Leu Pro Ser Pro Arg Ser Glu
820 825 830

<210> 158
<211> 638
<212> PRT
<213> Artificial Sequence

<220>
<223> DP47 Fc knob chain fused to monomeric hu 4-1BBL (71-254)

<400> 158

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala

145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Ala Ala Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Tyr Thr Leu Pro Pro Cys
340 345 350

Arg Asp Glu Leu Thr Lys Asn Gln Val Ser Leu Trp Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

```
Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Gly Gly Gly Gly
        435                 440                 445

Ser Gly Gly Gly Gly Ser Arg Glu Gly Pro Glu Leu Ser Pro Asp Asp
    450                 455                 460

Pro Ala Gly Leu Leu Asp Leu Arg Gln Gly Met Phe Ala Gln Leu Val
465                 470                 475                 480

Ala Gln Asn Val Leu Leu Ile Asp Gly Pro Leu Ser Trp Tyr Ser Asp
                485                 490                 495

Pro Gly Leu Ala Gly Val Ser Leu Thr Gly Gly Leu Ser Tyr Lys Glu
            500                 505                 510

Asp Thr Lys Glu Leu Val Val Ala Lys Ala Gly Val Tyr Tyr Val Phe
        515                 520                 525

Phe Gln Leu Glu Leu Arg Arg Val Val Ala Gly Glu Gly Ser Gly Ser
    530                 535                 540

Val Ser Leu Ala Leu His Leu Gln Pro Leu Arg Ser Ala Ala Gly Ala
545                 550                 555                 560

Ala Ala Leu Ala Leu Thr Val Asp Leu Pro Pro Ala Ser Ser Glu Ala
                565                 570                 575

Arg Asn Ser Ala Phe Gly Phe Gln Gly Arg Leu Leu His Leu Ser Ala
            580                 585                 590

Gly Gln Arg Leu Gly Val His Leu His Thr Glu Ala Arg Ala Arg His
        595                 600                 605

Ala Trp Gln Leu Thr Gln Gly Ala Thr Val Leu Gly Leu Phe Arg Val
    610                 615                 620

Thr Pro Glu Ile Pro Ala Gly Leu Pro Ser Pro Arg Ser Glu
625                 630                 635
```

<210>  159
<211>  1335
<212>  DNA

<213> Artificial Sequence

<220>
<223> nucleotide sequence of DP47 heavy chain (hu IgG1 PGLALA)

<400> 159

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagtgctag caccaagggc     360
ccatcggtct tccccctggc accctcctcc aagagcacct ctgggggcac agcggccctg     420
ggctgcctgg tcaaggacta cttccccgaa ccggtgacgg tgtcgtggaa ctcaggcgcc     480
ctgaccagcg gcgtgcacac cttcccggct gtcctacagt cctcaggact ctactccctc     540
agcagcgtgg tgaccgtgcc ctccagcagc ttgggcaccc agacctacat ctgcaacgtg     600
aatcacaagc ccagcaacac caaggtggac aagaaagttg agcccaaatc ttgtgacaaa     660
actcacacat gcccaccgtg cccagcacct gaagctgcag gggaccgtc agtcttcctc     720
ttccccccaa aacccaagga caccctcatg atctcccgga cccctgaggt cacatgcgtg     780
gtggtggacg tgagccacga agaccctgag gtcaagttca actggtacgt ggacggcgtg     840
gaggtgcata atgccaagac aaagccgcgg gaggagcagt acaacagcac gtaccgtgtg     900
gtcagcgtcc tcaccgtcct gcaccaggac tggctgaatg gcaaggagta caagtgcaag     960
gtctccaaca aagccctcgg cgcccccatc gagaaaacca tctccaaagc caaagggcag    1020
ccccgagaac cacaggtgta caccctgccc ccatcccggg atgagctgac caagaaccag    1080
gtcagcctga cctgcctggt caaaggcttc tatcccagcg acatcgccgt ggagtgggag    1140
agcaatgggc agccggagaa caactacaag accacgcctc ccgtgctgga ctccgacggc    1200
tccttcttcc tctacagcaa gctcaccgtg gacaagagca ggtggcagca ggggaacgtc    1260
ttctcatgct ccgtgatgca tgaggctctg cacaaccact acacgcagaa gagcctctcc    1320
ctgtctccgg gtaaa                                                    1335
```

<210> 160
<211> 445
<212> PRT
<213> Artificial Sequence

<220>
<223> DP47 heavy chain (hu IgG1 PGLALA)

<400> 160

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 161
<211> 1350
<212> DNA
<213> Artificial Sequence

<220>
<223> nucleotide sequence of PD1(0314) heavy chain (huIgG1 PGLALA)

<400> 161
gaagtgcagc tgctcgaaag cggcggggga ctggtccagc ccggcggttc cctgaggctg 60

tcttgcgccg cttcagggtt cagcttttcc tcttacacca tgagttgggt cagacaggca 120

cctggcaagg gactggagtg ggtcgccaca atcagcggtg gcgggcgcga catttattac 180

ccagattccg tgaaaggacg gttcaccatc tctagggaca actcaaagaa tactctgtat 240

ttgcagatga acagcctgag agctgaggat acagcagttt actactgtgt gctcctgacc 300

ggccgcgtct attttgccct tgactcctgg ggacaaggca ctctggtgac cgtatctagt 360

gctagcacca agggcccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaagc tgcaggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcggcgccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 162
<211> 450
<212> PRT
<213> Artificial Sequence

<220>
<223> PD1(0314) heavy chain (huIgG1 PGLALA)

<400> 162

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

```
Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Leu Leu Thr Gly Arg Val Tyr Phe Ala Leu Asp Ser Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300
```

Val Val Ser Val Leu Thr Val Leu His Gln Asp Trp Leu Asn Gly Lys
305 310 315 320

Glu Tyr Lys Cys Lys Val Ser Asn Lys Ala Leu Gly Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 163
<211> 163
<212> PRT
<213> Homo sapiens

<400> 163

Leu Gln Asp Pro Cys Ser Asn Cys Pro Ala Gly Thr Phe Cys Asp Asn
1 5 10 15

Asn Arg Asn Gln Ile Cys Ser Pro Cys Pro Pro Asn Ser Phe Ser Ser
20 25 30

Ala Gly Gly Gln Arg Thr Cys Asp Ile Cys Arg Gln Cys Lys Gly Val
35 40 45

Phe Arg Thr Arg Lys Glu Cys Ser Ser Thr Ser Asn Ala Glu Cys Asp
50 55 60

Cys Thr Pro Gly Phe His Cys Leu Gly Ala Gly Cys Ser Met Cys Glu
65 70 75 80

Gln Asp Cys Lys Gln Gly Gln Glu Leu Thr Lys Lys Gly Cys Lys Asp
85 90 95

Cys Cys Phe Gly Thr Phe Asn Asp Gln Lys Arg Gly Ile Cys Arg Pro
100 105 110

Trp Thr Asn Cys Ser Leu Asp Gly Lys Ser Val Leu Val Asn Gly Thr
115 120 125

Lys Glu Arg Asp Val Val Cys Gly Pro Ser Pro Ala Asp Leu Ser Pro
130 135 140

Gly Ala Ser Ser Val Thr Pro Pro Ala Pro Ala Arg Glu Pro Gly His
145 150 155 160

Ser Pro Gln

<210> 164
<211> 681
<212> DNA
<213> Artificial Sequence

<220>
<223> Nucleotide sequence of Fc hole chain

<400> 164
gacaaaactc acacatgccc accgtgccca gcacctgaac tcctgggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcccagcc cccatcgaga aaaccatctc caaagccaaa 360

gggcagcccc gagaaccaca ggtgtgcacc ctgcccccat cccgggatga gctgaccaag 420

aaccaggtca gcctctcgtg cgcagtcaaa ggcttctatc ccagcgacat cgccgtggag 480

tgggagagca atgggcagcc ggagaacaac tacaagacca cgcctcccgt gctggactcc 540

gacggctcct tcttcctcgt gagcaagctc accgtggaca agagcaggtg gcagcagggg 600

aacgtcttct catgctccgt gatgcatgag gctctgcaca accactacac gcagaagagc 660

ctctccctgt ctccgggtaa a 681

<210>  165
<211>  1266
<212>  DNA
<213>  Artificial Sequence

<220>
<223>  Nucleotide sequence of human 4-1BB antigen Fc knob chain

<400>  165
ctgcaggacc cctgcagcaa ctgccctgcc ggcaccttct gcgacaacaa ccggaaccag      60

atctgcagcc cctgcccccc caacagcttc agctctgccg gcggacagcg gacctgcgac      120

atctgcagac agtgcaaggg cgtgttcaga acccggaaag agtgcagcag caccagcaac      180

gccgagtgcg actgcacccc cggcttccat tgtctgggag ccggctgcag catgtgcgag      240

caggactgca agcagggcca ggaactgacc aagaagggct gcaaggactg ctgcttcggc      300

accttcaacg accagaagcg gggcatctgc cggccctgga ccaactgtag cctggacggc      360

aagagcgtgc tggtcaacgg caccaaagaa cgggacgtcg tgtgcggccc cagccctgct      420

gatctgtctc ctggggccag cagcgtgacc cctcctgccc ctgccagaga gcctggccac      480

tctcctcagg tcgacgaaca gttatatttt cagggcggct cacccaaatc tgcagacaaa      540

actcacacat gcccaccgtg cccagcacct gaactcctgg ggggaccgtc agtcttcctc      600

ttccccccaa aacccaagga caccctcatg atctcccgga cccctgaggt cacatgcgtg      660

gtggtggacg tgagccacga agaccctgag gtcaagttca actggtacgt ggacggcgtg      720

gaggtgcata atgccaagac aaagccgcgg gaggagcagt acaacagcac gtaccgtgtg      780

gtcagcgtcc tcaccgtcct gcaccaggac tggctgaatg gcaaggagta caagtgcaag      840

gtctccaaca aagccctccc agcccccatc gagaaaacca tctccaaagc caaagggcag      900

ccccgagaac cacaggtgta caccctgccc ccatgccggg atgagctgac caagaaccag      960

gtcagcctgt ggtgcctggt caaaggcttc tatcccagcg acatcgccgt ggagtgggag      1020

agcaatgggc agccggagaa caactacaag accacgcctc ccgtgctgga ctccgacggc      1080

tccttcttcc tctacagcaa gctcaccgtg gacaagagca ggtggcagca ggggaacgtc      1140

ttctcatgct ccgtgatgca tgaggctctg cacaaccact acacgcagaa gagcctctcc      1200

ctgtctccgg gtaaatccgg aggcctgaac gacatcttcg aggcccagaa gattgaatgg      1260

cacgag                                                                  1266


<210>  166
<211>  227
<212>  PRT
<213>  Artificial Sequence

<220>
<223>  Fc hole chain

<400> 166

Asp Lys Thr His Thr Cys Pro Pro Cys Pro Ala Pro Glu Leu Leu Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Pro Ala Pro Ile
100 105 110

Glu Lys Thr Ile Ser Lys Ala Lys Gly Gln Pro Arg Glu Pro Gln Val
115 120 125

Cys Thr Leu Pro Pro Ser Arg Asp Glu Leu Thr Lys Asn Gln Val Ser
130 135 140

Leu Ser Cys Ala Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Val Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Lys
225

<210> 167

<211> 422
<212> PRT
<213> Artificial Sequence

<220>
<223> human 4-1BB antigen Fc knob chain

<400> 167

Leu Gln Asp Pro Cys Ser Asn Cys Pro Ala Gly Thr Phe Cys Asp Asn
1               5                   10                  15

Asn Arg Asn Gln Ile Cys Ser Pro Cys Pro Pro Asn Ser Phe Ser Ser
            20                  25                  30

Ala Gly Gly Gln Arg Thr Cys Asp Ile Cys Arg Gln Cys Lys Gly Val
        35                  40                  45

Phe Arg Thr Arg Lys Glu Cys Ser Ser Thr Ser Asn Ala Glu Cys Asp
    50                  55                  60

Cys Thr Pro Gly Phe His Cys Leu Gly Ala Gly Cys Ser Met Cys Glu
65                  70                  75                  80

Gln Asp Cys Lys Gln Gly Gln Glu Leu Thr Lys Lys Gly Cys Lys Asp
                85                  90                  95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Val Thr Pro Pro Ala Pro Ala Arg Glu Pro Gly His
145                 150                 155                 160

Ser Pro Gln Val Asp Glu Gln Leu Tyr Phe Gln Gly Gly Ser Pro Lys
                165                 170                 175

Ser Ala Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

```
Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Cys Arg Asp Glu Leu Thr Lys Asn Gln
305                 310                 315                 320

Val Ser Leu Trp Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys Ser Gly Gly Leu Asn Asp Ile Phe Glu Ala Gln
                405                 410                 415

Lys Ile Glu Trp His Glu
            420


<210>  168
<211>  15
<212>  PRT
<213>  Artificial Sequence

<220>
<223>  avi tag

<400>  168
```

```
Gly Leu Asn Asp Ile Phe Glu Ala Gln Lys Ile Glu Trp His Glu
1               5                   10                  15


<210>  169
<211>  594
<212>  DNA
<213>  Artificial Sequence

<220>
<223>  nucleotide sequence of human 4-1BB His

<400>  169
ctgcaggacc cctgcagcaa ctgccctgcc ggcaccttct gcgacaacaa ccggaaccag        60

atctgcagcc cctgcccccc caacagcttc agctctgccg gcggacagcg gacctgcgac       120

atctgcagac agtgcaaggg cgtgttcaga acccggaaag agtgcagcag caccagcaac       180

gccgagtgcg actgcacccc cggcttccat tgtctgggag ccggctgcag catgtgcgag       240

caggactgca agcagggcca ggaactgacc aagaagggct gcaaggactg ctgcttcggc       300

accttcaacg accagaagcg gggcatctgc cggccctgga ccaactgtag cctggacggc       360

aagagcgtgc tggtcaacgg caccaaagaa cgggacgtcg tgtgcggccc cagccctgct       420

gatctgtctc ctggggccag cagcgtgacc cctcctgccc ctgccagaga gcctggccac       480

tctcctcagg tcgacgaaca gttatatttt cagggcggct caggcctgaa cgacatcttc       540

gaggcccaga agatcgagtg gcacgaggct cgagctcacc accatcacca tcac             594


<210>  170
<211>  198
<212>  PRT
<213>  Artificial Sequence

<220>
<223>  human 4-1BB His

<400>  170

Leu Gln Asp Pro Cys Ser Asn Cys Pro Ala Gly Thr Phe Cys Asp Asn
1               5                   10                  15


Asn Arg Asn Gln Ile Cys Ser Pro Cys Pro Pro Asn Ser Phe Ser Ser
            20                  25                  30


Ala Gly Gly Gln Arg Thr Cys Asp Ile Cys Arg Gln Cys Lys Gly Val
        35                  40                  45


Phe Arg Thr Arg Lys Glu Cys Ser Ser Thr Ser Asn Ala Glu Cys Asp
    50                  55                  60


Cys Thr Pro Gly Phe His Cys Leu Gly Ala Gly Cys Ser Met Cys Glu
65                  70                  75                  80
```

```
Gln Asp Cys Lys Gln Gly Gln Glu Leu Thr Lys Lys Gly Cys Lys Asp
                85                  90                  95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Val Thr Pro Pro Ala Pro Ala Arg Glu Pro Gly His
145                 150                 155                 160

Ser Pro Gln Val Asp Glu Gln Leu Tyr Phe Gln Gly Gly Ser Gly Leu
                165                 170                 175

Asn Asp Ile Phe Glu Ala Gln Lys Ile Glu Trp His Glu Ala Arg Ala
            180                 185                 190

His His His His His His
        195
```

**Description**

## FIELD OF THE INVENTION

The invention relates to novel TNF family ligand trimer-containing antigen binding molecules comprising (a) at least one moiety capable of specific binding to PD1 and (b) a first and a second polypeptide that are linked to each other by a disulfide bond, wherein the antigen binding molecules are characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof. The invention further relates to methods of producing these molecules and to methods of using the same.

## BACKGROUND

Ligands interacting with molecules of the TNF (tumor necrosis factor) receptor superfamily have pivotal roles in the organization and function of the immune system. While regulating normal functions such as immune responses, hematopoiesis and morphogenesis, the TNF family ligands (also called cytokines) play a role in tumorgenesis, transplant rejection, septic shock, viral replication, bone resorption, rheumatoid arthritis and diabetes (Aggarwal, 2003). The TNF ligand family comprises 18 genes encoding 19 type II (i.e. intracellular N terminus and extracellular C-terminus) transmembrane proteins, characterized by the presence of a conserved C-terminal domain coined the 'TNF homology domain' (THD). This domain is responsible for receptor binding and is thus critical for the biological activity of the TNF ligand family members. The sequence identity between family members is ~20-30% (Bodmer, 2002). Members of the TNF ligand family exert their biological function as self-assembling, noncovalent trimers (Banner et al, Cell 1993, 73, 431-445). Thus, the TNF family ligands form a trimer that is able to bind to and to activate the corresponding receptors of TNFR superfamily.

4-1BB (CD137), a member of the TNF receptor superfamily, has been first identified as a molecule whose expression is induced by T-cell activation (Kwon and Weissman, 1989). Subsequent studies demonstrated expression of 4-1BB in T- and B-lymphocytes (Snell et al., 2011; Zhang et al., 2010), NK-cells (Lin et al., 2008), NKT-cells (Kim et al., 2008), monocytes (Kienzle and von Kempis, 2000; Schwarz et al., 1995), neutrophils (Heinisch et al., 2000), mast (Nishimoto et al., 2005) and dendritic cells as well as cells of non-hematopoietic origin such as endothelial and smooth muscle cells (Broll et al., 2001; Olofsson et al., 2008). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., 2002; von Kempis et al., 1997; Zhang et al., 2010).

Expression of 4-1BB ligand (4-1BBL or CD137L) is more restricted and is observed on professional antigen presenting cells (APC) such as B-cells, dendritic cells (DCs) and macrophages. Inducible expression of 4-1BBL is characteristic for T-cells, including both $\alpha\beta$ and $\gamma\delta$ T-cell subsets, and endothelial cells (reviewed in Shao and Schwarz, 2011).

CD 137 signaling is known to stimulate IFN$\gamma$ secretion and proliferation of NK cells (Buechele et al., 2012; Lin et al., 2008; Melero et al., 1998) as well as to promote DC activation as indicated by their increased survival and capacity to secret cytokines and upregulate co-stimulatory molecules (Choi et al., 2009; Futagawa et al., 2002; Wilcox et al., 2002). However, CD 137 is best characterized as a co-stimulatory molecule which modulates TCR-induced activation in both the CD4+ and CD8+ subsets of T-cells. In combination with TCR triggering, agonistic 4-1BB-specific antibodies enhance proliferation of T-cells, stimulate lymphokine secretion and decrease sensitivity of T-lymphocytes to activation-induced cells death (reviewed in (reviewed in Snell et al., 2011).

In line with these co-stimulatory effects of 4-1BB antibodies on T-cells in vitro, their administration to tumor bearing mice leads to potent anti-tumor effects in many experimental tumor models (Melero et al., 1997; Narazaki et al., 2010). However, 4-1BB usually exhibits its potency as an anti-tumor agent only when administered in combination with other immunomodulatory compounds (Curran et al., 2011; Guo et al., 2013; Morales-Kastresana et al., 2013; Teng et al., 2009; Wei et al., 2013), chemotherapeutic reagents (Ju et al., 2008; Kim et al., 2009), tumor-specific vaccination (Cuadros et al., 2005; Lee et al., 2011) or radiotherapy (Shi and Siemann, 2006). In vivo depletion experiments demonstrated that CD8+ T-cells play the most critical role in anti-tumoral effect of 4-1BB-specific antibodies. However, depending on the tumor model or combination therapy, which includes anti-4-1BB, contributions of other types of cells such as DCs, NK-cells or CD4+ T-cells have been reported (Melero et al., 1997; Murillo et al., 2009; Narazaki et al., 2010; Stagg et al., 2011).

In addition to their direct effects on different lymphocyte subsets, 4-1BB agonists can also induce infiltration and retention of activated T-cells in the tumor through 4-1BB-mediated upregulation of intercellular adhesion molecule 1 (ICAM1) and vascular cell adhesion molecule 1 (VCAM1) on tumor vascular endothelium (Palazon et al., 2011).

4-1BB triggering may also reverse the state of T-cell anergy induced by exposure to soluble antigen that may contribute to disruption of immunological tolerance in the tumor microenvironment or during chronic infections (Wilcox et al., 2004).

It appears that the immunomodulatory properties of 4-1BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li and Ravetch, 2011; Teng et al., 2009). However, systemic administration of 4-1BB-specific agonistic antibodies with the functionally active Fc domain also induces expansion of CD8+ T-cells associated with liver toxicity (Dubrot et al., 2010) that is diminished or significantly ameliorated in the absence of functional Fc-receptors in mice. In human clinical trials (ClinicalTrials.gov, NCT00309023), Fc-competent 4-1BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone and Ascierto, 2012).

Collectively, the available pre-clinical and clinical data clearly demonstrate that there is a high clinical need for effective 4-1BB agonists. However, new generation drug candidates should not only effectively engage 4-1BB on the surface of hematopoietic and endothelial cells but also be capable of achieving that through mechanisms other than binding to Fc-receptors in order to avoid uncontrollable side effects. The latter may be accomplished through preferential binding to and oligomerization on tumor-specific or tumor-associated moieties.

Fusion proteins composed of one extracellular domain of a 4-1BB ligand and a single chain antibody fragment (Mueller et al., 2008; Hornig et al., 2012) or a single 4-1BB ligand fused to the C-terminus of a heavy chain (Zhang et al, 2007) have been made. WO 2010/010051 discloses the generation of fusion proteins that consist of three TNF ligand ectodomains linked to each other and fused to an antibody part.

Programmed cell death protein 1 (PD1 or CD279) is an inhibitory member of the CD28 family of receptors, that also includes CD28, CTLA-4, ICOS and BTLA. PD1 is a cell surface receptor and is expressed on activated B cells, T cells, and myeloid cells (Okazaki et al (2002) Curr. Opin. Immunol. 14: 391779-82; Bennett et al. (2003) J Immunol 170:711-8). The structure of PD1 is a monomeric type 1 transmembrane protein, consisting of one immunoglobulin variable-like extracellular domain and a cytoplasmic domain containing an immunoreceptor tyrosine-based inhibitory motif (ITIM) and an immunoreceptor tyrosine-based switch motif (ITSM). Activated T cells transiently express PD1, but sustained hyperexpression of PD1 and its ligand PDL1 promote immune exhaustion, leading to persistence of viral infections, tumor evasion, increased infections and mortality. PD1 expression is induced by antigen recognition via the T-cell receptor and its expression is maintained primarily through continuous T-cell receptor signaling. After prolonged antigen exposure, the PD1 locus fails to be remethylated, which promotes continuous hyperexpression. Blocking the PD1 pathway can restore the exhausted T-cell functionality in cancer and chronic viral infections (Sheridan, Nature Biotechnology 30 (2012), 729-730). Monoclonal antibodies to PD1 have been described, for example, in WO 2003/042402, WO 2004/004771, WO 2004/056875, WO 2004/072286, WO 2004/087196, WO 2006/121168, WO 2006/133396, WO 2007/005874, WO 2008/083174, WO 2008/156712, WO 2009/024531, WO 2009/014708, WO 2009/101611, WO 2009/114335, WO 2009/154335, WO 2010/027828, WO 2010/027423, WO 2010/029434, WO 2010/029435, WO 2010/036959, WO 2010/063011, WO 2010/089411, WO 2011/066342, WO 2011/110604, WO 2011/110621, WO 2012/145493, WO 2013/014668, WO 2014/179664, and WO 2015/112900. Two PD1 monoclonal antibodies, Opdivo (nivolumab) and Keytruda (pembrolizumab), are already on the market for the treatment of certain cancers. However, despite of the compelling anti-tumor activity, relaps of tumor growth after therapeutic PD1 blockade has increasingly been observed (Koyama et al., Nature Communications 2016, DOI: 10.1038/ncomms10501).

It has been shown that dual targeting of CD137/PD1 in the tumor microenvironment abolishes the local suppression and leads to functional rescue and expansion of effector T cells that subsequently kill cancer cells (Wei et al., 2014). Administering the combination of an anti-PD1 antibody and a 4-1BB agonistic antibody in a tumor mouse model led to an effective long-lasting systemic antitumor response, however the known toxicity of the 4-1BB agonist as given alone was slightly increased (Chen et al., 2015).

Thus, there is a need of new antigen binding molecules that combine a PD 1 moiety with a moiety that is capable of forming a costimulatory TNF ligand trimer and that is sufficiently stable to be pharmaceutically useful. The antigen binding molecules of the present invention comprise both and surprisingly they provide a trimeric and thus biologically active TNF ligand, although one of the trimerizing TNF ligand ectodomains is located on another polypeptide than the other two TNF ligand ectodomains of the molecule. Targeted by the anti-PD1 moiety the antigen binding molecules of the present invention have an increased activity in the tumor microenvironment and should thus impose less safety issues compared to conventional 4-1BB agonistic antibodies alone.

## SUMMARY OF THE INVENTION

In one aspect, the invention provides a TNF family ligand trimer-containing antigen binding molecule comprising

(a) at least one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or a fragment thereof that are connected to each other by a peptide linker and in that the second polypeptide comprises only one ectodomain of said TNF ligand family member or a fragment thereof.

In a particular aspect, the invention provides a TNF family ligand trimer-containing antigen binding molecule comprising

(a) at least one moiety capable of specific binding to PD1,
(b) a first and a second polypeptide that are linked to each other by a disulfide bond,
wherein the antigen binding molecule is characterized in that the first polypeptide comprises two ectodomains of a TNF ligand family member or a fragment thereof that are connected to each other by a peptide linker and in that the second polypeptide comprises only one ectodomain of said TNF ligand family member or a fragment thereof, and
(c) an Fc domain composed of a first and a second subunit capable of stable association.

In a further aspect, the invention provides a TNF family ligand trimer-containing antigen binding molecule, comprising

(a) at least one moiety capable of specific binding to PD1 and
(b) a first and a second polypeptide that are linked to each other by a disulfide bond,

wherein the antigen binding molecule is characterized in that

(i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or two fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or
(ii) the first polypeptide contains a CH3 domain and the second polypeptide contains a CH3 domain, respectively, and wherein the first polypeptide comprises two ectodomains of a TNF ligand family member or two fragments thereof that are connected to each other and to the C-terminus of the CH3 domain by a peptide linker and wherein the second polypeptide comprises only one ectodomain of said TNF ligand family member or a fragment thereof connected via a peptide linker to C-terminus of the CH3 domain of said polypeptide, or
(iii) the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to to VH or VL by a peptide linker and wherein the second polypeptide comprises one ectodomain of said TNF ligand family member or a fragment thereof connected via a peptide linker to VL or VH of said polypeptide.

In a particular aspect, the invention provides a TNF family ligand trimer-containing antigen binding molecule, comprising

(a) at least one moiety capable of specific binding to PD1 and
(b) a first and a second polypeptide that are linked to each other by a disulfide bond,

wherein the antigen binding molecule is characterized in that

(i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or two fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or
(ii) the first polypeptide contains a CH3 domain and the second polypeptide contains a CH3 domain, respectively, and wherein the first polypeptide comprises two ectodomains of a TNF ligand family member or two fragments thereof that are connected to each other and to the C-terminus of the CH3 domain by a peptide linker and wherein the second polypeptide comprises only one ectodomain of said TNF ligand family member or a fragment thereof connected via a peptide linker to C-terminus of the CH3 domain of said polypeptide.

In one aspect, the TNF ligand family member is one that costimulates human T-cell activation. Thus, the TNF family ligand trimer-containing antigen binding molecule comprises (a) at least one moiety capable of specific binding to a target cell antigen and (b) a first and a second polypeptide that are linked to each other by a disulfide bond, wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof, wherein the TNF ligand family member costimulates human T-cell activation. More particularly, the TNF ligand family member is selected from 4-1BBL and OX40L.

In a particular aspect, the TNF ligand family member is 4-1BBL.

In a further aspect, the ectodomain of a TNF ligand family member comprises the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO:5. More particularly, the ectodomain of a TNF ligand family member comprises the amino acid sequence of SEQ ID NO:5.

In a further aspect, the TNF family ligand trimer-containing antigen binding molecule of the invention comprises

(a) at least one moiety capable of specific binding to PD1 and
(b) a first and a second polypeptide that are linked to each other by a disulfide bond,

wherein the antigen binding molecule is characterized in that the first polypeptide comprises the amino acid sequence selected from the group consisting of SEQ ID NO:9, SEQ ID NO: 11, SEQ ID NO:12 and SEQ ID NO:10 and in that the second polypeptide comprises the amino acid sequence selected from the group consisting of SEQ ID NO:1, SEQ ID NO:3, SEQ ID NO:4 and SEQ ID NO:5.

In one aspect, the TNF family ligand trimer-containing antigen binding molecule of the invention comprises

(a) at least one moiety capable of specific binding to PD1 and
(b) a first and a second polypeptide that are linked to each other by a disulfide bond,

wherein the antigen binding molecule is characterized in that the first polypeptide comprises the amino acid sequence of SEQ ID NO:10 and in that the second polypeptide comprises the amino acid sequence of SEQ ID NO:5.

In another aspect, the TNF family ligand trimer-containing antigen binding molecule of the invention comprises

(a) at least one moiety capable of specific binding to PD1,
(b) a first polypeptide containing a CH1 or CL domain and a second polypeptide containing a CL or CH1 domain, respectively, wherein the second polypeptide is linked to the first polypeptide by a disulfide bond between the CH1 and CL domain,

and wherein the antigen binding molecule is characterized in that the first polypeptide comprises two ectodomains of a TNF ligand family member or two fragments thereof that are connected to each other and to the CH1 or CL domain by a peptide linker and in that the second polypeptide comprises only one ectodomain of said TNF ligand family member or a fragment thereof connected by a peptide linker to the CL or CH1 domain of said polypeptide.

In one aspect, provided is a TNF family ligand trimer-containing antigen binding molecule comprising

(a) at least one moiety capable of specific binding to PD1,
(b) a first polypeptide containing a CH1 domain and a second polypeptide containing a CL domain, wherein the second polypeptide is linked to the first polypeptide by a disulfide bond between the CH1 and CL domain,

and wherein the antigen binding molecule is characterized in that the first polypeptide comprises two ectodomains of a TNF ligand family member or fragments thereof that are connected to each other and to the CH1 domain by a peptide linker and in that the second polypeptide comprises one ectodomain of said TNF ligand family member or a fragment thereof connected via a peptide linker to the CL domain of said polypeptide.

In another aspect, provided is a TNF family ligand trimer-containing antigen binding molecule comprising

(a) at least one moiety capable of specific binding to PD1,
(b) a first polypeptide containing a CL domain and a second polypeptide containing a CH1 domain, wherein the second polypeptide is linked to the first polypeptide by a disulfide bond between the CH1 and CL domain,

and wherein the antigen binding molecule is characterized in that the first polypeptide comprises two ectodomains of a TNF ligand family member or fragments thereof that are connected to each other and to the CL domain by a peptide linker and in that the second polypeptide comprises one ectodomain of said TNF ligand family member or a fragment thereof connected via a peptide linker to the CH1 domain of said polypeptide.

In a further aspect, the invention provides a TNF family ligand trimer-containing antigen binding molecule as defined herein before, wherein the moiety capable of specific binding PD1 is selected from the group consisting of an antibody, an antibody fragment and a scaffold antigen binding protein. In particular, the moiety capable of specific binding to PD1 is selected from the group consisting of an antibody fragment, a Fab molecule, a crossover Fab molecule, a single chain Fab molecule, a Fv molecule, a scFv molecule, a single domain antibody, an aVH and a scaffold antigen binding protein.

In one aspect, the invention provides a TNF family ligand trimer-containing antigen binding molecule as defined herein before, wherein the moiety capable of specific binding to PD is an antibody fragment. In particular, the moiety capable of specific binding to PD is selected from the group consisting of a Fab molecule, a crossover Fab molecule, a single chain Fab molecule, a Fv molecule, a scFv molecule, a single domain antibody and an aVH..

In one aspect, the invention provides a TNF family ligand trimer-containing antigen binding molecule as defined herein before, wherein the moiety capable of specific binding to PD is a scaffold antigen binding protein.

In a particular aspect, the invention is concerned with a TNF family ligand trimer-containing antigen binding molecule as defined above, wherein the moiety capable of specific binding to PD is a Fab molecule capable of specific binding to a target cell antigen.

The invention provides a TNF family ligand trimer-containing antigen binding molecule that comprises at least one moiety capable of specific binding to PD1. In a particular aspect, the TNF family ligand trimer-containing antigen binding molecule comprises one moiety capable of specific binding to PD1, meaning the TNF family ligand trimer-containing antigen binding molecule is monovalent. In another aspect, the invention provides a TNF family ligand trimer-containing antigen binding molecule comprising two moieties capable of specific binding to PD1, meaning the TNF family ligand trimer-containing antigen binding molecule is bivalent.

In one aspect, the invention provides a TNF family ligand trimer-containing antigen binding molecule, wherein the moiety capable of specific binding to PD1 comprises

(a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16 (v) HVR-L2 comprising the amino acid sequence of SEQ ID NO: 17, and (vi) HVR-L3 comprising the amino acid sequence of SEQ ID NO:18,
(b) a VH domain comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28, and a VL domain comprising (iv) HVR-L1 comprising the amino acid sequence of SEQ ID NO:29 (v) HVR-L2 comprising the amino acid sequence of SEQ ID NO:30, and (vi) HVR-L3 comprising the amino acid sequence of SEQ ID NO:31,
(c) a VH domain comprising (i) HVR-H1 comprising the amino acid sequence of SEQ ID NO:34, (ii) HVR-H2 comprising the amino acid sequence of SEQ ID NO:35, and (iii) HVR-H3 comprising the amino acid sequence of SEQ ID NO:36, and a VL domain comprising (iv) HVR-L1 comprising the amino acid sequence of SEQ ID NO:37 (v) HVR-L2 comprising the amino acid sequence of SEQ ID NO:38, and (vi) HVR-L3 comprising the amino acid sequence of SEQ ID NO:39,
(d) a VH domain comprising (i) HVR-H1 comprising the amino acid sequence of SEQ ID NO:42, (ii) HVR-H2 comprising the amino acid sequence of SEQ ID NO:43, and (iii) HVR-H3 comprising the amino acid sequence of SEQ ID NO:44, and a VL domain comprising (iv) HVR-L1 comprising the amino acid sequence of SEQ ID NO:45 (v) HVR-L2 comprising the amino acid sequence of SEQ ID NO:46, and (vi) HVR-L3 comprising the amino acid sequence of SEQ ID NO:47,
(e) a VH domain comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52, and a VL domain comprising (iv) HVR-L1 comprising the amino acid sequence of SEQ ID NO:53 (v) HVR-L2 comprising the amino acid sequence of SEQ ID NO:54, and (vi) HVR-L3 comprising the amino acid sequence of SEQ ID NO:55,
(f) a VH domain comprising (i) HVR-H1 comprising the amino acid sequence of SEQ ID NO:58, (ii) HVR-H2 comprising the amino acid sequence of SEQ ID NO:59, and (iii) HVR-H3 comprising the amino acid sequence of SEQ ID NO:60, and a VL domain comprising (iv) HVR-L1 comprising the amino acid sequence of SEQ ID NO:61 (v) HVR-L2 comprising the amino acid sequence of SEQ ID NO:62, and (vi) HVR-L3 comprising the amino acid sequence of SEQ ID NO:63, or

(g) a VH domain comprising (i) HVR-H1 comprising the amino acid sequence of SEQ ID NO:66, (ii) HVR-H2 comprising the amino acid sequence of SEQ ID NO:67, and (iii) HVR-H3 comprising the amino acid sequence of SEQ ID NO:68, and a VL domain comprising (iv) HVR-L1 comprising the amino acid sequence of SEQ ID NO:69 (v) HVR-L2 comprising the amino acid sequence of SEQ ID NO:70, and (vi) HVR-L3 comprising the amino acid sequence of SEQ ID NO:71.

In a further aspect, the invention provides a a TNF family ligand trimer-containing antigen binding molecule, wherein the moiety capable of specific binding to PD comprises

(a) a VH domain comprising an amino acid sequence of SEQ ID NO:19 and a VL domain comprising an amino acid sequence of SEQ ID NO:20,

(b) a VH domain comprising an amino acid sequence of SEQ ID NO:21 and a VL domain comprising an amino acid sequence of SEQ ID NO:22,

(c) a VH domain comprising an amino acid sequence of SEQ ID NO:21 and a VL domain comprising an amino acid sequence of SEQ ID NO:23,

(d) a VH domain comprising an amino acid sequence of SEQ ID NO:21 and a VL domain comprising an amino acid sequence of SEQ ID NO:24,

(e) a VH domain comprising an amino acid sequence of SEQ ID NO:21 and a VL domain comprising an amino acid sequence of SEQ ID NO:25,

(f) a VH domain comprising an amino acid sequence of SEQ ID NO:32 and a VL domain comprising an amino acid sequence of SEQ ID NO:33,

(g) a VH domain comprising an amino acid sequence of SEQ ID NO:40 and a VL domain comprising an amino acid sequence of SEQ ID NO:41,

(h) a VH domain comprising an amino acid sequence of SEQ ID NO:48 and a VL domain comprising an amino acid sequence of SEQ ID NO:49,

(i) a VH domain comprising an amino acid sequence of SEQ ID NO:56 and a VL domain comprising an amino acid sequence of SEQ ID NO:57,

(k) a VH domain comprising an amino acid sequence of SEQ ID NO:64 and a VL domain comprising an amino acid sequence of SEQ ID NO:65, or

(l) a VH domain comprising an amino acid sequence of SEQ ID NO:72 and a VL domain comprising an amino acid sequence of SEQ ID NO:73.

In one aspect, the invention provides a TNF family ligand trimer-containing antigen binding molecule, wherein the moiety capable of specific binding to PD comprises (a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO:18.

In a particular aspect, the invention provides a TNF family ligand trimer-containing antigen binding molecule, wherein the moiety capable of specific binding to PD1 comprises

(a) a VH domain comprising an amino acid sequence of SEQ ID NO:19 and a VL domain comprising an amino acid sequence of SEQ ID NO:20,

(b) a VH domain comprising an amino acid sequence of SEQ ID NO:21 and a VL domain comprising an amino acid sequence of SEQ ID NO:22,

(c) a VH domain comprising an amino acid sequence of SEQ ID NO:21 and a VL domain comprising an amino acid sequence of SEQ ID NO:23,

(d) a VH domain comprising an amino acid sequence of SEQ ID NO:21 and a VL domain comprising an amino acid sequence of SEQ ID NO:24, or

(e) a VH domain comprising an amino acid sequence of SEQ ID NO:21 and a VL domain comprising an amino acid sequence of SEQ ID NO:25.

In one aspect, provided is a TNF family ligand trimer-containing antigen binding molecule as defined herein before, wherein the moiety capable of specific binding to PD comprises a variable heavy chain comprising an amino acid sequence of SEQ ID NO:21 and a variable light chain comprising an amino acid sequence of SEQ ID NO:22 or wherein the moiety capable of specific binding to FAP comprises a variable heavy chain comprising an amino acid sequence of SEQ ID NO:21 and a variable light chain comprising an amino acid sequence of SEQ ID NO:24.

In a particular aspect, the invention relates to a TNF family ligand trimer-containing antigen binding molecule as defined above, wherein the peptide linker is $(G4S)_2$, i.e. a peptide linker of SEQ ID NO: 117. In one aspect, the peptide linker in all instances is $(G4S)_2$.

The invention is further concerned with a TNF family ligand trimer-containing antigen binding molecule as defined herein before, comprising an Fc domain composed of a first and a second subunit capable of stable association.

In one aspect, the TNF family ligand trimer-containing antigen binding molecule of the invention comprising (c) an Fc domain composed of a first and a second subunit capable of stable association further comprises (a) a Fab molecule capable of specific binding to a target cell antigen, wherein the Fab heavy chain is fused at the C-terminus to the N-terminus of a CH2 domain in the Fc domain.

In a further aspect, the Fc domain is an IgG, particularly an IgG1 Fc domain or an IgG4 Fc domain.

More particularly, the Fc domain is an IgG1 Fc domain. In a particular aspect, the Fc domain comprises a modification promoting the association of the first and second subunit of the Fc domain. In a specific aspect, the invention provides a TNF family ligand trimer-containing antigen binding molecule, wherein the first subunit of the Fc domain comprises the amino acid substitutions S354C and T366W (numbering according to Kabat EU index) and the second subunit of the Fc domain comprises the amino acid substitutions Y349C, T366S, L368A and Y407V (numbering according to Kabat EU index).

In another aspect, the invention is concerned with a TNF family ligand trimer-containing antigen binding molecule as defined herein before, comprising (c) an Fc domain composed of a first and a second subunit capable of stable association, wherein the Fc domain comprises one or more amino acid substitution that reduces binding to an Fc receptor, in particular towards Fcγ receptor.

In particular, the Fc domain comprises amino acid substitutions at positions 234 and 235 (EU numbering) and/or 329 (EU numbering) of the IgG heavy chains. More particularly, provided is a trimeric TNF family ligand-containing antigen binding molecule according to the invention which comprises an IgG1 Fc domain comprising the amino acid substitutions at positions 234 and 235 (EU numbering) and/or 329 (EU numbering), in particular the amino acid substitutions L234A, L235A and P329G (EU numbering).

In a further aspect, the invention provides a TNF family ligand trimer-containing antigen binding molecule, wherein the antigen binding molecule comprises a first heavy chain and a first light chain, both comprising a Fab molecule capable of specific binding to PD1,

a first peptide comprising two ectodomains of a TNF ligand family member or fragments thereof connected to each other by a first peptide linker fused at its C-terminus by a second peptide linker to a second heavy or light chain,

and a second peptide comprising one ectodomain of said TNF ligand family member fused at its C-terminus by a third peptide linker to a second light or heavy chain, respectively.

In another aspect, provided is a TNF family ligand trimer-containing antigen binding molecule, wherein the first peptide comprising two ectodomains of a TNF ligand family member or fragments thereof connected to each other by a first peptide linker is fused at its C-terminus by a second peptide linker to a CH1 domain that is part of a heavy chain, and the second peptide comprising one ectodomain of said TNF ligand family member or a fragment thereof is fused at its C-terminus by a third peptide linker to a CL domain that is part of a light chain.

In yet another aspect, provided is a TNF family ligand trimer-containing antigen binding molecule, wherein the first peptide comprising two ectodomains of a TNF ligand family member or fragments thereof connected to each other by a first peptide linker is fused at its C-terminus by a second peptide linker to a CL domain that is part of a heavy chain, and the second peptide comprising one ectodomain of said TNF ligand family member or a fragment thereof is fused at its C-terminus by a third peptide linker to a CH1 domain that is part of a light chain.

In a further aspect, the invention provides a TNF family ligand trimer-containing antigen binding molecule, wherein the first peptide comprising two ectodomains of a TNF ligand family member or fragments thereof connected to each other by a first peptide linker is fused at its C-terminus by a second peptide linker to a VH domain that is part of a heavy chain, and the second peptide comprising one ectodomain of said TNF ligand family member or a fragment thereof is fused at its C-terminus by a third peptide linker to a VL domain that is part of a light chain.

In a particular aspect, the invention relates to a TNF family ligand trimer-containing antigen binding molecule as defined above, wherein the peptide linker is $(G4S)_2$, i.e. a peptide linker of SEQ ID NO: 117. In one aspect, the first, second and third peptide linker is $(G4S)_2$.

Provided is further a TNF family ligand trimer-containing antigen binding molecule, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

In a further aspect, provided is a TNF family ligand trimer-containing antigen binding molecule as described herein before, wherein the antigen binding molecule comprises

(a) a first heavy chain and a first light chain, both comprising a Fab molecule capable of specific binding to PD1,
(b) a second heavy chain comprising an amino acid sequence selected from the group consisting of SEQ ID NO:9, SEQ ID NO: 11, SEQ ID NO:12 and SEQ ID NO: 10, and

a second light chain comprising an amino acid sequence selected from the group consisting of SEQ ID NO:1, SEQ ID NO:3, SEQ ID NO:4 and SEQ ID NO:5.

In one aspect, the invention provides a TNF family ligand trimer-containing antigen binding molecule, wherein the antigen binding molecule comprises

(a) a Fab molecule capable of specific binding to PD1, and
(b) a second heavy chain comprising an amino acid sequence selected from the group consisting of SEQ ID NO:9, SEQ ID NO: 11, SEQ ID NO:12 and SEQ ID NO: 10, and

a second light chain comprising an amino acid sequence selected from the group consisting of SEQ ID NO:1, SEQ ID NO:3, SEQ ID NO:4 and SEQ ID NO:5.

In a particular aspect, provided is a TNF family ligand trimer-containing antigen binding molecule, wherein the antigen binding molecule comprises

(i) a first heavy chain comprising the VH domain comprising the amino acid sequence of SEQ ID NO:19 and a first light chain comprising the VL domain comprising the amino acid sequence of SEQ ID NO:20 or
a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
(ii) a second heavy chain comprising the amino acid sequence selected from the group consisting of SEQ ID NO:74, SEQ ID NO:76, SEQ ID NO:78 and SEQ ID NO:80, and
(iii) a second light chain comprising the amino acid sequence selected from the group consisting of SEQ ID NO:75, SEQ ID NO:77, SEQ ID NO:79 and SEQ ID NO:81..

In another aspect, the invention provides a TNF family ligand trimer-containing antigen binding molecule, wherein the antigen binding molecule comprises

(a) a first heavy chain comprising the amino acid sequence of SEQ ID NO:82, a first light chain comprising the amino acid sequence of SEQ ID NO:83, a second heavy chain comprising the amino acid sequence of SEQ ID NO:74 and a second light chain comprising the amino acid sequence of SEQ ID NO:75,
(b) a first heavy chain comprising the amino acid sequence of SEQ ID NO:82, a first light chain comprising the amino acid sequence of SEQ ID NO:83, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:83, a second heavy chain comprising the amino acid sequence of SEQ ID NO:80 and a second light chain comprising the amino acid sequence of SEQ ID NO:81.

In yet another aspect, the invention provides a TNF family ligand trimer-containing antigen binding molecule, comprising

(a) at least one moiety capable of specific binding to PD1, and
(b) a first and a second polypeptide that are linked to each other by a disulfide bond,

wherein the antigen binding molecule is characterized in that the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises one ectodomain of said TNF ligand family member or a fragment thereof connected via a peptide linker to C-terminus of the CH3 domain of said polypeptide.

In particular, such a TNF family ligand trimer-containing antigen binding molecule comprises two Fab domains capable of specific binding to PD1.

In particular, provided is a TNF family ligand trimer-containing antigen binding molecule as described herein before comprises

(a) a first heavy chain comprising the amino acid sequence of SEQ ID NO:84, a second heavy chain comprising the amino acid sequence of SEQ ID NO:85, and two light chains comprising the amino acid sequence of SEQ ID NO:83, or
(b) a first heavy chain comprising the amino acid sequence of SEQ ID NO:86, a second heavy chain comprising the amino acid sequence of SEQ ID NO:87, and two light chains comprising the amino acid sequence of SEQ ID NO:83.

In another particular aspect, the invention provides a TNF family ligand trimer-containing antigen binding molecule as described herein before, wherein the TNF family ligand trimer-containing antigen binding molecule comprises one Fab domain capable of specific binding to PD 1.
In such aspect, provided is a TNF family ligand trimer-containing antigen binding molecule, wherein the antigen binding molecule comprises

(i) a fusion polypeptide comprising a first subunit of a Fc domain and two ectodomains of a TNF ligand family member or fragments thereof that are connected to each other and to the C-terminus of the CH3 domain by a peptide linker, (ii) a heavy chain comprising the VH domain of the Fab domain capable of specific binding to PD1, a second subunit of the Fc domain and one ectodomain of said TNF ligand family member or a fragment thereof connected via a peptide linker to C-terminus of the CH3 domain, and (iii) a light chain comprising the VL domain of the Fab domain capable of specific binding to PD1, or
(i) a fusion polypeptide comprising a first subunit of a Fc domain and one ectodomain of a TNF ligand family member or a fragment thereof that is connected to the C-terminus of the CH3 domain by a peptide linker, (ii) a heavy chain comprising the VH domain of the Fab domain capable of specific binding to PD1, a second subunit of the Fc domain and two ectodomains of said TNF ligand family member or fragments thereof that are connected to each other and to the C-terminus of the CH3 domain via a peptide linker, and (iii) a light chain comprising the VL domain of the Fab domain capable of specific binding to PD1.

In a particular aspect, provided is a TNF family ligand trimer-containing antigen binding molecule, wherein the antigen binding molecule comprises

(a) a fusion polypeptide comprising the amino acid sequence of SEQ ID NO:88, a heavy chain comprising the amino acid sequence of SEQ ID NO:87 and a light chain comprising the amino acid sequence of SEQ ID NO:83,
(b) a fusion polypeptide comprising the amino acid sequence of SEQ ID NO:89, a heavy chain comprising the amino acid sequence of SEQ ID NO:90 and a light chain comprising the amino acid sequence of SEQ ID NO:83,
(c) a fusion polypeptide comprising the amino acid sequence of SEQ ID NO:91, a heavy chain comprising the amino acid sequence of SEQ ID NO:86 and a light chain comprising the amino acid sequence of SEQ ID NO:83,
(b) a fusion polypeptide comprising the amino acid sequence of SEQ ID NO:93, a heavy chain comprising the amino acid sequence of SEQ ID NO:92 and a light chain comprising the amino acid sequence of SEQ ID NO:83.

According to another aspect of the invention, there is provided an isolated polynucleotide encoding a TNF family ligand trimer-containing antigen binding molecule as defined herein before. The invention further provides a vector, particularly an expression vector, comprising the isolated polynucleotide of the invention and a host cell comprising the isolated polynucleotide or the vector of the invention. In some embodiments the host cell is a eukaryotic cell, particularly a mammalian cell.
In another aspect, provided is a method for producing the TNF family ligand trimer-containing antigen binding molecule of the invention, comprising the steps of (i) culturing the host cell of the invention under conditions suitable for expression of the antigen binding molecule, and (ii) recovering the antigen binding molecule. The invention also encompasses a TNF family ligand trimer-containing antigen binding molecule produced by the method of the invention.
The invention further provides a pharmaceutical composition comprising the TNF family ligand trimer-containing antigen binding molecule of the invention and at least one pharmaceutically acceptable excipient.
Also encompassed by the invention is the TNF family ligand trimer-containing antigen binding molecule of the invention, or the pharmaceutical composition of the invention, for use as a medicament. In one aspect is provided the TNF family ligand trimer-containing antigen binding molecule of the invention, or the pharmaceutical composition of the invention, for use in the treatment of a disease in an individual in need thereof. In a specific embodiment, provided is the TNF family ligand trimer-containing antigen binding molecule of the invention, or the pharmaceutical composition of the invention, for use in the treatment of cancer.
Also provided is the use of the TNF family ligand trimer-containing antigen binding molecule of the invention for the manufacture of a medicament for the treatment of a disease in an individual in need thereof, in particular for the manufacture of a medicament for the treatment of cancer, as well as a method of treating a disease in an individual, comprising administering to said individual a therapeutically effective amount of a composition comprising the TNF family ligand trimer-containing antigen binding molecule of the invention in a pharmaceutically acceptable form. In a specific embodiment, the disease is cancer. In any of the above embodiments the individual is preferably a mammal, particularly a human.

## BRIEF DESCRIPTION OF THE DRAWINGS

**Figure 1** shows that blockade of PD1 with chimeric antibody clone PD1-0103 strongly enhances IFN-gamma secretion by allogenic stimulated primary human T cells. IFN-gamma secretion is higher compared to reference antibodies.

In **Figure 2** it is shown that blockade of PD1 humanized clones PD1-0103-0312 and PD 1-103-314 even stronger increases interferon-gamma (IFN-g) secretion by allogenic stimulated primary human T cells as compared to clone PD-0103.

**Figure 3** demonstrates that the blockade of PD1 with chimeric antibody clone PD1-0103 and humanized clones PD1-0103-0312 and PD1-103-314 strongly increases tumor necrosis factor alpha (TNF) secretion by allogenic stimulated primary human T cells.

**Figure 4A** shows the frequency of $CD4^+$ T cells producing Granzyme B in the presence of increasing concentrations of different anti-PD1 antibodies and **Figure 4B** shows the amount of IFN-$\gamma$ detected by absorbance (Optical Density, O.D.) in the supernatant of the MLR in presence of increasing concentrations of different anti-PD1 antibodies.

In **Figure 5A** is shown the impact of PD1/PD-L1 blockade on reactivation of suppressed T cell receptor signaling in presence of different anti-PD1 antibodies and in **Figure 5B** the impact of PD1/PD-L1 blockade on reactivation of suppressed T cell receptor signalig in presence of different anti-PD1 antibodies is presented.

**Figure 6** shows the components for the assembly of split trimeric human 4-1BB ligands. Figure (6A) shows the dimeric 4-1BB(71-254) ligand that is fused at the C-terminus to a human CL domain with mutations E123R and Q124K (charged variant) and Figure (6B) shows the monomeric 4-1BB(71-254) ligand fused at its C-terminus to human CH1 domain with mutations K147E and K213E (charged variant). Figure (6C) shows the dimeric 4-1BB(71-254) ligand that is fused at the C-terminus to a human CL domain without the mutations and Figure (6D) shows the monomeric 4-1BB(71-254) ligand fused at its C-terminus to human CH1 domain without the mutations. Figure (1E) shows the dimeric 4-1BB(71-254)ligand that is fused at the N-terminus to a human IgG1 Fc hole domain and Figure (1F) shows the monomeric 4-1BB(71-254)ligand fused at the N-terminus to a human IgG1 Fc knob domain.

**Figure 7** illustrates schematically the structures of the 4-1BBL-trimer-containing antigen binding molecules Constructs 7.1 to 7.6 of the invention. The preparation and production of these constructs is described in Example 2. The VH and VL domains are those of anti-PD1 antibody 0103-0314, the thick black point stands for the knob-into-hole modification. * symbolizes amino acid modifications in the CH1 and CL domain (so-called charged variant).

**Figure 8** shows the components for the assembly of split trimeric human 4-1BB ligands. Figure (8A) shows the dimeric 4-1BB(71-248) ligand that is fused at the C-terminus to a human CL domain with mutations E123R and Q124K (charged variant) and Figure (8B) shows the monomeric 4-1BB(71-248) ligand fused at its C-terminus to human CH1 domain with mutations K147E and K213E (charged variant). Figure (8C) shows the dimeric 4-1BB(71-248) ligand that is fused at the C-terminus to a human CL domain without the mutations and Figure (8D) shows the monomeric 4-1BB(71-248) ligand fused at its C-terminus to human CH1 domain without the mutations. Figure (8E) shows the dimeric 4-1BB(71-248) ligand that is fused at the N-terminus to a human IgG1 Fc hole domain and Figure (8F) shows the monomeric 4-1BB(71-248)ligand fused at the N-terminus to a human IgG1 Fc knob domain.

**Figure 9** shows the components for the assembly of split trimeric human 4-1BB ligand, wherein each of dimeric 4-1BB ligand and the monomeric 4-1BB ligand is fused at its N-terminus to another polypeptide chain, in particular one of the subunits of an Fc domain. Figure (9A) shows the dimeric ligand that is fused at the N-terminus and Figure (9B) shows the monomeric ligand fused at the N-terminus. Schematic drawings of the 4-1BBL-trimer-containing antigen binding molecules Constructs 7.11 and 7.12 are shown in Figures (9C) and (9D), respectively. In Figure (9C) is shown an antigen binding molecule, wherein the Fab domain capable of specific binding to PD1 is attached to the Fc knob chain, the dimeric 4-1BBL is fused to the C-terminus of the Fc hole chain and the monomeric 4-1BBL is fused to the C-terminus of the Fc knob chain. Figure (9D) shows an antigen binding molecule, wherein the Fab domain capable of specific binding to PD1 is attached to the Fc knob chain, the dimeric 4-1BBL is also fused to the C-terminus of the Fc knob chain and the monomeric 4-1BBL is fused to the C-terminus of the Fc hole chain.

Schematic drawings of Constructs 7.13 and 7.14 are shown in **Figures (10A) and (10B),** respectively. In Figure (10A) is shown an antigen binding molecule, wherein the Fab domain capable of specific binding to PD1 is attached to the Fc hole chain, the dimeric 4-1BBL is fused to the C-terminus of the Fc hole chain and the monomeric 4-1BBL is fused to the C-terminus of the Fc knob chain. In Figure (10B) is shown an antigen binding molecule, wherein wherein the Fab domain capable of specific binding to a target cell antigen is attached to the Fc hole chain, the dimeric 4-1BBL is also fused to the C-terminus of the Fc knob chain and the monomeric 4-1BBL is fused to the C-terminus of the Fc hole chain.

In **Figures 11A to 11D** schematic drawings of the control molecules are shown. Figure (11A) and Figure (11B) show the "untargeted" variants of Constructs 7.1 and 7.3, wherein the anti-PD1 Fab domains are replaced by a DP47 (germline) Fab domain. The molecules are named Control B and Control C, respectively. Figure (11C) shows the untargeted variant of Construct 7.4 named Control D. The preparation of the control molecules is described in Example 2.9. Figure (11D) is a drawing of monospecific IgG1 molecules with the PGLALA mutations, wherein the Fab domains are DP47 Fab domains (Control F) or PD1 0103-0314 Fab domains (Control M). These molecules are described in Example 2.10.

**Figure 12A** shows the setup of the SPR experiments for simultaneous binding of the PD1 targeted split trimeric C-terminal 4-1BB ligand-containing antigen binding molecules of the invention. The simultaneous binding to human 4-1BB and human PD1 of the PD1(0314)-targeted split 4-1BBL (71-254) Fc kih antigen binding molecule Construct 7.1 is shown in Figure (12B). Simultaneous binding to human 4-1BB and human PD1 of the bivalent PD1(0314) targeted split 4-1BBL (71-254) Fc kih antigen binding molecule (Construct 7.3) is shown in Figure (12C). Simultaneous binding to human 4-1BB and human PD1 of the PD1(0314) targeted split 4-1BBL (71-248) Fc kih antigen binding molecule (construct 7.5) is shown in Figure (12D) and simultaneous binding to human 4-1BB and human PD1 of the bivalent PD1 (0314) targeted split 4-1BBL (71-248) Fc kih antigen binding molecule (Construct 7.6) is shown in Figure (12E).

**Figures 13A to 13H** show the binding of PD1-targeted 4-1BB split trimeric ligand Fc fusion antigen binding molecules to freshly isolated PBMCs as described in Example 5.1. Shown is the geo mean of fluorescence intensity of a PE-conjugated secondary detection antibody (y-axis) versus the concentration of tested constructs (x-axis). For a better overview the graphs were split in two, Figures (13A), (13B), (13C) and (13D) and Figures (13E), (13F), (13G) and (13H), respectively, whereby the curves for Control F and Control M were shown in all graphs as references. Binding was monitored on fresh human $CD45^+$ $CD3^+$ $CD4^+$ T cells (Figures (13A) and (13E)), fresh human $CD45^+$ $CD3^+$ $CD8^+$ T cells (Figures (13B) and (13F)), fresh human $CD45^+$ $CD3^+$ $CD4^{neg}$ $CD8^{neg}$ $\gamma\delta$ T cells (Figures (13C) and (13G)) and $CD45^+$ $CD3^{neg}$ $CD19^+$ B cells (Figure (13D) and (13H)).

**Figures 14A to 14H** illustrate the binding of PD1-targeted split trimeric 4-1BB ligand Fc fusion antigen binding molecules to activated human PBMCs which was tested as described in Example 5.1. Shown is the geo mean of fluorescence intensity of PE-conjugated secondary detection antibody (y-axis) versus the concentration of tested constructs (x-axis). As negative control control F was used. For a better overview the graphs were split in two, i.e. in Figures (14A), (14B), (14C) and (14D) and in Figures (14E), (14F), (14G) and (14H) whereby the curves of Control F and Control M are shown in all graphs as references. After activation with agonistic anti-human CD28 and anti-human CD3 antibody 4-1BB expression is upregulated on the majority of $CD45^+$ $CD3^+$ $CD8^+$ T cells (see Figures (14A) or (14E)) or $CD45^+$ $CD3^+$ $CD4^+$ T cells (see Figures (14B) and (14F)) and to a lesser extend to the majority of human $CD45^+$ $CD3^+$ $CD4^{neg}$ $CD8^{neg}$ $\gamma\delta$ T cells (Figures (14C) or (14G)) or $CD45^+$ $CD3^{neg}$ $CD19^+$ B cells (Figures (14D) or (14H)

**Figures 15A and 15B** show the binding of PD1-targeted or untargeted split trimeric human 4-1BB ligand Fc (kih) fusion antigen binding molecules to human-PD1 expressing CHO-k1-huPD1 clone 5 cells. The method is described in Example 5.2. Binding was detected with R-Phycoerythrin-fluorochrome conjugated anti-human IgG Fc$\gamma$-specific goat IgG F(ab')2 fragment and the geo mean measured by flow cytometry is shown on the y-axis. On the x-axis the used concentration of PD1 targeted 4-1BB split trimeric ligand Fc fusion antigen binding molecules and their controls are shown. For a better overview the graphs were split in two (Figure (15A) and Figure (15B)) whereby the curves for control F and control M are shown in both as references.

**Figures 16A and 16B** show the binding of PD1-targeted or untargeted split trimeric human 4-1BB ligand Fc (kih) fusion antigen binding molecules to human-4-1BB expressing HeLa-hu4-1BBL-NFkB-luc clone 26 cells as tested as described in Example 5.2. Binding was detected with R-Phycoerythrin-fluorochrome conjugated anti-human IgG Fcγ-specific goat IgG F(ab')2 fragment and the geo mean measured by flow cytometry is shown on the y-axis. On the x-axis the used concentration of PD1 targeted 4-1BB split trimeric ligand Fc fusion antigen binding molecules and their controls are shown. For a better overview the graphs were split in two, named Figures (16A) and (16B), whereby the curves of control F and control M are shown in both graphs as references.

**Figure 17** shows a scheme that illustrates the general principal of the NFkB activity assay described in Example 6 using a reporter cell line. Shown is the activation assay set up with human 4-1BB expressing HeLa reporter cell line. A crosslinking of 4-1BB expressed on the reporter cells induces NFκB activation and NFκB-mediated luciferase expression. After lysis of the cells luciferase can catalyze the oxidation of luciferin to oxyluciferin. This chemical reaction correlates positively with the strength of NFκB-mediated luciferase expression and can be measured by the strength of light emission (units of released light).

**Figures 18A to 18D** shows the NFκB-activation-induced luciferase expression and activity as measured with the assay described in Example 6. Units of released light (URL) are measured for 0.5 s/well and plotted against the used concentration of PD1-targeted or untargeted split trimeric human 4-1BB ligand Fc (kih) constructs. Human 4-1BB-expressing HeLa-reporter cells were incubated for 6 h in the absence (Figures (18A) and (18B)) or presence of crosslinking human-PD1 expressing CHO-k1-huPD1 clone 5 cells (Figure (18C) and (18D). The cell ratio of human 4-1BB-expressing HeLa reporter cell to CHO-k1-huPD1 clone 5 cells is indicated in each graph.

## DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term **"antigen binding molecule"** refers in its broadest sense to a molecule that specifically binds an antigenic determinant. Examples of antigen binding molecules are antibodies, antibody fragments and scaffold antigen binding proteins.

As used herein, the term **"moiety capable of specific binding to a target cell antigen"** refers to a polypeptide molecule that specifically binds to an antigenic determinant. In one aspect, the antigen binding moiety is able to activate signaling through its target cell antigen. In a particular aspect, the antigen binding moiety is able to direct the entity to which it is attached (e.g. the TNF family ligand trimer) to a target site, for example to a specific type of tumor cell or tumor stroma bearing the antigenic determinant or on T cells. Moieties capable of specific binding to a target cell antigen include antibodies and fragments thereof as further defined herein. In addition, moieties capable of specific binding to a target cell antigen include scaffold antigen binding proteins as further defined herein, e.g. binding domains which are based on designed repeat proteins or designed repeat domains (see e.g. WO 2002/020565).

In relation to an antibody or fragment thereof, the term "moiety capable of specific binding to a target cell antigen" refers to the part of the molecule that comprises the area which specifically binds to and is complementary to part or all of an antigen. A moiety capable of specific antigen binding may be provided, for example, by one or more antibody variable domains (also called antibody variable regions). Particularly, a moiety capable of specific antigen binding comprises an antibody light chain variable region (VL) and an antibody heavy chain variable region (VH).

The term **"antibody"** herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term **"monoclonal antibody"** as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term **"monospecific"** antibody as used herein denotes an antibody that has one or more binding sites each of which bind to the same epitope of the same antigen. The term **"bispecific"** means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term **"valent"** as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule.

The terms "full length antibody", "intact antibody", and "whole antibody" are used herein interchangeably to refer to an antibody having a structure substantially similar to a native antibody structure. **"Native antibodies"** refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma$1 (IgG1), $\gamma$2 (IgG2), $\gamma$3 (IgG3), $\gamma$4 (IgG4), $\alpha$1 (IgA1) and $\alpha$2 (IgA2). The light chain of an antibody may be assigned to one of two types, called kappa ($\kappa$) and lambda ($\lambda$), based on the amino acid sequence of its constant domain.

An **"antibody fragment"** refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term **"Fab fragment"** refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an F(ab')2 fragment that has two antigen-combining sites (two Fab fragments) and a part of the Fc region.

The term **"cross-Fab fragment"** or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab $_{(VLVH)}$. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab $_{(CLCH1)}$.

A "single chain Fab fragment" or **"scFab"** is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or **"x-scFab"** is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A **"single-chain variable fragment (scFv)"** is a fusion protein of the variable regions of the heavy ($V_H$) and light chains ($V_L$) of an antibody, connected with a short linker peptide of ten to about 25 amino acids. The linker is usually rich in glycine for flexibility, as well as serine or threonine for solubility, and can either connect the N-terminus of the $V_H$ with the C-terminus of the $V_L$, or *vice versa.* This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

**"Scaffold antigen binding proteins"** are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), $V_{NAR}$ fragments, a fibronectin (AdNectin), a C-type lectin domain (Tetranectin); a variable domain of a new antigen receptor beta-lactamase ($V_{NAR}$ fragments), a human gamma-crystallin or ubiquitin (Affilin molecules); a kunitz type domain of human protease inhibitors, microbodies such as the proteins from the knottin family, peptide aptamers and fibronectin (adnectin).

CTLA-4 (Cytotoxic T Lymphocyte-associated Antigen 4) is a CD28-family receptor expressed on mainly CD4+ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies (e.g. US7166697B1). Evibodies are around the same size as the isolated variable region of an antibody (e.g. a domain antibody). For further details see Journal of Immunological Methods 248 (1-2), 31-45 (2001).

Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633.

An affibody is a scaffold derived from Protein A of Staphylococcus aureus which can be engineered to bind to antigen. The domain consists of a three-helical bundle of approximately 58 amino acids. Libraries have been generated by randomization of surface residues. For further details see Protein Eng. Des. Sel. 17, 455-462 (2004) and EP 1641818A1.

Avimers are multidomain proteins derived from the A-domain scaffold family. The native domains of approximately 35 amino acids adopt a defined disulfide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007).

A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

A single-domain antibody is an antibody fragment consisting of a single monomeric variable antibody domain. The first single domain were derived from the variable domain of the antibody heavy chain from camelids (nanobodies or $V_H$H fragments). Furthermore, the term single-domain antibody includes an autonomous human heavy chain variable domain (aVH) or $V_{NAR}$ fragments derived from sharks.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the .beta.-sandwich can be engineered to enable an Adnectin to specifically recognize a therapeutic target of interest. For further details see Protein Eng. Des. Sel. 18, 435- 444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataBI and conotoxin and knottins. The microproteins have a loop which can beengineered to include upto 25 amino acids without affecting the overall fold of the microprotein. For further details of engineered knottin domains, see WO2008098796.

An **"antigen binding molecule that binds to the same epitope"** as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term **"antigen binding domain"** refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term **"antigenic determinant"** is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

The term **"capable of specific binding to PD1"** refers to an antigen binding molecule that is capable of binding PD with sufficient affinity such that the antigen binding molecule is useful as a diagnostic and/or therapeutic agent in targeting PD1. The antigen binding molecule includes but is not limited to, antibodies, Fab molecules, crossover Fab molecules, single chain Fab molecules, Fv molecules, scFv molecules, single domain antibodies, and VH and scaffold antigen binding protein. In one aspect, the extent of binding of an anti-PD1 1 antigen binding molecule to an unrelated, non-PD1 protein is less than about 10% of the binding of the antigen binding molecule to PD1 as measured, e.g., by a radioimmunoassay (RIA). In particular, an antigen binding molecule that is capable of specific binding to PD1 has a dissociation constant ($K_d$) of $\leq 1\ \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In certain embodiments, an anti-PD1 antigen binding molecule binds to PD1 from different species. In particular, the anti-PD1 antigen binding molecule binds to human and cynomolgus PD1.

By **"specific binding"** is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of ≤ 1 μM, ≤ 100 nM, ≤ 10 nM, ≤ 1 nM, ≤ 0.1 nM, ≤ 0.01 nM, or ≤ 0.001 nM (e.g. $10^{-8}$ M or less, e.g. from $10^{-8}$ M to $10^{-13}$ M, e.g. from $10^{-9}$ M to $10^{-13}$ M).

**"Affinity"** or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants ($k_{off}$ and $k_{on}$, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A **"target cell antigen"** as used herein refers to an antigenic determinant presented on the surface of a target cell, for example a T-cell or B-cell, a cell in a tumor such as a cancer cell or a cell of the tumor stroma. In certain embodiments, the target cell antigen is an antigen on the surface of T cell. In one embodiment, the target cell antigen is PD1.

The term **"PD1",** also known as Programmed cell death protein 1, is a type I membrane protein of 288 amino acids that was first described in 1992 (Ishida et al., EMBO J., 11 (1992), 3887-3895). PD1 is a member of the extended CD28/CTLA-4 family of T cell regulators and has two ligands, PD-L1 (B7-H1, CD274) and PD-L2 (B7-DC, CD273). The protein's structure includes an extracellular IgV domain followed by a transmembrane region and an intracellular tail. The intracellular tail contains two phosphorylation sites located in an immunoreceptor tyrosine-based inhibitory motif and an immunoreceptor tyrosine-based switch motif, which suggests that PD1 negatively regulates TCR signals. This is consistent with binding of SHP-1 and SHP-2 phosphatases to the cytoplasmic tail of PD1 upon ligand binding. While PD1 is not expressed on naïve T cells, it is upregulated following T cell receptor (TCR)-mediated activation and is observed on both activated and exhausted T cells (Agata et al., Int. Immunology 8 (1996), 765-772). These exhausted T-cells have a dysfunctional phenotype and are unable to respond appropriately. Although PD1 has a relatively wide expression pattern its most important role is likely as a coinhibitory receptor on T cells (Chinai et al, Trends in Pharmacological Sciences 36 (2015), 587-595). Current therapeutic approaches thus focus on blocking the interaction of PD1 with its ligands to enhance T cell response. The terms "Programmed Death 1," "Programmed Cell Death 1," "Protein PD-1," "PD-1," PD1," "PDCD1," "hPD-1" and "hPD-1" can be used interchangeably, and include variants, isoforms, species homologs of human PD1, and analogs having at least one common epitope with PD1. The amino acid sequence of human PD1 is shown in UniProt (www.uniprot.org) accession no. Q15116 (SEQ ID NO:94).

The term **"variable region"** or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen binding specificity.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987)). Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypemariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE A. CDR Definitions[1]**

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$ CDR1 | 24-34 | 26-32 | 24-34 |
| $V_L$ CDR2 | 50-56 | 50-52 | 50-56 |
| $V_L$ CDR3 | 89-97 | 91-96 | 89-97 |

[1] Numbering of all CDR definitions in Table A is according to the numbering conventions set forth by Kabat et al. (see below).
[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

As used herein, the term **"affinity matured"** in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

**"Framework"** or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An **"acceptor human framework"** for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term **"chimeric"** antibody refers to an antibody in which a portion of the heavy and/or light chain is derived from a particular source or species, while the remainder of the heavy and/or light chain is derived from a different source or species.

The **"class"** of an antibody refers to the type of constant domain or constant region possessed by its heavy chain. There are five major classes of antibodies: IgA, IgD, IgE, IgG, and IgM, and several of these may be further divided into subclasses (isotypes), e.g. $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$ respectively..

A **"humanized"** antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A **"humanized form"** of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A **"human"** antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or **"Fc region"** herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333, expressly incorporated herein by reference). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The **"knob-into-hole"** technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)). The numbering is according to EU index of Kabat et al, Sequences of Proteins of Immunological Interest, 5th Ed. Public Health Service, National Institutes of Health, Bethesda, MD, 1991.

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term **"effector functions"** refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

An **"activating Fc receptor"** is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcαRI (CD89). A particular activating Fc receptor is human FcγRIIIa (see UniProt accession no. P08637, version 141).

The term **"TNF ligand family member"** or "TNF family ligand" refers to a proinflammatory cytokine. Cytokines in general, and in particular the members of the TNF ligand family, play a crucial role in the stimulation and coordination of the immune system. At present, nineteen cyctokines have been identified as members of the TNF (tumour necrosis factor) ligand superfamily on the basis of sequence, functional, and structural similarities. All these ligands are type II transmembrane proteins with a C-terminal extracellular domain (ectodomain), N-terminal intracellular domain and a single transmembrane domain. The C-terminal extracellular domain, known as TNF homology domain (THD), has 20-30% amino acid identity between the superfamily members and is responsible for binding to the receptor. The TNF ectodomain is also responsible for the TNF ligands to form trimeric complexes that are recognized by their specific receptors.

Members of the TNF ligand family are selected from the group consisting of Lymphotoxin α (also known as LTA or TNFSF1), TNF (also known as TNFSF2), LTβ (also known as TNFSF3), OX40L (also known as TNFSF4), CD40L (also known as CD154 or TNFSF5), FasL (also known as CD95L, CD178 or TNFSF6), CD27L (also known as CD70 or TNFSF7), CD30L (also known as CD153 or TNFSF8), 4-1BBL (also known as TNFSF9), TRAIL (also known as APO2L, CD253 or TNFSF10), RANKL (also known as CD254 or TNFSF11), TWEAK (also known as TNFSF12), APRIL (also known as CD256 or TNFSF13), BAFF (also known as CD257 or TNFSF13B), LIGHT (also known as CD258 or TNFSF14), TL1A (also known as VEGI or TNFSF15), GITRL (also known as TNFSF18), EDA-A1 (also known as ectodysplasin A1) and EDA-A2 (also known as ectodysplasin A2). The term refers to any native TNF family ligand from any vertebrate source, including mammals such as primates (e.g. humans), non-human primates (e.g. cynomolgus monkeys) and rodents (e.g. mice and rats), unless otherwise indicated. In specific embodiments of the invention, the TNF ligand family member is selected from the group consisting of OX40L, FasL, CD27L, TRAIL, 4-1BBL, CD40L and GITRL. In a particular embodiment, the TNF ligand family member is selected from 4-1BBL and OX40L.

Further information, in particular sequences, of the TNF ligand family members may be obtained from publically accessible databases such as Uniprot (www.uniprot.org). For instance, the human TNF ligands have the following amino acid sequences: human Lymphotoxin α (UniProt accession no. P01374, SEQ ID NO:95), human TNF (UniProt accession no. P01375, SEQ ID NO:96), human Lymphotoxin β (UniProt accession no. Q06643, SEQ ID NO:97), human OX40L (UniProt accession no. P23510, SEQ ID NO:98), human CD40L (UniProt accession no. P29965, SEQ ID NO:99), human FasL (UniProt accession no. P48023, SEQ ID NO: 100), human CD27L (UniProt accession no. P32970, SEQ ID NO:101), human CD30L (UniProt accession no. P32971, SEQ ID NO:102) 4-1BBL (UniProt accession no. P41273, SEQ ID NO:103), TRAIL (UniProt accession no. P50591, SEQ ID NO:104), RANKL (UniProt accession no. 014788, SEQ ID NO: 105), TWEAK (UniProt accession no. 043508, SEQ ID NO:106), APRIL (UniProt accession no. 075888, SEQ ID NO:107), BAFF (UniProt accession no. Q9Y275, SEQ ID NO:108), LIGHT (UniProt accession no. 043557, SEQ ID NO:109), TL1A (UniProt accession no. 095150, SEQ ID NO: 110), GITRL (UniProt accession no. Q9UNG2, SEQ ID NO:111) and ectodysplasin A (UniProt accession no. Q92838, SEQ ID NO:112).

An **"ectodomain"** is the domain of a membrane protein that extends into the extracellular space (i.e. the space outside the target cell). Ectodomains are usually the parts of proteins that initiate contact with surfaces, which leads to signal transduction. The ectodomain of TNF ligand family member as defined herein thus refers to the part of the TNF ligand protein that extends into the extracellular space (the extracellular domain), but also includes shorter parts or fragments thereof that are responsible for the trimerization and for the binding to the corresponding TNF receptor. The term "ectodomain of a TNF ligand family member or a fragment thereof" thus refers to the extracellular domain of the TNF ligand family member that forms the extracellular domain or to parts thereof that are still able to bind to the receptor (receptor binding domain).

The term **"costimulatory TNF ligand family member"** or "costimulatory TNF family ligand" refers to a subgroup of TNF ligand family members, which are able to costimulate proliferation and cytokine production of T-cells. These TNF family ligands can costimulate TCR signals upon interaction with their corresponding TNF receptors and the interaction with their receptors leads to recruitment of TNFR-associated factors (TRAF), which initiate signalling cascades that result in T-cell activation. Costimulatory TNF family ligands are selected from the group consisting of 4-1BBL, OX40L, GITRL, CD70, CD30L and LIGHT, more particularly the costimulatory TNF ligand family member is selected from 4-1BBL and OX40L.

As described herein before, 4-1BBL is a type II transmembrane protein and one member of the TNF ligand family. Complete or full length 4-1BBL having the amino acid sequence of SEQ ID NO: 103 has been described to form trimers on the surface of cells. The formation of trimers is enabled by specific motives of the ectodomain of 4-1BBL. Said motives are designated herein as "trimerization region". The amino acids 50-254 of the human 4-1BBL sequence (SEQ ID NO: 113) form the extracellular domain of 4-1BBL, but even fragments thereof are able to form the trimers. In specific embodiments of the invention, the term "ectodomain of 4-1BBL or a fragment thereof" refers to a polypeptide having an amino acid sequence selected from SEQ ID NO:4 (amino acids 52-254 of human 4-1BBL), SEQ ID NO:1 (amino acids 71-254 of human 4-1BBL), SEQ ID NO:3 (amino acids 80-254 of human 4-1BBL) and SEQ ID NO:2 (amino acids 85-254 of human 4-1BBL) or a polypeptide having an amino acid sequence selected from SEQ ID NO:5 (amino acids 71-248 of human 4-1BBL), SEQ ID NO:8 (amino acids 52-248 of human 4-1BBL), SEQ ID NO:7 (amino acids 80-248 of human 4-1BBL) and SEQ ID NO:6 (amino acids 85-248 of human 4-1BBL), but also other fragments of the ectodomain capable of trimerization are included herein.

As described herein before, OX40L is another type II transmembrane protein and a further member of the TNF ligand family. Complete or full length human OX40L has the amino acid sequence of SEQ ID NO:98. The amino acids 51-183 of the human OX40L sequence (SEQ ID NO:114) form the extracellular domain of OX40L, but even fragments thereof that are able to form the trimers. In specific embodiments of the invention, the term "ectodomain of OX40L or a fragment thereof" refers to a polypeptide having an amino acid sequence selected from SEQ ID NO:114 (amino acids 51-183 of human OX40L) or SEQ ID NO:115 (amino acids 52-183 of human OX40L), but also other fragments of the ectodomain capable of trimerization are included herein.

The term **"peptide linker"** refers to a peptide comprising one or more amino acids, typically about 2 to 20 amino acids. Peptide linkers are known in the art or are described herein. Suitable, non-immunogenic linker peptides are, for example, $(G_4S)_n$, $(SG_4)_n$ or $G_4(SG_4)_n$ peptide linkers, wherein "n" is generally a number between 1 and 10, typically between 1 and 4, in particular 2, i.e. the peptides selected from the group consisting of GGGGS (SEQ ID NO: 116), GGGGSGGGGS (SEQ ID NO:117), SGGGGSGGGG (SEQ ID NO:118), $(G_4S)_3$ or GGGGSGGGGSGGGGS (SEQ ID NO:119), GGGGSGGGGSGGGG or $G4(SG4)_2$ (SEQ ID NO:120), and $(G_4S)_4$ or GGGGSGGGGSGGGGSGGGGS (SEQ ID NO:121), but also include the sequences GSPGSSSSGS (SEQ ID NO: 122), GSGSGSGS (SEQ ID NO: 123), GSGSGNGS (SEQ ID NO:124), GGSGSGSG (SEQ ID NO:125), GGSGSG (SEQ ID NO:126), GGSG (SEQ ID NO: 127), GGSGNGSG (SEQ ID NO: 128), GGNGSGSG (SEQ ID NO: 129) and GGNGSG (SEQ ID NO: 130). Peptide linkers of particular interest are $(G4S)_1$ or GGGGS (SEQ ID NO:86), $(G_4S)_2$ or GGGGSGGGGS (SEQ ID NO: 117) and GSPGSSSSGS (SEQ ID NO: 122), more particularly $(G_4S)_2$ or GGGGSGGGGS (SEQ ID NO: 117).

The term **"amino acid"** as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

A **"fusion polypeptide"** or "single fusion polypeptide" as used herein refers to a single chain polypeptide composed of one or two ectodomains of said TNF ligand family member fused to a part of antigen binding moiety or Fc part. The fusion may occur by directly linking the N or C-terminal amino acid of the antigen binding moiety via a peptide linker to the C- or N-terminal amino acid of the ectodomain of said TNF ligand family member.

By "fused" or "connected" is meant that the components (e.g. a polypeptide and an ectodomain of said TNF ligand family member) are linked by peptide bonds, either directly or via one or more peptide linkers.

**"Percent (%) amino acid sequence identity"** with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the TNF ligand trimer-containing antigen binding molecules provided herein are contemplated. For example, it may be desirable to improve the binding affinity and/or other biological properties of the TNF ligand trimer-containing antigen binding molecules. Amino acid sequence variants of the TNF ligand trimer-containing antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE B**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | He |
| Lys (K) | Arg; Gln; Asn | Arg |
| Met (M) | Leu; Phe; He | Leu |
| Phe (F) | Trp; Leu; Val; He; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

The term **"amino acid sequence variants"** includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include TNF family ligand trimer-containing antigen binding molecule with an N-terminal methionyl residue. Other insertional variants of the molecule include the fusion to the N- or C-terminus to a polypeptide which increases the serum half-life of the TNF ligand trimer-containing antigen binding molecules.

In certain embodiments, the TNF family ligand trimer-containing antigen binding molecules provided herein are altered to increase or decrease the extent to which the antibody is glycosylated. Glycosylation variants of the molecules may be conveniently obtained by altering the amino acid sequence such that one or more glycosylation sites is created or removed. Where the TNF ligand trimer-containing antigen binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in TNF family ligand trimer-containing antigen binding molecule may be made in order to create variants with certain improved properties. In one aspect, variants of TNF family ligand trimer-containing antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of the TNF family ligand trimer-containing antigen binding molecules of the invention include those with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain embodiments, it may be desirable to create **cysteine engineered variants** of the TNF family ligand trimer-containing antigen binding molecule of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the TNF family ligand trimer-containing antigen binding molecules provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the bispecific antibody derivative will be used in a therapy under defined conditions, etc. In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

In another aspect, immunoconjugates of the TNF family ligand trimer-containing antigen binding molecules provided herein maybe obtained. An **"immunoconjugate"** is an antibody conjugated to one or more heterologous molecule(s), including but not limited to a cytotoxic agent.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term **"expression cassette"** refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms **"host cell",** "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An **"effective amount"** of an agent refers to the amount that is necessary to result in a physiological change in the cell or tissue to which it is administered.

A **"therapeutically effective amount"** of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An **"individual"** or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term **"pharmaceutical composition"** refers to a preparation which is in such form as to permit the biological activity of an active ingredient contained therein to be effective, and which contains no additional components which are unacceptably toxic to a subject to which the formulation would be administered.

A **"pharmaceutically acceptable excipient"** refers to an ingredient in a pharmaceutical composition, other than an active ingredient, which is nontoxic to a subject. A pharmaceutically acceptable excipient includes, but is not limited to, a buffer, a stabilizer, or a preservative.

The term **"package insert"** is used to refer to instructions customarily included in commercial packages of therapeutic products, that contain information about the indications, usage, dosage, administration, combination therapy, contraindications and/or warnings concerning the use of such therapeutic products.

As used herein, **"treatment"** (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the invention are used to delay development of a disease or to slow the progression of a disease.

The term **"cancer"** as used herein refers to proliferative diseases, such as lymphomas, carcinoma, lymphoma, blastoma, sarcoma, leukemia, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colorectal cancer (CRC), pancreatic cancer, breast cancer, triple-negative breast cancer , uterine cancer, carcinoma of the fallopian tubes, carcinoma of the endometrium, carcinoma of the cervix, carcinoma of the vagina, carcinoma of the vulva, Hodgkin’s Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, melanoma, multiple myeloma, B-cell cancer (lymphoma), chronic lymphocytic leukemia (CLL), acute lymphoblastic leukemia (ALL), hairy cell leukemia, chronic myeloblastic leukemia, including refractory versions of any of the above cancers, or a combination of one or more of the above cancers.

**TNF family ligand trimer-containing antigen binding molecules of the invention**

The invention provides novel TNF family ligand trimer-containing antigen binding molecules with particularly advantageous properties such as producibility, stability, binding affinity, biological activity, targeting efficiency and reduced toxicity.

In a first aspect, the invention provides a TNF family ligand trimer-containing antigen binding molecule comprising

(a) at least one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or a fragment thereof that are connected to each other by a peptide linker and in that the second polypeptide comprises only one ectodomain of said TNF ligand family member or a fragment thereof.

In a particular aspect, the invention provides a TNF family ligand trimer-containing antigen binding molecule comprising

(a) at least one moiety capable of specific binding to PD1,
(b) a first and a second polypeptide that are linked to each other by a disulfide bond,
wherein the antigen binding molecule is characterized in that the first polypeptide comprises two ectodomains of a TNF ligand family member or a fragment thereof that are connected to each other by a peptide linker and in that the second polypeptide comprises only one ectodomain of said TNF ligand family member or a fragment thereof, and
(c) an Fc domain composed of a first and a second subunit capable of stable association.

In a particular aspect, the TNF family ligand trimer-containing antigen binding molecule comprises

(a) at least one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof, wherein the TNF ligand family member is costimulates human T-cell activation.

In another particular aspect, the TNF family ligand trimer-containing antigen binding molecule comprises

(a) at least one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof, wherein the ectodomains of a TNF ligand family member are identical in all instances.

In a further aspect, provided is a TNF family ligand trimer-containing antigen binding molecule as defined herein before, comprising

(a) at least one moiety capable of specific binding to a PD1 and
(b) a first and a second polypeptide that are linked to each other by a disulfide bond,

wherein the antigen binding molecule is characterized in that

(i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or two fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or
(ii) the first polypeptide contains a CH3 domain and the second polypeptide contains a CH3 domain, respectively, and wherein the first polypeptide comprises two ectodomains of a TNF ligand family member or two fragments thereof that are connected to each other and to the C-terminus of the CH3 domain by a peptide linker and wherein the second polypeptide comprises only one ectodomain of said TNF ligand family member or a fragment thereof connected via a peptide linker to C-terminus of the CH3 domain of said polypeptide, or

(iii) the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to to VH or VL by a peptide linker and wherein the second polypeptide comprises one ectodomain of said TNF ligand family member or a fragment thereof connected via a peptide linker to VL or VH of said polypeptide.

In another aspect, provided is a TNF family ligand trimer-containing antigen binding molecule of as defined herein before, comprising

(a) at least one moiety capable of specific binding to a PD1 and
(b) a first and a second polypeptide that are linked to each other by a disulfide bond,

wherein the antigen binding molecule is characterized in that

(i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or two fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or

(ii) the first polypeptide contains a CH3 domain and the second polypeptide contains a CH3 domain, respectively, and wherein the first polypeptide comprises two ectodomains of a TNF ligand family member or two fragments thereof that are connected to each other and to the C-terminus of the CH3 domain by a peptide linker and wherein the second polypeptide comprises only one ectodomain of said TNF ligand family member or a fragment thereof connected via a peptide linker to C-terminus of the CH3 domain of said polypeptide.

In one aspect, the TNF family ligand trimer-containing antigen binding molecule comprises a TNF ligand family member that costimulates human T-cell activation which is selected from 4-1BBL and OX40L. More particularly, the TNF ligand family member is 4-1BBL.

In another aspect, wherein the ectodomain of a TNF ligand family member comprises the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO:5. More particularly, the ectodomain of a TNF ligand family member comprises the amino acid sequence of SEQ ID NO:5.

In a further aspect, the TNF family ligand trimer-containing antigen binding molecule of the invention comprises

(a) at least one moiety capable of specific binding to PD1 and
(b) a first and a second polypeptide that are linked to each other by a disulfide bond,

wherein the antigen binding molecule is characterized in that the first polypeptide comprises the amino acid sequence selected from the group consisting of SEQ ID NO:9, SEQ ID NO: 11, SEQ ID NO:12 and SEQ ID NO:10 and in that the second polypeptide comprises the amino acid sequence selected from the group consisting of SEQ ID NO:1, SEQ ID NO:3, SEQ ID NO:4 and SEQ ID NO:5.

In one aspect, the TNF family ligand trimer-containing antigen binding molecule of the invention comprises

(a) at least one moiety capable of specific binding to PD1 and
(b) a first and a second polypeptide that are linked to each other by a disulfide bond,

wherein the antigen binding molecule is characterized in that the first polypeptide comprises the amino acid sequence of SEQ ID NO:10 and in that the second polypeptide comprises the amino acid sequence of SEQ ID NO:5.

In a further aspect, the TNF family ligand trimer-containing antigen binding molecule of the invention comprises

(a) at least one moiety capable of specific binding to PD1 and
(b) a first and a second polypeptide that are linked to each other by a disulfide bond,

wherein the antigen binding molecule is characterized in that the first polypeptide comprises the amino acid sequence of SEQ ID NO:9 and in that the second polypeptide comprises the amino acid sequence of SEQ ID NO: 1.

In another aspect, the TNF ligand family member is OX40L. In a particular aspect, provided is TNF family ligand trimer-containing antigen binding molecule, wherein the ectodomain of a TNF ligand family member comprises the amino acid sequence of SEQ ID NO: 114 or SEQ ID NO: 115, particularly the amino acid sequence of SEQ ID NO: 114.

In another aspect, the TNF family ligand trimer-containing antigen binding molecule of the invention comprises

(a) at least one moiety capable of specific binding to PD1,
(b) a first polypeptide containing a CH1 or CL domain and a second polypeptide containing a CL or CH1 domain, respectively, wherein the second polypeptide is linked to the first polypeptide by a disulfide bond between the CH1 and CL domain,

and wherein the antigen binding molecule is characterized in that the first polypeptide comprises two ectodomains of a TNF ligand family member or two fragments thereof that are connected to each other and to the CH1 or CL domain by a peptide linker and in that the second polypeptide comprises only one ectodomain of said TNF ligand family member or a fragment thereof connected by a peptide linker to the CL or CH1 domain of said polypeptide.

In one aspect, provided is a TNF family ligand trimer-containing antigen binding molecule comprising

(a) at least one moiety capable of specific binding to PD1,
(b) a first polypeptide containing a CH1 domain and a second polypeptide containing a CL domain, wherein the second polypeptide is linked to the first polypeptide by a disulfide bond between the CH1 and CL domain,

and wherein the antigen binding molecule is characterized in that the first polypeptide comprises two ectodomains of a TNF ligand family member or fragments thereof that are connected to each other and to the CH1 domain by a peptide linker and in that the second polypeptide comprises one ectodomain of said TNF ligand family member or a fragment thereof connected via a peptide linker to the CL domain of said polypeptide.

In another aspect, provided is a TNF family ligand trimer-containing antigen binding molecule comprising

(a) at least one moiety capable of specific binding to PD1,
(b) a first polypeptide containing a CL domain and a second polypeptide containing a CH1 domain, wherein the second polypeptide is linked to the first polypeptide by a disulfide bond between the CH1 and CL domain,

and wherein the antigen binding molecule is characterized in that the first polypeptide comprises two ectodomains of a TNF ligand family member or fragments thereof that are connected to each other and to the CL domain by a peptide linker and in that the second polypeptide comprises one ectodomain of said TNF ligand family member or a fragment thereof connected via a peptide linker to the CH1 domain of said polypeptide.

In another aspect, the invention provides a TNF family ligand trimer-containing antigen binding molecule comprising

(a) one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof.

In yet another aspect, the invention provides a TNF family ligand trimer-containing antigen binding molecule comprising

(a) more than one moiety capable of specific binding to PD1 and
(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof connected via a peptide linker to said polypeptide.

In one aspect, the invention provides a TNF family ligand trimer-containing antigen binding molecule comprising

(a) two moities capable of specific binding to PD1 and

(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof.

In a particular aspect, provided is a TNF family ligand trimer-containing antigen binding molecule, comprising

(a) at least one moiety capable of specific binding to PD1, and
(b) a first and a second polypeptide that are linked to each other by a disulfide bond,

wherein the antigen binding molecule is characterized in that the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises one ectodomain of said TNF ligand family member or a fragment thereof connected via a peptide linker to C-terminus of the CH3 domain of said polypeptide. Particularly, such TNF family ligand trimer-containing antigen binding molecule comprises two moieties capable of specific binding to a target cell antigen.

In one aspect, the invention provides a TNF family ligand trimer-containing antigen binding molecule comprising

(a) two moities capable of specific binding to PD1 and
(b) a first and a second polypeptide that are linked to each other by a disulfide bond,

wherein the antigen binding molecule is characterized in that the first polypeptide comprises two ectodomains of a TNF ligand family member or two fragments thereof that are connected to each other by a peptide linker and in that the second polypeptide comprises one ectodomain of said TNF ligand family member or a fragment thereof,
wherein the two moieties capable of specific binding to a target cell antigen bind to two different target cell antigens.

In a further aspect, the invention provides a TNF family ligand trimer-containing antigen binding molecule as defined herein before, wherein the moiety capable of specific binding to a target cell antigen is selected from the group consisting of an antibody, an antibody fragment and a scaffold antigen binding protein.

In one aspect, provided is a TNF family ligand trimer-containing antigen binding molecule as described herein before, wherein the moiety capable of specific binding to PD1 is selected from the group consisting of an antibody fragment, a Fab molecule, a crossover Fab molecule, a single chain Fab molecule, a Fv molecule, a scFv molecule, a single domain antibody, an aVH and a scaffold antigen binding protein. In one aspect, the moiety capable of specific binding to PD is an aVH or a scaffold antigen binding protein. In one aspect, the moiety capable of specific binding to a target cell antigen is a scaffold antigen binding protein capable of specific binding to a target cell antigen.

In particular, the TNF family ligand trimer-containing antigen binding molecule comprises one or two moieties capable of specific binding to PD1.

In a particular aspect, provided is a TNF family ligand trimer-containing antigen binding molecule, wherein the moiety capable of specific binding to PD is a Fab molecule or a crossover Fab molecule capable of specific binding to PD1. In particular, the moiety capable of specific binding to a target cell antigen is a Fab capable of specific binding to PD 1.

In a further aspect, provided is a TNF family ligand trimer-containing antigen binding molecule according to the invention, wherein a peptide comprising two ectodomains of a TNF ligand family member or fragments thereof connected to each other by a first peptide linker is fused at its C-terminus to the CH1 domain of a heavy chain by a second peptide linker and wherein one ectodomain of said TNF ligand family member or a fragment thereof is fused at the its C-terminus to the CL domain on a light chain by a third peptide linker.

In another aspect, provided is a TNF family ligand trimer-containing antigen binding molecule according to the invention, wherein a peptide comprising two ectodomains of a TNF ligand family member or fragments thereof connected to each other by a first peptide linker is fused at its C-terminus to the CL domain of a heavy chain by a second peptide linker and wherein one ectodomain of said TNF ligand family member or a fragment thereof is fused at the its C-terminus to the CH1 domain on a light chain by a third peptide linker.

In a further aspect, the invention is concerned with a TNF family ligand trimer-containing antigen binding molecule according to the invention, wherein a peptide comprising two ectodomains of a TNF ligand family member or fragments thereof connected to each other by a first peptide linker is fused at its C-terminus to the CL domain of a light chain by a second peptide linker and wherein one ectodomain of said TNF ligand family member or a fragment thereof is fused at the its C-terminus to the CH1 domain of the heavy chain by a third peptide linker.

In a particular aspect, the invention relates to a TNF family ligand trimer-containing antigen binding molecule as defined above, wherein the peptide linker is $(G4S)_2$. In one aspect, the first peptide linker is $(G_4S)_2$ (SEQ ID NO: 117), the second peptide linker is GSPGSSSSGS (SEQ ID NO: 122) and the third peptide linker is $(G_4S)_2$ (SEQ ID NO:

117). In particular, the invention relates to a TNF ligand trimer-containing antigen binding molecule as defined above, wherein the first peptide linker is $(G_4S)_2$ (SEQ ID NO: 117), the second peptide linker is $(G_4S)_2$ (SEQ ID NO: 117), and the third peptide linker is $(G_4S)_2$ (SEQ ID NO: 117).

In another aspect, the TNF family ligand trimer-containing antigen binding molecule as defined herein before comprises an Fc domain composed of a first and a second subunit capable of stable association.

In particular, the TNF family ligand trimer-containing antigen binding molecule of the invention comprises (a) a Fab molecule capable of specific binding to PD1, wherein the Fab heavy chain is fused at the C-terminus to the N-terminus of a CH2 domain in the Fc domain and (c) an Fc domain composed of a first and a second subunit capable of stable association.

In a further aspect, the Fc domain is an IgG, particularly an IgG1 Fc domain or an IgG4 Fc domain. More particularly, the Fc domain is an IgG1 Fc domain. In a particular aspect, the Fc domain comprises a modification promoting the association of the first and second subunit of the Fc domain.

**Fc domain modifications reducing Fc receptor binding and/or effector function**

The Fc domain of the TNF family ligand trimer-containing antigen binding molecules of the invention consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

The Fc domain confers favorable pharmacokinetic properties to the antigen binding molecules of the invention, including a long serum half-life which contributes to good accumulation in the target tissue and a favorable tissue-blood distribution ratio. At the same time it may, however, lead to undesirable targeting of the bispecific antibodies of the invention to cells expressing Fc receptors rather than to the preferred antigen-bearing cells. Accordingly, in particular aspects, the Fc domain of the TNF family ligand trimer-containing antigen binding molecule of the invention exhibits reduced binding affinity to an Fc receptor and/or reduced effector function, as compared to a native IgG1 Fc domain. In one aspect, the Fc does not substantially bind to an Fc receptor and/or does not induce effector function. In a particular aspect the Fc receptor is an Fcγ receptor. In one aspect, the Fc receptor is a human Fc receptor. In a specific aspect, the Fc receptor is an activating human Fcγ receptor, more specifically human FcγRIIIa, FcγRI or FcγRIIa, most specifically human FcγRIIIa. In one aspect, the Fc domain does not induce effector function. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

In certain aspects, one or more amino acid modifications may be introduced into the Fc region of a TNF family ligand trimer-containing antigen binding molecule provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In a particular aspect, the invention provides a TNF family ligand trimer-containing antigen binding molecule comprising

(a) at least one moiety capable of specific binding to PD1,
(b) a first and a second polypeptide that are linked to each other by a disulfide bond,
wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof, and
(c) an Fc domain composed of a first and a second subunit capable of stable association, wherein the Fc domain comprises one or more amino acid substitution that reduces binding to an Fc receptor, in particular towards Fcγ receptor.

In one aspect, the Fc domain of the TNF family ligand trimer-containing antigen binding molecule of the invention comprises one or more amino acid mutation that reduces the binding affinity of the Fc domain to an Fc receptor and/or effector function. Typically, the same one or more amino acid mutation is present in each of the two subunits of the Fc domain. In particular, the Fc domain comprises an amino acid substitution at a position of E233, L234, L235, N297, P331 and P329 (EU numbering). In particular, the Fc domain comprises amino acid substitutions at positions 234 and 235 (EU numbering) and/or 329 (EU numbering) of the IgG heavy chains. More particularly, provided is a trimeric TNF family ligand-containing antigen binding molecule according to the invention which comprises an Fc domain with the amino acid substitutions L234A, L235A and P329G ("P329G LALA", EU numbering) in the IgG heavy chains. The amino acid substitutions L234A and L235A refer to the so-called LALA mutation. The "P329G LALA" combination of amino acid substitutions almost completely abolishes Fcγ receptor binding of a human IgG1 Fc domain and is described in International Patent Appl. Publ. No. WO 2012/130831 A1 which also describes methods of preparing such mutant Fc domains and methods for determining its properties such as Fc receptor binding or effector functions. "EU numbering" refers to the numbering according to EU index of Kabat et al , Sequences of Proteins of Immunological Interest, 5th Ed. Public Health Service, National Institutes of Health, Bethesda, MD, 1991.

Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In another aspect, the Fc domain is an IgG4 Fc domain. IgG4 antibodies exhibit reduced binding affinity to Fc receptors and reduced effector functions as compared to IgG1 antibodies. In a more specific aspect, the Fc domain is an IgG4 Fc domain comprising an amino acid substitution at position S228 (Kabat numbering), particularly the amino acid substitution S228P. In a more specific aspect, the Fc domain is an IgG4 Fc domain comprising amino acid substitutions L235E and S228P and P329G (EU numbering). Such IgG4 Fc domain mutants and their Fcγ receptor binding properties are also described in WO 2012/130831.

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or bispecific antibodies of the invention comprising an Fc domain, can be measured by methods known in the art. A suitable assay for measuring ADCC is described herein. Other examples of in vitro assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the bispecific antibodies of the invention is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

In a particular aspect, the Fc domain comprises a modification promoting the association of the first and second subunit of the Fc domain.

**Fc domain modifications promoting heterodimerization**

In one aspect, the TNF family ligand trimer-containing antigen binding molecules of the invention comprise (a) at least one moiety capable of specific binding to a PD1, (b) a first and a second polypeptide that are linked to each other by a disulfide bond, wherein the antigen binding molecule is characterized in that the first polypeptide comprises two ectodomains of a TNF ligand family member or two fragments thereof that are connected to each other by a peptide linker and in that the second polypeptide comprises only one ectodomain of said TNF ligand family member or a fragment thereof, and (c) an Fc domain composed of a first and a second subunit capable of stable association, wherein the Fc domain comprises one or more amino acid substitution that reduces binding to an Fc receptor, in particular towards Fcγ receptor. Thus, they comprise different moieties, fused to one or the other of the two subunits of the Fc domain that are typically comprised in two non-identical polypetide chains ("heavy chains"). Recombinant co-expression of these polypeptides and subsequent dimerization leads to several possible combinations of the two polypeptides. To improve the yield and purity of the TNF family ligand trimer-containing antigen binding molecules in recombinant production, it will thus be advantageous to introduce in the Fc domain of the TNF family ligand trimer-containing antigen binding molecules of the invention a modification promoting the association of the desired polypeptides.

Accordingly, the Fc domain of the TNF family ligand trimer-containing antigen binding molecules of the invention comprises a modification promoting the association of the first and the second subunit of the Fc domain. The site of most extensive protein-protein interaction between the two subunits of a human IgG Fc domain is in the CH3 domain of the Fc domain. Thus, said modification is particularly in the CH3 domain of the Fc domain.

In a specific aspect, said modification is a so-called "knob-into-hole" modification, comprising a "knob" modification in one of the two subunits of the Fc domain and a "hole" modification in the other one of the two subunits of the Fc domain. Thus, in a particular aspect, the invention relates to a TNF family ligand trimer-containing antigen binding molecule as described herein before which comprises an IgG molecule, wherein the Fc part of the first heavy chain comprises a first dimerization module and the Fc part of the second heavy chain comprises a second dimerization module allowing a heterodimerization of the two heavy chains of the IgG molecule and the first dimerization module comprises knobs and the second dimerization module comprises holes according to the knob into hole technology.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular aspect, in the CH3 domain of the first subunit of the Fc domain of the TNF family ligand trimer-containing antigen binding molecules of the invention an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). More particularly, in the second subunit of the Fc domain additionally the threonine residue at position 366 is replaced with a serine residue (T366S) and the leucine residue at position 368 is replaced with an alanine residue (L368A). More particularly, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). The introduction of these two cysteine residues results in the formation of a disulfide bridge between the two subunits of the Fc domain. The disulfide bridge further stabilizes the dimer (Carter, J Immunol Methods 248, 7-15 (2001)).

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

## Modifications in the CH1/CL domains

To further improve correct pairing, the TNF family ligand trimer-containing antigen binding molecules can contain different charged amino acid substitutions (so-called "charged residues"). These modifications are introduced in the crossed or non-crossed CH1 and CL domains. In a particular aspect, the invention relates to a TNF family ligand trimer-containing antigen binding molecule, wherein in one of CL domains the amino acid at position 123 (EU numbering) has been replaced by arginine (R) and the amino acid at position 124 (EU numbering) has been substituted by lysine (K) and wherein in one of the CH1 domains the the amino acids at position 147 (EU numbering) and at position 213 (EU numbering) have been substituted by glutamic acid (E).

More particularly, the invention relates to a TNF family ligand trimer-containing antigen binding molecule, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

Thus, in a particular aspect, provided is a TNF family ligand trimer-containing antigen binding molecule comprising

(a) at least one moiety capable of specific binding to PD1,
(b) a first polypeptide containing a CL domain comprising the amino acid mutations E123R and Q124K and a second polypeptide containing a CH1 domain comprising the amino acid mutations K147E and K213E, wherein the second polypeptide is linked to the first polypeptide by a disulfide bond between the CH1 and CL domain,

and wherein the antigen binding molecule is characterized in that the first polypeptide comprises two ectodomains of a TNF ligand family member or fragments thereof that are connected to each other and to the CL domain by a peptide linker and in that the second polypeptide comprises one ectodomain of said TNF ligand family member or a fragment thereof connected via a peptide linker to the CH1 domain of said polypeptide.

**Particular TNF family ligand trimer-containing antigen binding molecules**

In another aspect, the invention provides a TNF family ligand trimer-containing antigen binding molecule, wherein the antigen binding molecule comprises
a first heavy chain and a first light chain, both comprising a Fab molecule capable of specific binding to PD1,
a first peptide comprising two ectodomains of a TNF ligand family member or fragments thereof connected to each other by a first peptide linker fused at its C-terminus by a second peptide linker to a second heavy or light chain,
and a second peptide comprising one ectodomain of said TNF ligand family member fused at its C-terminus by a third peptide linker to a second light or heavy chain, respectively.

In a further aspect, provided is a TNF family ligand trimer-containing antigen binding molecule, wherein the first peptide comprising two ectodomains of a TNF ligand family member or fragments thereof connected to each other by a first peptide linker is fused at its C-terminus by a second peptide linker to a CH1 domain that is part of a heavy chain, and the second peptide comprising one ectodomain of said TNF ligand family member or a fragment thereof is fused at its C-terminus by a third peptide linker to a CL domain that is part of a light chain.

In yet another aspect, provided is a TNF family ligand trimer-containing antigen binding molecule, wherein the first peptide comprising two ectodomains of a TNF ligand family member or fragments thereof connected to each other by a first peptide linker is fused at its C-terminus by a second peptide linker to a CL domain that is part of a heavy chain, and the second peptide comprising one ectodomain of said TNF ligand family member or a fragment thereof that is fused at its C-terminus by a third peptide linker to a CH1 domain that is part of a light chain.

In one aspect, the invention provides a TNF family ligand trimer-containing antigen binding molecule, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E). These modifications lead to so-called charged residues with advantageaous properties that avoid undesired effects such as for example mispairing.

In particular, the CL domain comprises the amino acid mutations E123R and Q124K and the CH1 domain comprises the amino acid mutations K147E and K213E.

In one aspect, the invention provides a TNF family ligand trimer-containing antigen binding molecule as defined herein before, wherein the moiety capable of specific binding to PD1 is a scaffold antigen binding protein.

In a particular aspect, the invention is concerned with a TNF family ligand trimer-containing antigen binding molecule as defined above, wherein the moiety capable of specific binding to PD1 is a Fab molecule capable of specific binding to a target cell antigen.

The invention provides a TNF family ligand trimer-containing antigen binding molecule that comprises at least one moiety capable of specific binding to PD 1. In a particular aspect, the TNF family ligand trimer-containing antigen binding molecule comprises one moiety capable of specific binding to PD1, meaning the TNF family ligand trimer-containing antigen binding molecule is monovalent. In another aspect, the invention provides a TNF family ligand trimer-containing antigen binding molecule comprising two moieties capable of specific binding to PD1, meaning the TNF family ligand trimer-containing antigen binding molecule is bivalent.

In one aspect, the invention provides a TNF family ligand trimer-containing antigen binding molecule, wherein the moiety capable of specific binding to PD1 comprises

(a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16

(v) HVR-L2 comprising the amino acid sequence of SEQ ID NO: 17, and (vi) HVR-L3 comprising the amino acid sequence of SEQ ID NO:18,
(b) a VH domain comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28, and a VL domain comprising (iv) HVR-L1 comprising the amino acid sequence of SEQ ID NO:29 (v) HVR-L2 comprising the amino acid sequence of SEQ ID NO:30, and (vi) HVR-L3 comprising the amino acid sequence of SEQ ID NO:31,
(c) a VH domain comprising (i) HVR-H1 comprising the amino acid sequence of SEQ ID NO:34, (ii) HVR-H2 comprising the amino acid sequence of SEQ ID NO:35, and (iii) HVR-H3 comprising the amino acid sequence of SEQ ID NO:36, and a VL domain comprising (iv) HVR-L1 comprising the amino acid sequence of SEQ ID NO:37 (v) HVR-L2 comprising the amino acid sequence of SEQ ID NO:38, and (vi) HVR-L3 comprising the amino acid sequence of SEQ ID NO:39,
(d) a VH domain comprising (i) HVR-H1 comprising the amino acid sequence of SEQ ID NO:42, (ii) HVR-H2 comprising the amino acid sequence of SEQ ID NO:43, and (iii) HVR-H3 comprising the amino acid sequence of SEQ ID NO:44, and a VL domain comprising (iv) HVR-L1 comprising the amino acid sequence of SEQ ID NO:45 (v) HVR-L2 comprising the amino acid sequence of SEQ ID NO:46, and (vi) HVR-L3 comprising the amino acid sequence of SEQ ID NO:47,
(e) a VH domain comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52, and a VL domain comprising (iv) HVR-L1 comprising the amino acid sequence of SEQ ID NO:53 (v) HVR-L2 comprising the amino acid sequence of SEQ ID NO:54, and (vi) HVR-L3 comprising the amino acid sequence of SEQ ID NO:55,
(f) a VH domain comprising (i) HVR-H1 comprising the amino acid sequence of SEQ ID NO:58, (ii) HVR-H2 comprising the amino acid sequence of SEQ ID NO:59, and (iii) HVR-H3 comprising the amino acid sequence of SEQ ID NO:60, and a VL domain comprising (iv) HVR-L1 comprising the amino acid sequence of SEQ ID NO:61 (v) HVR-L2 comprising the amino acid sequence of SEQ ID NO:62, and (vi) HVR-L3 comprising the amino acid sequence of SEQ ID NO:63, or
(g) a VH domain comprising (i) HVR-H1 comprising the amino acid sequence of SEQ ID NO:66, (ii) HVR-H2 comprising the amino acid sequence of SEQ ID NO:67, and (iii) HVR-H3 comprising the amino acid sequence of SEQ ID NO:68, and a VL domain comprising (iv) HVR-L1 comprising the amino acid sequence of SEQ ID NO:69 (v) HVR-L2 comprising the amino acid sequence of SEQ ID NO:70, and (vi) HVR-L3 comprising the amino acid sequence of SEQ ID NO:71.

In a further aspect, the invention provides a a TNF family ligand trimer-containing antigen binding molecule, wherein the moiety capable of specific binding to PD 1 comprises

(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24,
(e) a VH domain comprising an amino acid sequence of SEQ ID NO:21 and a VL domain comprising an amino acid sequence of SEQ ID NO:25,
(f) a VH domain comprising an amino acid sequence of SEQ ID NO:32 and a VL domain comprising an amino acid sequence of SEQ ID NO:33,
(g) a VH domain comprising an amino acid sequence of SEQ ID NO:40 and a VL domain comprising an amino acid sequence of SEQ ID NO:41,
(h) a VH domain comprising an amino acid sequence of SEQ ID NO:48 and a VL domain comprising an amino acid sequence of SEQ ID NO:49,
(i) a VH domain comprising an amino acid sequence of SEQ ID NO:56 and a VL domain comprising an amino acid sequence of SEQ ID NO:57,
(k) a VH domain comprising an amino acid sequence of SEQ ID NO:64 and a VL domain comprising an amino acid sequence of SEQ ID NO:65, or
(l) a VH domain comprising an amino acid sequence of SEQ ID NO:72 and a VL domain comprising an amino acid sequence of SEQ ID NO:73.

In one aspect, the invention provides a TNF family ligand trimer-containing antigen binding molecule, wherein the moiety capable of specific binding to PD 1 comprises (a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO:18.

In a particular aspect, the invention provides a TNF family ligand trimer-containing antigen binding molecule, wherein the moiety capable of specific binding to PD1 comprises

(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

In a further aspect, the moiety capable of specific binding to PD1 comprises a heavy chain variable region comprising an amino acid sequence that is at least about 95%, 96%, 97%, 98%, 99% or 100% identical to the amino acid sequence of SEQ ID NO:21 and a light chain variable region comprising an amino acid sequence that is at least about 95%, 96%, 97%, 98%, 99% or 100% identical to the amino acid sequence of SEQ ID NO:22.

In another aspect, the moiety capable of specific binding to PD1 comprises a heavy chain variable region comprising an amino acid sequence that is at least about 95%, 96%, 97%, 98%, 99% or 100% identical to the amino acid sequence of SEQ ID NO:21 and a light chain variable region comprising an amino acid sequence that is at least about 95%, 96%, 97%, 98%, 99% or 100% identical to the amino acid sequence of SEQ ID NO:24.

In one aspect, provided is a TNF family ligand trimer-containing antigen binding molecule as defined herein before, wherein the moiety capable of specific binding to PD comprises a variable heavy chain comprising an amino acid sequence of SEQ ID NO:21 and a variable light chain comprising an amino acid sequence of SEQ ID NO:22 or wherein the moiety capable of specific binding to FAP comprises a variable heavy chain comprising an amino acid sequence of SEQ ID NO:21 and a variable light chain comprising an amino acid sequence of SEQ ID NO:24.

In a particular aspect, the moiety capable of specific binding to PD1 comprises a heavy chain variable region comprising the amino acid sequence of SEQ ID NO:21 and a light chain variable region comprising the amino acid sequence of SEQ ID NO:22. In another particular aspect, the moiety capable of specific binding to PD 1 comprises a heavy chain variable region comprising the amino acid sequence of SEQ ID NO:21 and a light chain variable region comprising the amino acid sequence of SEQ ID NO:24 In a specific aspect, the moiety capable of specific binding to PD 1 comprises a VH domain consisting of amino acid sequence of SEQ ID NO:21 and a VL domain consisting of the amino acid sequence of SEQ ID NO:22 or a VH domain consisting of amino acid sequence of SEQ ID NO:21 and a VL domain consisting of the amino acid sequence of SEQ ID NO:24.

In a further aspect, the TNF family ligand trimer-containing antigen binding molecule of the invention comprises

(a) at least one moiety capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a second heavy chain comprising an amino acid sequence selected from the group consisting of SEQ ID NO:9, SEQ ID NO: 11, SEQ ID NO:12 and SEQ ID NO: 10, and

a second light chain comprising an amino acid sequence selected from the group consisting of SEQ ID NO:1, SEQ ID NO:3, SEQ ID NO:4 and SEQ ID NO:5.

In a particular aspect, the TNF family ligand trimer-containing antigen binding molecule of the invention comprises

(a) at least one moiety capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a first and a second polypeptide that are linked to each other by a disulfide bond,

wherein the antigen binding molecule is characterized in that the first polypeptide comprises the the amino acid sequence of SEQ ID NO:10 and the second polypeptide comprises the amino acid sequence of SEQ ID NO:5.

In a further aspect, provided is a TNF family ligand trimer-containing antigen binding molecule as described herein before, wherein the antigen binding molecule comprises

(a) a first heavy chain and a first light chain, both comprising a Fab molecule capable of specific binding to PD1,
(b) a second heavy chain comprising an amino acid sequence selected from the group consisting of SEQ ID NO:9, SEQ ID NO: 11, SEQ ID NO:12 and SEQ ID NO: 10, and

a second light chain comprising an amino acid sequence selected from the group consisting of SEQ ID NO:1, SEQ ID NO:3, SEQ ID NO:4 and SEQ ID NO:5.

In one aspect, the invention provides a TNF family ligand trimer-containing antigen binding molecule, wherein the antigen binding molecule comprises

(a) a Fab molecule capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a second heavy chain comprising an amino acid sequence selected from the group consisting of SEQ ID NO:9, SEQ ID NO: 11, SEQ ID NO:12 and SEQ ID NO: 10, and

a second light chain comprising an amino acid sequence selected from the group consisting of SEQ ID NO:1, SEQ ID NO:3, SEQ ID NO:4 and SEQ ID NO:5.

In one aspect, the invention provides a TNF family ligand trimer-containing antigen binding molecule, wherein the antigen binding molecule comprises

(a) (i) a first heavy chain comprising the VH domain comprising the amino acid sequence of SEQ ID NO:19 and a first light chain comprising the VL domain comprising the amino acid sequence of SEQ ID NO:20 or
a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25, and
(b) a second heavy chain comprising an amino acid sequence selected from the group consisting of SEQ ID NO:9, SEQ ID NO:11, SEQ ID NO:12 and SEQ ID NO:10, and

a second light chain comprising an amino acid sequence selected from the group consisting of SEQ ID NO:1, SEQ ID NO:3, SEQ ID NO:4 and SEQ ID NO:5.

In a particular aspect, provided is a TNF family ligand trimer-containing antigen binding molecule, wherein the antigen binding molecule comprises

(i) a first heavy chain comprising the VH domain comprising the amino acid sequence of SEQ ID NO:19 and a first light chain comprising the VL domain comprising the amino acid sequence of SEQ ID NO:20 or
a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
(ii) a second heavy chain comprising the amino acid sequence selected from the group consisting of SEQ ID NO:74, SEQ ID NO:76, SEQ ID NO:78 and SEQ ID NO:80, and
(iii) a second light chain comprising the amino acid sequence selected from the group consisting of SEQ ID NO:75, SEQ ID NO:77, SEQ ID NO:79 and SEQ ID NO:81..

In another aspect, the invention provides a TNF family ligand trimer-containing antigen binding molecule, wherein the antigen binding molecule comprises

(a) a first heavy chain comprising the amino acid sequence of SEQ ID NO:82, a first light chain comprising the amino acid sequence of SEQ ID NO:83, a second heavy chain comprising the amino acid sequence of SEQ ID NO:74 and a second light chain comprising the amino acid sequence of SEQ ID NO:75,

(b) a first heavy chain comprising the amino acid sequence of SEQ ID NO:82, a first light chain comprising the amino acid sequence of SEQ ID NO:83, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:83, a second heavy chain comprising the amino acid sequence of SEQ ID NO:80 and a second light chain comprising the amino acid sequence of SEQ ID NO:81.

In particular, provided is a TNF family ligand trimer-containing antigen binding molecule, wherein the antigen binding molecule comprises a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79.
In yet another aspect, the invention provides a TNF family ligand trimer-containing antigen binding molecule, comprising

(a) at least one moiety capable of specific binding to PD1, and
(b) a first and a second polypeptide that are linked to each other by a disulfide bond,

wherein the antigen binding molecule is characterized in that the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises one ectodomain of said TNF ligand family member or a fragment thereof connected via a peptide linker to C-terminus of the CH3 domain of said polypeptide.
In particular, such a TNF family ligand trimer-containing antigen binding molecule comprises two Fab domains capable of specific binding to PD 1.
In particular, provided is a TNF family ligand trimer-containing antigen binding molecule as described herein before comprises

(a) a first heavy chain comprising the amino acid sequence of SEQ ID NO:84, a second heavy chain comprising the amino acid sequence of SEQ ID NO:85, and two light chains comprising the amino acid sequence of SEQ ID NO:83, or
(b) a first heavy chain comprising the amino acid sequence of SEQ ID NO:86, a second heavy chain comprising the amino acid sequence of SEQ ID NO:87, and two light chains comprising the amino acid sequence of SEQ ID NO:83.

In another particular aspect, the invention provides a TNF family ligand trimer-containing antigen binding molecule as described herein before, wherein the TNF family ligand trimer-containing antigen binding molecule comprises one Fab domain capable of specific binding to PD 1.
In such aspect, provided is a TNF family ligand trimer-containing antigen binding molecule, wherein the antigen binding molecule comprises

(i) a fusion polypeptide comprising a first subunit of a Fc domain and two ectodomains of a TNF ligand family member or fragments thereof that are connected to each other and to the C-terminus of the CH3 domain by a peptide linker, (ii) a heavy chain comprising the VH domain of the Fab domain capable of specific binding to PD1, a second subunit of the Fc domain and one ectodomain of said TNF ligand family member or a fragment thereof connected via a peptide linker to C-terminus of the CH3 domain, and (iii) a light chain comprising the VL domain of the Fab domain capable of specific binding to PD1, or
(i) a fusion polypeptide comprising a first subunit of a Fc domain and one ectodomain of a TNF ligand family member or a fragment thereof that is connected to the C-terminus of the CH3 domain by a peptide linker, (ii) a heavy chain comprising the VH domain of the Fab domain capable of specific binding to PD1, a second subunit of the Fc domain and two ectodomains of said TNF ligand family member or fragments thereof that are connected to each other and to the C-terminus of the CH3 domain via a peptide linker, and (iii) a light chain comprising the VL domain of the Fab domain capable of specific binding to PD 1.

In a particular aspect, provided is a TNF family ligand trimer-containing antigen binding molecule, wherein the antigen binding molecule comprises

(a) a fusion polypeptide comprising the amino acid sequence of SEQ ID NO:88, a heavy chain comprising the amino acid sequence of SEQ ID NO:87 and a light chain comprising the amino acid sequence of SEQ ID NO:83,
(b) a fusion polypeptide comprising the amino acid sequence of SEQ ID NO:89, a heavy chain comprising the amino acid sequence of SEQ ID NO:90 and a light chain comprising the amino acid sequence of SEQ ID NO:83,
(c) a fusion polypeptide comprising the amino acid sequence of SEQ ID NO:91, a heavy chain comprising the amino acid sequence of SEQ ID NO:86 and a light chain comprising the amino acid sequence of SEQ ID NO:83,
(b) a fusion polypeptide comprising the amino acid sequence of SEQ ID NO:93, a heavy chain comprising the amino acid sequence of SEQ ID NO:92 and a light chain comprising the amino acid sequence of SEQ ID NO:83.

**Polynucleotides**

The invention further provides isolated polynucleotides encoding a TNF family ligand trimer-containing antigen binding molecule as described herein or a fragment thereof.

The isolated polynucleotides encoding TNF ligand trimer-containing antigen binding molecules of the invention may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes the entire TNF family ligand trimer-containing antigen binding molecule according to the invention as described herein. In parrticular, the isolated polynucleotide encodes a polypeptide comprised in the TNF family ligand trimer-containing antigen binding molecule according to the invention as described herein.

In one aspect, the present invention is directed to isolated polynucleotides encoding a TNF family ligand trimer-containing antigen binding molecule, wherein the polynucleotide comprises (a) a sequence that encodes a moiety capable of specific binding to a PD1, (b) a sequence that encodes a polypeptide comprising two ectodomains of a TNF ligand family member or two fragments thereof that are connected to each other by a peptide linker and (c) a sequence that encodes a polypeptide comprising one ectodomain of said TNF ligand family member or a fragment thereof.

In another aspect, provided is an isolated polynucleotide encoding a 4-1BB ligand trimer-containing antigen binding molecule, wherein the polynucleotide comprises (a) a sequence that encodes a moiety capable of specific binding to PD1, (b) a sequence that encodes a polypeptide comprising two ectodomains of 4-1BBL or two fragments thereof that are connected to each other by a peptide linker and (c) a sequence that encodes a polypeptide comprising one ectodomain of 4-1BBL or a fragment thereof.

In a further aspect, the invention is directed to an isolated polynucleotide comprising a sequence that encodes a polypeptide comprising two 4-1BBL fragments comprising an amino acid sequence that is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 or SEQ ID NO:8, and to a polynucleotide comprising a sequence that encodes a polypeptide comprising one 4-1BBL fragment comprising an amino acid sequence that is is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 or SEQ ID NO: 8.

Furthermore, provided is an isolated polynucleotide encoding a OX40 ligand trimer-containing antigen binding molecule, wherein the polynucleotide comprises (a) a sequence that encodes a moiety capable of specific binding to PD1, (b) a sequence that encodes a polypeptide comprising two ectodomains of OX40L or two fragments thereof that are connected to each other by a peptide linker and (c) a sequence that encodes a polypeptide comprising one ectodomain of OX40L or a fragment thereof.

In further aspects, the invention relates to the polynucleotides comprising a sequence that is at least about 90%, 95%, 98% or 100% identical to the specific cDNA sequences disclosed herein. In a particular aspect, the invention relates to a polynucleotide comprising a sequence that is identical to one of the specific cDNA sequences disclosed herein.

In other aspects, the nucleic acid molecule comprises or consists of a nucleotide sequence that encodes an amino acid sequence as set forth in any one of SEQ ID NOs: 19, 20, 21, 22, 23, 24, 25, 32, 33, 40, 41, 48, 49, 56, 57, 64, 65, 72 or 73. In a further aspect, the nucleic acid molecule comprises or consists of a nucleotide sequence that encodes an amino acid sequence as set forth in any one of SEQ ID NOs:9, 10, 11 or 12.

In still other aspects, the nucleic acid molecule comprises or consists of a nucleotide sequence selected from the group consisting of SEQ ID NOs: 131, 132, 133, 134, 135, 136, 137, 138, 139, 140, 141, 142, 143, 144, 145, 146, 147, 148, 149 or 150.

In certain aspects, the polynucleotide or nucleic acid is DNA. In other embodiments, a polynucleotide of the present invention is RNA, for example, in the form of messenger RNA (mRNA). RNA of the present invention may be single stranded or double stranded.

**Recombinant Methods**

TNF family ligand trimer-containing antigen binding molecules of the invention may be obtained, for example, by solid-state peptide synthesis (e.g. Merrifield solid phase synthesis) or recombinant production. For recombinant production one or more polynucleotide encoding the TNF family ligand trimer-containing antigen binding molecule or polypeptide fragments thereof, e.g., as described above, is isolated and inserted into one or more vectors for further cloning and/or expression in a host cell. Such polynucleotide may be readily isolated and sequenced using conventional procedures. In one aspect of the invention, a vector, preferably an expression vector, comprising one or more of the polynucleotides of the invention is provided. Methods which are well known to those skilled in the art can be used to construct expression vectors containing the coding sequence of the TNF family ligand trimer-containing antigen binding molecule (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the TNF family ligand trimer-containing antigen binding molecule or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the TNF family ligand trimer-containing antigen binding molecule of the invention or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the TNF family ligand trimer-containing antigen binding molecule or polypeptide fragments thereof is desired, DNA encoding a signal sequence may be placed upstream of the nucleic acid encoding a TNF family ligand trimer-containing antigen binding molecule of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a TNF family ligand trimer-containing antigen binding molecule of the invention or polypeptide fragments thereof.

In a further aspect of the invention, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) a TNF family ligand trimer-containing antigen binding molecule of the invention of the invention. As used herein, the term "host cell" refers to any kind of cellular system which can be engineered to generate the fusion proteins of the invention or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In one aspect, a method of producing a TNF family ligand trimer-containing antigen binding molecule of the invention or polypeptide fragments thereof is provided, wherein the method comprises culturing a host cell comprising polynucleotides encoding the TNF family ligand trimer-containing antigen binding molecule of the invention or polypeptide fragments thereof, as provided herein, under conditions suitable for expression of the TNF family ligand trimer-containing antigen binding molecule of the invention or polypeptide fragments thereof, and recovering the TNF family ligand trimer-containing antigen binding molecule of the invention or polypeptide fragments thereof from the host cell (or host cell culture medium).

In the TNF family ligand trimer-containing antigen binding molecule of the invention, the components (at least one moiety capable of specific binding to a target cell antigen, one polypeptide comprising two ectodomains of a TNF ligand family member or fragments thereof and a polypeptide comprising one ectodomain of said TNF family ligand family member or a fragment thereof) are not genetically fused to each other. The polypeptides are designed such that its components (two ectodomains of a TNF ligand family member or fragments thereof and other components such as CH or CL) are fused to each other directly or through a linker sequence. The composition and length of the linker may be determined in accordance with methods well known in the art and may be tested for efficacy. Examples of linker sequences between different components of the antigen binding molecules of the invention are found in the sequences provided herein. Additional sequences may also be included to incorporate a cleavage site to separate the individual components of the fusion protein if desired, for example an endopeptidase recognition sequence.

In certain embodiments the moieties capable of specific binding to a target cell antigen (e.g. Fab fragments) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used in the invention. Non-limiting immunoglobulins useful in the present invention can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins according to the invention are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the moieties capable of specific binding to a target cell antigen (e.g. Fab fragments) comprised in the antigen binding molecules of the present invention are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules of the invention to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain embodiments, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch.14 (Cold Spring Harbor Laboratory, Cold Spring Harbor, NY).

TNF ligand trimer-containing antigen binding molecules of the invention prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the TNF ligand trimer-containing antigen binding molecule binds. For example, for affinity chromatography purification of fusion proteins of the invention, a matrix with protein A or protein G may be used. Sequential Protein A or G affinity chromatography and size exclusion chromatography can be used to isolate an antigen binding molecule essentially as described in the Examples. The purity of the TNF ligand trimer-containing antigen binding molecule or fragments thereof can be determined by any of a variety of well-known analytical methods including gel electrophoresis, high pressure liquid chromatography, and the like. For example, the TNF ligand trimer-containing antigen binding molecules expressed as described in the Examples were shown to be intact and properly assembled as demonstrated by reducing and non-reducing SDS-PAGE.

## Assays

The antigen binding molecules provided herein may be identified, screened for, or characterized for their physical/chemical properties and/or biological activities by various assays known in the art. Biological activity may include, e.g., the ability to enhance the activation and/or proliferation of different immune cells especially T-cells. E.g. they enhance secretion of immunomodulating cytokines (e.g. interferon-gamma (IFN-gamma) and/or tumor necrosis factor alpha (TNF alpha)). Other immunomodulating cytokines which are or can be enhanced are e.g IL12, Granzyme B etc. Biological activity may also include, cynomolgus binding crossreactivity, as well as binding to different cell types. Antigen binding molecules having such biological activity in vivo and/or in vitro are also provided.

### 1. Affinity assays

The affinity of the TNF family ligand trimer-containing antigen binding molecule provided herein for the corresponding TNF receptor can be determined in accordance with the methods set forth in the Examples by surface plasmon resonance (SPR), using standard instrumentation such as a BIAcore instrument (GE Healthcare), and receptors or target proteins such as may be obtained by recombinant expression. The affinity of the TNF family ligand trimer-containing antigen binding molecule for PD1 can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 4. According to one aspect, $K_d$ is measured by surface plasmon resonance using a BIACORE® T100 machine (GE Healthcare) at 25 °C.

### 2. Binding assays and other assays

Binding of the TNF family ligand trimer-containing antigen binding molecule provided herein to the corresponding receptor expressing cells may be evaluated using cell lines expressing the particular receptor or target antigen, for example by flow cytometry (FACS). In one aspect, fresh peripheral blood mononuclear cells (PBMCs) expressing the TNF receptor are used in the binding assay. These cells are used directly after isolation (naive PMBCs) or after stimulation (activated PMBCs). In another aspect, activated mouse splenocytes (expressing the TNF receptor molecule) were used to demonstrate the binding of the TNF family ligand trimer-containing antigen binding molecule of the invention to the corresponding TNF receptor expressing cells.

In a further aspect, cell lines expressing PD1 were used to demonstrate the binding of the antigen binding molecules to this target cell antigen.

In another aspect, competition assays may be used to identify an antigen binding molecule that competes with a specific antibody or antigen binding molecule for binding to the target or TNF receptor, respectively. In certain embodiments, such a competing antigen binding molecule binds to the same epitope (e.g., a linear or a conformational epitope) that is bound by a specific anti-target antibody or a specific anti-TNF receptor antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

### 3. Activity assays

In one aspect, assays are provided for identifying TNF family ligand trimer-containing antigen binding molecules that bind to a specific target cell antigen and to a specific TNF receptor having biological activity. Biological activity may include, e.g., agonistic signalling through the TNF receptor on cells expressing the target cell antigen. TNF family ligand trimer-containing antigen binding molecules identified by the assays as having such biological activity in vitro are also provided.

In certain aspects, a TNF family ligand trimer-containing antigen binding molecule of the invention is tested for such biological activity. Assays for detecting the biological activity of the molecules of the invention are those described in Examples 5 and 6. Furthermore, assays for detecting cell lysis (e.g. by measurement of LDH release), induced apoptosis kinetics (e.g. by measurement of Caspase 3/7 activity) or apoptosis (e.g. using the TUNEL assay) are well known in the art. In addition the biological activity of such complexes can be assessed by evaluating their effects on survival, proliferation and lymphokine secretion of various lymphocyte subsets such as NK cells, NKT-cells or $\gamma\delta$ T-cells or assessing their capacity to modulate phenotype and function of antigen presenting cells such as dendritic cells, monocytes/macrophages or B-cells.

### Pharmaceutical Compositions, Formulations and Routes of Administation

In a further aspect, the invention provides pharmaceutical compositions comprising any of the TNF family ligand trimer-containing antigen binding molecules provided herein, e.g., for use in any of the below therapeutic methods. In one embodiment, a pharmaceutical composition comprises any of the TNF family ligand trimer-containing antigen binding molecules provided herein and at least one pharmaceutically acceptable excipient. In another embodiment, a pharmaceutical composition comprises any of the TNF family ligand trimer-containing antigen binding molecules provided herein and at least one additional therapeutic agent, e.g., as described below.

Pharmaceutical compositions of the present invention comprise a therapeutically effective amount of one or more TNF family ligand trimer-containing antigen *binding* molecules dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one TNF family ligand trimer-containing antigen binding molecule and optionally an additional active ingredient will be known to those of skill in the art in light of the present disclosure, as exemplified by Remington's Pharmaceutical Sciences, 18th Ed. Mack Printing Company, 1990, incorporated herein by reference. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the TNF family ligand trimer-containing antigen binding molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the fusion proteins may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the fusion proteins of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The TNF family ligand trimer-containing antigen binding molecules may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides;

or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

**Therapeutic methods and compositions**

Any of the TNF family ligand trimer-containing antigen binding molecules provided herein may be used in therapeutic methods.

For use in therapeutic methods, TNF family ligand trimer-containing antigen binding molecules of the invention can be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, TNF family ligand trimer-containing antigen binding molecules of the invention for use as a medicament are provided. In further aspects, TNF family ligand trimer-containing antigen binding molecules of the invention for use in treating a disease, in particular for use in the treatment of cancer, are provided. In certain aspects, TNF family ligand trimer-containing antigen binding molecules of the invention for use in a method of treatment are provided. In one aspect, the invention provides a TNF family ligand trimer-containing antigen binding molecule as described herein for use in the treatment of a disease in an individual in need thereof. In certain aspects, the invention provides a TNF family ligand trimer-containing antigen binding molecule for use in a method of treating an individual having a disease comprising administering to the individual a therapeutically effective amount of the fusion protein. In certain aspects, the disease to be treated is cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia. Thus, a TNF family ligand trimer-containing antigen binding molecule as described herein for use in the treatment of cancer is provided. The subject, patient, or "individual" in need of treatment is typically a mammal, more specifically a human.

In another aspect, provided is a TNF family ligand trimer-containing antigen binding molecule as described herein for use in the treatment of infectious diseases, in particular for the treatment of viral infections. In a further aspect, provided is a TNF family ligand trimer-containing antigen binding molecule as described herein for use in the treatment of autoimmune diseases such as for example Lupus disease.

In a further aspect, the invention relates to the use of a TNF family ligand trimer-containing antigen binding molecule in the manufacture or preparation of a medicament for the treatment of a disease in an individual in need thereof. In one aspect, the medicament is for use in a method of treating a disease comprising administering to an individual having the disease a therapeutically effective amount of the medicament. In certain embodiments the disease to be treated is a proliferative disorder, particularly cancer. Thus, in one aspect, the invention relates to the use of a TNF family ligand trimer-containing antigen binding molecule of the invention in the manufacture or preparation of a medicament for the treatment of cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia.. Other cell proliferation disorders that can be treated using a TNF family ligand trimer-containing antigen binding molecule of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan may recognize that in some cases the TNF family ligand trimer-containing antigen binding molecule may not provide a cure but may only provide partial benefit. In some aspects, a physiological change having some benefit is also considered therapeutically beneficial. Thus, in some aspects, an amount of TNF family ligand trimer-containing antigen binding molecule that provides a physiological change is considered an "effective amount" or a "therapeutically effective amount".

In a further aspect, the invention relates to the use of a TNF family ligand trimer-containing antigen binding molecule as described herein in the manufacture or preparation of a medicament for the treatment of infectious diseases, in particular for the treatment of viral infections or for the treatment of autoimmune diseases, for example Lupus disease.

In a further aspect, the invention provides a method for treating a disease in an individual, comprising administering to said individual a therapeutically effective amount of a TNF family ligand trimer-containing antigen binding molecule of the invention. In one aspect a composition is administered to said individual, comprising a fusion protein of the invention in a pharmaceutically acceptable form. In certain aspects, the disease to be treated is a proliferative disorder. In a particular aspect, the disease is cancer. In another aspect, the disease is an infectious disease or an autoimmune disease. In certain aspects, the method further comprises administering to the individual a therapeutically effective amount of at least one additional therapeutic agent, e.g. an anti-cancer agent if the disease to be treated is cancer. An "individual" according to any of the above embodiments may be a mammal, preferably a human.

For the prevention or treatment of disease, the appropriate dosage of a TNF family ligand trimer-containing antigen binding molecule of the invention (when used alone or in combination with one or more other additional therapeutic agents) will depend on the type of disease to be treated, the route of administration, the body weight of the patient, the type of antigen binding molecule, the severity and course of the disease, whether the fusion protein is administered for preventive or therapeutic purposes, previous or concurrent therapeutic interventions, the patient's clinical history and response to the fusion protein, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The TNF family ligand trimer-containing antigen binding molecule is suitably administered to the patient at one time or over a series of treatments. Depending on the type and severity of the disease, about 1 μg/kg to 15 mg/kg (e.g. 0.1 mg/kg - 10 mg/kg) of TNF family ligand trimer-containing antigen binding molecule can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the fusion protein would be in the range from about 0.005 mg/kg to about 10 mg/kg. In other examples, a dose may also comprise from about 1 μg/kg body weight, about 5 μg/kg body weight, about 10 μg/kg body weight, about 50 μg/kg body weight, about 100 μg/kg body weight, about 200 μg/kg body weight, about 350 μg/kg body weight, about 500 μg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg body weight or more per administration, and any range derivable therein. In examples of a derivable range from the numbers listed herein, a range of about 5 mg/kg body weight to about 100 mg/kg body weight, about 5 μg/kg body weight to about 500 mg/kg body weight etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the fusion protein). An initial higher loading dose, followed by one or more lower doses may be administered. However, other dosage regimens may be useful. The progress of this therapy is easily monitored by conventional techniques and assays.

The TNF family ligand trimer-containing antigen binding molecules of the invention will generally be used in an amount effective to achieve the intended purpose. For use to treat or prevent a disease condition, the TNF family ligand trimer-containing antigen binding molecules of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from in vitro assays, such as cell culture assays. A dose can then be formulated in animal models to achieve a circulating concentration range that includes the $IC_{50}$ as determined in cell culture. Such information can be used to more accurately determine useful doses in humans.

Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the TNF family ligand trimer-containing antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the TNF family ligand trimer-containing antigen binding molecule may not be related to plasma concentration. One skilled in the art will be able to optimize therapeutically effective local dosages without undue experimentation.

A therapeutically effective dose of the TNF family ligand trimer-containing antigen binding molecules described herein will generally provide therapeutic benefit without causing substantial toxicity. Toxicity and therapeutic efficacy of a fusion protein can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. TNF family ligand trimer-containing antigen binding molecules that exhibit large therapeutic indices are preferred. In one embodiment, the TNF family ligand trimer-containing antigen binding molecule according to the present invention exhibits a high therapeutic index. The data obtained from cell culture assays and animal studies can be used in formulating a range of dosages suitable for use in humans. The dosage lies preferably within a range of circulating concentrations that include the $ED_{50}$ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1, incorporated herein by reference in its entirety).

The attending physician for patients treated with fusion proteins of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

**Other agents and treatments**

The TNF family ligand trimer-containing antigen binding molecules of the invention may be administered in combination with one or more other agents in therapy. For instance, a fusion protein of the invention may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is another anti-cancer agent.

Such other agents are suitably present in combination in amounts that are effective for the purpose intended. The effective amount of such other agents depends on the amount of fusion protein used, the type of disorder or treatment, and other factors discussed above. The TNF family ligand trimer-containing antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the TNF family ligand trimer-containing antigen binding molecule of the invention can occur prior to, simultaneously, and/or following, administration of the additional therapeutic agent and/or adjuvant.

**Articles of Manufacture**

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is a TNF ligand trimer-containing antigen binding molecule of the invention.

The label or package insert indicates that the composition is used for treating the condition of choice. Moreover, the article of manufacture may comprise (a) a first container with a composition contained therein, wherein the composition comprises a TNF ligand trimer-containing antigen binding molecule of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQ ID NO: | Description | Sequence |
|---|---|---|
| 1 | Human (hu) 4-1BBL (71-254) | REGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 2 | hu 4-1BBL (85-254) | LDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 3 | hu 4-1BBL (80-254) | DPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 4 | hu 4-1BBL (52-254) | PWAVSGARASPGSAASPRLREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 5 | Human (hu) 4-1BBL (71-248) | REGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 6 | hu 4-1BBL (85-248) | LDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 7 | hu 4-1BBL (80-248) | DPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |

(continued)

| SEQ ID NO: | Description | Sequence |
|---|---|---|
| 8 | hu 4-1BBL (52-248) | PWAVSGARASPGSAASPRLREGPELSPDDP AGLLDLRQGMFAQLVAQNVLLIDGPLSW YSDPGLAGVSLTGGLSYKEDTKELVVAKA |
| | | GVYYVFFQLELRRVVAGEGSGSVSLALHL QPLRSAAGAAALALTVDLPPASSEARNSA FGFQGRLLHLSAGQRLGVHLHTEARARHA WQLTQGATVLGLFRVTPEIPAGL |
| 9 | dimeric hu 4-1BBL (71-254) connected by $(G4S)_2$ linker | REGPELSPDDPAGLLDLRQGMFAQLVAQN VLLIDGPLSWYSDPGLAGVSLTGGLSYKE DTKELVVAKAGVYYVFFQLELRRVVAGE GSGSVSLALHLQPLRSAAGAAALALTVDL PPASSEARNSAFGFQGRLLHLSAGQRLGV HLHTEARARHAWQLTQGATVLGLFRVTP EIPAGLPSPRSEGGGGSGGGGSREGPELSP DDPAGLLDLRQGMFAQLVAQNVLLIDGPL SWYSDPGLAGVSLTGGLSYKEDTKELVVA KAGVYYVFFQLELRRVVAGEGSGSVSLAL HLQPLRSAAGAAALALTVDLPPASSEARN SAFGFQGRLLHLSAGQRLGVHLHTEARAR HAWQLTQGATVLGLFRVTPEIPAGLPSPRS E |
| 10 | dimeric hu 4-1BBL (71-248) connected by $(G4S)_2$ linker | REGPELSPDDPAGLLDLRQGMFAQLVAQN VLLIDGPLSWYSDPGLAGVSLTGGLSYKE DTKELVVAKAGVYYVFFQLELRRVVAGE GSGSVSLALHLQPLRSAAGAAALALTVDL PPASSEARNSAFGFQGRLLHLSAGQRLGV HLHTEARARHAWQLTQGATVLGLFRVTP EIPAGLGGGGSGGGGSREGPELSPDDPAGL LDLRQGMFAQLVAQNVLLIDGPLSWYSDP GLAGVSLTGGLSYKEDTKELVVAKAGVY YVFFQLELRRVVAGEGSGSVSLALHLQPL RSAAGAAALALTVDLPPASSEARNSAFGF QGRLLHLSAGQRLGVHLHTEARARHAWQ LTQGATVLGLFRVTPEIPAGL |
| 11 | dimeric hu 4-1BBL (80-254) connected by $(G4S)_2$ linker | DPAGLLDLRQGMFAQLVAQNVLLIDGPLS WYSDPGLAGVSLTGGLSYKEDTKELVVA KAGVYYVFFQLELRRVVAGEGSGSVSLAL HLQPLRSAAGAAALALTVDLPPASSEARN SAFGFQGRLLHLSAGQRLGVHLHTEARAR HAWQLTQGATVLGLFRVTPEIPAGLPSPRS EGGGGSGGGGSDPAGLLDLRQGMFAQLV AQNVLLIDGPLSWYSDPGLAGVSLTGGLS YKEDTKELVVAKAGVYYVFFQLELRRVV AGEGSGSVSLALHLQPLRSAAGAAALALT VDLPPASSEARNSAFGFQGRLLHLSAGQR LGVHLHTEARARHAWQLTQGATVLGLFR VTPEIPAGLPSPRSE |

(continued)

| SEQ ID NO: | Description | Sequence |
|---|---|---|
| 12 | dimeric hu 4-1BBL (52-254) connected by $(G4S)_2$ linker | PWAVSGARASPGSAASPRLREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSEGGGGSGGGGSPWAVSGARASPGSAASPRLREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 13 | heavy chain HVR-H1, PD1-0103 | GFSFSSY |
| 14 | heavy chain HVR-H2, PD1-0103 | GGR |
| 15 | heavy chain HVR-H3, PD1-0103 | TGRVYFALD |
| 16 | light chain HVR-L1, PD1-0103 | SESVDTSDNSF |
| 17 | light chain HVR-L2, PD1-0103 | RSS |
| 18 | light chain HVR-L3, PD1-0103 | NYDVPW |
| 19 | heavy chain variable domain VH, PD1-0103 | EVILVESGGGLVKPGGSLKLSCAASGFSFSSYTMSWVRQTPEKRLDWVATISGGGRDIYYPDSVKGRFTISRDNAKNTLYLEMSSLMSEDTALYYCVLLTGRVYFALDSWGQGTSVTVSS |
| 20 | light chain variable domain VL, PD1-0103 | KIVLTQSPASLPVSLGQRATISCRASESVDTSDNSFIHWYQQRPGQSPKLLIYRSSTLESGVPARFSGSGSRTDFTLTIDPVEADDVATYYCQQNYDVPWTFGGGTKLEIK |
| 21 | humanized variant -heavy chain variable domain VH of PD1-0103-0312, -0313,-0314 and -0315 | EVQLLESGGGLVQPGGSLRLSCAASGFSFSSYTMSWVRQAPGKGLEWVATISGGGRDIYYPDSVKGRFTISRDNSKNTLYLQMNSLRAEDTAVYYCVLLTGRVYFALDSWGQGTLVTVSS |
| 22 | humanized variant -light chain variable domain VL of PD1-0103-0312 | DIVMTQSPDSLAVSLGERATINCKASESVDTSDNSFIHWYQQKPGQSPKLLIYRSSTLESGVPDRFSGSGSGTDFTLTISSLQAEDVAVYYCQQNYDVPWTFGQGTKVEIK |
| 23 | humanized variant -light chain variable domain VL of PD1-0103-0313 | DVVMTQSPLSLPVTLGQPASISCRASESVDTSDNSFIHWYQQRPGQSPRLLIYRSSTLESGVPDRFSGSGSGTDFTLKISRVEAEDVGVYYCQQNYDVPWTFGQGTKVEIK |

(continued)

| SEQ ID NO: | Description | Sequence |
|---|---|---|
| 24 | humanized variant -light chain variable domain VL of PD1-0103-0314 | EIVLTQSPATLSLSPGERATLSCRASESVDTSDNSFIHWYQQKPGQSPRLLIYRSSTLESGIPARFSGSGSGTDFTLTISSLEPEDFAVYYCQQNYDVPWTFGQGTKVEIK |
| 25 | humanized variant -light chain variable domain VL of PD1-0103-0315 | EIVLTQSPATLSLSPGERATLSCRASESVDTSDNSFIHWYQQKPGQSPRLLIYRSSTLESGIPARFSGSGSGTDFTLTISSLEPEDFAVYYCQQNYDVPWTFGQGTKVEIK |
| 26 | heavy chain HVR-H1, PD1-0098 | GYSITSDY |
| 27 | heavy chain HVR-H2, PD1-0098 | YSG |
| 28 | heavy chain HVR-H3, PD1-0098 | HGSAPWYFD |
| 29 | light chain HVR-L1, PD1-0098 | SQNIVHSDGNTY |
| 30 | light chain HVR-L2, PD1-0098 | KVS |
| 31 | light chain HVR-L3, PD1-0098 | GSHFPL |
| 32 | heavy chain variable domain VH, PD1-0098 | DVQLQESGPGLVKPSQSLSLTCTVTGYSITSDYAWNWIRQFPGDKLEWLGYITYSGFTNYNPSLKSRISISRDTSKNQFFLQLNSVATED |
| | | TATYYCARWHGSAPWYFDYWGRGTTLTVSS |
| 33 | light chain variable domain VL, PD1-0098 | DVLMTQTPLSLPVSLGDQASISCRSSQNIVHSDGNTYLEWYLQKPGQSPNLLIYKVSRRFSGVPDRFSGSGSGTDFTLKISRVEAEDLGVYYCFQGSHFPLTFGAGTKLELK |
| 34 | heavy chain HVR-H1, PD1-0050 | GYSITSDY |
| 35 | heavy chain HVR-H2, PD1-0050 | YTG |
| 36 | heavy chain HVR-H3, PD1-0050 | MDYYGSTLD |
| 37 | light chain HVR-L1, PD1-0050 | SESVDRYGNSF |
| 38 | light chain HVR-L2, PD1-0050 | RAS |
| 39 | light chain HVR-L3, PD1-0050 | NNEDPY |
| 40 | heavy chain variable domain VH, PD1-0050 | DVQLQESGPGLVKPSQSLSLTCTVT**GYSITSDY**AWNWIRQFPGNKLEWMGYIT**YTG**RTSYNPSLKSRISITRDTSKNQFFLQLNSVTTEDTATYYCARE**MDYYGSTLD**YWGQGTTLTVSS |
| 41 | light chain variable domain VL, PD1-0050 | KIVLTQSPASLAVSLRQRATISCRA**SESVDRYGNSF**IHWYQQKPGQPPKVLIY**RAS**NLESGFPARFSGSGSRTDFTLTIDPVEADDAATYYCQQ**NNEDPY**TFGSGTKLEIK |
| 42 | heavy chain HVR-H1, PD1-0069 | GYTFTDY |
| 43 | heavy chain HVR-H2, PD1-0069 | YSG |
| 44 | heavy chain HVR-H3, PD1-0069 | GITTGFA |

(continued)

| SEQ ID NO: | Description | Sequence |
|---|---|---|
| 45 | light chain HVR-L1, PD1-0069 | SKGVSTSSYSF |
| 46 | light chain HVR-L2, PD 1-0069 | YAS |
| 47 | light chain HVR-L3, PD 1-0069 | SREFPW |
| 48 | heavy chain variable domain VH, PD1-0069 | QVQLQQSGPELVRPGVSVKISCKGS**GYTFTDY**AMHWVKQSHARTLEWIGVIST**YSG**DTNYNQKFKDKATMTVDKSSSTAYLELARMTSEDSAIYYCARL**GITTGFA**YWGQGTLVTVSA |
| 49 | light chain variable domain VL, PD1-0069 | DIVLTQSPASLAVSLGQRATISCRA**SKGVSTSSYSF**MHWYQQKPRQPPKLLIK**YAS**YLESGVPARFSGSGSGTDFTLNIHPVEEEDAATYYCHH**SREFPW**TFGGGTKLEIK |
| 50 | heavy chain HVR-H1, PD1-0073 | GFTFSNY |
| 51 | heavy chain HVR-H2, PD1-0073 | GGR |
| 52 | heavy chain HVR-H3, PD1-0073 | YYGID |
| 53 | light chain HVR-L1, PD1-0073 | SQDVTTA |
| 54 | light chain HVR-L2, PD1-0073 | WAS |
| 55 | light chain HVR-L3, PD1-0073 | HYSIPW |
| 56 | heavy chain variable domain VH, PD1-0073 | EVKLVESGGGLVKPGGSLKLSCAAS**GFTFSNY**GMSWIRQTPEKGLEWVATISG**GGR**DTYYPDSVKGRFTISRDNVKNNLYLQMSSLRSEDTAFYYCASY**YYGID**YWGQGTSVTVSS |
| 57 | light chain variable domain VL, PD1-0073 | DIVMTQPHKFMSTSVGDRVRITCKA**SQDVTTA**VAWYQQKPGQSPKLLIY**WAS**TRHTG |
| | | VPDRFTGSGSGTEFTLTISSVQAEDLALYYCQQ**HYSIPW**TFGGGTKLEIK |
| 58 | heavy chain HVR-H1, PD1-0078 | GYTFTST |
| 59 | heavy chain HVR-H2, PD1-0078 | SDS |
| 60 | heavy chain HVR-H3, PD1-0078 | PFD |
| 61 | light chain HVR-L1, PD1-0078 | SQDVSTA |
| 62 | light chain HVR-L2, PD1-0078 | SAS |
| 63 | light chain HVR-L3, PD1-0078 | HYSHPF |
| 64 | heavy chain variable domain VH, PD1-0078 | QVQLQQPGAELVKPGASVKMSCKAS**GYTFTST**WMHWVKQRPGQGLEWIGAIDP**SDS**YTTYNQKFKGKATLTVDTSSTTAYMQLSSLTSEDSAVYYCTRS**PFD**YWGQGTTLTVSS |

(continued)

| SEQ ID NO: | Description | Sequence |
|---|---|---|
| 65 | light chain variable domain VL, PD1-0078 | DIVMTQSHKFMSTSVGDRVSITCKA**SQDVSTA**VAWYQQKPGQSPKLLIY**SAS**YRYTGVPDRFTGSGSGTDFTFAISSVQAEDLAVYYCQQ**HYSHPF**TFGSGTKLEIK |
| 66 | heavy chain HVR-H1, PD1-0102 | GYSITSGY |
| 67 | heavy chain HVR-H2, PD1-0102 | SSG |
| 68 | heavy chain HVR-H3, PD1-0102 | RNWYFD |
| 69 | light chain HVR-L1, PD1-0102 | SQSLLNSGTQKNY |
| 70 | light chain HVR-L2, PD1-0102 | WAS |
| 71 | light chain HVR-L3, PD1-0102 | DYTFPL |
| 72 | heavy chain variable domain VH, PD1-0102 | DVQLQESGPDLVKPSQSLSLTCTVT**GYSITSGY**SWHWIRQFPGNKLEWMGFIH**SSG**DTNYNPSLKSRISFTRDTSKNQFFLQLSSLTDEDTATYYCATY**RNWYFD**VWGAGTTVTVSS |
| 73 | light chain variable domain VL, PD1-0102 | DIVMTQSPSSLTVTAGEKVTMRCKS**SQSLLNSGTQKNY**LTWYQQKPGQPPKLLIY**WAS**TRESGVPNRFTGSGSGTDFTLTISSVQAEDLSVYYCQS**DYTFPL**TFGGGTKLELK |
| 74 | Dimeric 4-1BB ligand (71-254) - CL* Fc knob chain | see Table 8 |
| 75 | Monomeric 4-1BB ligand (71-254)-CH1* | see Table 8 |
| 76 | Dimeric 4-1BB ligand (71-254) - CL Fc knob chain | see Table 9 |
| 77 | Monomeric 4-1BB ligand (71-254) -CH1 | see Table 9 |
| 78 | Dimeric 4-1BB ligand (71-248) - CL* Fc knob chain | see Table 11 |
| 79 | Monomeric 4-1BB ligand (71-248)-CH1* | see Table 11 |
| 80 | Dimeric 4-1BB ligand (71-248) - CL Fc knob chain | see Table 12 |
| 81 | Monomeric 4-1BB ligand (71-248)-CH1 | see Table 12 |
| 82 | anti-PD1(314) Fc hole chain | see Table 8 |
| 83 | anti- PD1(314) light chain | see Table 8 |
| 84 | anti-PD1(314) Fc hole dimeric 4-1BB ligand (71-254) | see Table 10 |
| 85 | anti-PD1(314) Fc knob monomeric 4-1BB ligand (71-254) | see Table 10 |
| 86 | anti-PD1(314) Fc hole dimeric 4-1BB ligand (71-248) | see Table 13 |
| 87 | anti-PD1(314) Fc knob monomeric 4-1BB ligand (71-248) | see Table 13 |

(continued)

| SEQ ID NO: | Description | Sequence |
|---|---|---|
| 88 | Fc hole dimeric 4-1BB ligand (71-248) | see Table 14 |
| 89 | Fc hole monomeric 4-1BB ligand (71-248) | see Table 15 |
| 90 | anti-PD1(314) Fc knob dimeric 4-1BB ligand (71-248) | see Table 15 |
| 91 | Fc knob chain fused to monomeric 4-1BB ligand (71-248) chain | see Table 16 |
| 92 | anti-PD1(314) Fc hole monomeric 4-1BB ligand (71-248) chain | see Table 17 |
| 93 | Fc knob dimeric 4-1BB ligand (71-248) | see Table 17 |
| 94 | Human PD1 | UniProt no. Q15116 |
| 95 | human Lymphotoxin $\alpha$ | UniProt no. P01374 |
| 96 | human TNF | UniProt no. P01375 |
| 97 | human Lymphotoxin $\beta$ | UniProt no. Q06643 |
| 98 | human OX40L | UniProt no. P23510 |
| 99 | human CD40L | UniProt no. P29965 |
| 100 | human FasL | UniProt no. P48023 |
| 101 | human CD27L | UniProt no. P32970 |
| 102 | human CD30L | UniProt no. P32971 |
| 103 | human 4-1BBL | UniProt no. P41273 |
| 104 | human TRAIL | UniProt no. P50591 |
| 105 | human RANKL | UniProt no. 014788 |
| 106 | human TWEAK | UniProt no. 043508 |
| 107 | human APRIL | UniProt no. 075888 |
| 108 | human BAFF | UniProt no. Q9Y275 |
| 109 | human LIGHT | UniProt no. 043557 |
| 110 | human TL1A | UniProt no. 095150 |
| 111 | human GITRL | UniProt no. Q9UNG2 |
| 112 | human ectodysplasin A | UniProt no. Q92838 |
| 113 | human 4-1BBL(50-254) | ACPWAVSGARASPGSAASPRLREGPE<br>LSPDDPAGLLDLRQGMFAQLVAQNVL<br>LIDGPLSWYSDPGLAGVSLTGGLSYKE<br>DTKELVVAKAGVYYVFFQLELRRVVA<br>GEGSGSVSLALHLQPLRSAAGAAALA<br>LTVDLPPASSEARNSAFGFQGRLLHLS<br>AGQRLGVHLHTEARARHAWQLTQGA<br>TVLGLFRVTPEIPAGLPSPRSE |
| 114 | human OX40L (51-183) | QVSHRYPRIQSIKVQFTEYKKEKGFILT<br>SQKEDEIMKVQNNSVIINCDGFYLISLK<br>GYFSQEVNISLHYQKDEEPLFQLKKVR<br>SVNSLMVASLTYKDKVYLNVTTDNTS |

(continued)

| SEQ ID NO: | Description | Sequence |
|---|---|---|
| | | LDDFHVNGGELILIHQNPGEFCVL |
| 115 | human OX40L (52-183) | VSHRYPRIQSIKVQFTEYKKEKGFILTS QKEDEIMKVQNNSVIINCDGFYLISLK GYFSQEVNISLHYQKDEEPLFQLKKVR SVNSLMVASLTYKDKVYLNVTTDNTS LDDFHVNGGELILIHQNPGEFCVL |
| 116 | Petpide linker G4S | GGGGS |
| 117 | Peptide linker $(G4S)_2$ | GGGGSGGGGS |
| 118 | Peptide linker $(SG4)_2$ | SGGGGSGGGG |
| 119 | Peptide linker $(G_4S)_3$ | GGGGSGGGGSGGGGS |
| 120 | Peptide linker $G4(SG4)_2$ | GGGGSGGGGSGGGG |
| 121 | Peptide linker $(G_4S)_4$ | GGGGSGGGGSGGGGSGGGGS |
| 122 | Peptide linker | GSPGSSSSGS |
| 123 | Peptide linker | GSGSGSGS |
| 124 | Peptide linker | GSGSGNGS |
| 125 | Peptide linker | GGSGSGSG |
| 126 | Peptide linker | GGSGSG |
| 127 | Peptide linker | GGSG |
| 128 | Peptide linker | GGSGNGSG |
| 129 | Peptide linker | GGNGSGSG |
| 130 | Peptide linker | GGNGSG |
| 131 | nucleotide sequence of dimeric hu 4-1BBL (71-254) - CL* Fc knob chain | see Table 8 |
| 132 | nucleotide sequence of monomeric hu 4-1BBL (71-254) - CH1* | see Table 8 |
| 133 | nucleotide sequence of anti-PD1(0314) Fc hole chain | see Table 8 |
| 134 | nucleotide sequence of anti-PD1(0314) light chain | see Table 8 |
| 135 | nucleotide sequence of dimeric hu 4-1BBL (71-254) - CL Fc knob chain | see Table 9 |
| 136 | nucleotide sequence of monomeric hu 4-1BBL (71-254) - CH1 | see Table 9 |
| 137 | nucleotide sequence of anti-PD1(0314) Fc hole chain fused to dimeric hu 4-1BBL (71-254) | see Table 10 |
| 138 | nucleotide sequence of anti-PD1(0314) Fc knob chain fused to monomeric hu 4-1BBL (71-254) | see Table 10 |
| 139 | nucleotide sequence of dimeric hu 4-1BBL (71-248) - CL* Fc knob chain | see Table 11 |

(continued)

| SEQ ID NO: | Description | Sequence |
|---|---|---|
| 140 | nucleotide sequence of monomeric hu 4-1BBL (71-248) - CH1* | see Table 11 |
| 141 | nucleotide sequence of dimeric hu 4-1BBL (71-248) - CL Fc knob chain | see Table 12 |
| 142 | nucleotide sequence of monomeric hu 4-1BBL (71-248) - CH1 | see Table 12 |
| 143 | nucleotide sequence of anti-PD1(0314) Fc | see Table 13 |
| | hole chain fused to dimeric hu 4-1BBL (71-248) | |
| 144 | nucleotide sequence of anti-PD1(0314) Fc knob chain fused to monomeric hu 4-1BBL (71-248) | see Table 13 |
| 145 | nucleotide sequence of Fc hole dimeric ligand (71-248) chain | see Table 14 |
| 146 | nucleotide sequence of Fc hole monomeric ligand (71-248) chain | see Table 15 |
| 147 | nucleotide sequence of anti-PD1(0314) Fc knob chain fused to dimeric hu 4-1BBL (71-248) | see Table 15 |
| 148 | nucleotide sequence of Fc knob chain fused to monomeric hu 4-1BBL (71-248) | see Table 16 |
| 149 | nucleotide sequence of anti-PD1(0314) Fc hole monomeric ligand (71-248) chain | see Table 17 |
| 150 | nucleotide sequence of Fc knob chain fused to dimeric hu 4-1BBL (71-248) | see Table 17 |
| 151 | nucleotide sequence of DP47 Fc hole chain | see Table 18 |
| 152 | nucleotide sequence of DP47 light chain | see Table 18 |
| 153 | DP47 Fc hole chain | see Table 18 |
| 154 | DP47 light chain | see Table 18 |
| 155 | nucleotide sequence of DP47 Fc hole chain fused to dimeric hu 4-1BBL (71-254) | see Table 19 |
| 156 | nucleotide sequence of DP47 Fc knob chain fused to monomeric hu 4-1BBL (71-254) | see Table 19 |
| 157 | DP47 Fc hole chain fused to dimeric hu 4-1BBL (71-254) | see Table 19 |
| 158 | DP47 Fc knob chain fused to monomeric hu 4-1BBL (71-254) | see Table 19 |
| 159 | nucleotide sequence of DP47 heavy chain (hu IgG1 PGLALA) | see Table 21 |
| 160 | DP47 heavy chain (hu IgG1 PGLALA) | see Table 21 |
| 161 | nucleotide sequence of PD 1(0314) heavy chain (huIgG1 PGLALA) | see Table 22 |
| 162 | PD1(0314) heavy chain (huIgG1 PGLALA) | see Table 22 |

(continued)

| SEQ ID NO: | Description | Sequence |
|---|---|---|
| 163 | human 4-1BB ECD, aa 24-186 of Q07011 | |
| 164 | Nucleotide sequence of Fc hole chain | see Table 24 |
| 165 | Nucleotide sequence of human 4-1BB antigen Fc knob chain | see Table 24 |
| 166 | Fc hole chain | see Table 24 |
| 167 | human 4-1BB antigen Fc knob chain | see Table 24 |
| 168 | avi tag | GLNDIFEAQKIEWHE |
| 169 | nucleotide sequence of human 4-1BB His | see Table 25 |
| 170 | human 4-1BB His | see Table 25 |

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the EU numbering systems according to Kabat (Kabat, E.A., et al., Sequences of Proteins of Immunological Interest, 5th ed., Public Health Service, National Institutes of Health, Bethesda, MD (1991)) as defined above.

The following are aspects of the invention:

- A TNF family ligand trimer-containing antigen binding molecule comprising

  (a) at least one moiety capable of specific binding to PD1 and
  (b) a first and a second polypeptide that are linked to each other by a disulfide bond, wherein the antigen binding molecule is characterized in that the first polypeptide comprises two ectodomains of a TNF ligand family member or a fragment thereof that are connected to each other by a peptide linker and in that the second polypeptide comprises only one ectodomain of said TNF ligand family member or a fragment thereof.

- The TNF family ligand trimer-containing antigen binding molecule of point 1, further comprising

  (c) an Fc domain composed of a first and a second subunit capable of stable association.

- The TNF family ligand trimer-containing antigen binding molecule of points 1 or 2, comprising

  (a) at least one moiety capable of specific binding to PD1 and
  (b) a first and a second polypeptide that are linked to each other by a disulfide bond, wherein the antigen binding molecule is characterized in that

    o the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or
    o the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises only one ectodomain of said TNF ligand family member or a fragment thereof connected via a peptide linker to the C-terminus of the CH3 domain of said polypeptide, or
    o the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to to VH or VL by a peptide linker and wherein the second polypeptide comprises one ectodomain of said TNF ligand family member or a fragment thereof connected via a peptide linker to VL or VH of said polypeptide.

- The TNF family ligand trimer-containing antigen binding molecule of any one of points 1 to 3, wherein the TNF ligand family member costimulates human T-cell activation.

- The TNF family ligand trimer-containing antigen binding molecule of any one of points 1 to 4, wherein the TNF ligand family member is selected from 4-1BBL and OX40L.

- The TNF family ligand trimer-containing antigen binding molecule of any one of points 1 to 5, wherein the TNF ligand family member is 4-1BBL.

- The TNF family ligand trimer-containing antigen binding molecule of any one of points 1 to 6, wherein the ectodomain of a TNF ligand family member comprises the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO: 5.

- The TNF family ligand trimer-containing antigen binding molecule of any one of points 1 to 7, wherein the ectodomain of a TNF ligand family member comprises the amino acid sequence of SEQ ID NO:5.

- The TNF family ligand trimer-containing antigen binding molecule of any one of points 1 to 8, comprising

  (a) at least one moiety capable of specific binding to PD1 and
  (b) a first and a second polypeptide that are linked to each other by a disulfide bond, wherein the antigen binding molecule is characterized in that the first polypeptide comprises the amino acid sequence selected from the group consisting of SEQ ID NO:9, SEQ ID NO: 11, SEQ ID NO: 12 and SEQ ID NO: 10 and in that the second polypeptide comprises the amino acid sequence selected from the group consisting of SEQ ID NO:1, SEQ ID NO: 3, SEQ ID NO:4 and SEQ ID NO:5.

- The TNF family ligand trimer-containing antigen binding molecule of any one of points 1 to 9, comprising

  (a) at least one moiety capable of specific binding to PD1, and
  (b) a first polypeptide containing a CH1 or CL domain and a second polypeptide containing a CL or CH1 domain, respectively, wherein the second polypeptide is linked to the first polypeptide by a disulfide bond between the CH1 and CL domain,

  and wherein the antigen binding molecule is characterized in that the first polypeptide comprises two ectodomains of a TNF ligand family member or fragments thereof that are connected to each other and to the CH1 or CL domain by a peptide linker and in that the second polypeptide comprises only one ectodomain of said TNF ligand family member or a fragment thereof connected via a peptide linker to the CL or CH1 domain of said polypeptide.

- The TNF family ligand trimer-containing antigen binding molecule of any one of points 1 to 10, wherein the moiety capable of specific binding to PD1 is selected from the group consisting of an antibody fragment, a Fab molecule, a crossover Fab molecule, a single chain Fab molecule, a Fv molecule, a scFv molecule, a single domain antibody, an aVH and a scaffold antigen binding protein.

- The TNF family ligand trimer-containing antigen binding molecule of any one of points 1 to 11, wherein the molecule comprises one moiety capable of specific binding to PD1.

- The TNF family ligand trimer-containing antigen binding molecule of any one of points 1 to 12, wherein the moiety capable of specific binding to PD 1 is a Fab molecule capable of specific binding to PD1.

- The TNF family ligand trimer-containing antigen binding molecule of any one of points 1 to 13, wherein the moiety capable of specific binding to PD1 comprises

(a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16 (v) HVR-L2 comprising the amino acid sequence of SEQ ID NO: 17, and (vi) HVR-L3 comprising the amino acid sequence of SEQ ID NO:18,
(b) a VH domain comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28, and a VL domain comprising (iv) HVR-L1 comprising the amino acid sequence of SEQ ID NO:29 (v) HVR-L2 comprising the amino acid sequence of SEQ ID NO:30, and (vi) HVR-L3 comprising the amino acid sequence of SEQ ID NO:31,
(c) a VH domain comprising (i) HVR-H1 comprising the amino acid sequence of SEQ ID NO:34, (ii) HVR-H2 comprising the amino acid sequence of SEQ ID NO:35, and (iii) HVR-H3 comprising the amino acid sequence of SEQ ID NO:36, and a VL domain comprising (iv) HVR-L1 comprising the amino acid sequence of SEQ ID NO:37 (v) HVR-L2 comprising the amino acid sequence of SEQ ID NO:38, and (vi) HVR-L3 comprising the amino acid sequence of SEQ ID NO:39,
(d) a VH domain comprising (i) HVR-H1 comprising the amino acid sequence of SEQ ID NO:42, (ii) HVR-H2 comprising the amino acid sequence of SEQ ID NO:43, and (iii) HVR-H3 comprising the amino acid sequence of SEQ ID NO:44, and a VL domain comprising (iv) HVR-L1 comprising the amino acid sequence of SEQ ID NO:45 (v) HVR-L2 comprising the amino acid sequence of SEQ ID NO:46, and (vi) HVR-L3 comprising the amino acid sequence of SEQ ID NO:47,
(e) a VH domain comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52, and a VL domain comprising (iv) HVR-L1 comprising the amino acid sequence of SEQ ID NO:53 (v) HVR-L2 comprising the amino acid sequence of SEQ ID NO:54, and (vi) HVR-L3 comprising the amino acid sequence of SEQ ID NO:55,
(f) a VH domain comprising (i) HVR-H1 comprising the amino acid sequence of SEQ ID NO:58, (ii) HVR-H2 comprising the amino acid sequence of SEQ ID NO:59, and (iii) HVR-H3 comprising the amino acid sequence of SEQ ID NO:60, and a VL domain comprising (iv) HVR-L1 comprising the amino acid sequence of SEQ ID NO:61 (v) HVR-L2 comprising the amino acid sequence of SEQ ID NO:62, and (vi) HVR-L3 comprising the amino acid sequence of SEQ ID NO:63, or
(g) a VH domain comprising (i) HVR-H1 comprising the amino acid sequence of SEQ ID NO:66, (ii) HVR-H2 comprising the amino acid sequence of SEQ ID NO:67, and (iii) HVR-H3 comprising the amino acid sequence of SEQ ID NO:68, and a VL domain comprising (iv) HVR-L1 comprising the amino acid sequence of SEQ ID NO:69 (v) HVR-L2 comprising the amino acid sequence of SEQ ID NO:70, and (vi) HVR-L3 comprising the amino acid sequence of SEQ ID NO:71.

- The TNF family ligand trimer-containing antigen binding molecule of any one of claims 1 to 14, wherein the moiety capable of specific binding to PD1 comprises

(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24,
(e) a VH domain comprising an amino acid sequence of SEQ ID NO:21 and a VL domain comprising an amino acid sequence of SEQ ID NO:25,
(f) a VH domain comprising an amino acid sequence of SEQ ID NO:32 and a VL domain comprising an amino acid sequence of SEQ ID NO:33,
(g) a VH domain comprising an amino acid sequence of SEQ ID NO:40 and a VL domain comprising an amino acid sequence of SEQ ID NO:41,
(h) a VH domain comprising an amino acid sequence of SEQ ID NO:48 and a VL domain comprising an amino acid sequence of SEQ ID NO:49,
(i) a VH domain comprising an amino acid sequence of SEQ ID NO:56 and a VL domain comprising an amino acid sequence of SEQ ID NO:57,
(k) a VH domain comprising an amino acid sequence of SEQ ID NO:64 and a VL domain comprising an amino acid sequence of SEQ ID NO:65, or
(l) a VH domain comprising an amino acid sequence of SEQ ID NO:72 and a VL domain comprising an amino acid sequence of SEQ ID NO:73.

- The TNF family ligand trimer-containing antigen binding molecule of any one of points 1 to 14, wherein the moiety capable of specific binding to PD1 comprises (a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO:18.

- The TNF family ligand trimer-containing antigen binding molecule of any one of claims 1 to 16, wherein the moiety capable of specific binding to PD1 comprises

(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

- The TNF family ligand trimer-containing antigen binding molecule of any one of points 2 to 17, wherein the Fc domain is an IgG, particularly an IgG1 Fc domain or an IgG4 Fc domain.

- The TNF family ligand trimer-containing antigen binding molecule of any one of points 2 to 18, wherein the Fc domain is an IgG1 Fc domain comprising the amino acid substitutions at positions 234 and 235 (EU numbering) and/or 329 (EU numbering).

- The TNF family ligand trimer-containing antigen binding molecule of any one of points 1 to 19, wherein the first subunit of the Fc domain comprises the amino acid substitutions S354C and T366W (numbering according to Kabat EU index) and the second subunit of the Fc domain comprises the amino acid substitutions Y349C, T366S, L368A and Y407V (numbering according to Kabat EU index).

- The TNF family ligand trimer-containing antigen binding molecule of any one of points 1 to 20, wherein the antigen binding molecule comprises
a first heavy chain and a first light chain, both comprising a Fab molecule capable of specific binding to PD1,
a first peptide comprising two ectodomains of a TNF ligand family member or fragments thereof connected to each other by a first peptide linker fused at its C-terminus by a second peptide linker to a second heavy or light chain,
and a second peptide comprising one ectodomain of said TNF ligand family member fused at its C-terminus by a third peptide linker to a second light or heavy chain, respectively.

- The TNF family ligand trimer-containing antigen binding molecule of any one of points 1 to 20, wherein the first peptide comprising two ectodomains of a TNF ligand family member or fragments thereof connected to each other by a first peptide linker is fused at its C-terminus by a second peptide linker to a CH1 domain that is part of a heavy chain, and the second peptide comprising one ectodomain of said TNF ligand family member or a fragment thereof is fused at its C-terminus by a third peptide linker to a CL domain that is part of a light chain.

- The TNF family ligand trimer-containing antigen binding molecule of any one of points 1 to 20, wherein the first peptide comprising two ectodomains of a TNF ligand family member or fragments thereof connected to each other by a first peptide linker is fused at its C-terminus by a second peptide linker to a CL domain that is part of a heavy chain, and the second peptide comprising one ectodomain of said TNF ligand family member or a fragment thereof is fused at its C-terminus by a third peptide linker to a CH1 domain that is part of a light chain.

- The TNF family ligand trimer-containing antigen binding molecule of any one of points 1 to 20, wherein the first peptide comprising two ectodomains of a TNF ligand family member or fragments thereof connected to each other by a first peptide linker is fused at its C-terminus by a second peptide linker to a VH domain that is part of a heavy chain, and the second peptide comprising one ectodomain of said TNF ligand family member or a fragment thereof is fused at its C-terminus by a third peptide linker to a VL domain that is part of a light chain.

- The TNF family ligand trimer-containing antigen binding molecule of points 21 to 23, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

- The TNF family ligand trimer-containing antigen binding molecule of any one of points 1 to 20, wherein the antigen binding molecule comprises

  (a) a first heavy chain and a first light chain, both comprising a Fab molecule capable of specific binding to PD1,
  (b) a second heavy chain comprising an amino acid sequence selected from the group consisting of SEQ ID NO:9, SEQ ID NO: 11, SEQ ID NO:12 and SEQ ID NO: 10, and a second light chain comprising an amino acid sequence selected from the group consisting of SEQ ID NO:1, SEQ ID NO:3, SEQ ID NO:4 and SEQ ID NO:5.

- The TNF family ligand trimer-containing antigen binding molecule of any one of points 1 to 20 or 26, wherein the antigen binding molecule comprises

  (i) a first heavy chain comprising the VH domain comprising the amino acid sequence of SEQ ID NO:19 and a first light chain comprising the VL domain comprising the amino acid sequence of SEQ ID NO:20 or
  a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
  (ii) a second heavy chain comprising the amino acid sequence selected from the group consisting of SEQ ID NO:74, SEQ ID NO:76, SEQ ID NO:78 and SEQ ID NO:80, and
  (iii) a second light chain comprising the amino acid sequence selected from the group consisting of SEQ ID NO:75, SEQ ID NO:77, SEQ ID NO:79 and SEQ ID NO:81.

- The TNF family ligand trimer-containing antigen binding molecule of any one of points 1 to 21, wherein the antigen binding molecule comprises

  (a) a first heavy chain comprising the amino acid sequence of SEQ ID NO:82, a first light chain comprising the amino acid sequence of SEQ ID NO:83, a second heavy chain comprising the amino acid sequence of SEQ ID NO:74 and a second light chain comprising the amino acid sequence of SEQ ID NO:75,
  (b) a first heavy chain comprising the amino acid sequence of SEQ ID NO:82, a first light chain comprising the amino acid sequence of SEQ ID NO:83, a second heavy chain comprising the amino acid sequence of SEQ ID NO:76 and a second light chain comprising the amino acid sequence of SEQ ID NO:77,
  (c) a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79, or
  (d) a first heavy chain comprising the amino acid sequence of SEQ ID NO:82, a first light chain comprising the amino acid sequence of SEQ ID NO:83, a second heavy chain comprising the amino acid sequence of SEQ ID NO:80 and a second light chain comprising the amino acid sequence of SEQ ID NO:81.

- The TNF family ligand trimer-containing antigen binding molecule of any one of points 1 to 20, comprising

  (a) at least one Fab domain capable of specific binding to PD1, and
  (b) a first and a second polypeptide that are linked to each other by a disulfide bond, wherein the antigen binding molecule is characterized in that the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises one ectodomain of said TNF ligand family member or a fragment thereof connected via a peptide linker to C-terminus of the CH3 domain of said polypeptide.

- The TNF family ligand trimer-containing antigen binding molecule of point 29, comprising two Fab domains capable of specific binding to PD1.
- The TNF family ligand trimer-containing antigen binding molecule of point 29, wherein the antigen binding molecule comprises

  (a) a first heavy chain comprising the amino acid sequence of SEQ ID NO:84, a second heavy chain comprising the amino acid sequence of SEQ ID NO:85, and two light chains comprising the amino acid sequence of SEQ ID NO:83, or
  (b) a first heavy chain comprising the amino acid sequence of SEQ ID NO:86, a second heavy chain comprising the amino acid sequence of SEQ ID NO:87, and two light chains comprising the amino acid sequence of SEQ ID NO:83.

- The TNF family ligand trimer-containing antigen binding molecule of point 29, comprising one Fab domain capable of specific binding to PD1.
- The TNF family ligand trimer-containing antigen binding molecule of point 29 or 32, wherein the antigen binding molecule comprises

  (i) a fusion polypeptide comprising a first subunit of a Fc domain and two ectodomains of a TNF ligand family member or fragments thereof that are connected to each other and to the C-terminus of the CH3 domain by a peptide linker, (ii) a heavy chain comprising the VH domain of the Fab domain capable of specific binding to PD1, a second subunit of the Fc domain and one ectodomain of said TNF ligand family member or a fragment thereof connected via a peptide linker to C-terminus of the CH3 domain, and (iii) a light chain comprising the VL domain of the Fab domain capable of specific binding to PD1, or
  (i) a fusion polypeptide comprising a first subunit of a Fc domain and one ectodomain of a TNF ligand family member or a fragment thereof that is connected to the C-terminus of the CH3 domain by a peptide linker, (ii) a heavy chain comprising the VH domain of the Fab domain capable of specific binding to PD1, a second subunit of the Fc domain and two ectodomains of said TNF ligand family member or fragments thereof that are connected to each other and to the C-terminus of the CH3 domain via a peptide linker, and (iii) a light chain comprising the VL domain of the Fab domain capable of specific binding to PD1.

- The TNF family ligand trimer-containing antigen binding molecule of point 31, wherein the antigen binding molecule comprises

  (a) a fusion polypeptide comprising the amino acid sequence of SEQ ID NO:88, a heavy chain comprising the amino acid sequence of SEQ ID NO:87 and a light chain comprising the amino acid sequence of SEQ ID NO:83,
  (b) a fusion polypeptide comprising the amino acid sequence of SEQ ID NO:89, a heavy chain comprising the amino acid sequence of SEQ ID NO:90 and a light chain comprising the amino acid sequence of SEQ ID NO:83,
  (c) a fusion polypeptide comprising the amino acid sequence of SEQ ID NO:91, a heavy chain comprising the amino acid sequence of SEQ ID NO:86 and a light chain comprising the amino acid sequence of SEQ ID NO:83,
  (b) a fusion polypeptide comprising the amino acid sequence of SEQ ID NO:93, a heavy chain comprising the amino acid sequence of SEQ ID NO:92 and a light chain comprising the amino acid sequence of SEQ ID NO:83,

- An isolated polynucleotide encoding the TNF family ligand trimer-containing antigen binding molecule of any one of points 1 to 34.
- A vector, particularly an expression vector, comprising the isolated polynucleotide of point 35.
- A host cell comprising the isolated polynucleotide of claim 35 or the vector of point 36.
- A method for producing the TNF family ligand trimer-containing antigen binding molecule of any one of points 1 to 34, comprising the steps of

  (i) culturing the host cell of point 37 under conditions suitable for expression of the antigen binding molecule, and
  (ii) recovering the antigen binding molecule.

- A pharmaceutical composition comprising the TNF family ligand trimer-containing antigen binding molecule of any one of points 1 to 34 and at least one pharmaceutically acceptable excipient.

- The TNF family ligand trimer-containing antigen binding molecule of any one of points 1 to 34, or the pharmaceutical composition of point 39, for use as a medicament.

- The TNF family ligand trimer-containing antigen binding molecule of any one of claims 1 to 34, or the pharmaceutical composition of point 39, for use in the treatment of cancer.

- Use of the TNF family ligand trimer-containing antigen binding molecule of any one of points 1 to 34 for the manufacture of a medicament for the treatment of cancer.

- A method of treating a disease in an individual, comprising administering to said individual a therapeutically effective amount of a composition comprising a TNF family ligand trimer-containing antigen binding molecule of any one of points 1 to 34 in a pharmaceutically acceptable form.

- The method of point 43, wherein said disease is cancer.

## EXAMPLES

The following are examples of methods and compositions of the invention. It is understood that various other embodiments may be practiced, given the general description provided above.

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

**SDS-PAGE**

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

**Analytical size exclusion chromatography**

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM $KH_2PO_4/K_2HPO_4$, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

**Mass spectrometry**

This section describes the characterization of the multispecific antibodies with VH/VL exchange (VH/VL CrossMabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 μg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

**Determination of binding and binding affinity of multispecific antibodies to the respective antigens using surface plasmon resonance (SPR) (BIACORE)**

Binding of the generated antibodies to the respective antigens is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C). Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

**Example 1**

**1.1 Generation of anti-PD1 antibodies**

**Immunization of mice**

NMRI mice were immunized genetically, using a plasmid expression vector coding for full-length human PD1 by intradermal application of 100 ug vector DNA (plasmid15300_hPD1-fl), followed by Electroporation (2 square pulses of 1000 V/cm, duration 0.1 ms, interval 0.125 s; followed by 4 square pulses of 287.5 V/cm, duration 10 ms, interval 0.125 s. Mice received either 6 consecutive immunizations at days 0, 14, 28, 42, 56, 70, and 84. Blood was taken at days 36, 78 and 92 and serum prepared, which was used for titer determination by ELISA (see below). Animals with highest titers were selected for boosting at day 96, by intravenous injection of 50 ug of recombinant human PD 1 human Fc chimera, and monoclonal antibodies were isolated by hybridoma technology, by fusion of splenocytes to myeloma cell line 3 days after boost.

**Determination of serum titers (ELISA)**

Human recombinant PD1 human Fc chimera was immobilized on a 96-well NUNC Maxisorp plate at 0.3 ug/ml, 100 ul/well, in PBS, followed by: blocking of the plate with 2% Crotein C in PBS, 200 ul/well; application of serial dilutions of antisera, in duplicates, in 0.5% Crotein C in PBS, 100 ul/well; detection with HRP-conjugated goat anti-mouse antibody (Jackson Immunoresearch/Dianova 115-036-071; 1/16 000). For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well. Absorbance was read out at 450 nm, against 690 nm as reference. Titer was defined as dilution of antisera resulting in half-maximal signal.

**1.2 Characterization anti-PD1 antibodies/ Binding of anti-PD1 antibodies to human PD1**

Elisa for hu PD1

Nunc maxisorp streptavidin coated plates (MicroCoat #11974998001) were coated with 25 μl/well biotinylated PD1-ECD-AviHis and incubated at 4°C over night. After washing (3x90 μl/well with PBST-buffer) 25 μl anti PD1 samples or reference antibodies (human anti PD1; Roche/mouse anti PD1; Biolegend; cat.:329912) were added and incubated 1h at RT. After washing (3x90 μl/well with PBST-buffer) 25μl/well goat-anti-human H+L-POD (JIR, JIR109-036-088)/ Sheep-anti-mouse-POD (GE Healthcare; NA9310) was added in 1:2000/1:1000 dilution and incubated at RT for 1 h on shaker. After washing (3x90 μl/well with PBST-buffer) 25 μl/well TMB substrate (Roche Catalogue No. 11835033001) was added and incubated until OD 2 - 3. Measurement took place at 370/492 nm.

ELISA results are listed as $EC_{50}$-values [ng/ml] in Summary Table 1 and 2 below.

Cell ELISA for PD1

Adherent CHO-K1 cell line stably transfected with plasmid 15311_hPD1-fl_pUC_Neo coding for full-length human PD1 and selection with G418 (Neomycin restistance marker on plasmid) were seeded at a concentration of 0.01x10E6 cells/well in 384-well flat bottom plates and grown over night.

The next day 25 μl/well PD1 sample or human anti PD1 (Roche)/mouse anti PD1(Biolegend; cat.:329912) reference antibody were added and incubated for 2h at 4°C (to avoid internalization). After washing carefully (1x90μl/well PBST) cells were fixed by adding 30μl/well 0,05% Glutaraldehyde (Sigma, Cat.No: G5882, 25%)diluted in 1xPBS-buffer and incubated for 10min at RT. After washing (3x90μl/well PBST) 25 μl/well secondary antibody was added for detection: goat-anti-human H+L-POD (JIR, JIR109-036-088)/Sheep-anti-mouse-POD (GE NA9310) followed by 1h incubation at RT on shaker. After washing (3x90μl/well PBST) 25 μl/well TMB substrate solution (Roche 11835033001) was added and incubated until OD 1.0 - 2.0. Plates were measured at 370/492 nm.

Cell ELISA results are listed as "$EC_{50}$ CHO-PD1"-values [ng/ml] in Summary Table 2 below.

ELISA for cyno PD1

Nunc maxisorp streptavidin coated plates (MicroCoat #11974998001) were coated with 25 μl/well biotinylated cyno PD1-ECD-Biotin and incubated at 4°C over night. After washing (3x90 μl/well with PBST-buffer) 25 μl anti PD1 samples or reference antibodies (human anti PD1; Roche) were added and incubated 1h at RT on shaker. After washing (3x90 μl/well with PBST-buffer) 25μl/well goat-anti-human H+L-POD (JIR, JIR109-036-088) was added in 1:1000 dilution and incubated at RT for 1 h on shaker. After washing (3x90 μl/well with PBST-buffer) 25 μl/well TMB substrate (Roche, 11835033001) was added and incubated until OD 2 - 3. Measurement took place at 370/492 nm.

ELISA results are listed as $EC_{50}$-values [ng/ml] in Summary Tables 1 and 2 below.

PD Ligand 1 replacing assay

Nunc maxisorp streptavidin coated plates (MicroCoat #11974998001) were coated with 25 μl/well biotinylated PD1-ECD-AviHis and incubated at 4°C over night. After washing (3x90 μl/well with PBST-buffer) 25 μl anti PD1 samples or reference antibodies (mouse anti PD1; Biolegend; cat.:329912) were added and incubated 1h at RT on shaker. After washing (3x90 μl/well with PBST-buffer) 25μl/well PD-L1 (Recombinant human B7-H1/PD-L1 Fc Chimera; 156-B7, R&D) was added and incubated 1h at RT on shaker. After washing (3x90 μl/well with PBST-buffer) 25μl/well goat-anti-human H+L-POD (JIR, 109-036-088) was added in 1:1000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as $IC_{50}$-values [ng/ml] in Summary Table 1 below.

PD Ligand 2 replacing assay

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (mouse anti huPD1; Roche) were added and incubated 1h at RT on shaker. After washing (3x90 µl/well with PBST-buffer) 25µl/well PD-L2 (Recombinant human B7-DC/PD-L2 Fc Chimera; 1224-PL-100, R&D) was added and incubated 1h at RT on shaker. After washing (3x90 µl/well with PBST-buffer) 25µl/well goat-anti-human H+L-POD (JIR, 109-036-088) was added in 1:2000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as $IC_{50}$-values [ng/ml] in summary Table 1 below.

Epitope mapping ELISA/ Binding competition assay

Nunc maxisorp plates (Nunc #464718) were coated with 25µl/well capture antibody (goat anti mouse IgG; JIR; 115-006-071) and incubated for 1h at RT on shaker. After washing (3x90µl/well with PBST-buffer) plates were blocked for 1h with 2% BSA containing PBS buffer at RT on shaker. After washing (3x90µl/well with PBST-buffer) 25µl mouse anti PD1 samples were added and incubated 1h at RT on shaker. After washing (3x90µl/well with PBST-buffer) capture antibody was blocked by 30µl/well mouse IgG (JIR; 015-000-003) for 1h at RT on shaker. At the same time biotinylated PD1-ECD-AviHis was preincubated with second sample antibody for 1h at RT on shaker. After washing assay plate (3x90 µl/well with PBST-buffer) the PD1 antibody mix was transferred to assay plate and incubated at RT for 1h on shaker. After washing (3x90 µl/well with PBST-buffer) 25µl/well streptavidin POD (Roche, #11089153001) was added in 1:4000 dilution and incubated at RT for 1 h on shaker. After washing (3x90 µl/well with PBST-buffer) 25 µl/well TMB substrate (Roche, #11089153001) was added and incubated until OD 1.5 - 2.5. Measurement took place at 370/492 nm. Epitope groups were defined by hierarchical clustering against reference antibodies.

**Table 1: Binding, PD-L1 inhibition and epitope region groups of exemplary PD1 antibodies (ELISA)**

| Antibody | ELISA huPD1 $EC_{50}$ [ng/ml] | ELISA cyPD1 $EC_{50}$ [ng/ml] | ELISA PD-L1 inhibition $IC_{50}$ [ng/ml] | ELISA PD-L2 inhibition $IC_{50}$ [ng/ml] | Epitope region group (by competition assay) |
|---|---|---|---|---|---|
| PD1- 0050 | 17.9 | 9.8 | 128 | 34 | 1 |
| PD1- 0069 | 45.7 | 22.7 | 225 | 89 | 6 |
| PD1- 0073 | 15.1 | 8.3 | 124 | 65 | 5 |
| PD1- 0078 | 26.3 | 22.4 | x | 86 | 2 |
| PD1-0098 | 50.8 | 54.6 | 174 | 45 | 5 |
| PD1-0102 | 34.2 | 52.7 | >35.5 µg/ml | 140 | 4 |
| PD1-0103 | 33.7 | 36.9 | 182 | 51 | 5 |

**Table 2: Biochemial- and Cell-binding of humanized PD1 antibodies derived from parental mouse antibody PD1-0103 (ELISA)**

| Humanized antibody | ELISA huPD1 $EC_{50}$ [ng/ml] | ELISA cyPD1 $EC_{50}$ [ng/ml] | ELISA CHO-PD1 $EC_{50}$ [ng/ml] |
|---|---|---|---|
| PD1-103-0312 | 11 | 8.3 | 10.1 |
| PD1-103-0313 | 15 | 11 | 10.8 |
| PD1-103-0314 | 11 | 8.3 | 7.7 |

(continued)

| Humanized antibody | ELISA huPD1 $EC_{50}$ [ng/ml] | ELISA cyPD1 $EC_{50}$ [ng/ml] | ELISA CHO-PD1 $EC_{50}$ [ng/ml] |
|---|---|---|---|
| PD1-103-0315 | 10 | 7.9 | 7.3 |

Biacore characterization of the humanized anti-PD1 antibodies

A surface plasmon resonance (SPR) based assay has been used to determine the kinetic parameters of the binding between several murine PD 1 binders as well as commercial human PD1 binding references. Therefore, an anti-human IgG was immobilized by amine coupling to the surface of a (Biacore) CM5 sensor chip. The samples were then captured and hu PD1-ECD was bound to them. The sensor chip surface was regenerated after each analysis cycle. The equilibrium constant and kinetic rate constants were finally gained by fitting the data to a 1:1 1 langmuir interaction model.

About 2000 response units (RU) of 20 μg/ml anti-human IgG (GE Healthcare #BR-1008-39) were coupled onto the flow cells 1 and 2 (alternatively: 3 and 4) of a CM5 sensor chip in a Biacore T200 at pH 5.0 by using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

The samples were injected for 20 seconds with a concentration of 10 nM and bound to the second flow cell. Then a complete set of human PD1-ECD concentrations (144 nM, 48 nM, 16 nM, 5.33 nM, 1.78 nM, 0.59 nM, 0.20 nM and 0 nM) was injected over each sample for 120s followed by a dissociation time of 30/300s and two 20s regeneration steps with 3 M $MgCl_2$, of which the last one contained an "extra wash after injection" with running buffer.

Finally the double referenced data was fitted to a 1:1 langmuir interaction model with the Biacore T200 Evaluation Software. Resulting $K_D$, $k_a$ and $k_d$ values are shown in Table 3.

**Table 3: Kinetic rate constants and equilibrium constant for chimeric PD1-0103 and humanized PD1-Abs determined by Biacore**

| Ligand | $k_a$ [$M^{-1}s^{-1}$] | $k_d$ [$s^{-1}$] | **$K_D$ [nM]** |
|---|---|---|---|
| chimeric PD1-0103 | 3.86E+05 | 3.07E-04 | **0.8** |
| PD1-0103-0312 | 1.95E+05 | 3.45E-04 | **1.8** |
| PD1-0103-0313 | 1.60E+05 | 3.67E-04 | **2.3** |
| PD1-0103-0314 | 1.87E+05 | 2.79E-04 | **1.5** |
| PD1-0103-0315 | 1.89E+05 | 2.91E-04 | **1.5** |

As shown in Table 3, all the humanized versions of chimeric PD1-0103 (generation see Example 1.3) display kinetic properties similar to the parental antibody (chimeric PD1-0103).

Kinetics

A CM5 sensor series S was mounted into the Biacore 4000 System and the detection spots were hydrodynamically addressed according to the manufacturer's instructions.

The polyclonal rabbit IgG antibody <IgGFCγM>R (Jackson ImmunoResearch Laboratories Inc.) was immobilized at 10 000 Ru on the detection spots 1 and 5 in the flow cells 1,2,3 and 4. Coupling was done via EDC/NHS chemistry according to the manufacturer's instructions. The remaining spots in the flow cells served as a reference. The sample buffer was the system buffer supplemented with 1 mg/ml carboxymethyldextrane.

In one embodiment the assay was driven at 25 °C. In another embodiment the assay was driven at 37 °C. 50 nM of each murine monoclonal antibody was captured on the sensor surface by a 1 min injection at 10 μl/min. Subsequently the respective antigens were injected in a concentration series of 100 nM, 2x 33 nM, 11 nM, 4 nM, 1 nM and system buffer 0 nM at 30μl/min for 4 min association phase time. The dissociation was monitored for another 4 min. The capture system was regenerated using a 3 min injection of 10 mM glycine pH 1.5 at 30 μl/min. Relevant kinetic data was calculated using the Biacore evaluation software according to the manufacturer's instructions.

Epitope Mapping

A Biacore 4000 instrument was mounted with a Biacore CAP sensor and was prepared like recommended by the manufacturer. The instrument buffer was HBS-ET (10 mM HEPES pH 7.4, 150 mM NaCl, 3 mM EDTA, 0.005% w/v Tween 20). The instrument was running at 25 °C.

All samples were diluted in system buffer. A 35kDa biotinylated antigen PD1-ECD-AviHis was captured at 200 RU on the CAP sensor surface by a 1 min injection at 30μl/min in the flow cells 1, 2, 3 and 4 in the spots 1 and 5. Spots 2, 3 and 4 served as a reference. In another embodiment, a 35 kDa biotinylated antigen PD1-ECD-AviHis was captured at 200 RU on the CAP sensor in the same manner.

Subsequently a primary antibody was injected at 100 nM for 3 min at 30 μl/min followed by the injection of a secondary antibody at 100 nM for 3 min at 30 μl/min. The primary antibody was injected until full saturation of the surface presented antigen. At the end of the primary and secondary antibody injection phases report points " Binding Late" (BL) were set to monitor the binding response of the respective antibodies. The Molar Ratio, a quotient between the secondary antibody binding response "BL2" and the primary antibody response "BL1" was calculated. The Molar Ratio was used as an indicator of the antigen accessibility of the secondary antibody, when the antigen was already complexed by the primary antibody.

The complexes were completely removed from the sensor surface by an injection for 2 min at 30μ/min 2M guanidine-HCL 250 mM NaOH regeneration buffer as recommended by the manufacturer, followed by a 1 min injection at 30μl /min of system buffer.

**1.3 Effect of different anti-PD1 Antibodies on Cytokine Production in a Mixed Lymphocyte Reaction (MLR)**

3A) The Mixed Lymphocyte Reaction (MLR) is a immune cell assay which measures the activation of lymphocytes from one individual (donor X) to lymphocytes from another individual (donor Y). A mixed lymphocyte reaction was used to demonstrate the effect of blocking the PD1 pathway to lymphocyte effector cells. T cells in the assay were tested for activation and theier IFN-gamma secretion in the presence or absence of an anti-PD1 mAbs.
To perform an allogeneic MLR, peripheral blood mononuclear cells (PBMCs) from at least four healthy donors of unknown HLA type were isolated by density gradient centrifugation using Leukosep (Greiner Bio One, 227 288). Briefly, heparinized blood samples were diluted with the three fold volume of PBS and 25 ml aliquots of the diluted blood were layered in 50 ml Leukosep tubes. After centrifugation at 800 x g for 15 min at room temperature (w/o break) the lymphocyte containing fractions were harvested, washed in PBS and used directly in functional assay or resuspended in freezing medium (10% DMSO, 90 %FCS) at 1.0E+07 cells/ml and stored in liquid nitrogen. Individual 2-way MLR reactions were set up by mixing PBMCs from two different donors at a 1:1 stimulator/responder cell ratio and co-cultures were done at least in duplicate in flat-bottomed 96-well plates for 6 days at 37oC, 5% CO2, in the presence or w/o of a different concentration range of purified anti-PD1 monoclonal antibodies PD1-0050, PD1-0069, PD1-0073, PD1-0078, PD1-0098, PD1-0102, PD1-0103. As reference anti-PD1 antibodies antibodies comprising the VH and VL domains of either nivolumab (also known as MDX-5C4 or MDX-1106) or pembrolizumab (also known as MK-3475 or Org 1.09A) were synthesized and cloned with backbones of human IgG1 (with mutations L234A, L235A and P329G (EU index of Kabat)). Either no antibody or an isotype control antibody was used as a negative control and rec hu IL-2 (20 EU/ml) was used as positive control. After day 6 100 μl of medium was taken from each culture for cytokine measurement. The levels of IFN-gamma were measured using OptEIA ELISA kit (BD Biosciences).
The results are shown in Table 4 (IFN-γ secretion/release). The anti-PD1 monoclonal antibodies promoted T cell activation and IFN-gamma secretion in concentration dependent manner. The value of % increase of IFNg secretion was calculated in relation to IFN-γ production of MLR w/o adding of any blocking mAbs (basal allogeneic stimulation induced IFNg value as E-c) and MLR with adding of 20 EU/ml rec hu IL-2 (positive control = 100% IFNg value as E+c) and was calculated according to formula: Rel.Stimulation [%] = ((Example-E-c)/(E+c - E-c)*100).

**Table 4: Percentage of of IFN gamma secretion after allogenic stimulation and treatment with anti-PD1 antibody in comparison to effect of recombinant human IL-2 treatment (20 EU/ml) ( = 100% increase) as positive control**

| | Concentration (μg/ml) | 1:12 | 1:120 | 1:1200 | Effect in MLR |
|---|---|---|---|---|---|
| PD1-0050 | 44 | 136 | 96 | 33 | +++ |
| PD1-0069 | 60 | 76 | 71 | 55 | +++ |
| PD1-0073 | 43 | 103 | 63 | 38 | ++ |
| PD1-0078 | 64 | 99 | 72 | 21 | ++ |

Several PD1 blocking antibodies PD1-0050, PD1-0069, PD1-0073, PD1-0078, PD1-0098, PD1-0102, PD1-0103 demonstrated strong immune modulating activity by enhancing secretion of interferon gamma (IFN-y) (data not shown for all antibodies).

**3B)** In a further experiment chimeric PD1-0103 (human IgG1 isotype with mutations L234A, L235A and P329G (EU index of Kabat)) was evaluated. Blockade of PD1 with chimeric PD1-0103 strongly enhances IFN-gamma secretion by allogenic stimulated primary human T cells. Chimeric PD1-0103 is more potent than reference anti-PD1 antibodies (see **Figure 1).**

For comparison the reference anti-PD1 antibodies comprising the VH and VL domains of either nivolumab (also known as MDX5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

**3C)** In additional experiments the immune modulating activity of the humanized variants of anti-PD1 antibody PD1-0103 (humanized antibodies PD1-0103-0312, PD1-0103-0314, in figures 2 and 3) the a) IFNγ release (secretion) b) TNF-alpha release (secretion) was evaluated in MLR as described above. The effect of the chimeric PD1-0103 antibody and its humanized versions were compared to the reference anti-PD1 antibodies comprising the VH and VL domains of either nivolumab (also known as MDX5C4 or MDX-1106) and pembrolizumab (also known as MK-3475 or Org 1.09A) with backbones of human IgG1 (with mutations L234A, L235A and P329G (EU index of Kabat)). After 6 days of MLR culture 50 μl of supernatant was taken and multiple cytokines were measured in a single culture using Bio-Plex Pro™ Human Cytokine Th1/Th2 Assay (Bio-Rad Laboratories Inc.). (data not shown for all cytokines).

The chimeric PD1-0103 antibody and its humanized versions (PD1-0103_0312 and PD1-0103_0314) were more potent compared to the reference anti-PD1 antibodies in enhancing the T cell activation and IFN-gamma secretion (see **Figure 2).**

Furthermore, the chimeric PD1-0103 antibody and its humanization variants increase tumor necrosis factor alpha (TNF alpha) (see **Figure 3)** and IL-12 (data not shown) secretion by antigen presenting cells and encance capacity of monocytes /macrophages or antigen presenting cells to stimulate a T cell.

### 1.4 Effect of anti-PD1 blockade on cytotoxic Granzyme B release and IFN-γ secretion by human CD4 T cells cocultured with allogeneic mature dendritic cells

To further investigate the effect of anti-PD1 treatment in an allogeneic setting we developed an assay in which freshly purified CD4 T cells are cocultured for 5 days in presence of monocyte-derived allogeneic mature dendritic cells (mDCs). Monocytes were isolated from fresh PBMCs one week before through plastic adherence followed by the removal of the non-adherent cells. We then generated immature DCs from the monocytes by culturing them for 5 days in media containing GM-CSF (50 ng/ml) and IL-4 (100 ng/ml). To induce iDCs maturation, we added TNF-α, IL-1β and IL-6 (50 ng/ml each) to the culturing media for 2 additional days. We then assessed DCs maturation by measuring their surface expression of Major Histocompatibility Complex Class II (MHCII), CD80, CD83 and CD86 thorugh flow cytometry (LSR-Fortessa, BD Biosciences).

On the day of the minimal mixed lymphocyte reaction (mMLR), CD4 T cells were enriched via a microbead kit (Miltenyi Biotec) from $10^8$ PBMCs obtained from an unrelated donor. Prior culture, CD4 T cells were labeled with 5μM of Carboxy-Fluorescein-Succinimidyl Esther (CFSE). $10^5$CD4 T cells were then plated in a 96 well plate together with mature allo-DCs (5:1) in presence or absence of blocking anti-PD1 antibody (either PD1-0103, chimeric PD1-0103, or humanized antibodies PD1-0103-0312, PD1-0103-0313, PD1-0103-0314, PD1-0103-0315, abbreviated as 0312, 0313, 0314, 0315 in **figures 4A and 4 B),** at the concentration of 10 μg/ml if not differentely indicated in the figures.

Five days later we collected the cell-culture supernatants, used later to measure the IFN-γ levels by ELISA (R&D systems), and left the cells at 37 C degrees for additional 5 hours in presence of Golgi Plug (Brefeldin A) and Golgi Stop (Monensin). The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience), IFN-γ and IL-2 (both from eBioscience).

We also tested different concentrations of the humanized variants PD1-0103 (humanized antibodies PD1-0103-0312, PD1-0103-0313, PD1-0103-0314, PD1-0103-0315, abbreviated as 0312, 0313, 0314, 0315 in the figures, see also Example 1.6 below) and found them to be equally good in enhancing granzyme B and interferon gamma. DP47 is a non binding human IgG with a LALA mutation in the Fc portion to avoid recognition by FcyR and was used as negative control. Results are shown in **Figures 4A and 4 B.**

### 1.5 Chimeric antibodies derivatives

Chimeric PD antibodies were generated by amplifying the variable heavy and light chain regions of the anti-PD1 mouse antibodies PD1-0098, PD1-0103 via PCR and cloning them into heavy chain expression vectors as fusion proteins with human IgG1 backbones / human CH1-Hinge-CH2-CH3 with mutations L234A, L235A and P329G (EU index of Kabat)) (Leucine 234 to Alanine, Leucine 235 to Alanine, Proline 329 to Glycine) abrogating effector functions and light chain expression vectors as fusion proteins to human C-kappa. LC and HC Plasmids were then cotransfected into HEK293 and purified after 7 days from supertnatants by standard methods for antibody purification. The chimeric PD1-antibodies were renamed chimeric chiPD1-0098 (chiPD1-0098) and chimeric PD1-0103 (chiPD1-0103). For comparison the reference anti-PD1 antibodies comprising the VH and VL domains of either nivolumab (also known as MDX-5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

**1.6 Generation, Expression and Purification of humanized variants of anti-PD1 antibody PD-0103 (huMab PD-0103) and characterization**

Humanization of the VH and VL domains of murine anti-PD1 antibody 0103

Based upon the amino acid sequence of the murine VH and VL domains of murine anti-PD1 antibody PD1-0103 (SEQ ID NO: 43 and 44), humanized anti- anti-PD1 antibody variants were generated.

The humanized VH-variant is based on the human germline IMGT_hVH_3_23 in combination with the human J-element germline IGHJ5-01 with several mutations. (resulting in SEQ ID NO: 45).

The humanized variants of VL are based on the human germlines IMGT_hVK_4_1, IMGT_hVK_2_30, IMGT_hVK_3_11 and IMGT_hVK_1_39 in combination with the human J-element germline IGKJ1-01. Different muations resulted in humanized variants of SEQ ID NO: 46 to SEQ ID NO: 49.

The humanized amino acid sequences for heavy and light chain variable regions of PD1-0103 were backtranslated in to DNA and the resulting cNDA were synthesized (GenArt) and then cloned into heavy chain expression vectors as fusion proteins with human IgG1 backbones /human CH1-Hinge-CH2-CH3 with LALA and PG mutations (Leucine 234 to Alanine, Leucine 235 to Alanine, Proline 329 to Glycine) abrogating effector functions or into light chain expression vectors as fusion proteins to human C-kappa. LC and HC Plasmids were then cotransfected into HEK293 and purified after 7 days from supertnatants by standard methods for antibody purification. The resulting humanized PD1-antibodies named as follows:

**Table 5: VH and VL sequences of humanized variant antibodies of PD1-0103**

| Humanized antibodies of PD1-0103 | humanized variant of VH/SEQ ID NO: | humanized variant of VL/SEQ ID NO: |
|---|---|---|
| PD1-0103-0312 | SEQ ID NO: 21 | SEQ ID NO: 22 |
| PD1-0103-0313 | SEQ ID NO: 21 | SEQ ID NO: 23 |
| PD1-0103-0314 | SEQ ID NO: 21 | SEQ ID NO: 24 |
| PD1-0103-0315 | SEQ ID NO: 21 | SEQ ID NO: 25 |

**Table 6: HVR sequences of humanized variant antibodies of PD1-0103**

| Humanized antibodies of PD1-0103 | HVR-H1, HVR-H2, and HVR-H3 of humanized variant/SEQ ID NO: | HVR-L1, HVR-L2, and HVR-L3 of humanized variant/SEQ ID NO: |
|---|---|---|
| PD-0103-0312 | SEQ ID NOs: 13 , 14 and 15 | SEQ ID NOs: 16, 17 and 18 |
| PD-0103-0313 | SEQ ID NOs: 13 , 14 and 15 | SEQ ID NOs: 16 , 17 and 18 |
| PD-0103-0314 | SEQ ID NOs: 13 , 14 and 15 | SEQ ID NOs: 16 , 17 and 18 |
| PD-0103-0315 | SEQ ID NOs: 13 , 14 and 15 | SEQ ID NOs: 16 , 17 and 18 |

Humanized PD1-0103 antibody variants and parental chimeric PD1-0103 were characterized as descibed above. Results are shown in Table 7.

**Table 7: Summary of results for humanized PD1-0103 antibody variants and parental chimeric PD1-0103**

| Assay | chimeric PD1-0103 | PD-0103-0312 | PD-0103-0313 | PD-0103-0314 | PD-0103-0315 |
|---|---|---|---|---|---|
| Affinity $K_D$ [nM] at 37°C | 2.0 / 0.8 | 1.5 / 1.8 | 1.9 / 2.3 | 1.6 / 1.5 | 1.7 / 1.5 |
| ELISA $EC_{50}$ [nM] | 0,2 | 0,1 | 0,07 | 0,07 | 0,06 |
| CHO-PD1 $EC_{50}$ | + | + | + | + | + |
| $IC_{50}$ PD-L1, 2 [nM] | 1.35 | tbd | tbd | tbd | tbd |
| Mixed Lymphocyte Reaction assay | +++ | +++ | +++ | ++++ | ++ |
| cynomolgus crossreactivity ($EC_{50}$ [nm] | + | 0,08 | 0,06 | 0,05 | 0,04 |

**1.7 Neutralizing potency of PD1 antibodies**

To test the neutralizing potency of inhouse generated PD antibodies in mimicking a restoration of a suppressed T cell response in vitro a commercially available PD1/PD-L1 reporter assay (Promega) was used. This system consists of PD1+ NFAT Jurkat cells and a PD-L1+ CHO counterpart, which also gives the activation signal. In principle, the reporter system is based on three steps: (1) TCR-mediated NFAT activation, (2) inhibition of NFAT signal upon activation by the PD-1/PD-L1 axis and (3) recovery of the NFAT signal by PD1 blocking antibodies.

Material and Methods:

- PD-L1 Medium: PAN Biotech (#P04-03609); FBS (10%) and L-Gln (4mM)
- Assay Medium: RPMI 1640 (#31870; Invitrogen), 25mM HEPES, 2mM L-Gln, FBS (2%)
- Cells used for this assay (both cell types purchased by Promega):

  PD-L1+ CHO cells (batch no. #139147): 2-3x104 cells/96well

  PD1+ NFAT Jurkat cells (batch no. #133024: 3.5x104 cells/well

On day 1, PD-L1+ cells were thawed, seeded at the indicated cell concentration in the above mentioned medium and cultured over night at 37°C and 5% $CO_2$. On the next day, medium was removed and PD-L1+ cells were incubated with the prepared antibodies at indicated concentrations (in Assay Medium). In parallel, PD1+ NFAT Jurkat cells were thawed and above mentioned cell numbers were transferred to and co-cultured with the PD-L1+ cells. After an incubation of 6 hrs at 37 °C and 5% $CO_2$, Bio-Glo substrate was warmed to room temperature (1-2 hrs prior addition). The cell culture plate was removed from the incubator and adjusted to room temperature (10min) before 80μl Bio-Glo solution was added per well, incubated for 5-10 min before the luminescence was measured at a Tecan Infinite reader according to the kit's manufacturer's recommendation. Results can be seen in the Figures 5A and 5B where the restoration of a PD1/PD-L1 mediated suppression of the NFAT signal by different PD1 antibodies upon TCR stimulation is shown: **Figure 5 A:** Chimeric PD1_0103 showed a reproducibly superior effect when compared to a reference antibody. As reference an anti-PD1 antibody comprising the VH and VL domains nivolumab (also known as MDX-5C4 or MDX-1106) was synthesized and cloned with backbones of human IgG1 (with mutations L234A, L235A and P329G (EU index of Kabat)). **Figure 5B:** The four humanized variants of PD1_0103 demonstrated a similar in vitro potency to the lead antibody and were also slightly superior to the reference antibody.

**Example 2**

**Preparation of PD1- targeted 4-1BB ligand trimer-containing Fc fusion antigen binding molecules**

Different fragments of the DNA sequence encoding part of the ectodomain (amino acids 71-254 and 71-248) of human 4-1BB ligand were synthetized according to the P41273 sequence of Uniprot database (SEQ ID NO:103).

**2.1 Preparation of monovalent PD1 (0314) targeted 4-1BB ligand (71-254) trimer-containing Fc (kih) fusion antigen binding molecule with crossed CH1-CL domains with charged residues (Construct 7.1)**

A polypeptide containing two ectodomains of 4-1BB ligand (71-254), separated by (G4S)2 linkers, and fused to the human IgG1-CL domain, was cloned as depicted in **Figure 6A:** human 4-1BB ligand, (G4S)2 connector, human 4-1BB ligand, (G4S)2 connector, human CL.

A polypeptide containing one ectodomain of 4-1BB ligand (71-254) and fused to the human IgG1-CH1 domain, was cloned as described in **Figure 6B:** human 4-1BB ligand, (G4S)2 connector, human CH.

To improve correct pairing the following mutations have been introduced in the crossed CH-CL. In the dimeric 4-1BB ligand fused to human CL the mutations E123R and Q124K were introduced. In the monomeric 4-1BB ligand fused to human CH1, the mutations K147E and K213E were cloned into the human CH1 domain as described in International Patent Appl. Publ. No. WO 2015/150447.

The variable region of heavy and light chain DNA sequences encoding a binder specific for PD1, clone 0314 (or 0103-0314), were subcloned in frame with either the constant heavy chain of the hole or the constant light chain of human IgG1. The generation and preparation of the PD1 binders is described in Example 1.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831.

For all constructs the knobs into hole heterodimerization technology was used with the the S354C/T366W mutations in the knob chain and the corresponding Y349C/T366S/L368A/Y407V mutations in the hole chain.

Combination of the dimeric ligand-Fc knob chain containing the S354C/T366W mutations, the monomeric CH1 fusion, the targeted anti-PD1-Fc hole chain containing the Y349C/T366S/L368A/Y407V mutations and the anti-PD1 light chain allows generation of a heterodimer, which includes an assembled trimeric 4-1BB ligand and a PD1 binding Fab (**Figure** 7, Construct 7.1).

Table 8 shows the cDNA and amino acid sequences of the monovalent PD1(0314)-human 4-1BB ligand (71-254) Fc (kih) fusion antigen binding molecule containing CH1-CL crossover and charged residues (Construct 7.1).

**Table 8: Sequences of monovalent PD1(0314)-targeted human 4-1BB ligand (71-254) containing Fc (kih) fusion molecule Construct 7.1**

| SEQ ID NO: | Description | Sequence |
|---|---|---|
| 131 | nucleotide sequence of dimeric hu 4-1BBL (71-254)-CL* Fc knob chain | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGAAGCGCT GCTGGCGCTGCAGCTCTGGCACTGACAGTGGATCTGCCT CCTGCCAGCTCCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGCCTGCCTTCT CCAAGAAGCGAAGGCGGAGGCGGATCTGGCGGCGGAG GATCTAGAGAGGGACCCGAACTGTCCCCTGACGATCCA GCCGGGCTGCTGGATCTGAGACAGGGAATGTTCGCCCA GCTGGTGGCTCAGAATGTGCTGCTGATTGACGGACCTCT GAGCTGGTACTCCGACCCAGGGCTGGCAGGGGTGTCCC TGACTGGGGGACTGTCCTACAAAGAAGATACAAAAGAA CTGGTGGTGGCTAAAGCTGGGGTGTACTATGTGTTTTT CAGCTGGAACTGAGGCGGGTGGTGGCTGGGGAGGGCTC AGGATCTGTGTCCCTGGCTCTGCATCTGCAGCCACTGCG CTCTGCTGCTGGCGCAGCTGCACTGGCTCTGACTGTGGA CCTGCCACCAGCCTCTAGCGAGGCCAGAAACAGCGCCT TCGGGTTCCAAGGACGCCTGCTGCATCTGAGCGCCGGAC AGCGCCTGGGAGTGCATCTGCATACTGAAGCCAGAGCC CGGCATGCTTGGCAGCTGACTCAGGGGGCAACTGTGCTG GGACTGTTTCGCGTGACACCTGAGATCCCTGCCGGACTG CCAAGCCCTAGATCAGAAGGGGGCGGAGGTTCCGGAGG GGGAGGATCTCGTACGGTGGCTGCACCATCTGTCTTTAT CTTCCCACCCAGCGACCGGAAGCTGAAGTCTGGCACAG CCAGCGTCGTGTGCCTGCTGAATAACTTCTACCCCCGCG AGGCCAAGGTGCAGTGGAAGGTGGACAATGCCCTGCAG AGCGGCAACAGCCAGGAAAGCGTGACCGAGCAGGACA GCAAGGACTCCACCTACAGCCTGAGCAGCACCCTGACC CTGAGCAAGGCCGACTACGAGAAGCACAAGGTGTACGC CTGCGAAGTGACCCACCAGGGCCTGTCTAGCCCCGTGAC CAAGAGCTTCAACCGGGGCGAGTGCGACAAGACCCACA CCTGTCCTCCATGCCCTGCCCCTGAAGCTGCTGGCGGCC CTAGCGTGTTCCTGTTCCCCCCAAAGCCCAAGGACACCC TGATGATCAGCCGGACCCCTGAAGTGACCTGCGTGGTGG TGGATGTGTCCCACGAGGACCCTGAAGTGAAGTTCAATT GGTACGTGGACGGCGTGGAAGTGCACAATGCCAAGACC AAGCCGCGGGAGGAGCAGTACAACAGCACGTACCGTGT GGTCAGCGTCCTCACCGTCCTGCACCAGGACTGGCTGAA TGGCAAGGAGTACAAGTGCAAGGTCTCCAACAAAGCCC TCGGCGCCCCCATCGAGAAAACCATCTCCAAAGCCAAA |

(continued)

| SEQ ID NO: | Description | Sequence |
|---|---|---|
| | | GGGCAGCCCCGAGAACCACAGGTGTACACCCTGCCCCC ATGCCGGGATGAGCTGACCAAGAACCAGGTCAGCCTGT GGTGCCTGGTCAAAGGCTTCTATCCCAGCGACATCGCCG TGGAGTGGGAGAGCAATGGGCAGCCGGAGAACAACTAC AAGACCACGCCTCCCGTGCTGGACTCCGACGGCTCCTTC TTCCTCTACAGCAAGCTCACCGTGGACAAGAGCAGGTG GCAGCAGGGGAACGTCTTCTCATGCTCCGTGATGCATGA GGCTCTGCACAACCACTACACGCAGAAGAGCCTCTCCCT GTCTCCGGGTAAA |
| 132 | nucleotide sequence of monomeric hu 4-1BBL (71-254)-CH1* | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGAAGCGCT GCTGGCGCTGCAGCTCTGGCTCTGACAGTGGATCTGCCT CCTGCCAGCTCCGAGGCCCGGAATAGCGCATTTGGGTTT CAAGGCCGGCTGCTGCACCTGTCTGCCGGCCAGAGACT GGGAGTGCATCTGCACACAGAGGCCAGAGCCAGGCACG CCTGGCAGCTGACACAGGGCGCTACAGTGCTGGGCCTG TTCAGAGTGACCCCCGAGATTCCTGCCGGCCTGCCTAGC CCTAGATCTGAAGGCGGCGGAGGTTCCGGAGGCGGAGG ATCTGCTAGCACAAAGGGCCCCAGCGTGTTCCCTCTGGC CCCTAGCAGCAAGAGCACATCTGGCGGAACAGCCGCCC TGGGCTGCCTGGTGGAAGATTACTTCCCCGAGCCCGTGA CCGTGTCCTGGAATTCTGGCGCCCTGACAAGCGGCGTGC ACACCTTTCCAGCCGTGCTGCAGAGCAGCGGCCTGTACT CTCTGAGCAGCGTCGTGACAGTGCCCAGCAGCTCTCTGG GCACCCAGACCTACATCTGCAACGTGAACCACAAGCCC AGCAACACCAAGGTGGACGAGAAGGTGGAACCCAAGTC CTGC |

(continued)

| SEQ ID NO: | Description | Sequence |
|---|---|---|
| 133 | nucleotide sequence of anti-PD1(0314) Fc hole chain | GAAGTGCAGCTGCTCGAAAGCGGCGGGGGACTGGTCCA GCCCGGCGGTTCCCTGAGGCTGTCTTGCGCCGCTTCAGG GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA AAGGACGGTTCACCATCTCTAGGGACAACTCAAAGAAT ACTCTGTATTTGCAGATGAACAGCCTGAGAGCTGAGGAT ACAGCAGTTTACTACTGTGTGCTCCTGACCGGCCGCGTC TATTTTGCCCTTGACTCCTGGGGACAAGGCACTCTGGTG ACCGTATCTAGTGCTAGCACCAAGGGCCCCTCCGTGTTC CCCCTGGCCCCCAGCAGCAAGAGCACCAGCGGCGGCAC AGCCGCTCTGGGCTGCCTGGTCAAGGACTACTTCCCCGA GCCCGTGACCGTGTCCTGGAACAGCGGAGCCCTGACCTC CGGCGTGCACACCTTCCCCGCCGTGCTGCAGAGTTCTGG CCTGTATAGCCTGAGCAGCGTGGTCACCGTGCCTTCTAG CAGCCTGGGCACCCAGACCTACATCTGCAACGTGAACC ACAAGCCCAGCAACACCAAGGTGGACAAGAAGGTGGA GCCCAAGAGCTGCGACAAAACTCACACATGCCCACCGT GCCCAGCACCTGAAGCTGCAGGGGGACCGTCAGTCTTC CTCTTCCCCCCAAAACCCAAGGACACCCTCATGATCTCC |
| | | CGGACCCCTGAGGTCACATGCGTGGTGGTGGACGTGAG CCACGAAGACCCTGAGGTCAAGTTCAACTGGTACGTGG ACGGCGTGGAGGTGCATAATGCCAAGACAAAGCCGCGG GAGGAGCAGTACAACAGCACGTACCGTGTGGTCAGCGT CCTCACCGTCCTGCACCAGGACTGGCTGAATGGCAAGG AGTACAAGTGCAAGGTCTCCAACAAAGCCCTCGGCGCC CCCATCGAGAAAACCATCTCCAAAGCCAAAGGGCAGCC CCGAGAACCACAGGTGTGCACCCTGCCCCCATCCCGGG ATGAGCTGACCAAGAACCAGGTCAGCCTCTCGTGCGCA GTCAAAGGCTTCTATCCCAGCGACATCGCCGTGGAGTGG GAGAGCAATGGGCAGCCGGAGAACAACTACAAGACCAC GCCTCCCGTGCTGGACTCCGACGGCTCCTTCTTCCTCGT GAGCAAGCTCACCGTGGACAAGAGCAGGTGGCAGCAGG GGAACGTCTTCTCATGCTCCGTGATGCATGAGGCTCTGC ACAACCACTACACGCAGAAGAGCCTCTCCCTGTCTCCGG GTAAA |

(continued)

| SEQ ID NO: | Description | Sequence |
|---|---|---|
| 134 | nucleotide sequence of anti-PD1 (0314) light chain | GAGATCGTGCTGACCCAGAGCCCCGCCACACTCTCCCTG TCTCCTGGCGAAAGGGCTACCCTTTCATGCAGAGCAAGC GAGTCCGTGGACACTTCTGATAACAGTTTCATTCACTGG TATCAGCAGAAGCCAGGGCAGAGCCCCCGCCTGCTCAT CTATCGGTCCTCTACACTGGAGTCAGGGATTCCTGCCAG GTTTAGCGGCTCCGGTTCTGGGACCGACTTCACTTTGAC CATCAGTAGCCTGGAACCAGAGGATTTTGCTGTGTACTA TTGTCAACAGAATTACGACGTCCCCTGGACATTCGGCCA GGGAACCAAAGTGGAGATAAAGCGTACGGTGGCTGCAC CATCTGTCTTCATCTTCCCGCCATCTGATGAGCAGTTGA AATCTGGAACTGCCTCTGTTGTGTGCCTGCTGAATAACT TCTATCCCAGAGAGGCCAAAGTACAGTGGAAGGTGGAT AACGCCCTCCAATCGGGTAACTCCCAGGAGAGTGTCAC AGAGCAGGACAGCAAGGACAGCACCTACAGCCTCAGCA GCACCCTGACGCTGAGCAAAGCAGACTACGAGAAACAC AAAGTCTACGCCTGCGAAGTCACCCATCAGGGCCTGAG CTCGCCCGTCACAAAGAGCTTCAACAGGGGAGAGTGT |
| 74 | Dimeric hu 4-1BBL (71-254)-CL* Fc knob chain | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSREGPEL SPDDPAGLLDLRQGMFAQLVAQNVLLIDGPLSWYSDPGLA GVSLTGGLSYKEDTKELVVAKAGVYYVFFQLELRRVVAG EGSGSVSLALHLQPLRSAAGAAALALTVDLPPASSEARNS AFGFQGRLLHLSAGQRLGVHLHTEARARHAWQLTQGATV LGLFRVTPEIPAGLPSPRSEGGGGSGGGGSRTVAAPSVFIFP PSDRKLKSGTASVVCLLNNFYPREAKVQWKVDNALQSGN SQESVTEQDSKDSTYSLSSTLTLSKADYEKHKVYACEVTH QGLSSPVTKSFNRGECDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGK |
| 75 | Monomeric hu 4-1BBL (71-254)-CH1* | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSASTKGP SVFPLAPSSKSTSGGTAALGCLVEDYFPEPVTVSWNSGALT SGVHTFPAVLQSSGLYSLSSVVTVPSSSLGTQTYICNVNHK PSNTKVDEKVEPKSC |

(continued)

| SEQ ID NO: | Description | Sequence |
|---|---|---|
| 82 | anti-PD1 (0314) Fc hole chain | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVCTLPPSRDELTK NQVSLSCAVKGFYPSDIAVEWESNGQPENNYKTTPPVLDS DGSFFLVSKLTVDKSRWQQGNVFSCSVMHEALHNHYTQK SLSLSPGK |
| 83 | anti-PD1 (0314) light chain | EIVLTQSPATLSLSPGERATLSCRASESVDTSDNSFIHWYQQ KPGQSPRLLIYRSSTLESGIPARFSGSGSGTDFTLTISSLEPED FAVYYCQQNYDVPWTFGQGTKVEIKRTVAAPSVFIFPPSD EQLKSGTASVVCLLNNFYPREAKVQWKVDNALQSGNSQE SVTEQDSKDSTYSLSSTLTLSKADYEKHKVYACEVTHQGL SSPVTKSFNRGEC |

**2. 2 Preparation of monovalent PD1 (0314) targeted 4-1BB ligand (71-254) trimer-containing Fc (kih) fusion antigen binding molecule with crossed CH1-CL domains without charged residues (Construct 7.2)**

A polypeptide containing two ectodomains of 4-1BB ligand (71-254), separated by (G4S)2 linkers, and fused to the human IgG1-CL domain, was cloned as depicted in **Figure 6C:** human 4-1BB ligand, (G4S)2 connector, human 4-1BB ligand, (G4S)2 connector, human CL.

A polypeptide containing one ectodomain of 4-1BB ligand (71-254) and fused to the human IgG1-CH1 domain, was cloned as described in **Figure 6D:** human 4-1BB ligand, (G4S)2 connector, human CH1.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors (WO 2012/130831).

Combination of the dimeric ligand-Fc knob chain containing the S354C/T366W mutations, the monomeric CH1 fusion, the targeted anti-FAP-Fc hole chain containing the Y349C/T366S/L368A/Y407V mutations and the anti-FAP light chain allows generation of a heterodimer, which includes an assembled trimeric 4-1BB ligand and a FAP binding Fab (**Figure 7,** Construct 7.2).

Table 9 shows the cDNA and amino acid sequences of the monovalent anti-PD1(0314) human 4-1BB ligand (71-254) Fc (kih) fusion antigen binding molecule containing CH1-CL crossover without charged residues (Construct 7.2).

**Table 9: Sequences of monovalent PD1(0314)-targeted human 4-1BB ligand (71-254) containing Fc (kih) fusion molecule Construct 7.2**

| SEQ ID NO: | Description | Sequence |
|---|---|---|
| 135 | nucleotide sequence of dimeric hu 4-1BBL (71-254)-CL Fc knob chain | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGAAGCGCT GCTGGCGCTGCAGCTCTGGCACTGACAGTGGATCTGCCT CCTGCCAGCTCCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGCCTGCCTTCT CCAAGAAGCGAAGGCGGAGGCGGATCTGGCGGCGGAG GATCTAGAGAGGGACCCGAACTGTCCCCTGACGATCCA GCCGGGCTGCTGGATCTGAGACAGGGAATGTTCGCCCA GCTGGTGGCTCAGAATGTGCTGCTGATTGACGGACCTCT GAGCTGGTACTCCGACCCAGGGCTGGCAGGGGTGTCCC TGACTGGGGGACTGTCCTACAAAGAAGATACAAAAGAA CTGGTGGTGGCTAAAGCTGGGGTGTACTATGTGTTTTT CAGCTGGAACTGAGGCGGGTGGTGGCTGGGGAGGGCTC AGGATCTGTGTCCCTGGCTCTGCATCTGCAGCCACTGCG CTCTGCTGCTGGCGCAGCTGCACTGGCTCTGACTGTGGA CCTGCCACCAGCCTCTAGCGAGGCCAGAAACAGCGCCT TCGGGTTCCAAGGACGCCTGCTGCATCTGAGCGCCGGAC AGCGCCTGGGAGTGCATCTGCATACTGAAGCCAGAGCC CGGCATGCTTGGCAGCTGACTCAGGGGGCAACTGTGCTG GGACTGTTTCGCGTGACACCTGAGATCCCTGCCGGACTG CCAAGCCCTAGATCAGAAGGGGGCGGAGGTTCCGGAGG GGGAGGATCTCGTACGGTGGCCGCTCCCTCCGTGTTTAT CTTTCCCCCATCCGATGAACAGCTGAAAAGCGGCACCGC CTCCGTCGTGTGTCTGCTGAACAATTTTTACCCTAGGGA AGCTAAAGTGCAGTGGAAAGTGGATAACGCACTGCAGT |

(continued)

| SEQ ID NO: | Description | Sequence |
|---|---|---|
| | | CCGGCAACTCCCAGGAATCTGTGACAGAACAGGACTCC AAGGACAGCACCTACTCCCTGTCCTCCACCCTGACACTG TCTAAGGCTGATTATGAGAAACACAAAGTCTACGCCTGC GAAGTCACCCATCAGGGCCTGAGCTCGCCCGTCACAAA GAGCTTCAACAGGGGAGAGTGTGACAAGACCCACACCT GTCCCCCTTGTCCTGCCCCTGAAGCTGCTGGCGGCCCTT CTGTGTTCCTGTTCCCCCCAAAGCCCAAGGACACCCTGA TGATCAGCCGGACCCCCGAAGTGACCTGCGTGGTGGTG GATGTGTCCCACGAGGACCCTGAAGTGAAGTTCAATTGG TACGTGGACGGCGTGGAAGTGCACAATGCCAAGACCAA GCCGCGGGAGGAGCAGTACAACAGCACGTACCGTGTGG TCAGCGTCCTCACCGTCCTGCACCAGGACTGGCTGAATG GCAAGGAGTACAAGTGCAAGGTCTCCAACAAAGCCCTC GGCGCCCCCATCGAGAAAACCATCTCCAAAGCCAAAGG GCAGCCCCGAGAACCACAGGTGTACACCCTGCCCCCAT GCCGGGATGAGCTGACCAAGAACCAGGTCAGCCTGTGG TGCCTGGTCAAAGGCTTCTATCCCAGCGACATCGCCGTG GAGTGGGAGAGCAATGGGCAGCCGGAGAACAACTACAA GACCACGCCTCCCGTGCTGGACTCCGACGGCTCCTTCTT CCTCTACAGCAAGCTCACCGTGGACAAGAGCAGGTGGC AGCAGGGGAACGTCTTCTCATGCTCCGTGATGCATGAGG CTCTGCACAACCACTACACGCAGAAGAGCCTCTCCCTGT CTCCGGGTAAA |
| 136 | nucleotide sequence of monomeric hu 4-1BBL (71-254)-CH1 | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGAAGCGCT GCTGGCGCTGCAGCTCTGGCTCTGACAGTGGATCTGCCT CCTGCCAGCTCCGAGGCCCGGAATAGCGCATTTGGGTTT CAAGGCCGGCTGCTGCACCTGTCTGCCGGCCAGAGACT GGGAGTGCATCTGCACACAGAGGCCAGAGCCAGGCACG CCTGGCAGCTGACACAGGGCGCTACAGTGCTGGGCCTG TTCAGAGTGACCCCCGAGATTCCTGCCGGCCTGCCTAGC CCTAGATCTGAAGGCGGCGGAGGTTCCGGAGGCGGAGG ATCTGCTAGCACCAAAGGCCCTTCCGTGTTTCCTCTGGC TCCTAGCTCCAAGTCCACCTCTGGAGGCACCGCTGCTCT CGGATGCCTCGTGAAGGATTATTTTCCTGAGCCTGTGAC AGTGTCCTGGAATAGCGGAGCACTGACCTCTGGAGTGC ATACTTTCCCCGCTGTGCTGCAGTCCTCTGGACTGTACA GCCTGAGCAGCGTGGTGACAGTGCCCAGCAGCAGCCTG GGCACCCAGACCTACATCTGCAACGTGAACCACAAGCC CAGCAACACCAAGGTGGACAAGAAGGTGGAACCCAAGT CTTGT |

(continued)

| SEQ ID NO: | Description | Sequence |
|---|---|---|
| 133 | nucleotide sequence of anti-PD1(0314) Fc | see Table 8 |
| | hole chain | |
| 134 | nucleotide sequence of anti-PD 1(0314) light chain | see Table 8 |
| 76 | Dimeric hu 4-1BBL (71-254)-CL Fc knob chain | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSREGPEL SPDDPAGLLDLRQGMFAQLVAQNVLLIDGPLSWYSDPGLA GVSLTGGLSYKEDTKELVVAKAGVYYVFFQLELRRVVAG EGSGSVSLALHLQPLRSAAGAAALALTVDLPPASSEARNS AFGFQGRLLHLSAGQRLGVHLHTEARARHAWQLTQGATV LGLFRVTPEIPAGLPSPRSEGGGGSGGGGSRTVAAPSVFIFP PSDEQLKSGTASVVCLLNNFYPREAKVQWKVDNALQSGN SQESVTEQDSKDSTYSLSSTLTLSKADYEKHKVYACEVTH QGLSSPVTKSFNRGECDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGK |
| 77 | Monomeric hu 4-1BBL (71-254)-CH1 | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSASTKGP SVFPLAPSSKSTSGGTAALGCLVKDYFPEPVTVSWNSGALT SGVHTFPAVLQSSGLYSLSSVVTVPSSSLGTQTYICNVNHK PSNTKVDKKVEPKSC |
| 82 | anti-PD1(0314) Fc hole chain | see Table 8 |
| 83 | anti-PD1(0314) light chain | see Table 8 |

**2.3 Preparation of bivalent PD1(0314) targeted 4-1BB ligand (71-254) trimer-containing Fc (kih) fusion antigen binding molecule with the dimeric and monomeric 4-1BB ligands fused at the C-terminus of each heavy chain (Construct 7.3)**

A polypeptide containing two ectodomains of 4-1BB ligand (71-254), separated by (G4S)2 linkers was fused to the C-terminus of human IgG1 Fc hole chain, as depicted in **Figure 6E:** human IgG1 Fc hole, (G4S)2 connector, human 4-1BB ligand, (G4S)2 connector, human 4-1BB ligand. A polypeptide containing one ectodomain of 4-1BB ligand (71-254) was fused to the C-terminus of human IgG1 Fc knob chain as described in **Figure 6F:** human IgG1 Fc knob, (G4S)2 connector, human 4-1BB ligand.

The variable region of heavy and light chain DNA sequences encoding a binder specific for PD1, clone 0103-0314, were subcloned in frame with either the constant heavy chain of the hole, the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-FAP huIgG1 hole dimeric ligand chain containing the Y349C/T366S/L368A/Y407V mutations, the anti-FAP huIgG1 knob monomeric ligand chain containing the S354C/T366W mutations and the anti-FAP light chain allows generation of a heterodimer, which includes an assembled trimeric 4-1BB ligand and two PD1 binding Fabs

**(Figure 7,** Construct 7.3)

Table 10 shows the cDNA and amino acid sequences of the bivalent PD1(0314)-targeted 4-1BB ligand trimer-containing Fc (kih) fusion molecule Construct 7.3 (PD1 split trimer with 2 anti-PD1 Fabs, dimeric and monomeric 4-1BB ligand fused at the C-terminus of each heavy chain, respectively).

**Table 10: Sequences of bivalent PD1(0314)-targeted human 4-1BB ligand (71-254) containing Fc (kih) fusion molecule Construct 7.3**

| SEQ ID NO: | Description | Sequence |
|---|---|---|
| 137 | nucleotide sequence of anti-PD1(0314) Fc hole chain fused to dimeric hu 4-1BBL (71-254) | GAAGTGCAGCTGCTCGAAAGCGGCGGGGGACTGGTCCA GCCCGGCGGTTCCCTGAGGCTGTCTTGCGCCGCTTCAGG GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA AAGGACGGTTCACCATCTCTAGGGACAACTCAAAGAAT ACTCTGTATTTGCAGATGAACAGCCTGAGAGCTGAGGAT ACAGCAGTTTACTACTGTGTGCTCCTGACCGGCCGCGTC TATTTTGCCCTTGACTCCTGGGGACAAGGCACTCTGGTG ACCGTATCTAGTGCTAGCACCAAGGGCCCCTCCGTGTTC CCCCTGGCCCCCAGCAGCAAGAGCACCAGCGGCGGCAC AGCCGCTCTGGGCTGCCTGGTCAAGGACTACTTCCCCGA GCCCGTGACCGTGTCCTGGAACAGCGGAGCCCTGACCTC CGGCGTGCACACCTTCCCCGCCGTGCTGCAGAGTTCTGG CCTGTATAGCCTGAGCAGCGTGGTCACCGTGCCTTCTAG |

(continued)

| SEQ ID NO: | Description | Sequence |
|---|---|---|
| | | CAGCCTGGGCACCCAGACCTACATCTGCAACGTGAACC ACAAGCCCAGCAACACCAAGGTGGACAAGAAGGTGGA GCCCAAGAGCTGCGACAAAACTCACACATGCCCACCGT GCCCAGCACCTGAAGCTGCAGGGGGACCGTCAGTCTTC CTCTTCCCCCCAAAACCCAAGGACACCCTCATGATCTCC CGGACCCCTGAGGTCACATGCGTGGTGGTGGACGTGAG CCACGAAGACCCTGAGGTCAAGTTCAACTGGTACGTGG ACGGCGTGGAGGTGCATAATGCCAAGACAAAGCCGCGG GAGGAGCAGTACAACAGCACGTACCGTGTGGTCAGCGT CCTCACCGTCCTGCACCAGGACTGGCTGAATGGCAAGG AGTACAAGTGCAAGGTCTCCAACAAAGCCCTCGGCGCC CCCATCGAGAAAACCATCTCCAAAGCCAAAGGGCAGCC CCGAGAACCACAGGTGTGCACCCTGCCCCCATCCCGGG ATGAGCTGACCAAGAACCAGGTCAGCCTCTCGTGCGCA GTCAAAGGCTTCTATCCCAGCGACATCGCCGTGGAGTGG GAGAGCAATGGGCAGCCGGAGAACAACTACAAGACCAC GCCTCCCGTGCTGGACTCCGACGGCTCCTTCTTCCTCGT GAGCAAGCTCACCGTGGACAAGAGCAGGTGGCAGCAGG GGAACGTCTTCTCATGCTCCGTGATGCATGAGGCTCTGC ACAACCACTACACGCAGAAGAGCCTCTCCCTGTCTCCGG GTGGAGGCGGCGGAAGCGGAGGAGGAGGATCCAGAGA GGGCCCTGAGCTGAGCCCCGATGATCCTGCTGGACTGCT GGACCTGCGGCAGGGCATGTTTGCTCAGCTGGTGGCCCA GAACGTGCTGCTGATCGATGGCCCCCTGTCCTGGTACAG CGATCCTGGACTGGCTGGCGTGTCACTGACAGGCGGCCT GAGCTACAAAGAGGACACCAAAGAACTGGTGGTGGCCA AGGCCGGCGTGTACTACGTGTTCTTTCAGCTGGAACTGC GGAGAGTGGTGGCCGGCGAAGGATCTGGCTCTGTGTCTC TGGCCCTGCATCTGCAGCCTCTGAGAAGCGCTGCTGGCG CTGCAGCTCTGGCACTGACAGTGGATCTGCCTCCTGCCA GCTCCGAGGCCCGGAATAGCGCATTTGGGTTTCAAGGCA GGCTGCTGCACCTGTCTGCCGGCCAGAGGCTGGGAGTG CATCTGCACACAGAGGCCAGGGCTAGACACGCCTGGCA GCTGACACAGGGCGCTACAGTGCTGGGCCTGTTCAGAGT GACCCCCGAGATTCCAGCCGGCCTGCCTTCTCCAAGAAG CGAAGGCGGAGGCGGATCTGGCGGCGGAGGATCTAGAG AGGGACCCGAACTGTCCCCTGACGATCCAGCCGGGCTG CTGGATCTGAGACAGGGAATGTTCGCCCAGCTGGTGGCT CAGAATGTGCTGCTGATTGACGGACCTCTGAGCTGGTAC TCCGACCCAGGGCTGGCAGGGGTGTCCCTGACTGGGGG ACTGTCCTACAAAGAAGATACAAAAGAACTGGTGGTGG CTAAAGCTGGGGTGTACTATGTGTTTTTCAGCTGGAAC TGAGGCGGGTGGTGGCTGGGGAGGGCTCAGGATCTGTG TCCCTGGCTCTGCATCTGCAGCCACTGCGCTCTGCTGCT GGCGCAGCTGCACTGGCTCTGACTGTGGACCTGCCACCA GCCTCTAGCGAGGCCAGAAACAGCGCCTTCGGGTTCCA AGGACGCCTGCTGCATCTGAGCGCCGGACAGCGCCTGG GAGTGCATCTGCATACTGAAGCCAGAGCCCGGCATGCTT GGCAGCTGACTCAGGGGGCAACTGTGCTGGGACTGTTTC GCGTGACACCTGAGATCCCTGCCGGACTGCCAAGCCCTA GATCAGAA |

(continued)

| SEQ ID NO: | Description | Sequence |
|---|---|---|
| 138 | nucleotide sequence of anti- | GAAGTGCAGCTGCTCGAAAGCGGCGGGGGACTGGTCCA GCCCGGCGGTTCCCTGAGGCTGTCTTGCGCCGCTTCAGG |

(continued)

| SEQ ID NO: | Description | Sequence |
|---|---|---|
| | PD1(0314) Fc knob chain fused to monomeric hu 4-1BBL (71-254) | GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA AAGGACGGTTCACCATCTCTAGGGACAACTCAAAGAAT ACTCTGTATTTGCAGATGAACAGCCTGAGAGCTGAGGAT ACAGCAGTTTACTACTGTGTGCTCCTGACCGGCCGCGTC TATTTTGCCCTTGACTCCTGGGGACAAGGCACTCTGGTG ACCGTATCTAGTGCTAGCACCAAGGGCCCATCGGTCTTC CCCCTGGCACCCTCCTCCAAGAGCACCTCTGGGGGCACA GCGGCCCTGGGCTGCCTGGTCAAGGACTACTTCCCCGAA CCGGTGACGGTGTCGTGGAACTCAGGCGCCCTGACCAG CGGCGTGCACACCTTCCCGGCTGTCCTACAGTCCTCAGG ACTCTACTCCCTCAGCAGCGTGGTGACCGTGCCCTCCAG CAGCTTGGGCACCCAGACCTACATCTGCAACGTGAATCA CAAGCCCAGCAACACCAAGGTGGACAAGAAAGTTGAGC CCAAATCTTGTGACAAAACTCACACATGCCCACCGTGCC CAGCACCTGAAGCTGCAGGGGGACCGTCAGTCTTCCTCT TCCCCCCAAAACCCAAGGACACCCTCATGATCTCCCGGA CCCCTGAGGTCACATGCGTGGTGGTGGACGTGAGCCAC GAAGACCCTGAGGTCAAGTTCAACTGGTACGTGGACGG CGTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGTA CAAGTGCAAGGTCTCCAACAAAGCCCTCGGCGCCCCCA TCGAGAAAACCATCTCCAAAGCCAAAGGGCAGCCCCGA GAACCACAGGTGTACACCCTGCCCCCCTGCAGAGATGA GCTGACCAAGAACCAGGTGTCCCTGTGGTGTCTGGTCAA GGGCTTCTACCCCAGCGATATCGCCGTGGAGTGGGAGA GCAACGGCCAGCCTGAGAACAACTACAAGACCACCCCC CCTGTGCTGGACAGCGACGGCAGCTTCTTCCTGTACTCC AAACTGACCGTGGACAAGAGCCGGTGGCAGCAGGGCAA CGTGTTCAGCTGCAGCGTGATGCACGAGGCCCTGCACA ACCACTACACCCAGAAGTCCCTGAGCCTGAGCCCCGGC GGAGGCGGCGGAAGCGGAGGAGGAGGATCCAGAGAGG GCCCTGAGCTGAGCCCCGATGATCCTGCTGGACTGCTGG ACCTGCGGCAGGGCATGTTTGCTCAGCTGGTGGCCCAGA ACGTGCTGCTGATCGATGGCCCCCTGTCCTGGTACAGCG ATCCTGGACTGGCTGGCGTGTCACTGACAGGCGGCCTGA GCTACAAAGAGGACACCAAAGAACTGGTGGTGGCCAAG GCCGGCGTGTACTACGTGTTCTTTCAGCTGGAACTGCGG AGAGTGGTGGCCGGCGAAGGATCTGGCTCTGTGTCTCTG GCCCTGCATCTGCAGCCTCTGAGAAGCGCTGCTGGCGCT GCAGCTCTGGCACTGACAGTGGATCTGCCTCCTGCCAGC TCCGAGGCCCGGAATAGCGCATTTGGGTTTCAAGGCAG GCTGCTGCACCTGTCTGCCGGCCAGAGGCTGGGAGTGC ATCTGCACACAGAGGCCAGGGCTAGACACGCCTGGCAG CTGACACAGGGCGCTACAGTGCTGGGCCTGTTCAGAGTG ACCCCCGAGATTCCAGCCGGCCTGCCTTCTCCAAGAAGC GAA |

(continued)

| SEQ ID NO: | Description | Sequence |
|---|---|---|
| 134 | nucleotide sequence of anti-PD 1(0314) light | see Table 8 |
| | chain | |
| 84 | anti-PD1(0314) Fc hole chain fused to dimeric hu 4-1BBL (71-254) | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVCTLPPSRDELTK NQVSLSCAVKGFYPSDIAVEWESNGQPENNYKTTPPVLDS DGSFFLVSKLTVDKSRWQQGNVFSCSVMHEALHNHYTQK SLSLSPGGGGGSGGGGSREGPELSPDDPAGLLDLRQGMFA QLVAQNVLLIDGPLSWYSDPGLAGVSLTGGLSYKEDTKEL VVAKAGVYYVFFQLELRRVVAGEGSGSVSLALHLQPLRSA AGAAALALTVDLPPASSEARNSAFGFQGRLLHLSAGQRLG VHLHTEARARHAWQLTQGATVLGLFRVTPEIPAGLPSPRSE GGGGSGGGGSREGPELSPDDPAGLLDLRQGMFAQLVAQN VLLIDGPLSWYSDPGLAGVSLTGGLSYKEDTKELVVAKAG VYYVFFQLELRRVVAGEGSGSVSLALHLQPLRSAAGAAAL ALTVDLPPASSEARNSAFGFQGRLLHLSAGQRLGVHLHTE ARARHAWQLTQGATVLGLFRVTPEIPAGLPSPRSE |
| 85 | anti-PD1(0314) Fc knob chain fused to monomeric hu 4-1BBL (71-254) | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVYTLPPCRDELTK NQVSLWCLVKGFYPSDIAVEWESNGQPENNYKTTPPVLDS DGSFFLYSKLTVDKSRWQQGNVFSCSVMHEALHNHYTQK SLSLSPGGGGGSGGGGSREGPELSPDDPAGLLDLRQGMFA QLVAQNVLLIDGPLSWYSDPGLAGVSLTGGLSYKEDTKEL VVAKAGVYYVFFQLELRRVVAGEGSGSVSLALHLQPLRSA AGAAALALTVDLPPASSEARNSAFGFQGRLLHLSAGQRLG VHLHTEARARHAWQLTQGATVLGLFRVTPEIPAGLPSPRSE |
| 83 | anti-PD1(0314) light chain | see Table 21 |

**2.4 Preparation of monovalent PD1(0314) targeted 4-1BB ligand (71-248) trimer-containing Fc (kih) fusion antigen binding molecule with crossed CH1-CL domains with charged residues (Construct 7.4)**

A polypeptide containing two ectodomains of 4-1BB ligand (71-248), separated by (G4S)2 linkers, and fused to the human IgG1-CL domain, was cloned as depicted in **Figure 8A:** human 4-1BB ligand, (G4S)2 connector, human 4-1BB ligand, (G4S)2 connector, human CL. A polypeptide containing one ectodomain of 4-1BB ligand (71-248) and fused to the human IgG1-CH domain, was cloned as described in **Figure 8B:** human 4-1BB ligand, (G4S)2 connector, human CH1.

To improve correct pairing the following mutations have been introduced in the crossed CH-CL. In the dimeric 4-1BB ligand fused to human CL the mutations E123R and Q124K were introduced. In the monomeric 4-1BB ligand fused to human CH1, the mutations K147E and K213E were cloned into the human CH1 domain as described in International Patent Appl. Publ. No. WO 2015/150447.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the dimeric ligand-Fc knob chain containing the S354C/T366W mutations, the monomeric CH1 fusion, the targeted anti-PD1(0314)-Fc hole chain containing the Y349C/T366S/L368A/Y407V mutations and the anti-PD1(0314) light chain allows generation of a heterodimer, which includes an assembled trimeric 4-1BB ligand and a FAP binding Fab (**Figure 7,** Construct 7.4).

**Table 11** shows the cDNA and amino acid sequences of the monovalent PD1(0314)-human 4-1BB ligand (71-248) Fc (kih) fusion antigen binding molecule containing CH1-CL crossover with charged residues (Construct 7.4).

**Table 11: Sequences of monovalent PD1(0314)-targeted human 4-1BB ligand (71-248) containing Fc (kih) fusion molecule Construct 7.4**

| SEQ ID NO: | Description | Sequence |
|---|---|---|
| 139 | nucleotide sequence of dimeric hu 4-1BBL (71-248)-CL*Fc knob chain | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGATCTGCT GCTGGCGCCGCTGCTCTGGCACTGACAGTGGATCTGCCT CCTGCCAGCAGCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGACTGGGAGG CGGCGGATCTGGCGGCGGAGGATCTAGAGAAGGACCCG AGCTGTCCCCTGACGATCCAGCCGGGCTGCTGGATCTGA GACAGGGAATGTTCGCCCAGCTGGTGGCTCAGAATGTG CTGCTGATTGACGGACCTCTGAGCTGGTACTCCGACCCA GGGCTGGCAGGGGTGTCCCTGACTGGGGGACTGTCCTAC AAAGAAGATACAAAAGAACTGGTGGTGGCTAAAGCTGG GGTGTACTATGTGTTTTTCAGCTGGAACTGAGGCGGGT GGTGGCTGGGGAGGGCTCAGGATCTGTGTCCCTGGCTCT GCATCTGCAGCCACTGCGCTCTGCAGCAGGGGCTGCAG CACTGGCCCTGACTGTGGACCTGCCCCCAGCTTCTTCCG AGGCCAGAAACAGCGCCTTCGGGTTCCAAGGACGCCTG CTGCATCTGAGCGCCGGACAGCGCCTGGGAGTGCATCT GCATACTGAAGCCAGAGCCCGGCATGCTTGGCAGCTGA CTCAGGGGGCAACTGTGCTGGGACTGTTTCGCGTGACAC CTGAGATCCCCGCTGGACTGGGCGGAGGCGGTTCCGGA GGGGGAGGATCTCGTACGGTGGCTGCACCATCTGTCTTT ATCTTCCCACCCAGCGACCGGAAGCTGAAGTCTGGCAC AGCCAGCGTCGTGTGCCTGCTGAATAACTTCTACCCCCG CGAGGCCAAGGTGCAGTGGAAGGTGGACAATGCCCTGC AGAGCGGCAACAGCCAGGAAAGCGTGACCGAGCAGGA CAGCAAGGACTCCACCTACAGCCTGAGCAGCACCCTGA CCCTGAGCAAGGCCGACTACGAGAAGCACAAGGTGTAC GCCTGCGAAGTGACCCACCAGGGCCTGTCTAGCCCCGTG ACCAAGAGCTTCAACCGGGGCGAGTGCGACAAGACCCA CACCTGTCCTCCATGCCCTGCCCCTGAAGCTGCTGGCGG CCCTAGCGTGTTCCTGTTCCCCCCAAAGCCCAAGGACAC CCTGATGATCAGCCGGACCCCTGAAGTGACCTGCGTGGT GGTGGATGTGTCCCACGAGGACCCTGAAGTGAAGTTCA ATTGGTACGTGGACGGCGTGGAAGTGCACAATGCCAAG ACCAAGCCGCGGGAGGAGCAGTACAACAGCACGTACCG TGTGGTCAGCGTCCTCACCGTCCTGCACCAGGACTGGCT GAATGGCAAGGAGTACAAGTGCAAGGTCTCCAACAAAG CCCTCGGCGCCCCCATCGAGAAAACCATCTCCAAAGCC |

(continued)

| SEQ ID NO: | Description | Sequence |
|---|---|---|
| | | AAAGGGCAGCCCCGAGAACCACAGGTGTACACCCTGCCCCCATGCCGGGATGAGCTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA |
| 140 | nucleotide sequence of monomeric hu 4-1BBL (71-248)-CH1* | AGAGAGGGCCCTGAGCTGAGCCCCGATGATCCTGCTGGACTGCTGGACCTGCGGCAGGGCATGTTTGCTCAGCTGGTGGCCCAGAACGTGCTGCTGATCGATGGCCCCCTGTCCTGGTACAGCGATCCTGGACTGGCTGGCGTGTCACTGACAGGCGGCCTGAGCTACAAAGAGGACACCAAAGAACTGGTGGTGGCCAAGGCCGGCGTGTACTACGTGTTCTTTCAGCTGGAACTGCGGAGAGTGGTGGCCGGCGAAGGATCTGGCTCTGTGTCTCTGGCCCTGCATCTGCAGCCTCTGAGATCTGCTGCTGGCGCCGCTGCTCTGGCACTGACAGTGGATCTGCCTCCTGCCAGCAGCGAGGCCCGGAATAGCGCATTTGGGTTTCAAGGCAGGCTGCTGCACCTGTCTGCCGGCCAGAGGCTGGGAGTGCATCTGCACACAGAGGCCAGGGCTAGACACGCCTGGCAGCTGACACAGGGCGCTACAGTGCTGGGCCTGTTCAGAGTGACCCCCGAGATTCCAGCCGGACTGGGAGGCGGAGGTTCCGGAGGCGGAGGATCTGCTAGCACAAAGGGCCCCAGCGTGTTCCCTCTGGCCCCTAGCAGCAAGAGCACATCTGGCGGAACAGCCGCCCTGGGCTGCCTGGTGGAAGATTACTTCCCCGAGCCCGTGACCGTGTCCTGGAATTCTGGCGCCCTGACAAGCGGCGTGCACACCTTTCCAGCCGTGCTGCAGAGCAGCGGCCTGTACTCTCTGAGCAGCGTCGTGACAGTGCCCAGCAGCTCTCTGGGCACCCAGACCTACATCTGCAACGTGAACCACAAGCCCAGCAACACCAAGGTGGACGAGAAGGTGGAACCCAAGTCCTGC |
| 133 | nucleotide sequence of anti-PD1(0314) Fc hole chain | see Table 8 |
| 134 | nucleotide sequence of anti-PD 1(0314) light chain | see Table 8 |
| 78 | Dimeric hu 4-1BBL (71-248)-CL* Fc knob chain | REGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAALALTVDLPPASSEARNSAFGFQGRLLHLSAGQRLGVHLHTEARARHAWQLTQGATVLGLFRVTPEIPAGLGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSV |

(continued)

| SEQ ID NO: | Description | Sequence |
|---|---|---|
| | | SLALHLQPLRSAAGAAALALTVDLPPASSEARNSAFGFQG RLLHLSAGQRLGVHLHTEARARHAWQLTQGATVLGLFRV TPEIPAGLGGGGSGGGGSRTVAAPSVFIFPPSDRKLKSGTAS VVCLLNNFYPREAKVQWKVDNALQSGNSQESVTEQDSKD STYSLSSTLTLSKADYEKHKVYACEVTHQGLSSPVTKSFNR GECDKTHTCPPCPAPEAAGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALGAPIEKTI SKAKGQPREPQVYTLPPCRDELTKNQVSLWCLVKGFYPSD IAVEWESNGQPENNYKTTPPVLDSDGSFFLYSKLTVDKSR WQQGNVFSCSVMHEALHNHYTQKSLSLSPGK |
| 79 | Monomeric hu 4-1BBL (71-248)-CH1* | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLGGGGSGGGGSASTKGPSVFPLA PSSKSTSGGTAALGCLVEDYFPEPVTVSWNSGALTSGVHTF PAVLQSSGLYSLSSVVTVPSSSLGTQTYICNVNHKPSNTKV DEKVEPKSC |
| 82 | anti-PD1(0314) Fc hole chain | see Table 8 |
| 83 | anti-PD1(0314) light chain | see Table 8 |

**2.5 Preparation of monovalent PD1(0314) targeted 4-1BB ligand (71-248) trimer-containing Fc (kih) fusion antigen binding molecule with crossed CH1-CL domains without charged residues (Construct 7.5)**

A polypeptide containing two ectodomains of 4-1BB ligand (71-248), separated by (G4S)2 linkers, and fused to the human IgG1-CL domain, was cloned as depicted in **Figure 8C:** human 4-1BB ligand, (G4S)2 connector, human 4-1BB ligand, (G4S)2 connector, human CL. A polypeptide containing one ectodomain of 4-1BB ligand (71-248) and fused to the human IgG1-CH domain, was cloned as described in **Figure 8D:** human 4-1BB ligand, (G4S)2 connector, human CH.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the dimeric ligand-Fc knob chain containing the S354C/T366W mutations, the monomeric CH1 fusion, the targeted anti-PD1 Fc hole chain containing the Y349C/T366S/L368A/Y407V mutations and the anti-PD1 light chain allows generation of a heterodimer, which includes an assembled trimeric 4-1BB ligand and a FAP binding Fab

(Figure 7, Construct 7.5)

Table 12 shows the cDNA and amino acid sequences of the monovalent PD1(0314)-human 4-1BB ligand (71-248) Fc (kih) fusion antigen binding molecule containing CH1-CL crossover without charged residues (Construct 7.5).

**Table 12: Sequences of monovalent PD1(0314)-targeted human 4-1BB ligand (71-248) containing Fc (kih) fusion molecule Construct 7.5**

| SEQ ID NO: | Description | Sequence |
|---|---|---|
| 141 | nucleotide sequence of dimeric hu 4-1BBL (71-248)-CL Fc knob chain | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGATCTGCT GCTGGCGCCGCTGCTCTGGCACTGACAGTGGATCTGCCT CCTGCCAGCAGCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGACTGGGAGG CGGCGGATCTGGCGGCGGAGGATCTAGAGAAGGACCCG AGCTGTCCCCTGACGATCCAGCCGGGCTGCTGGATCTGA GACAGGGAATGTTCGCCCAGCTGGTGGCTCAGAATGTG CTGCTGATTGACGGACCTCTGAGCTGGTACTCCGACCCA GGGCTGGCAGGGGTGTCCCTGACTGGGGGACTGTCCTAC AAAGAAGATACAAAAGAACTGGTGGTGGCTAAAGCTGG GGTGTACTATGTGTTTTTTCAGCTGGAACTGAGGCGGGT GGTGGCTGGGGAGGGCTCAGGATCTGTGTCCCTGGCTCT GCATCTGCAGCCACTGCGCTCTGCAGCAGGGGCTGCAG CACTGGCCCTGACTGTGGACCTGCCCCCAGCTTCTTCCG AGGCCAGAAACAGCGCCTTCGGGTTCCAAGGACGCCTG CTGCATCTGAGCGCCGGACAGCGCCTGGGAGTGCATCT GCATACTGAAGCCAGAGCCCGGCATGCTTGGCAGCTGA CTCAGGGGGCAACTGTGCTGGGACTGTTTCGCGTGACAC CTGAGATCCCCGCTGGACTGGGCGGAGGCGGTTCCGGA |

(continued)

| SEQ ID NO: | Description | Sequence |
|---|---|---|
| | | GGGGGAGGATCTCGTACGGTGGCCGCTCCCTCCGTGTTT<br>ATCTTTCCCCCATCCGATGAACAGCTGAAAAGCGGCACC<br>GCCTCCGTCGTGTGTCTGCTGAACAATTTTTACCCTAGG<br>GAAGCTAAAGTGCAGTGGAAAGTGGATAACGCACTGCA<br>GTCCGGCAACTCCCAGGAATCTGTGACAGAACAGGACT<br>CCAAGGACAGCACCTACTCCCTGTCCTCCACCCTGACAC<br>TGTCTAAGGCTGATTATGAGAAACACAAAGTCTACGCCT<br>GCGAAGTCACCCATCAGGGCCTGAGCTCGCCCGTCACA<br>AAGAGCTTCAACAGGGGAGAGTGTGACAAGACCCACAC<br>CTGTCCCCCTTGTCCTGCCCCTGAAGCTGCTGGCGGCCC<br>TTCTGTGTTCCTGTTCCCCCCAAAGCCCAAGGACACCCT<br>GATGATCAGCCGGACCCCCGAAGTGACCTGCGTGGTGG<br>TGGATGTGTCCCACGAGGACCCTGAAGTGAAGTTCAATT<br>GGTACGTGGACGGCGTGGAAGTGCACAATGCCAAGACC<br>AAGCCGCGGGAGGAGCAGTACAACAGCACGTACCGTGT<br>GGTCAGCGTCCTCACCGTCCTGCACCAGGACTGGCTGAA<br>TGGCAAGGAGTACAAGTGCAAGGTCTCCAACAAAGCCC<br>TCGGCGCCCCCATCGAGAAAACCATCTCCAAAGCCAAA<br>GGGCAGCCCCGAGAACCACAGGTGTACACCCTGCCCCC<br>ATGCCGGGATGAGCTGACCAAGAACCAGGTCAGCCTGT<br>GGTGCCTGGTCAAAGGCTTCTATCCCAGCGACATCGCCG<br>TGGAGTGGGAGAGCAATGGGCAGCCGGAGAACAACTAC<br>AAGACCACGCCTCCCGTGCTGGACTCCGACGGCTCCTTC<br>TTCCTCTACAGCAAGCTCACCGTGGACAAGAGCAGGTG<br>GCAGCAGGGGAACGTCTTCTCATGCTCCGTGATGCATGA<br>GGCTCTGCACAACCACTACACGCAGAAGAGCCTCTCCCT<br>GTCTCCGGGTAAA |

(continued)

| SEQ ID NO: | Description | Sequence |
|---|---|---|
| 142 | nucleotide sequence of monomeric hu 4-1BBL (71-248)-CH1 | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGATCTGCT GCTGGCGCCGCTGCTCTGGCACTGACAGTGGATCTGCCT CCTGCCAGCAGCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGACTGGGAGG CGGAGGTTCCGGAGGCGGAGGATCTGCTAGCACCAAAG GCCCTTCCGTGTTTCCTCTGGCTCCTAGCTCCAAGTCCAC CTCTGGAGGCACCGCTGCTCTCGGATGCCTCGTGAAGGA TTATTTCCTGAGCCTGTGACAGTGTCCTGGAATAGCGG AGCACTGACCTCTGGAGTGCATACTTTCCCCGCTGTGCT GCAGTCCTCTGGACTGTACAGCCTGAGCAGCGTGGTGAC AGTGCCCAGCAGCAGCCTGGGCACCCAGACCTACATCT GCAACGTGAACCACAAGCCCAGCAACACCAAGGTGGAC AAGAAGGTGGAACCCAAGTCTTGT |
| 133 | nucleotide sequence of anti-PD1(0314) Fc hole chain | see Table 8 |
| 134 | nucleotide sequence of anti-PD 1(0314) light chain | see Table 8 |
| 80 | Dimeric hu 4-1BBL (71-248)-CL Fc knob chain | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLGGGGSGGGGSREGPELSPDDPA GLLDLRQGMFAQLVAQNVLLIDGPLSWYSDPGLAGVSLTG GLSYKEDTKELVVAKAGVYYVFFQLELRRVVAGEGSGSV SLALHLQPLRSAAGAAALALTVDLPPASSEARNSAFGFQG RLLHLSAGQRLGVHLHTEARARHAWQLTQGATVLGLFRV TPEIPAGLGGGGSGGGGSRTVAAPSVFIFPPSDEQLKSGTAS VVCLLNNFYPREAKVQWKVDNALQSGNSQESVTEQDSKD STYSLSSTLTLSKADYEKHKVYACEVTHQGLSSPVTKSFNR GECDKTHTCPPCPAPEAAGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALGAPIEKTI SKAKGQPREPQVYTLPPCRDELTKNQVSLWCLVKGFYPSD IAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 81 | Monomeric hu 4-1BBL (71-248)-CH1 | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLGGGGSGGGGSASTKGPSVFPLA PSSKSTSGGTAALGCLVKDYFPEPVTVSWNSGALTSGVHT FPAVLQSSGLYSLSSVVTVPSSSLGTQTYICNVNHKPSNTK VDKKVEPKSC |
| 82 | anti-PD1(0314) Fc hole chain | see Table 8 |
| 83 | anti-PD1(0314) light chain | see Table 8 |

**2.6 Preparation of bivalent PD1(0314) targeted 4-1BB ligand (71-248) trimer-containing Fc (kih) fusion antigen binding molecule with the dimeric and monomeric 4-1BB ligands fused at the C-terminus of each heavy chain (Construct 7.6)**

A polypeptide containing two ectodomains of 4-1BB ligand (71-248), separated by (G4S)2 linkers was fused to the C-terminus of human IgG1 Fc hole chain, as depicted in **Figure 8E:** human IgG1 Fc hole, (G4S)2 connector, human 4-1BB ligand, (G4S)2 connector, human 4-1BB ligand. A polypeptide containing one ectodomain of 4-1BB ligand (71-248) and fused to the C-terminus of human IgG1 Fc knob chain as described in **Figure 8F:** human IgG1 Fc knob, (G4S)2 connector, human 4-1BB ligand.

The variable region of heavy and light chain DNA sequences encoding a binder specific for PD1, clone 0103-0314, were subcloned in frame with either the constant heavy chain of the hole, the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-PD1 huIgG1 hole dimeric ligand chain containing the Y349C/T366S/L368A/Y407V mutations, the anti-PD1 huIgG1 knob monomeric ligand chain containing the S354C/T366W mutations and the anti-PD1 light chain allows generation of a heterodimer, which includes an assembled trimeric 4-1BB ligand and two PD1 binding Fabs (**Figure 7**, Construct 7.6).

Table 13 shows the cDNA and amino acid sequences of the bivalent PD1(0314)-targeted 4-1BB ligand trimer-containing Fc (kih) fusion molecule Construct 7.6 (PD1 split trimer with 2 anti-PD1 Fabs, dimeric and monomeric 4-1BB ligand fused at the C-terminus of each heavy chain, respectively).

**Table 13: Sequences of bivalent PD1(0314)-targeted human 4-1BB ligand (71-248) containing Fc (kih) fusion molecule (Construct 7.6)**

| SEQ ID NO: | Description | Sequence |
|---|---|---|
| 143 | nucleotide sequence of anti-PD1(0314) Fc hole chain fused | GAAGTGCAGCTGCTCGAAAGCGGCGGGGGACTGGTCCA GCCCGGCGGTTCCCTGAGGCTGTCTTGCGCCGCTTCAGG GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA |

(continued)

| SEQ ID NO: | Description | Sequence |
|---|---|---|
| | to dimeric hu 4-1BBL (71-248) | AAGGACGGTTCACCATCTCTAGGGACAACTCAAAGAAT<br>ACTCTGTATTTGCAGATGAACAGCCTGAGAGCTGAGGAT<br>ACAGCAGTTTACTACTGTGTGCTCCTGACCGGCCGCGTC<br>TATTTTGCCCTTGACTCCTGGGGACAAGGCACTCTGGTG<br>ACCGTATCTAGTGCTAGCACCAAGGGCCCCTCCGTGTTC<br>CCCCTGGCCCCCAGCAGCAAGAGCACCAGCGGCGGCAC<br>AGCCGCTCTGGGCTGCCTGGTCAAGGACTACTTCCCCGA<br>GCCCGTGACCGTGTCCTGGAACAGCGGAGCCCTGACCTC<br>CGGCGTGCACACCTTCCCCGCCGTGCTGCAGAGTTCTGG<br>CCTGTATAGCCTGAGCAGCGTGGTCACCGTGCCTTCTAG<br>CAGCCTGGGCACCCAGACCTACATCTGCAACGTGAACC<br>ACAAGCCCAGCAACACCAAGGTGGACAAGAAGGTGGA<br>GCCCAAGAGCTGCGACAAAACTCACACATGCCCACCGT<br>GCCCAGCACCTGAAGCTGCAGGGGGACCGTCAGTCTTC<br>CTCTTCCCCCCAAAACCCAAGGACACCCTCATGATCTCC<br>CGGACCCCTGAGGTCACATGCGTGGTGGTGGACGTGAG<br>CCACGAAGACCCTGAGGTCAAGTTCAACTGGTACGTGG<br>ACGGCGTGGAGGTGCATAATGCCAAGACAAAGCCGCGG<br>GAGGAGCAGTACAACAGCACGTACCGTGTGGTCAGCGT<br>CCTCACCGTCCTGCACCAGGACTGGCTGAATGGCAAGG<br>AGTACAAGTGCAAGGTCTCCAACAAAGCCCTCGGCGCC<br>CCCATCGAGAAAACCATCTCCAAAGCCAAAGGGCAGCC<br>CCGAGAACCACAGGTGTGCACCCTGCCCCCATCCCGGG<br>ATGAGCTGACCAAGAACCAGGTCAGCCTCTCGTGCGCA<br>GTCAAAGGCTTCTATCCCAGCGACATCGCCGTGGAGTGG<br>GAGAGCAATGGGCAGCCGGAGAACAACTACAAGACCAC<br>GCCTCCCGTGCTGGACTCCGACGGCTCCTTCTTCCTCGT<br>GAGCAAGCTCACCGTGGACAAGAGCAGGTGGCAGCAGG<br>GGAACGTCTTCTCATGCTCCGTGATGCATGAGGCTCTGC<br>ACAACCACTACACGCAGAAGAGCCTCTCCCTGTCTCCGG<br>GTGGAGGCGGCGGAAGCGGAGGAGGAGGATCCAGAGA<br>GGGCCCTGAGCTGAGCCCTGATGATCCTGCCGGACTGCT<br>GGACCTGCGGCAGGGAATGTTTGCCCAGCTGGTGGCCC<br>AGAACGTGCTGCTGATCGATGGCCCCCTGTCCTGGTACA<br>GCGATCCTGGACTGGCTGGCGTGTCACTGACAGGCGGC<br>CTGAGCTACAAAGAGGACACCAAAGAACTGGTGGTGGC<br>CAAGGCCGGCGTGTACTACGTGTTCTTTCAGCTGGAACT<br>GCGGAGAGTGGTGGCCGGCGAAGGATCTGGCTCTGTGT<br>CTCTGGCCCTGCATCTGCAGCCTCTGAGATCTGCTGCTG<br>GCGCCGCTGCTCTGGCACTGACAGTGGATCTGCCTCCTG<br>CCAGCAGCGAGGCCCGGAATAGCGCATTTGGGTTTCAA<br>GGCAGGCTGCTGCACCTGTCTGCCGGCCAGAGGCTGGG<br>AGTGCATCTGCACACAGAGGCCAGGGCTAGACACGCCT<br>GGCAGCTGACACAGGGCGCTACAGTGCTGGGCCTGTTC<br>AGAGTGACCCCCGAGATTCCAGCAGGCCTGGGAGGCGG<br>CGGATCTGGCGGCGGAGGATCTAGAGAAGGACCCGAGC<br>TGTCCCCCGACGATCCCGCTGGGCTGCTGGATCTGAGAC<br>AGGGCATGTTCGCTCAGCTGGTGGCTCAGAATGTGCTGC<br>TGATTGACGGACCTCTGAGCTGGTACTCCGACCCAGGGC<br>TGGCAGGGGTGTCCCTGACTGGGGGACTGTCCTACAAA<br>GAAGATACAAAAGAACTGGTGGTGGCTAAAGCTGGGGT<br>GTACTATGTGTTTTTCAGCTGGAACTGAGGCGGGTGGT<br>GGCTGGGGAGGGCTCAGGATCTGTGTCCCTGGCTCTGCA<br>TCTGCAGCCACTGCGCTCTGCAGCAGGGGCTGCAGCACT |

(continued)

| SEQ ID NO: | Description | Sequence |
|---|---|---|
| | | GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |

(continued)

| SEQ ID NO: | Description | Sequence |
|---|---|---|
| 144 | nucleotide sequence of anti-PD1(0314) Fc knob chain fused to monomeric hu 4-1BBL (71-248) | GAAGTGCAGCTGCTCGAAAGCGGCGGGGGACTGGTCCA<br>GCCCGGCGGTTCCCTGAGGCTGTCTTGCGCCGCTTCAGG<br>GTTCAGCTTTTCCTCTTACACCATGAGTTGGGTCAGACA<br>GGCACCTGGCAAGGGACTGGAGTGGGTCGCCACAATCA<br>GCGGTGGCGGGCGCGACATTTATTACCCAGATTCCGTGA<br>AAGGACGGTTCACCATCTCTAGGGACAACTCAAAGAAT<br>ACTCTGTATTTGCAGATGAACAGCCTGAGAGCTGAGGAT<br>ACAGCAGTTTACTACTGTGTGCTCCTGACCGGCCGCGTC<br>TATTTTGCCCTTGACTCCTGGGGACAAGGCACTCTGGTG<br>ACCGTATCTAGTGCTAGCACCAAGGGCCCATCGGTCTTC<br>CCCCTGGCACCCTCCTCCAAGAGCACCTCTGGGGGCACA<br>GCGGCCCTGGGCTGCCTGGTCAAGGACTACTTCCCCGAA<br>CCGGTGACGGTGTCGTGGAACTCAGGCGCCCTGACCAG<br>CGGCGTGCACACCTTCCCGGCTGTCCTACAGTCCTCAGG<br>ACTCTACTCCCTCAGCAGCGTGGTGACCGTGCCCTCCAG<br>CAGCTTGGGCACCCAGACCTACATCTGCAACGTGAATCA<br>CAAGCCCAGCAACACCAAGGTGGACAAGAAAGTTGAGC<br>CCAAATCTTGTGACAAAACTCACACATGCCCACCGTGCC<br>CAGCACCTGAAGCTGCAGGGGGACCGTCAGTCTTCCTCT<br>TCCCCCCAAAACCCAAGGACACCCTCATGATCTCCCGGA<br>CCCCTGAGGTCACATGCGTGGTGGTGGACGTGAGCCAC<br>GAAGACCCTGAGGTCAAGTTCAACTGGTACGTGGACGG<br>CGTGGAGGTGCATAATGCCAAGACAAAGCCGCGGGAGG<br>AGCAGTACAACAGCACGTACCGTGTGGTCAGCGTCCTC<br>ACCGTCCTGCACCAGGACTGGCTGAATGGCAAGGAGTA<br>CAAGTGCAAGGTCTCCAACAAAGCCCTCGGCGCCCCCA<br>TCGAGAAAACCATCTCCAAAGCCAAAGGGCAGCCCCGA<br>GAACCACAGGTGTACACCCTGCCCCCCTGCAGAGATGA<br>GCTGACCAAGAACCAGGTGTCCCTGTGGTGTCTGGTCAA<br>GGGCTTCTACCCCAGCGATATCGCCGTGGAGTGGGAGA<br>GCAACGGCCAGCCTGAGAACAACTACAAGACCACCCCC<br>CCTGTGCTGGACAGCGACGGCAGCTTCTTCCTGTACTCC<br>AAACTGACCGTGGACAAGAGCCGGTGGCAGCAGGGCAA<br>CGTGTTCAGCTGCAGCGTGATGCACGAGGCCCTGCACA<br>ACCACTACACCCAGAAGTCCCTGAGCCTGAGCCCCGGC<br>GGAGGCGGCGGAAGCGGAGGAGGAGGATCCAGAGAGG<br>GCCCTGAGCTGAGCCCTGATGATCCTGCCGGACTGCTGG<br>ACCTGCGGCAGGGAATGTTTGCCCAGCTGGTGGCCCAG<br>AACGTGCTGCTGATCGATGGCCCCCTGTCCTGGTACAGC<br>GATCCTGGACTGGCTGGCGTGTCACTGACAGGCGGCCTG<br>AGCTACAAAGAGGACACCAAAGAACTGGTGGTGGCCAA<br>GGCCGGCGTGTACTACGTGTTCTTTCAGCTGGAACTGCG<br>GAGAGTGGTGGCCGGCGAAGGATCTGGCTCTGTGTCTCT<br>GGCCCTGCATCTGCAGCCTCTGAGATCTGCTGCTGGCGC<br>CGCTGCTCTGGCACTGACAGTGGATCTGCCTCCTGCCAG<br>CAGCGAGGCCCGGAATAGCGCATTTGGGTTTCAAGGCA<br>GGCTGCTGCACCTGTCTGCCGGCCAGAGGCTGGGAGTG |

(continued)

| SEQ ID NO: | Description | Sequence |
|---|---|---|
| | | CATCTGCACACAGAGGCCAGGGCTAGACACGCCTGGCA GCTGACACAGGGCGCTACAGTGCTGGGCCTGTTCAGAGT GACCCCCGAGATTCCTGCCGGGCTC |
| 134 | nucleotide sequence of anti-PD 1(0314) light chain | see Table 8 |
| 86 | anti-PD1(0314) Fc hole chain fused to dimeric hu 4-1BBL (71-248) | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVCTLPPSRDELTK NQVSLSCAVKGFYPSDIAVEWESNGQPENNYKTTPPVLDS DGSFFLVSKLTVDKSRWQQGNVFSCSVMHEALHNHYTQK SLSLSPGGGGGSGGGGSREGPELSPDDPAGLLDLRQGMFA QLVAQNVLLIDGPLSWYSDPGLAGVSLTGGLSYKEDTKEL VVAKAGVYYVFFQLELRRVVAGEGSGSVSLALHLQPLRSA AGAAALALTVDLPPASSEARNSAFGFQGRLLHLSAGQRLG VHLHTEARARHAWQLTQGATVLGLFRVTPEIPAGLGGGGS GGGGSREGPELSPDDPAGLLDLRQGMFAQLVAQNVLLIDG PLSWYSDPGLAGVSLTGGLSYKEDTKELVVAKAGVYYVF FQLELRRVVAGEGSGSVSLALHLQPLRSAAGAAALALTVD LPPASSEARNSAFGFQGRLLHLSAGQRLGVHLHTEARARH AWQLTQGATVLGLFRVTPEIPAGL |
| 87 | anti-PD1(0314) Fc knob chain fused to monomeric hu 4-1BBL (71-248) | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVYTLPPCRDELTK NQVSLWCLVKGFYPSDIAVEWESNGQPENNYKTTPPVLDS DGSFFLYSKLTVDKSRWQQGNVFSCSVMHEALHNHYTQK SLSLSPGGGGGSGGGGSREGPELSPDDPAGLLDLRQGMFA QLVAQNVLLIDGPLSWYSDPGLAGVSLTGGLSYKEDTKEL VVAKAGVYYVFFQLELRRVVAGEGSGSVSLALHLQPLRSA AGAAALALTVDLPPASSEARNSAFGFQGRLLHLSAGQRLG VHLHTEARARHAWQLTQGATVLGLFRVTPEIPAGL |
| 83 | anti-PD1(0314) light chain | see Table 8 |

**2.7 Preparation of monovalent PD1(0314) targeted 4-1BB ligand (71-248) trimer-containing Fc (kih) fusion antigen binding molecules with the dimeric and monomeric 4-1BB ligands fused at the C-terminus of each heavy chain (anti-PD1 on the knob chain, Constructs 7.11 and 7.12)**

A polypeptide containing two ectodomains of 4-1BB ligand, separated by (G4S)2 linkers was subcloned in frame to the C-terminus of human IgG1 Fc hole or knob chain, as depicted in **Figure 9A:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 9B:** human IgG1 Fc, (G4S)2 connector, human 4-1BB ligand..

The variable region of heavy and light chain DNA sequences encoding a binder specific for PD1, clone 0314, were subcloned in frame with either the constant heavy chain of the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the huIgG1 hole chain containing the Y349C/T366S/L368A/Y407V mutations, the anti-PD1 huIgG1 knob chain containing the S354C/T366W mutations and the anti-PD1 light chain allows generation of a heterodimer, which includes an assembled trimeric 4-1BB ligand and one PD1 binding Fab (**Figures 9C and D,** Constructs 7.11 and 7.12).

Table 14 shows the cDNA and amino acid sequences of the monovalent PD1(0314)-targeted 4-1BB ligand trimer-containing Fc (kih) fusion molecule Construct 7.11 (PD1 split trimer with one anti-PD1 Fab, dimeric 4-1BB ligand fused at the C-terminus of the Fc hole chain and monomeric 4-1BB ligand fused at the C-terminus of the Fc knob chain).

**Table 14: Sequences of monovalent PD1(0314)-targeted human 4-1BB ligand (71-248) containing Fc (kih) fusion molecule (Construct 7.11)**

| SEQ ID NO: | Description | Sequence |
|---|---|---|
| 145 | nucleotide sequence of Fc hole dimeric ligand (71-248) | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA |

(continued)

| SEQ ID NO: | Description | Sequence |
|---|---|---|
| | chain | CCAGGACTGGCTGAATGGCAAGGAGTACAAGTGCAAGG<br>TCTCCAACAAAGCCCTCGGCGCCCCCATCGAGAAAACC<br>ATCTCCAAAGCCAAAGGGCAGCCCCGAGAACCACAGGT<br>GTGCACCCTGCCCCCATCCCGGGATGAGCTGACCAAGA<br>ACCAGGTCAGCCTCTCGTGCGCAGTCAAAGGCTTCTATC<br>CCAGCGACATCGCCGTGGAGTGGGAGAGCAATGGGCAG<br>CCGGAGAACAACTACAAGACCACGCCTCCCGTGCTGGA<br>CTCCGACGGCTCCTTCTTCCTCGTGAGCAAGCTCACCGT<br>GGACAAGAGCAGGTGGCAGCAGGGGAACGTCTTCTCAT<br>GCTCCGTGATGCATGAGGCTCTGCACAACCACTACACGC<br>AGAAGAGCCTCTCCCTGTCTCCGGGTGGAGGCGGCGGA<br>AGCGGAGGAGGAGGATCCAGAGAGGGCCCTGAGCTGAG<br>CCCTGATGATCCTGCCGGACTGCTGGACCTGCGGCAGGG<br>AATGTTTGCCCAGCTGGTGGCCCAGAACGTGCTGCTGAT<br>CGATGGCCCCCTGTCCTGGTACAGCGATCCTGGACTGGC<br>TGGCGTGTCACTGACAGGCGGCCTGAGCTACAAAGAGG<br>ACACCAAAGAACTGGTGGTGGCCAAGGCCGGCGTGTAC<br>TACGTGTTCTTTCAGCTGGAACTGCGGAGAGTGGTGGCC<br>GGCGAAGGATCTGGCTCTGTGTCTCTGGCCCTGCATCTG<br>CAGCCTCTGAGATCTGCTGCTGGCGCCGCTGCTCTGGCA<br>CTGACAGTGGATCTGCCTCCTGCCAGCAGCGAGGCCCG<br>GAATAGCGCATTTGGGTTTCAAGGCAGGCTGCTGCACCT<br>GTCTGCCGGCCAGAGGCTGGGAGTGCATCTGCACACAG<br>AGGCCAGGGCTAGACACGCCTGGCAGCTGACACAGGGC<br>GCTACAGTGCTGGGCCTGTTCAGAGTGACCCCCGAGATT<br>CCAGCAGGCCTGGGAGGCGGCGGATCTGGCGGCGGAGG<br>ATCTAGAGAAGGACCCGAGCTGTCCCCCGACGATCCCG<br>CTGGGCTGCTGGATCTGAGACAGGGCATGTTCGCTCAGC<br>TGGTGGCTCAGAATGTGCTGCTGATTGACGGACCTCTGA<br>GCTGGTACTCCGACCCAGGGCTGGCAGGGGTGTCCCTG<br>ACTGGGGGACTGTCCTACAAAGAAGATACAAAAGAACT<br>GGTGGTGGCTAAAGCTGGGGTGTACTATGTGTTTTTCA<br>GCTGGAACTGAGGCGGGTGGTGGCTGGGGAGGGCTCAG<br>GATCTGTGTCCCTGGCTCTGCATCTGCAGCCACTGCGCT<br>CTGCAGCAGGGGCTGCAGCACTGGCCCTGACTGTGGAC<br>CTGCCCCCAGCTTCTTCCGAGGCCAGAAACAGCGCCTTC<br>GGGTTCCAAGGACGCCTGCTGCATCTGAGCGCCGGACA<br>GCGCCTGGGAGTGCATCTGCATACTGAAGCCAGAGCCC<br>GGCATGCTTGGCAGCTGACTCAGGGGGCAACTGTGCTG<br>GGACTGTTTCGCGTGACACCTGAGATCCCAGCCGGGCTC |
| 144 | nucleotide sequence of anti-PD1(0314) Fc knob chain fused to monomeric hu 4-1BBL (71-248) | see Table 13 |
| 134 | nucleotide sequence of anti-PD 1(0314) light | see Table 8 |
| | chain | |

(continued)

| SEQ ID NO: | Description | Sequence |
|---|---|---|
| 88 | Fc hole dimeric ligand (71-248) chain | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVCTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGGGGGSGGGGSREGPE LSPDDPAGLLDLRQGMFAQLVAQNVLLIDGPLSWYSDPGL AGVSLTGGLSYKEDTKELVVAKAGVYYVFFQLELRRVVA GEGSGSVSLALHLQPLRSAAGAAALALTVDLPPASSEARN SAFGFQGRLLHLSAGQRLGVHLHTEARARHAWQLTQGAT VLGLFRVTPEIPAGLGGGGSGGGGSREGPELSPDDPAGLLD LRQGMFAQLVAQNVLLIDGPLSWYSDPGLAGVSLTGGLSY KEDTKELVVAKAGVYYVFFQLELRRVVAGEGSGSVSLAL HLQPLRSAAGAAALALTVDLPPASSEARNSAFGFQGRLLH LSAGQRLGVHLHTEARARHAWQLTQGATVLGLFRVTPEIP AGL |
| 87 | anti-PD1(0314) Fc knob chain fused to monomeric hu 4-1BBL (71-248) | see Table 13 |
| 83 | anti-PD1(0314) light chain | see Table 8 |

Table 15 shows the cDNA and amino acid sequences of the monovalent PD1(0314)-targeted 4-1BB ligand trimer-containing Fc (kih) fusion molecule Construct 7.12 (PD1 split trimer with one anti-PD1 Fab, dimeric 4-1BB ligand fused at the C-terminus of the Fc knob chain and monomeric 4-1BB ligand fused at the C-terminus of the Fc hole chain).

**Table 15: Sequences of monovalent PD1(0314)-targeted human 4-1BB ligand (71-248) containing Fc (kih) fusion molecule (Construct 7.12)**

| SEQ ID NO: | Description | Sequence |
|---|---|---|
| 146 | nucleotide sequence of Fc hole monomeric ligand (71-248) | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA |

(continued)

| SEQ ID NO: | Description | Sequence |
|---|---|---|
| | chain | CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAGAGAGGGCCCT GAGCTGAGCCCTGATGATCCTGCCGGACTGCTGGACCTG CGGCAGGGAATGTTTGCCCAGCTGGTGGCCCAGAACGT GCTGCTGATCGATGGCCCCCTGTCCTGGTACAGCGATCC TGGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGATCTGCTGCTGGCGCCGCTG CTCTGGCACTGACAGTGGATCTGCCTCCTGCCAGCAGCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC CCGAGATTCCTGCCGGGCTC |

(continued)

| SEQ ID NO: | Description | Sequence |
|---|---|---|
| 147 | nucleotide sequence of anti-PD1(0314) Fc knob chain fused to dimeric hu 4-1BBL (71-248) | GAAGTGCAGCTGCTCGAAAGCGGCGGGGGACTGGTCCA GCCCGGCGGTTCCCTGAGGCTGTCTTGCGCCGCTTCAGG GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA AAGGACGGTTCACCATCTCTAGGGACAACTCAAAGAAT ACTCTGTATTTGCAGATGAACAGCCTGAGAGCTGAGGAT ACAGCAGTTTACTACTGTGTGCTCCTGACCGGCCGCGTC TATTTTGCCCTTGACTCCTGGGGACAAGGCACTCTGGTG ACCGTATCTAGTGCTAGCACCAAGGGCCCATCGGTCTTC CCCCTGGCACCCTCCTCCAAGAGCACCTCTGGGGGCACA GCGGCCCTGGGCTGCCTGGTCAAGGACTACTTCCCCGAA CCGGTGACGGTGTCGTGGAACTCAGGCGCCCTGACCAG CGGCGTGCACACCTTCCCGGCTGTCCTACAGTCCTCAGG ACTCTACTCCCTCAGCAGCGTGGTGACCGTGCCCTCCAG CAGCTTGGGCACCCAGACCTACATCTGCAACGTGAATCA CAAGCCCAGCAACACCAAGGTGGACAAGAAAGTTGAGC CCAAATCTTGTGACAAAACTCACACATGCCCACCGTGCC CAGCACCTGAAGCTGCAGGGGGACCGTCAGTCTTCCTCT TCCCCCCAAAACCCAAGGACACCCTCATGATCTCCCGGA CCCCTGAGGTCACATGCGTGGTGGTGGACGTGAGCCAC GAAGACCCTGAGGTCAAGTTCAACTGGTACGTGGACGG CGTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGTA CAAGTGCAAGGTCTCCAACAAAGCCCTCGGCGCCCCCA TCGAGAAAACCATCTCCAAAGCCAAAGGGCAGCCCCGA GAACCACAGGTGTACACCCTGCCCCCCTGCAGAGATGA |

(continued)

| SEQ ID NO: | Description | Sequence |
|---|---|---|
| | | GCTGACCAAGAACCAGGTGTCCCTGTGGTGTCTGGTCAA GGGCTTCTACCCCAGCGATATCGCCGTGGAGTGGGAGA GCAACGGCCAGCCTGAGAACAACTACAAGACCACCCCC CCTGTGCTGGACAGCGACGGCAGCTTCTTCCTGTACTCC AAACTGACCGTGGACAAGAGCCGGTGGCAGCAGGGCAA CGTGTTCAGCTGCAGCGTGATGCACGAGGCCCTGCACA ACCACTACACCCAGAAGTCCCTGAGCCTGAGCCCCGGC AGAGAGGGCCCTGAGCTGAGCCCTGATGATCCTGCCGG ACTGCTGGACCTGCGGCAGGGAATGTTTGCCCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGATCTGCT GCTGGCGCCGCTGCTCTGGCACTGACAGTGGATCTGCCT CCTGCCAGCAGCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCAGGCCTGGGAGG CGGCGGATCTGGCGGCGGAGGATCTAGAGAAGGACCCG AGCTGTCCCCCGACGATCCCGCTGGGCTGCTGGATCTGA GACAGGGCATGTTCGCTCAGCTGGTGGCTCAGAATGTGC TGCTGATTGACGGACCTCTGAGCTGGTACTCCGACCCAG GGCTGGCAGGGGTGTCCCTGACTGGGGGACTGTCCTACA AAGAAGATACAAAAGAACTGGTGGTGGCTAAAGCTGGG GTGTACTATGTGTTTTTCAGCTGGAACTGAGGCGGGTG GTGGCTGGGGAGGGCTCAGGATCTGTGTCCCTGGCTCTG CATCTGCAGCCACTGCGCTCTGCAGCAGGGGCTGCAGC ACTGGCCCTGACTGTGGACCTGCCCCCAGCTTCTTCCGA GGCCAGAAACAGCGCCTTCGGGTTCCAAGGACGCCTGC TGCATCTGAGCGCCGGACAGCGCCTGGGAGTGCATCTG CATACTGAAGCCAGAGCCCGGCATGCTTGGCAGCTGAC TCAGGGGGCAACTGTGCTGGGACTGTTTCGCGTGACACC TGAGATCCCAGCCGGGCTC |
| 134 | nucleotide sequence of anti-PD 1(0314) light chain | see Table 8 |

(continued)

| SEQ ID NO: | Description | Sequence |
|---|---|---|
| 89 | Fc hole monomeric ligand (71-248) chain | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVCTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGREGPELSPDDPAGLL DLRQGMFAQLVAQNVLLIDGPLSWYSDPGLAGVSLTGGLS YKEDTKELVVAKAGVYYVFFQLELRRVVAGEGSGSVSLA LHLQPLRSAAGAAALALTVDLPPASSEARNSAFGFQGRLL HLSAGQRLGVHLHTEARARHAWQLTQGATVLGLFRVTPEI |
| | | PAGL |
| 90 | anti-PD1(0314)Fcknob chain fused to dimeric hu 4-1BBL (71-248) | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVYTLPPCRDELTK NQVSLWCLVKGFYPSDIAVEWESNGQPENNYKTTPPVLDS DGSFFLYSKLTVDKSRWQQGNVFSCSVMHEALHNHYTQK SLSLSPGREGPELSPDDPAGLLDLRQGMFAQLVAQNVLLID GPLSWYSDPGLAGVSLTGGLSYKEDTKELVVAKAGVYYV FFQLELRRVVAGEGSGSVSLALHLQPLRSAAGAAALALTV DLPPASSEARNSAFGFQGRLLHLSAGQRLGVHLHTEARAR HAWQLTQGATVLGLFRVTPEIPAGLGGGGSGGGGSREGPE LSPDDPAGLLDLRQGMFAQLVAQNVLLIDGPLSWYSDPGL AGVSLTGGLSYKEDTKELVVAKAGVYYVFFQLELRRVVA GEGSGSVSLALHLQPLRSAAGAAALALTVDLPPASSEARN SAFGFQGRLLHLSAGQRLGVHLHTEARARHAWQLTQGAT VLGLFRVTPEIPAGL |
| 83 | anti-PD1(0314) light chain | see Table 8 |

**2.8 Preparation of monovalent PD1(0314) targeted 4-1BB ligand (71-248) trimer-containing Fc (kih) fusion antigen binding molecules with the dimeric and monomeric 4-1BB ligands fused at the C-terminus of each heavy chain (anti-PD1 on the hole chain, Constructs 7.13 and 7.14)**

A polypeptide containing two ectodomains of 4-1BB ligand, separated by (G4S)2 linkers was subcloned in frame to the C-terminus of human IgG1 Fc hole or knob chain, as depicted in **Figure 9A:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 9B:** human IgG1 Fc, (G4S)2 connector, human 4-1BB ligand..

The variable region of heavy and light chain DNA sequences encoding a binder specific for PD1, clone 0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-PD1 huIgG1 hole chain containing the Y349C/T366S/L368A/ Y407V mutations, the huIgG1 knob chain containing the S354C/T366W mutations and the anti-PD1 light chain allows generation of a heterodimer, which includes an assembled trimeric 4-1BB ligand and one PD1 binding Fab (**Figures 10A and B,** Constructs 7.13 and 7.14).

Table 16 shows the cDNA and amino acid sequences of the monovalent PD1(0314)-targeted 4-1BB ligand trimer-containing Fc (kih) fusion molecule Construct 7.13 (PD1 split trimer with one anti-PD1 Fab, dimeric 4-1BB ligand fused at the C-terminus of the Fc hole chain and monomeric 4-1BB ligand fused at the C-terminus of the Fc knob chain).

**Table 16: Sequences of monovalent PD1(0314)-targeted human 4-1BB ligand (71-248) containing Fc (kih) fusion molecule (Construct 7.13)**

| SEQ ID NO: | Description | Sequence |
|---|---|---|
| 143 | nucleotide sequence of anti-PD1(0314) Fc hole dimeric ligand (71-248) chain | see Table 13 |
| 148 | nucleotide sequence of Fc knob chain fused to monomeric hu 4-1BBL (71-248) | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCCTGCAGAGATGAGCTGACCAAGA ACCAGGTGTCCCTGTGGTGTCTGGTCAAGGGCTTCTACC CCAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCGT GGACAAGAGCCGGTGGCAGCAGGGCAACGTGTTCAGCT GCAGCGTGATGCACGAGGCCCTGCACAACCACTACACC CAGAAGTCCCTGAGCCTGAGCCCCGGCGGAGGCGGCGG AAGCGGAGGAGGAGGATCCAGAGAGGGCCCTGAGCTGA |
| | | GCCCTGATGATCCTGCCGGACTGCTGGACCTGCGGCAGG GAATGTTTGCCCAGCTGGTGGCCCAGAACGTGCTGCTGA TCGATGGCCCCCTGTCCTGGTACAGCGATCCTGGACTGG CTGGCGTGTCACTGACAGGCGGCCTGAGCTACAAAGAG GACACCAAAGAACTGGTGGTGGCCAAGGCCGGCGTGTA CTACGTGTTCTTTCAGCTGGAACTGCGGAGAGTGGTGGC CGGCGAAGGATCTGGCTCTGTGTCTCTGGCCCTGCATCT GCAGCCTCTGAGATCTGCTGCTGGCGCCGCTGCTCTGGC ACTGACAGTGGATCTGCCTCCTGCCAGCAGCGAGGCCC GGAATAGCGCATTTGGGTTTCAAGGCAGGCTGCTGCACC TGTCTGCCGGCCAGAGGCTGGGAGTGCATCTGCACACA GAGGCCAGGGCTAGACACGCCTGGCAGCTGACACAGGG CGCTACAGTGCTGGGCCTGTTCAGAGTGACCCCCGAGAT TCCTGCCGGGCTC |
| 134 | nucleotide sequence of anti-PD 1(0314) light chain | see Table 8 |

(continued)

| SEQ ID NO: | Description | Sequence |
|---|---|---|
| 86 | anti-PD1(0314) Fc hole dimeric ligand (71-248) chain | see Table 13 |
| 91 | Fc knob chain fused to monomeric hu 4-1BBL (71-248) | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVYTLPPCRDELTKNQVSLWCLVKGFYPSDIAVE WESNGQPENNYKTTPPVLDSDGSFFLYSKLTVDKSRWQQ GNVFSCSVMHEALHNHYTQKSLSLSPGGGGGSGGGGSRE GPELSPDDPAGLLDLRQGMFAQLVAQNVLLIDGPLSWYSD PGLAGVSLTGGLSYKEDTKELVVAKAGVYYVFFQLELRRV VAGEGSGSVSLALHLQPLRSAAGAAALALTVDLPPASSEA RNSAFGFQGRLLHLSAGQRLGVHLHTEARARHAWQLTQG ATVLGLFRVTPEIPAGL |
| 83 | anti-PD1(0314) light chain | see Table 8 |

Table 17 shows the cDNA and amino acid sequences of the monovalent PD 1 (0314)-targeted 4-1BB ligand trimer-containing Fc (kih) fusion molecule Construct 7.14 (PD1 split trimer with one anti-PD1 Fab, dimeric 4-1BB ligand fused at the C-terminus of the Fc knob chain and monomeric 4-1BB ligand fused at the C-terminus of the Fc hole chain).

**Table 17: Sequences of monovalent PD1(0314)-targeted human 4-1BB ligand (71-248) containing Fc (kih) fusion molecule (Construct 7.14)**

| SEQ ID NO: | Description | Sequence |
|---|---|---|
| 149 | nucleotide sequence of anti-PD1(0314) Fc hole monomeric ligand (71-248) chain | GAAGTGCAGCTGCTCGAAAGCGGCGGGGGACTGGTCCA GCCCGGCGGTTCCCTGAGGCTGTCTTGCGCCGCTTCAGG GTTCAGCTTTTCCTCTTACACCATGAGTTGGGTCAGACA GGCACCTGGCAAGGGACTGGAGTGGGTCGCCACAATCA GCGGTGGCGGGCGCGACATTTATTACCCAGATTCCGTGA AAGGACGGTTCACCATCTCTAGGGACAACTCAAAGAAT ACTCTGTATTTGCAGATGAACAGCCTGAGAGCTGAGGAT ACAGCAGTTTACTACTGTGTGCTCCTGACCGGCCGCGTC TATTTTGCCCTTGACTCCTGGGGACAAGGCACTCTGGTG ACCGTATCTAGTGCTAGCACCAAGGGCCCCTCCGTGTTC CCCCTGGCCCCCAGCAGCAAGAGCACCAGCGGCGGCAC AGCCGCTCTGGGCTGCCTGGTCAAGGACTACTTCCCCGA GCCCGTGACCGTGTCCTGGAACAGCGGAGCCCTGACCTC CGGCGTGCACACCTTCCCCGCCGTGCTGCAGAGTTCTGG CCTGTATAGCCTGAGCAGCGTGGTCACCGTGCCTTCTAG CAGCCTGGGCACCCAGACCTACATCTGCAACGTGAACC ACAAGCCCAGCAACACCAAGGTGGACAAGAAGGTGGA GCCCAAGAGCTGCGACAAAACTCACACATGCCCACCGT GCCCAGCACCTGAAGCTGCAGGGGGACCGTCAGTCTTC CTCTTCCCCCCAAAACCCAAGGACACCCTCATGATCTCC CGGACCCCTGAGGTCACATGCGTGGTGGTGGACGTGAG CCACGAAGACCCTGAGGTCAAGTTCAACTGGTACGTGG ACGGCGTGGAGGTGCATAATGCCAAGACAAAGCCGCGG GAGGAGCAGTACAACAGCACGTACCGTGTGGTCAGCGT CCTCACCGTCCTGCACCAGGACTGGCTGAATGGCAAGG AGTACAAGTGCAAGGTCTCCAACAAAGCCCTCGGCGCC CCCATCGAGAAAACCATCTCCAAAGCCAAAGGGCAGCC CCGAGAACCACAGGTGTGCACCCTGCCCCCATCCCGGG ATGAGCTGACCAAGAACCAGGTCAGCCTCTCGTGCGCA GTCAAAGGCTTCTATCCCAGCGACATCGCCGTGGAGTGG GAGAGCAATGGGCAGCCGGAGAACAACTACAAGACCAC GCCTCCCGTGCTGGACTCCGACGGCTCCTTCTTCCTCGT GAGCAAGCTCACCGTGGACAAGAGCAGGTGGCAGCAGG GGAACGTCTTCTCATGCTCCGTGATGCATGAGGCTCTGC ACAACCACTACACGCAGAAGAGCCTCTCCCTGTCTCCGG GTGGAGGCGGCGGAAGCGGAGGAGGAGGATCCAGAGA GGGCCCTGAGCTGAGCCCTGATGATCCTGCCGGACTGCT GGACCTGCGGCAGGGAATGTTTGCCCAGCTGGTGGCCC AGAACGTGCTGCTGATCGATGGCCCCCTGTCCTGGTACA GCGATCCTGGACTGGCTGGCGTGTCACTGACAGGCGGC CTGAGCTACAAAGAGGACACCAAAGAACTGGTGGTGGC CAAGGCCGGCGTGTACTACGTGTTCTTTCAGCTGGAACT GCGGAGAGTGGTGGCCGGCGAAGGATCTGGCTCTGTGT CTCTGGCCCTGCATCTGCAGCCTCTGAGATCTGCTGCTG GCGCCGCTGCTCTGGCACTGACAGTGGATCTGCCTCCTG CCAGCAGCGAGGCCCGGAATAGCGCATTTGGGTTTCAA GGCAGGCTGCTGCACCTGTCTGCCGGCCAGAGGCTGGG |

(continued)

| SEQ ID NO: | Description | Sequence |
|---|---|---|
| | | AGTGCATCTGCACACAGAGGCCAGGGCTAGACACGCCT GGCAGCTGACACAGGGCGCTACAGTGCTGGGCCTGTTC AGAGTGACCCCCGAGATTCCTGCCGGGCTC |

(continued)

| SEQ ID NO: | Description | Sequence |
|---|---|---|
| 150 | nucleotide sequence of Fc knob chain fused to dimeric hu 4-1BBL (71-248) | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCCTGCAGAGATGAGCTGACCAAGA ACCAGGTGTCCCTGTGGTGTCTGGTCAAGGGCTTCTACC CCAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCGT GGACAAGAGCCGGTGGCAGCAGGGCAACGTGTTCAGCT GCAGCGTGATGCACGAGGCCCTGCACAACCACTACACC CAGAAGTCCCTGAGCCTGAGCCCCGGCGGAGGCGGCGG AAGCGGAGGAGGAGGATCCAGAGAGGGCCCTGAGCTGA GCCCTGATGATCCTGCCGGACTGCTGGACCTGCGGCAGG GAATGTTTGCCCAGCTGGTGGCCCAGAACGTGCTGCTGA TCGATGGCCCCCTGTCCTGGTACAGCGATCCTGGACTGG CTGGCGTGTCACTGACAGGCGGCCTGAGCTACAAAGAG GACACCAAAGAACTGGTGGTGGCCAAGGCCGGCGTGTA CTACGTGTTCTTTCAGCTGGAACTGCGGAGAGTGGTGGC CGGCGAAGGATCTGGCTCTGTGTCTCTGGCCCTGCATCT GCAGCCTCTGAGATCTGCTGCTGGCGCCGCTGCTCTGGC ACTGACAGTGGATCTGCCTCCTGCCAGCAGCGAGGCCC GGAATAGCGCATTTGGGTTTCAAGGCAGGCTGCTGCACC TGTCTGCCGGCCAGAGGCTGGGAGTGCATCTGCACACA GAGGCCAGGGCTAGACACGCCTGGCAGCTGACACAGGG CGCTACAGTGCTGGGCCTGTTCAGAGTGACCCCCGAGAT TCCAGCAGGCCTGGGAGGCGGCGGATCTGGCGGCGGAG GATCTAGAGAAGGACCCGAGCTGTCCCCCGACGATCCC GCTGGGCTGCTGGATCTGAGACAGGGCATGTTCGCTCAG CTGGTGGCTCAGAATGTGCTGCTGATTGACGGACCTCTG AGCTGGTACTCCGACCCAGGGCTGGCAGGGGTGTCCCT GACTGGGGGACTGTCCTACAAAGAAGATACAAAAGAAC TGGTGGTGGCTAAAGCTGGGGTGTACTATGTGTTTTTC AGCTGGAACTGAGGCGGGTGGTGGCTGGGGAGGGCTCA GGATCTGTGTCCCTGGCTCTGCATCTGCAGCCACTGCGC TCTGCAGCAGGGGCTGCAGCACTGGCCCTGACTGTGGA CCTGCCCCCAGCTTCTTCCGAGGCCAGAAACAGCGCCTT CGGGTTCCAAGGACGCCTGCTGCATCTGAGCGCCGGAC AGCGCCTGGGAGTGCATCTGCATACTGAAGCCAGAGCC CGGCATGCTTGGCAGCTGACTCAGGGGGCAACTGTGCTG GGACTGTTTCGCGTGACACCTGAGATCCCAGCCGGGCTC |

(continued)

| SEQ ID NO: | Description | Sequence |
|---|---|---|
| 134 | nucleotide sequence of anti-PD1(0314) light chain | see Table 8 |
| 92 | anti-PD1(0314) Fc hole monomeric ligand (71-248) chain | EVQLLESGGGLVQPGGSLRLSCAASGFSFSSYTMSWVRQA PGKGLEWVATISGGGRDIYYPDSVKGRFTISRDNSKNTLYL QMNSLRAEDTAVYYCVLLTGRVYFALDSWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALGAPIEKTISKAKGQPREPQVCTLPPSRDELTK NQVSLSCAVKGFYPSDIAVEWESNGQPENNYKTTPPVLDS DGSFFLVSKLTVDKSRWQQGNVFSCSVMHEALHNHYTQK SLSLSPGGGGGSGGGGSREGPELSPDDPAGLLDLRQGMFA QLVAQNVLLIDGPLSWYSDPGLAGVSLTGGLSYKEDTKEL VVAKAGVYYVFFQLELRRVVAGEGSGSVSLALHLQPLRSA AGAAALALTVDLPPASSEARNSAFGFQGRLLHLSAGQRLG VHLHTEARARHAWQLTQGATVLGLFRVTPEIPAGL |
| 93 | Fc knob chain fused to dimeric hu 4-1BBL (71-248) | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVYTLPPCRDELTKNQVSLWCLVKGFYPSDIAVE WESNGQPENNYKTTPPVLDSDGSFFLYSKLTVDKSRWQQ GNVFSCSVMHEALHNHYTQKSLSLSPGGGGGSGGGGSRE GPELSPDDPAGLLDLRQGMFAQLVAQNVLLIDGPLSWYSD PGLAGVSLTGGLSYKEDTKELVVAKAGVYYVFFQLELRRV VAGEGSGSVSLALHLQPLRSAAGAAALALTVDLPPASSEA RNSAFGFQGRLLHLSAGQRLGVHLHTEARARHAWQLTQG ATVLGLFRVTPEIPAGLGGGGSGGGGSREGPELSPDDPAGL LDLRQGMFAQLVAQNVLLIDGPLSWYSDPGLAGVSLTGGL SYKEDTKELVVAKAGVYYVFFQLELRRVVAGEGSGSVSL ALHLQPLRSAAGAAALALTVDLPPASSEARNSAFGFQGRL LHLSAGQRLGVHLHTEARARHAWQLTQGATVLGLFRVTP EIPAGL |
| 83 | anti-PD1(0314) light chain | see Table 8 |

### 2.9 Preparation of untargeted human 4-1BB ligand trimer-containing Fc fusion antigen binding molecules (Control molecules)

The control molecules were prepared as described above for the PD1-targeted antigen binding molecules, with the only difference that the anti-PD1 binder (VH and VL) was replaced by a germline control, termed DP47, not binding to the antigen.

Control B is an untargeted monovalent split trimeric human 4-1BB ligand (71-254) Fc (kih) antigen binding molecule (**Figure 11A**) containing a CH-CL crossover with charged residues. It corresponds to Construct 7.1, however the variable region of heavy and light chain DNA sequences of the PD1 binder were replaced with those of the germline control (DP47) and subcloned in frame with either the constant heavy chain of the hole or the constant light chain of human IgG1.

A bivalent variant Control C (**Figure 11B**) was prepared in analogy to the bivalent Constructs 7.3 and 7.6 and a monovalent variant Control D (**Figure 11C**) was prepared in analogy to Construct 7.3 (containing a 4-1BB ligand (71-248) trimer), with the only difference that the anti-PD1 binder (VH and VL) was replaced by a germline control, termed DP47, not binding to the antigen.

Table 18 shows the cDNA and amino acid sequences of the DP47-untargeted 4-1BB ligand (71-254) trimer-containing Fc (kih) fusion antigen binding molecule with CH1-CL crossover and charged residues in the 4-1BB ligand containing arms (Control B).

**Table 18: Sequences of DP47 untargeted 4-1BB ligand (71-254) trimer-containing Fc (kih) fusion antigen binding molecule (DP47 split 4-1BBL trimer) Control B**

| SEQ ID NO: | Description | Sequence |
|---|---|---|
| 131 | nucleotide sequence of dimeric hu 4-1BBL (71-254)-CL* Fc knob chain | see Table 8 |
| 132 | nucleotide sequence of monomeric hu 4-1BBL (71-254) -CH1* | see Table 8 |
| 151 | nucleotide sequence of DP47 Fc hole chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCATCGGTCTTCCCCCTGGCACCCTC CTCCAAGAGCACCTCTGGGGGCACAGCGGCCCTGGGCT GCCTGGTCAAGGACTACTTCCCCGAACCGGTGACGGTGT CGTGGAACTCAGGCGCCCTGACCAGCGGCGTGCACACC |

(continued)

| SEQID NO: | Description | Sequence |
|---|---|---|
| | | TTCCCGGCTGTCCTACAGTCCTCAGGACTCTACTCCCTC AGCAGCGTGGTGACCGTGCCCTCCAGCAGCTTGGGCAC CCAGACCTACATCTGCAACGTGAATCACAAGCCCAGCA ACACCAAGGTGGACAAGAAAGTTGAGCCCAAATCTTGT GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAAA |
| 152 | nucleotide sequence of DP47 light chain | GAAATCGTGTTAACGCAGTCTCCAGGCACCCTGTCTTTG TCTCCAGGGGAAAGAGCCACCCTCTCTTGCAGGGCCAGT CAGAGTGTTAGCAGCAGCTACTTAGCCTGGTACCAGCAG AAACCTGGCCAGGCTCCCAGGCTCCTCATCTATGGAGCA TCCAGCAGGGCCACTGGCATCCCAGACAGGTTCAGTGG CAGTGGATCCGGGACAGACTTCACTCTCACCATCAGCAG ACTGGAGCCTGAAGATTTTGCAGTGTATTACTGTCAGCA GTATGGTAGCTCACCGCTGACGTTCGGCCAGGGGACCA AAGTGGAAATCAAACGTACGGTGGCTGCACCATCTGTCT TCATCTTCCCGCCATCTGATGAGCAGTTGAAATCTGGAA CTGCCTCTGTTGTGTGCCTGCTGAATAACTTCTATCCCAG AGAGGCCAAAGTACAGTGGAAGGTGGATAACGCCCTCC AATCGGGTAACTCCCAGGAGAGTGTCACAGAGCAGGAC AGCAAGGACAGCACCTACAGCCTCAGCAGCACCCTGAC GCTGAGCAAAGCAGACTACGAGAAACACAAAGTCTACG CCTGCGAAGTCACCCATCAGGGCCTGAGCTCGCCCGTCA CAAAGAGCTTCAACAGGGGAGAGTGT |
| 74 | Dimeric hu 4-1BBL (71-254)-CL* Fc knob chain | see Table 8 |
| 75 | Monomeric hu 4-1BBL (71-254)-CH1* | see Table 8 |

(continued)

| SEQID NO: | Description | Sequence |
|---|---|---|
| 153 | DP47 Fc hole chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP |
| | | KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVCTLPPSRDELTKNQVS LSCAVKGFYPSDIAVEWESNGQPENNYKTTPPVLDSDGSFF LVSKLTVDKSRWQQGNVFSCSVMHEALHNHYTQKSLSLS PGK |
| 154 | DP47 light chain | EIVLTQSPGTLSLSPGERATLSCRASQSVSSSYLAWYQQKP GQAPRLLIYGASSRATGIPDRFSGSGSGTDFTLTISRLEPEDF AVYYCQQYGSSPLTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

Table 19 shows the cDNA and amino acid sequences of the bivalent DP47-untargeted split trimeric 4-1BB ligand (71-254) Fc (kih) fusion molecule Control C.

**Table 19: Sequences of bivalent DP47-untargeted human 4-1BB ligand (71-254) containing Fc (kih) fusion molecule Control C**

| SEQ ID NO: | Description | Sequence |
|---|---|---|
| 155 | nucleotide sequence of DP47 Fc hole chain fused to dimeric hu 4-1BBL (71-254) | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCCTCCGTGTTCCCCCTGGCCCCCAG CAGCAAGAGCACCAGCGGCGGCACAGCCGCTCTGGGCT GCCTGGTCAAGGACTACTTCCCCGAGCCCGTGACCGTGT CCTGGAACAGCGGAGCCCTGACCTCCGGCGTGCACACC TTCCCCGCCGTGCTGCAGAGTTCTGGCCTGTATAGCCTG AGCAGCGTGGTCACCGTGCCTTCTAGCAGCCTGGGCACC CAGACCTACATCTGCAACGTGAACCACAAGCCCAGCAA CACCAAGGTGGACAAGAAGGTGGAGCCCAAGAGCTGCG ACAAAACTCACACATGCCCACCGTGCCCAGCACCTGAA GCTGCAGGGGGACCGTCAGTCTTCCTCTTCCCCCCAAAA CCCAAGGACACCCTCATGATCTCCCGGACCCCTGAGGTC ACATGCGTGGTGGTGGACGTGAGCCACGAAGACCCTGA GGTCAAGTTCAACTGGTACGTGGACGGCGTGGAGGTGC ATAATGCCAAGACAAAGCCGCGGGAGGAGCAGTACAAC AGCACGTACCGTGTGGTCAGCGTCCTCACCGTCCTGCAC CAGGACTGGCTGAATGGCAAGGAGTACAAGTGCAAGGT CTCCAACAAAGCCCTCGGCGCCCCCATCGAGAAAACCA TCTCCAAAGCCAAAGGGCAGCCCCGAGAACCACAGGTG TGCACCCTGCCCCCATCCCGGGATGAGCTGACCAAGAA CCAGGTCAGCCTCTCGTGCGCAGTCAAAGGCTTCTATCC |

(continued)

| SEQ ID NO: | Description | Sequence |
|---|---|---|
| | | CAGCGACATCGCCGTGGAGTGGGAGAGCAATGGGCAGC CGGAGAACAACTACAAGACCACGCCTCCCGTGCTGGAC TCCGACGGCTCCTTCTTCCTCGTGAGCAAGCTCACCGTG GACAAGAGCAGGTGGCAGCAGGGGAACGTCTTCTCATG CTCCGTGATGCATGAGGCTCTGCACAACCACTACACGCA GAAGAGCCTCTCCCTGTCTCCGGGTGGAGGCGGCGGAA GCGGAGGAGGAGGATCCAGAGAGGGCCCTGAGCTGAGC CCCGATGATCCTGCTGGACTGCTGGACCTGCGGCAGGGC ATGTTTGCTCAGCTGGTGGCCCAGAACGTGCTGCTGATC GATGGCCCCCTGTCCTGGTACAGCGATCCTGGACTGGCT GGCGTGTCACTGACAGGCGGCCTGAGCTACAAAGAGGA CACCAAAGAACTGGTGGTGGCCAAGGCCGGCGTGTACT ACGTGTTCTTTCAGCTGGAACTGCGGAGAGTGGTGGCCG GCGAAGGATCTGGCTCTGTGTCTCTGGCCCTGCATCTGC AGCCTCTGAGAAGCGCTGCTGGCGCTGCAGCTCTGGCAC TGACAGTGGATCTGCCTCCTGCCAGCTCCGAGGCCCGGA ATAGCGCATTTGGGTTTCAAGGCAGGCTGCTGCACCTGT CTGCCGGCCAGAGGCTGGGAGTGCATCTGCACACAGAG GCCAGGGCTAGACACGCCTGGCAGCTGACACAGGGCGC TACAGTGCTGGGCCTGTTCAGAGTGACCCCCGAGATTCC AGCCGGCCTGCCTTCTCCAAGAAGCGAAGGCGGAGGCG GATCTGGCGGCGGAGGATCTAGAGAGGGACCCGAACTG TCCCCTGACGATCCAGCCGGGCTGCTGGATCTGAGACAG GGAATGTTCGCCCAGCTGGTGGCTCAGAATGTGCTGCTG ATTGACGGACCTCTGAGCTGGTACTCCGACCCAGGGCTG GCAGGGGTGTCCCTGACTGGGGGACTGTCCTACAAAGA AGATACAAAAGAACTGGTGGTGGCTAAAGCTGGGGTGT ACTATGTGTTTTTTCAGCTGGAACTGAGGCGGGTGGTGG CTGGGGAGGGCTCAGGATCTGTGTCCCTGGCTCTGCATC TGCAGCCACTGCGCTCTGCTGCTGGCGCAGCTGCACTGG CTCTGACTGTGGACCTGCCACCAGCCTCTAGCGAGGCCA GAAACAGCGCCTTCGGGTTCCAAGGACGCCTGCTGCATC TGAGCGCCGGACAGCGCCTGGGAGTGCATCTGCATACT GAAGCCAGAGCCCGGCATGCTTGGCAGCTGACTCAGGG GGCAACTGTGCTGGGACTGTTTCGCGTGACACCTGAGAT CCCTGCCGGACTGCCAAGCCCTAGATCAGAA |

(continued)

| SEQ ID NO: | Description | Sequence |
|---|---|---|
| 156 | nucleotide sequence of DP47 Fc knob chain fused to monomeric hu 4-1BBL (71-254) | GAGGTGCAATTGTTGGAGTCTGGGGGAGGCTTGGTACA<br>GCCTGGGGGGTCCCTGAGACTCTCCTGTGCAGCCTCCGG<br>ATTCACCTTTAGCAGTTATGCCATGAGCTGGGTCCGCCA<br>GGCTCCAGGGAAGGGGCTGGAGTGGGTCTCAGCTATTA<br>GTGGTAGTGGTGGTAGCACATACTACGCAGACTCCGTGA<br>AGGGCCGGTTCACCATCTCCAGAGACAATTCCAAGAAC<br>ACGCTGTATCTGCAGATGAACAGCCTGAGAGCCGAGGA<br>CACGGCCGTATATTACTGTGCGAAAGGCAGCGGATTTGA<br>CTACTGGGGCCAAGGAACCCTGGTCACCGTCTCGAGTGC<br>TAGCACCAAGGGCCCATCGGTCTTCCCCCTGGCACCCTC<br>CTCCAAGAGCACCTCTGGGGGCACAGCGGCCCTGGGCT<br>GCCTGGTCAAGGACTACTTCCCCGAACCGGTGACGGTGT<br>CGTGGAACTCAGGCGCCCTGACCAGCGGCGTGCACACC<br>TTCCCGGCTGTCCTACAGTCCTCAGGACTCTACTCCCTC<br>AGCAGCGTGGTGACCGTGCCCTCCAGCAGCTTGGGCAC<br>CCAGACCTACATCTGCAACGTGAATCACAAGCCCAGCA<br>ACACCAAGGTGGACAAGAAAGTTGAGCCCAAATCTTGT<br>GACAAAACTCACACATGCCCACCGTGCCCAGCACCTGA |

(continued)

| SEQ ID NO: | Description | Sequence |
|---|---|---|
| | | AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCCTGCAGAGATGAGCTGACCAAGA ACCAGGTGTCCCTGTGGTGTCTGGTCAAGGGCTTCTACC CCAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCGT GGACAAGAGCCGGTGGCAGCAGGGCAACGTGTTCAGCT GCAGCGTGATGCACGAGGCCCTGCACAACCACTACACC CAGAAGTCCCTGAGCCTGAGCCCCGGCGGAGGCGGCGG AAGCGGAGGAGGAGGATCCAGAGAGGGCCCTGAGCTGA GCCCCGATGATCCTGCTGGACTGCTGGACCTGCGGCAGG GCATGTTTGCTCAGCTGGTGGCCCAGAACGTGCTGCTGA TCGATGGCCCCCTGTCCTGGTACAGCGATCCTGGACTGG CTGGCGTGTCACTGACAGGCGGCCTGAGCTACAAAGAG GACACCAAAGAACTGGTGGTGGCCAAGGCCGGCGTGTA CTACGTGTTCTTTCAGCTGGAACTGCGGAGAGTGGTGGC CGGCGAAGGATCTGGCTCTGTGTCTCTGGCCCTGCATCT GCAGCCTCTGAGAAGCGCTGCTGGCGCTGCAGCTCTGGC ACTGACAGTGGATCTGCCTCCTGCCAGCTCCGAGGCCCG GAATAGCGCATTTGGGTTTCAAGGCAGGCTGCTGCACCT GTCTGCCGGCCAGAGGCTGGGAGTGCATCTGCACACAG AGGCCAGGGCTAGACACGCCTGGCAGCTGACACAGGGC GCTACAGTGCTGGGCCTGTTCAGAGTGACCCCCGAGATT CCAGCCGGCCTGCCTTCTCCAAGAAGCGAA |
| 152 | nucleotide sequence of DP47 light chain | see Table 18 |

(continued)

| SEQ ID NO: | Description | Sequence |
|---|---|---|
| 157 | DP47 Fc hole chain fused to dimeric hu 4-1BBL (71-254) | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVCTLPPSRDELTKNQVS LSCAVKGFYPSDIAVEWESNGQPENNYKTTPPVLDSDGSFF LVSKLTVDKSRWQQGNVFSCSVMHEALHNHYTQKSLSLS PGGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGLPSPRSEGGG GSGGGGSREGPELSPDDPAGLLDLRQGMFAQLVAQNVLLI DGPLSWYSDPGLAGVSLTGGLSYKEDTKELVVAKAGVYY |
| | | VFFQLELRRVVAGEGSGSVSLALHLQPLRSAAGAAALALT VDLPPASSEARNSAFGFQGRLLHLSAGQRLGVHLHTEARA RHAWQLTQGATVLGLFRVTPEIPAGLPSPRSE |
| 158 | DP47 Fc knob chain fused to monomeric hu 4-1BBL (71-254) | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGLPSPRSE |
| 154 | DP47 light chain | see Table 18 |

Table 20 shows, respectively, the cDNA and amino acid sequences of the monovalent DP47-untargeted split trimeric 4-1BB ligand (71-248) Fc (kih) fusion containing CH-CL cross with charged residues, control D.

**Table 20: cDNA and amino acid sequences of monovalent DP47 untargeted split trimeric human 4-1BB ligand (71-248) Fc (kih) fusion with CH-CL cross and with charged residues (control D). * charged residues**

| SEQ ID NO: | Description | Sequence |
|---|---|---|
| 139 | nucleotide sequence of dimeric hu 4-1BBL (71-248) - CL* Fc knob chain | see Table 11 |

(continued)

| SEQ ID NO: | Description | Sequence |
|---|---|---|
| 140 | nucleotide sequence of monomeric hu 4-1BBL (71-248) - CH1* | see Table 11 |
| 151 | nucleotide sequence DP47 Fc hole chain | see Table 18 |
| 152 | nucleotide sequence DP47 light chain | see Table 18 |
| 78 | Dimeric hu 4-1BBL (71-254) - CL* Fc knob chain | see Table 11 |
| 79 | Monomeric hu 4-1BBL (71-254) - CH1* | see Table 11 |
| 153 | DP47 Fc hole chain | see Table 18 |
| 154 | DP47 light chain | see Table 18 |

**2.10 Preparation of human IgG1 antigen binding molecules as controls**

An additional control molecule (Control F) used in the assays was an untargeted DP47, germline control, human IgG1, containing the Pro329Gly, Leu234Ala and Leu235Ala mutations, to abrogate binding to Fc gamma receptors according to the method described in International Patent Appl. Publ. No. WO 2012/130831).

Table 21 shows the cDNA and amino acid sequences of the cDNA and amino acid sequences of the untargeted DP47 huIgG1 PGLALA (Control F).

**Table 21: Sequences of untargeted DP47 huIgG1 (Control F)**

| SEQ ID NO: | Description | Sequence |
|---|---|---|
| 159 | nucleotide sequence of DP47 heavy chain (hu IgG1 PGLALA) | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCATCGGTCTTCCCCCTGGCACCCTC CTCCAAGAGCACCTCTGGGGGCACAGCGGCCCTGGGCT GCCTGGTCAAGGACTACTTCCCCGAACCGGTGACGGTGT CGTGGAACTCAGGCGCCCTGACCAGCGGCGTGCACACC TTCCCGGCTGTCCTACAGTCCTCAGGACTCTACTCCCTC AGCAGCGTGGTGACCGTGCCCTCCAGCAGCTTGGGCAC CCAGACCTACATCTGCAACGTGAATCACAAGCCCAGCA ACACCAAGGTGGACAAGAAAGTTGAGCCCAAATCTTGT GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCATCCCGGGATGAGCTGACCAAGA ACCAGGTCAGCCTGACCTGCCTGGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCTACAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAAA |
| 152 | nucleotide sequence of DP47 light chain | see Table 18 |
| 160 | DP47 heavy chain (hu IgG1 PGLALA) | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPSRDELTKNQVS LTCLVKGFYPSDIAVEWESNGQPENNYKTTPPVLDSDGSFF LYSKLTVDKSRWQQGNVFSCSVMHEALHNHYTQKSLSLS PGK |

(continued)

| SEQ ID NO: | Description | Sequence |
|---|---|---|
| 154 | DP47 light chain | see Table 18 |

**Table 22** shows the cDNA and amino acid sequences of the anti-PD1 huIgG1 PGLALA (clone 0314), i.e. control M.

**Table 22: cDNA and amino acid sequences of anti-PD1(0314) huIgG1 PGLALA (control M)**

| SEQ ID NO: | Description | Sequence |
|---|---|---|
| 161 | nucleotide sequence of PD1(0314) heavy chain (huIgG1 PGLALA) | GAAGTGCAGCTGCTCGAAAGCGGCGGGGGAC TGGTCCAGCCCGGCGGTTCCCTGAGGCTGTCT TGCGCCGCTTCAGGGTTCAGCTTTTCCTCTTAC ACCATGAGTTGGGTCAGACAGGCACCTGGCAA GGGACTGGAGTGGGTCGCCACAATCAGCGGTG GCGGGCGCGACATTTATTACCCAGATTCCGTG AAAGGACGGTTCACCATCTCTAGGGACAACTC AAAGAATACTCTGTATTTGCAGATGAACAGCC TGAGAGCTGAGGATACAGCAGTTTACTACTGT GTGCTCCTGACCGGCCGCGTCTATTTTGCCCTT GACTCCTGGGGACAAGGCACTCTGGTGACCGT ATCTAGTGCTAGCACCAAGGGCCCATCGGTCT TCCCCCTGGCACCCTCCTCCAAGAGCACCTCT GGGGGCACAGCGGCCCTGGGCTGCCTGGTCAA GGACTACTTCCCCGAACCGGTGACGGTGTCGT GGAACTCAGGCGCCCTGACCAGCGGCGTGCAC ACCTTCCCGGCTGTCCTACAGTCCTCAGGACTC TACTCCCTCAGCAGCGTGGTGACCGTGCCCTC CAGCAGCTTGGGCACCCAGACCTACATCTGCA ACGTGAATCACAAGCCCAGCAACACCAAGGT GGACAAGAAAGTTGAGCCCAAATCTTGTGACA AAACTCACACATGCCCACCGTGCCCAGCACCT GAAGCTGCAGGGGGACCGTCAGTCTTCCTCTT |

(continued)

| SEQ ID NO: | Description | Sequence |
|---|---|---|
| | | CCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTACACCCTGCCCCCATCCCGGGATGAGCTGACCAAGAACCAGGTCAGCCTGACCTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA |
| 134 | nucleotide sequence of anti-PD1(0314) light chain | see Table 8 |
| 162 | PD1(0314) heavy chain (huIgG1 PGLALA) | EVQLLESGGGLVQPGGSLRLSCAASGFSFSSYTMSWVRQAPGKGLEWVATISGGGRDIYYPDSVKGRFTISRDNSKNTLYLQMNSLRAEDTAVYYCVLLTGRVYFALDSWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 83 | anti-PD1(0314) light chain | see Table 8 |

**Example 3**

**Production and Purification of monovalent and bivalent anti-PD1 targeted split trimeric 4-1BB ligand Fc fusion antigen binding molecules and control molecules**

The targeted and untargeted split trimeric 4-1BB ligand Fc (kih) fusion molecule encoding sequences were cloned into a plasmid vector, which drives expression of the insert from an MPSV promoter and contains a synthetic polyA sequence located at the 3' end of the CDS. In addition, the vector contains an EBV OriP sequence for episomal maintenance of the plasmid.

The split trimeric 4-1BB ligand Fc (kih) fusion molecules were produced by co-transfecting HEK293-EBNA cells with the mammalian expression vectors using polyethylenimine. The cells were transfected with the corresponding expression vectors. For constructs 7.1, 7.2, 7.4, 7.6 and the corresponding control molecules control B and D, at a 1:1:1:1 ratio ("vector Fc hole chain": "vector PD1 light chain": "vector Fc knob chain": "vector 4-1BBL light chain"). For constructs 7.3, 7.6, 7.11, 7.12, 7.13, 7.14 and the control C, at a 1:1:1 ratio ("vector Fc hole chain": "vector Fc knob chain": "vector anti-PD1 light chain"). Human IgGs, used as control in the assay, were produced as for the bispecific constructs (for transfection only a vector for light and a vector for heavy chain were used at a 1:1 ratio).

For production in 500 mL shake flasks, 300 million HEK293 EBNA cells were seeded 24 hours before transfection. For transfection cells were centrifuged for 10 minutes at 210 x g, and the supernatant was replaced by 20mL pre-warmed CD CHO medium. Expression vectors (200 μg of total DNA) were mixed in 20 mL CD CHO medium. After addition of 540 μL PEI, the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% $CO_2$ atmosphere. After the incubation, 160 mL of Excell medium supplemented with 6mM L-Glutamine, 5g/L PEPSOY and 1.2mM valproic acid was added and cells were cultured for 24 hours. One day after transfection 12% Feed 7 and Glucose (final conc. 3g/L) were added. After culturing for 7 days, the supernatant was collected by centrifugation for 30-40 minutes at least 400 x g. The solution was sterile filtered (0.22 μm filter), supplemented with sodium azide to a final concentration of 0.01 % (w/v), and kept at 4 °C.

Secreted proteins were purified from cell culture supernatants by affinity chromatography using Protein A, followed by size exclusion chromatography. For affinity chromatography, the supernatant was loaded on a MabSelect Sure column (CV = 5-15 mL, resin from GE Healthcare) equilibrated with Sodium Phosphate (20 mM), Sodium Citrate (20 mM) buffer (pH 7.5). Unbound protein was removed by washing with at least 6 column volumes of the same buffer. The bound protein was eluted using either a linear gradient (20 CV) or a step elution (8 CV) with 20 mM sodium citrate, 100 mM Sodium chloride, 100 mM Glycine buffer (pH 2.5). For the linear gradient an additional 4 column volumes step elution was applied.

The pH of collected fractions was adjusted by adding 1/10 (v/v) of 0.5M sodium phosphate, pH8.0. The protein was concentrated prior to loading on a HiLoad Superdex 200 column (GE Healthcare) equilibrated with 20 mM Histidine, 140 mM sodium chloride, 0.01% (v/v) Tween20 solution of pH 6.0.

The protein concentration was determined by measuring the optical density (OD) at 280 nm, using a molar extinction coefficient calculated on the basis of the amino acid sequence. Purity and molecular weight of the targeted trimeric 4-1BB ligand Fc (kih) fusion was analyzed by SDS-PAGE in the presence and absence of a reducing agent (5 mM 1,4-dithiotreitol) and staining with Coomassie SimpleBlue™ SafeStain (Invitrogen USA) or CE-SDS using Caliper LabChip GXII (Perkin Elmer). The aggregate content of samples was analyzed using a TSKgel G3000 SW XL analytical size-exclusion column (Tosoh) equilibrated in 25 mM $K_2HPO_4$ 125 mM NaCl, 200 mM L-Arginine Monohydrocloride, 0.02 % (w/v) NaN3, pH 6.7 running buffer at 25 °C.

Table 23 summarizes the yield and final monomer content of the FAP (4B9) targeted and untargeted 4-1BB ligand trimer-containing Fc (kih) fusion antigen binding molecules and control molecules.

**Table 23 Biochemical analysis of PD1 targeted and untargeted 4-1BB ligand trimer-containing Fc (kih) fusion antigen binding molecules and control molecules**

| Construct | Monomer [%] (SEC) | Yield [mg/l] |
|---|---|---|
| Construct 7.1 | 97.2 | 110.2 |
| Construct 7.3 | 96.6 | 8.9 |
| Construct 7.5 | 85 | 5.1 |
| Construct 7.6 | 100 | 16.5 |
| Control B | 99 | 15.4 |
| Control C | 98 | 12.6 |
| Control D | 99.5 | 25.9 |
| Control M | 99 | 34.4 |
| Control F | 100 | 50 |

## Example 4

### Simultaneous binding of PD1-targeted 4-1BB split trimeric ligand Fc fusion antigen binding molecules by surface plasmon resonance

### 4.1 Preparation, purification and characterization of antigens for surface plasmon resonance

Preparation of 4-1BB Fc (kih) fusion molecule

A DNA sequence encoding the ectodomain of human 4-1BB (amino acids 24 to 186 of human 4-1BB according to Q07011, SEQ ID NO:163) were subcloned in frame with the human IgG1 heavy chain CH2 and CH3 domains on the knob. An AcTEV protease cleavage site was introduced between an antigen ectodomain and the Fc of human IgG1. An Avi tag for directed biotinylation was introduced at the C-terminus of the antigen-Fc knob. Combination of the antigen-Fc knob chain containing the S354C/T366W mutations, with a Fc hole chain containing the Y349C/T366S/L368A/Y407V mutations allows generation of a heterodimer which includes a single copy of 4-1BB ectodomain containing chain, thus creating a monomeric form of Fc-linked antigen. Table 24 shows the cDNA and amino acid sequences of the antigen Fc-fusion construct.

**Table 24: cDNA and Amino acid sequences of monomeric antigen Fc(kih) fusion molecules**

| SEQ ID NO: | Antigen | Sequence |
|---|---|---|
| 164 | Nucleotide sequence of Fc hole chain | GACAAAACTCACACATGCCCACCGTGCCCAGCACCTGA ACTCCTGGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCCCAGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAAA |
| 165 | Nucleotide sequence of | CTGCAGGACCCCTGCAGCAACTGCCCTGCCGGCACCTTC TGCGACAACAACCGGAACCAGATCTGCAGCCCCTGCCC CCCCAACAGCTTCAGCTCTGCCGGCGGACAGCGGACCT |

(continued)

| SEQ ID NO: | Antigen | Sequence |
|---|---|---|
| | human 4-1BB antigen Fc knob chain | GCGACATCTGCAGACAGTGCAAGGGCGTGTTCAGAACC<br>CGGAAAGAGTGCAGCAGCACCAGCAACGCCGAGTGCGA<br>CTGCACCCCCGGCTTCCATTGTCTGGGAGCCGGCTGCAG<br>CATGTGCGAGCAGGACTGCAAGCAGGGCCAGGAACTGA<br>CCAAGAAGGGCTGCAAGGACTGCTGCTTCGGCACCTTC<br>AACGACCAGAAGCGGGGCATCTGCCGGCCCTGGACCAA<br>CTGTAGCCTGGACGGCAAGAGCGTGCTGGTCAACGGCA<br>CCAAAGAACGGGACGTCGTGTGCGGCCCCAGCCCTGCT<br>GATCTGTCTCCTGGGGCCAGCAGCGTGACCCCTCCTGCC<br>CCTGCCAGAGAGCCTGGCCACTCTCCTCAGGTCGACGAA<br>CAGTTATATTTTCAGGGCGGCTCACCCAAATCTGCAGAC<br>AAAACTCACACATGCCCACCGTGCCCAGCACCTGAACTC<br>CTGGGGGGACCGTCAGTCTTCCTCTTCCCCCCAAAACCC<br>AAGGACACCCTCATGATCTCCCGGACCCCTGAGGTCACA<br>TGCGTGGTGGTGGACGTGAGCCACGAAGACCCTGAGGT<br>CAAGTTCAACTGGTACGTGGACGGCGTGGAGGTGCATA<br>ATGCCAAGACAAAGCCGCGGGAGGAGCAGTACAACAGC<br>ACGTACCGTGTGGTCAGCGTCCTCACCGTCCTGCACCAG<br>GACTGGCTGAATGGCAAGGAGTACAAGTGCAAGGTCTC<br>CAACAAAGCCCTCCCAGCCCCCATCGAGAAAACCATCT<br>CCAAAGCCAAAGGGCAGCCCCGAGAACCACAGGTGTAC<br>ACCCTGCCCCCATGCCGGGATGAGCTGACCAAGAACCA<br>GGTCAGCCTGTGGTGCCTGGTCAAAGGCTTCTATCCCAG<br>CGACATCGCCGTGGAGTGGGAGAGCAATGGGCAGCCGG<br>AGAACAACTACAAGACCACGCCTCCCGTGCTGGACTCC<br>GACGGCTCCTTCTTCCTCTACAGCAAGCTCACCGTGGAC<br>AAGAGCAGGTGGCAGCAGGGGAACGTCTTCTCATGCTC<br>CGTGATGCATGAGGCTCTGCACAACCACTACACGCAGA<br>AGAGCCTCTCCCTGTCTCCGGGTAAATCCGGAGGCCTGA<br>ACGACATCTTCGAGGCCCAGAAGATTGAATGGCACGAG |
| 166 | Fc hole chain | DKTHTCPPCPAPELLGGPSVFLFPPKPKDTLMISRTPEVTCV<br>VVDVSHEDPEVKFNWYVDGVEVHNAKTKPREEQYNSTYR<br>VVSVLTVLHQDWLNGKEYKCKVSNKALPAPIEKTISKAKG<br>QPREPQVCTLPPSRDELTKNQVSLSCAVKGFYPSDIAVEWE<br>SNGQPENNYKTTPPVLDSDGSFFLVSKLTVDKSRWQQGNV<br>FSCSVMHEALHNHYTQKSLSLSPGK |

(continued)

| SEQ ID NO: | Antigen | Sequence |
|---|---|---|
| 167 | human 4-1BB antigen Fc knob chain | LQDPCSNCPAGTFCDNNRNQICSPCPPNSFSSAGGQRTCDI CRQCKGVFRTRKECSSTSNAECDCTPGFHCLGAGCSMCEQ DCKQGQELTKKGCKDCCFGTFNDQKRGICRPWTNCSLDG KSVLVNGTKERDVVCGPSPADLSPGASSVTPPAPAREPGHS PQVDEQLYFQGGSPKSADKTHTCPPCPAPELLGGPSVFLFP PKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPCRDELTKNQV SLWCLVKGFYPSDIAVEWESNGQPENNYKTTPPVLDSDGS FFLYSKLTVDKSRWQQGNVFSCSVMHEALHNHYTQKSLS LSPGKSGGLNDIFEAQKIEWHE |

All 4-1BB-Fc-fusion molecule encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

For preparation of the biotinylated monomeric antigen/Fc fusion molecule, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 2 : 1 : 0.05 ratio ("antigen ECD-AcTEV- Fc knob" : "Fc hole" : "BirA").

For protein production in 500 ml shake flasks, 400 million HEK293 EBNA cells were seeded 24 hours before transfection. For transfection cells were centrifuged for 5 minutes at 210 g, and the supernatant was replaced by pre-warmed CD CHO medium. Expression vectors were resuspended in 20 mL of CD CHO medium containing 200 μg of vector DNA. After addition of 540 μL of polyethylenimine (PEI), the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5 % $CO_2$ atmosphere. After the incubation, 160 mL of F17 medium was added and cells were cultured for 24 hours. One day after transfection, 1 mM valproic acid and 7 % Feed 1 with supplements were added to the culture. After 7 days of culturing, the cell supernatant was collected by spinning down cells for 15 min at 210 g. The solution was sterile filtered (0.22 μm filter), supplemented with sodium azide to a final concentration of 0.01 % (w/v), and kept at 4°C.

Secreted proteins were purified from cell culture supernatants by affinity chromatography using Protein A, followed by size exclusion chromatography. For affinity chromatography, the supernatant was loaded on a HiTrap ProteinA HP column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate, 0.5 M sodium chloride containing buffer (pH 7.5). The bound protein was eluted using a linear pH-gradient of sodium chloride (from 0 to 500 mM) created over 20 column volumes of 20 mM sodium citrate, 0.01 % (v/v) Tween-20, pH 3.0. The column was then washed with 10 column volumes of 20 mM sodium citrate, 500 mM sodium chloride, 0.01 % (v/v) Tween-20, pH 3.0.

The pH of collected fractions was adjusted by adding 1/40 (v/v) of 2M Tris, pH8.0. The protein was concentrated and filtered prior to loading on a HiLoad Superdex 200 column (GE Healthcare) equilibrated with 2mM MOPS, 150 mM sodium chloride, 0.02 % (w/v) sodium azide solution of pH 7.4.

For affinity determination to the human receptor, the ectodomain of human 4-1BB was also subcloned in frame with an avi (GLNDIFEAQKIEWHE, SEQ ID NO:168) and a hexahistidine tag.

Protein production was performed as described above for the Fc-fusion protein. Secreted proteins were purified from cell culture supernatants by chelating chromatography, followed by size exclusion chromatography. The first chromatographic step was performed on a NiNTA Superflow Cartridge (5ml, Qiagen) equilibrated in 20 mM sodium phosphate, 500 nM sodium chloride, pH7.4. Elution was performed by applying a gradient over 12 column volume from 5 % to 45 % of elution buffer (20 mM sodium phosphate, 500 nM sodium chloride, 500 mM Imidazole, pH7.4). The protein was concentrated and filtered prior to loading on a HiLoad Superdex 75 column (GE Healthcare) equilibrated with 2 mM MOPS, 150 mM sodium chloride, 0.02 % (w/v) sodium azide solution of pH 7.4 (Table 25).

**Table 25: Sequences of monomeric human 4-1BB His molecule**

| SEQ ID NO: | antigen | Sequence |
|---|---|---|
| 169 | nucleotide sequence of human 4-1BB His | CTGCAGGACCCCTGCAGCAACTGCCCTGCCGGCACCTTCTG<br>CGACAACAACCGGAACCAGATCTGCAGCCCCTGCCCCCCC<br>AACAGCTTCAGCTCTGCCGGCGGACAGCGGACCTGCGACA<br>TCTGCAGACAGTGCAAGGGCGTGTTCAGAACCCGGAAAGA<br>GTGCAGCAGCACCAGCAACGCCGAGTGCGACTGCACCCCC<br>GGCTTCCATTGTCTGGGAGCCGGCTGCAGCATGTGCGAGC<br>AGGACTGCAAGCAGGGCCAGGAACTGACCAAGAAGGGCT<br>GCAAGGACTGCTGCTTCGGCACCTTCAACGACCAGAAGCG<br>GGGCATCTGCCGGCCCTGGACCAACTGTAGCCTGGACGGC<br>AAGAGCGTGCTGGTCAACGGCACCAAAGAACGGGACGTCG<br>TGTGCGGCCCCAGCCCTGCTGATCTGTCTCCTGGGGCCAGC<br>AGCGTGACCCCTCCTGCCCCTGCCAGAGAGCCTGGCCACTC<br>TCCTCAGGTCGACGAACAGTTATATTTCAGGGCGGCTCAG<br>GCCTGAACGACATCTTCGAGGCCCAGAAGATCGAGTGGCA<br>CGAGGCTCGAGCTCACCACCATCACCATCAC |
| 170 | human 4-1BB His | LQDPCSNCPAGTFCDNNRNQICSPCPPNSFSSAGGQRTCDICR<br>QCKGVFRTRKECSSTSNAECDCTPGFHCLGAGCSMCEQDCK<br>QGQELTKKGCKDCCFGTFNDQKRGICRPWTNCSLDGKSVLV<br>NGTKERDVVCGPSPADLSPGASSVTPPAPAREPGHSPQVDEQL<br>YFQGGSGLNDIFEAQKIEWHEARAHHHHHH |

Human PD1 Fc fusion antigen was bought from R&D systems (Cat N° 1086-PD-050).

**4.2 Determination of simultaneous binding of targeted C-terminal 4-1BB split trimeric ligand Fc fusion antigen binding molecules by surface plasmon resonance**

The capacity of binding simultaneously to human 4-1BB Fc(kih) and human PD1, was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated human 4-1BB Fc (kih) was directly coupled to a flow cell of a streptavidin (SA) sensor chip. Immobilization levels up to 200 resonance units (RU) were used. The targeted trimeric C-terminal 4-1BB ligand Fc (kih) fusion molecules were passed at a concentration range of 200 nM with a flow of 30 μL/minute through the flow cells over 90 seconds and dissociation was set to zero sec. Human PD1-Fc (R&D systems) was injected as second analyte with a flow of 30 μL/minute through the flow cells over 90 seconds at a concentration of 500 nM (**Figure 12A**). The dissociation was monitored for 120 sec. Bulk refractive index differences were corrected for by subtracting the response obtained in a reference flow cell, where no protein was immobilized. All bispecific constructs could bind simultaneously human 4-1BB and human PD1 (**Figures 12B, 12C, 12D and 12E**).

**Example 5**

**Functional characterization of the PD1 targeted 4-1BB ligand trimer-containing Fc fusion antigen binding molecules**

**5.1. Binding on fresh and activated human PMBCs of the PD1 targeted 4-1BB ligand trimer-containing Fc (kih) fusion antigen binding molecules**

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the diluted buffy coat contents were layered above the Histopaque 1077. The tubes were centrifuged for 30 min at 450 x g. PBMCs were then collected from the interface, washed three times with DPBS and resuspended in T cell medium consisting of RPMI 1640 medium (Gibco by Life Technology, Cat. No. 42401-042) supplied with 10 % (v/v) Fetal Bovine Serum (FBS, US-origin, PAN biotech, P30-2009, Lot P150307GI, gamma irradiated mycoplasma free, heat inactivated 35 min 56 °C), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 μM β-Mercaptoethanol (SIGMA, M3148).

PBMCs were used directly after isolation (binding on fresh human PBMCs) or they were stimulated to induce 4-1BB expression on the cell surface of T cells (binding on activated human PBMCs). To induce 4-1BB upregulation on human T cells, PBMCs were cultured for 3 days in RPMI 1640 (GIBCO by life technologies, Cat.-No. 42401-042) supplied with 10% Fetal Bovine Serum (FBS, US-origin, PAN biotech, P30-2009, Lot P150307GI, gamma irradiated mycoplasma free, heat inactivated 35 min 56 °C), 1% GlutaMAX-I (GIBCO by life technologies, Cat.-No. 35050-038), 200 U/mL Proleukin (Novartis Pharma Schweiz AG, CHCLB-P-476-700-10340, Lot AA4493BAL) and 2 ug/mL PHA-L (SIGMA, Cat.-No. L2769) at a concentration of $1x10^6$ cells/mL. After three days pre-activated human PBMCs were harvested, resuspended in RPMI 1640 supplied with 10 % FBS, 1 mM Sodium-Pyruvat (SIGMA, Cat.-No. S8636), 1% Gluta-MAX-I, 1% MEM-non essential amino acid Solution (SIGMA, Cat.-No. M7145) and 50 μM beta-Mercaptoethanol (Sigma M3148) to a final concentration of $1x10^6$ cells/mL. Afterwards cells were seeded in 6-well tissue culture plates (TTP, Cat.-No. 92006) which had been coated overnight at 4°C with 10 ug/mL anti-human CD3 (clone OKT3, Mouse IgG2a, BioLegend, Cat.-No. 317315) and 2 ug/mL anti-human CD28 (clone CD28.2, Mouse IgG1, BioLegend, Cat.-No. 302923). Cells were further incubated for 2 days at 37°C and 5% $CO_2$

For the binding assay 0.1 x $10^6$ fresh or activated PBMCs were added to each well of a round-bottom suspension cell 96-well plates (greiner bio-one, cellstar, Cat. No. 650185). Plates were centrifuged 5 minutes with 350 x g and at 4 °C and supernatant were discarded. Afterwards cells were stained in 100 μL/well DPBS containing 1:5000 diluted fixable viability dye eF660 (eBioscience, Cat.-No. 65-0864-18) for 30 minutes at 4°C in the dark. Cells were washed once with 200 μL ice cold cold DPBS buffer. Next, 50 μL/well of 4 °C cold FACS buffer (DPBS supplied with 2 % FBS, 5 mM EDTA pH8 (Amresco, Cat. No. E177) and 7.5 mM Sodium azide (Sigma-Aldrich, Cat.-No. S2002)) containing titrated concentrations of construct 7.1, construct 7.3, construct 7.5 and construct 7.6 and as controls control B, control C, control D, control M and control F were added and cells were incubated for 60 minutes at 4 °C, washed three times with 200 μL/well 4 °C FACS buffer. Cells were further resuspended in 50 μL/well of 4 °C cold FACS buffer containing 0.67 μg/mL anti-human CD45-AF488 (clone HI30, mouse igGlk, BioLegend, Cat.-No. 304019), 0.33 μg/mL anti-human CD8a-BV510 (clone SK1, mouse IgG1k, BioLegend, Cat.-No. 344732), 0.23 μg/mL anti-human CD4-BV421 (clone OKT4, mouse IgG2bκ, BioLegend, Cat.-No. 317434), 0.67 μg/mL anti-human CD3-PerCP-Cy5.5 (clone UCHT1, mouse IgG1k, BioLegend, Cat.-No. 300430), 0.67 μg/mL anti-human CD19-PE/Cy7 (clone HIB19, mouse IgG1k, BioLegend, Cat.-No. 302216), 5 μg/mL PE-conjugated AffiniPure anti-human IgG F(ab')2-fragment-specific goat F(ab')2 fragment (Jackson ImmunoResearch, Cat. No. 109 116 098) and incubated for 30 min at 4°C. Cells were washed twice with 200 μL/well 4 °C FACS buffer and then fixed with DPBS supplied with 1 % Formaldehyde (Sigma, HT501320-9.5L). Cells were resuspended in 100 μL/well FACS-buffer and acquired using the MACS Quant Analyzer 10 (Miltenyi Biotech). Data was analyzed using FlowJo V10 (FlowJo, LLC) and GraphPad Prism 6.04 (GraphPad Software, Inc)

Gates were set on living $CD45^+$ $CD3^+$ $CD19^{neg}$ $CD8^{neg}$ $CD4^+$ T cells, $CD45^+$ $CD3^+$ CD19 neg $CD4^{neg}$ $CD8^+$ T cells, $CD45^+$ $CD3^+$ $CD4^{neg}$ $CD8^{neg}$ γδ T cells and $CD45^+$ $CD3^{neg}$ $CD19^+$ B cells and geo means of fluorescence intensity of PE-conjugated AffiniPure anti-human IgG IgG Fcγ-fragment-specific goat F(ab')2 fragment were blotted against the titrated concentration of PD1-targeted or DP47-untargeted 4-1BB split trimeric ligand Fc fusion antigen binding molecules. As shown in **Figures 13A-H,** on resting fresh isolated human PBMCs from healthy donors only the PD1 targeted 4-1BB split trimeric ligand Fc fusion antigen binding molecules (construct 7.1., construct 7.3, construct 7.5 and construct 7.6) and the PD1 targeted human IgG control M bound to $CD3^+$ $CD4^+$, $CD3^+$ $CD8^+$ and $CD3^+$ $CD4^{neg}$ $CD8^{neg}$ γδ T cells. Whereas the DP47-untargeted 4-1BB split trimeric ligand Fc fusion antigen binding molecules (control B, C and D) did not bind to any freshly isolated PBMCs. Therefore on freshly isolated human PBMCs the majority of cells do not express 4-1BB whereas a population of T cells expresses a detectable amount of PD1. In Table 26 the $EC_{50}$ values are summarized for the binding curves to freshly isolated human PBMCs.

**Table 26: $EC_{50}$ values of binding curves to freshly isolated resting human PBMCs**

| | Control M | Construct 7.1 | Construct 7.3 | Construct 7.5 | Construct 7.6 |
|---|---|---|---|---|---|
| **$EC_{50}$ [nM] on activated CD4 T cells** | 0.03 | 0.13 | 0.11 | 0.28 | 0.11 |
| **$EC_{50}$ [nM] on activated CD8 T cells** | 0.07 | 0.21 | 0.17 | 0.38 | 0.19 |
| **$EC_{50}$ [nM] on activated γδ T cells** | 0.03 | 0.34 | 0.13 | 0.42 | 0.27 |

As shown in **Figures 14A-H,** after activation living $CD45^+$ $CD3^+$ $CD19^{neg}$ $CD8^{neg}$ $CD4^+$ T cells, $CD45^+$ $CD3^+$ CD19 $^{neg}$ $CD4^{neg}$ $CD8^+$ T cells, $CD45^+$ $CD3^+$ CD4neg $CD8^{neg}$ $\gamma\delta$ T cells and $CD45^+$ $CD3^{neg}$ $CD19^+$ B cells express human 4-1BB and human PD1. The only PD1 binding molecules (control M) or only human 4-1BB binding molecules (controls B, control C and control D) proof thereby, that PD1 is highly upregulated on activated $CD4^+$ T cells, $CD8^+$ T cells, $\gamma\delta$ T cells and B cells, whereas 4-1BB is mainly upregulated on $CD8^+$ T cells, $\gamma\delta$ T cells and B cells and to a lesser extent on $CD4^+$ T cells. PD1-targeted 4-1BB split trimeric ligand Fc fusion antigen binding molecules (construct 7.1., construct 7.3, construct 7.5 and construct 7.6) bind to PD1 and human 4-1BB and this is reflected by higher geo means of fluorescence intensity compared to the fitting controls. In Table 27 the $EC_{50}$ values are summarized for the binding curves to activated human PBMCs.

**Table 27: $EC_{50}$ values of binding curves to activated human PBMCs**

| | Control B | Control C | Control D | Control M | Construct 7.1 | Construct 7.3 | Construct 7.5 | Construct 7.6 |
|---|---|---|---|---|---|---|---|---|
| $EC_{50}$ [nM] on activated CD8 T cells | 0.19 | 0.15 | 0.08 | 0.30 | 0.15 | 0.09 | 0.62 | 0.25 |
| $EC_{50}$ [nM] on activated CD4 | 0.43 | 0.25 | 0.15 | 0.33 | 0.32 | 0.15 | 1.32 | 0.33 |
| T cells | | | | | | | | |
| $EC_{50}$ [nM] on activated $\gamma\delta$ T cells | 0.20 | 0.16 | 0.09 | 0.71 | 0.12 | 0.12 | 0.52 | 0.32 |
| $EC_{50}$ [nM] on activated CD 19 cells | 0.15 | 0.15 | 0.08 | 0.33 | 0.15 | 0.08 | 1.13 | 0.40 |

**5.2 Binding of human PD1 and human 4-1BB transfected tumor cells**

For binding assays on PD1 and 4-1BB expressing tumor cells two genetically modified tumor cells were used. The generation of human expressing HeLa-hu4-1BB-NFκB-luc clone 26 cells will be described in Example 5.3.1. For human PD1 expressing tumor cell lines ,CHO-k1 cells were transfected with human PD1 as following: parental adherent CHO-K1 cell line (ATCC CCL-61) was stably transfected with plasmid 15311_hPD1-fl_pUC Neo coding for full-length human PD1 and selection with G418 (Neomycin resistance marker on plasmid).

0.1 x $10^6$ tumor cells resuspended in DPBS (Gibco by Life Technologies, Cat. No. 14190 326) were added to each well of a round-bottom suspension cell 96-well plates (greiner bio-one, cellstar, Cat. No. 650185). Cells were washed once with 200 μL DPBS. Cells were resuspended in 100 μL/well of 4 °C cold DPBS buffer containing 1:5000 diluted Fixable Viability Dye eFluor 660 (eBioscience, Cat. No. 65-0864-18) and plates were incubated for 30 minutes at 4°C. Cells were washed once with 200 μL/well 4 °C cold DPBS buffer and resuspended in 50 μL/well of 4 °C cold FACS buffer (DPBS supplied with 2 % FBS, 5 mM EDTA pH8 (Amresco, Cat. No. E177) and 7.5 mM Sodium azide (Sigma-Aldrich S2002)) containing PD1-targeted 4-1BB split trimeric ligand Fc fusion antigen binding molecules at a series of concentrations, followed by incubation for 1 hour at 4 °C. After extensive washing, cells were further stained with 50 μL/well of 4 °C cold FACS buffer containing 5 μg/mL PE-conjugated AffiniPure anti-human IgG F(ab')2-fragment-specific goat F(ab')2 fragment (Jackson ImmunoResearch, Cat. No. 109 116 098) for 30 minutes at 4 °C. Cells were washed twice with 200 μL/well 4 °C FACS buffer and cells were fixed in 50 μL/well DPBS containing 1 % Formaldehyde (Sigma, HT501320-9.5L). Cells were resuspended in 100 μL/well FACS-buffer and acquired using the MACS Quant Analyzer 10 (Miltenyi Biotech). Data was analyzed using FlowJo V10 (FlowJo, LLC) and GraphPad Prism 6.04 (GraphPad Software, Inc).

Gates were set on living tumor cells and geo means of fluorescence intensity of PE-conjugated AffiniPure anti-human IgG IgG Fcγ-fragment-specific goat F(ab')2 fragment were blotted against the titrated concentration of PD1-targeted 4-1BB split trimeric ligand Fc fusion antigen binding molecules or their controls. As shown in **Figures 15A and 15B,** only the PD1-targeted split trimeric 4-1BB ligand fusion molecules construct 7.1., construct 7.3, construct 7.5 and construct 7.6 and control M bound to PD1-expressing CHO-k1-huPD1 clone 5 whereas the not PD 1 targeted control molecules control B, control C, control D and control F did not bind to the tumor cells. In **Figures 16A and 16B,** only the split trimeric 4-1BB ligand containing fusion molecules construct 7.1., construct 7.3, construct 7.5 and construct 7.6 and control B, control C and control D bound to human 4-1BB-expressing HeLa-hu4-1BB-NFkB-luc clone 26 whereas the controls containing no fused split trimeric 4-1BB ligand (control F and control M) did not bind to the human 4-1BB expressing tumor cells. In Table 28 the $EC_{50}$ values of the curves are listed.

**Table 28: $EC_{50}$ values of binding curves to human PD1 or human 4-1BB expressing tumor cells**

| | Control C | Control B | Control D | Control M | Construct 7.1 | Construct 7.3 | Construct 7.5 | Construct 7.6 |
|---|---|---|---|---|---|---|---|---|
| $EC_{50}$ [nM] on HeLa-huCD137-NFkB-luc clone 26 cells | 0.16 | 0.19 | 0.25 | n.d. | 0.14 | 0.28 | 0.36 | 0.32 |
| $EC_{50}$ [nM] on CHO-K1-huPD1 clone 5 cells | n.d. | n.d. | n.d. | 1.59 | 1.73 | 1.43 | 3.33 | 1.82 |

**Example 6**

**Biological activity of the PD1 targeted 4-1BB ligand trimer-containing Fc fusion antigen binding molecules**

**NF-κB activation of human 4-1BB expressing HeLa reporter cell line**

**6.1 Generation of HeLa cells expressing human 4-1BB and NF-κB-luciferase**

The human-papilloma-virus-18-induced cervix carcinoma cell line HeLa (ATCC CCL-2) was transduced with a plasmid based on the expression vector pETR10829, which contains the sequence of human 4-1BB (Uniprot accession Q07011) under control of a CMV-promoter and a puromycin resistance gene. Cells were cultured in DMEM-medium (Gibco by Life Technologies Cat. No. 42430-025) supplied with 10% Fetal Bovine Serum (FBS, GIBCO by Life Technologies, Cat. No. 16000-044, Lot. No. 941273, gamma-irradiated mycoplasma free, heat inactivated at 56 °C for 35 minutes), 1 % GlutaMAX-1 (GIBCO by Life Technologies, Cat.-No. 35050-038) and 3 μg/mL Puromycin (InvivoGen, Cat No. ant-pr-1).

4-1BB-transduced HeLa cells were tested for 4-1BB expression by flow cytometry: 0.2 x $10^6$ living cells were resuspended in 100 μL FACS buffer (DPBS supplied with 2 % FBS, 5 mM EDTA pH 8 (Amresco, Cat. No. E177) and 7.5 mM Sodium Azide (Sigma-Aldrich, Cat. No. S2002)) containing 0.1 μg PerCP/Cy5.5 conjugated anti-human 4-1BB mouse IgG1κ clone 4B4-1 (BioLegend Cat. No. 309814) or its isotype control (PerCP/Cy5.5 conjugated mouse IgG1κ isotype control antibody clone MOPC 21, BioLegend Cat. No. 400150) and incubated for 30 minutes at 4 °C. Cells were washed twice with FACS buffer, resuspended in 300 μL FACS buffer containing 0.06 μg DAPI (Santa Cruz Biotec, Cat. No. Sc-3598) and acquired using a 5-laser LSR-Fortessa (BD Bioscience, DIVA software). Limited dilutions were performed to generate single clones as following: Human-4-1BB transduced HeLa cells were resuspended in medium to a concentration of 10, 5 and 2.5 cells/mL and 200 μL of cell suspensions were transferred to round bottom tissue-culture treated 96-well plates (6 plates/cell concentration, TPP Cat. No. 92697). Single clones were harvested, expanded and tested for 4-1BB expression as described above. The clone with the highest expression of 4-1BB (clone 5) was chosen for subsequent transfection with the NFκB-luciferase expression-vector 5495p Tranlucent HygB. The vector confers transfected cells both with resistance to hygromycin B and capacity to express luciferase under control of NFkB-response element (Panomics, Cat. No. LR0051). Human-4-1BB HeLa clone 5 cells were cultured to 70% confluence. 50 μg (40 μL) linearized (restriction enzymes AseI and SalI) 5495p Tranlucent HygB expression vector were added to a sterile 0.4 cm Gene Pulser/MicroPulser Cuvette (Biorad, Cat.-No, 165-2081). 2.5 x $10^6$ human-4-1BB HeLa clone 5 cells in 400 μl supplement-free DMEM were added and mixed carefully with the plasmid solution. Transfection of cells was performed using a Gene Pulser Xcell total system (Biorad, Cat No. 165 2660) under the following settings: exponential pulse, capacitance 500 μF, voltage 160 V, resistance ∞. Immediately after the pulse transfected cells were transferred to a tissue culture flask 75 $cm^2$ (TPP, Cat. No. 90075) with 15 mL 37 °C warm DMEM-Medium (Gibco by Life Technologies Cat. No. 42430-025) supplied with 10 % FBS and 1 % GlutaMAX I (GIBCO by Life Technologies, Cat. No. 35050 038). Next day, culture medium containing 3 μg/mL Puromycin (InvivoGen, Cat No. ant-pr-1) and 200 μg/mL Hygromycin B (Roche, Cat. No. 10843555001) was added. Surviving cells were expanded and limited dilution was performed as described above to generate single clones. Clones were tested for 4-1BB expression as described above and for NFκB-Luciferase activity as following: Clones were harvested in selection medium and counted using a Cell Counter Vi-cell xr 2.03 (Beckman Coulter, Cat. No. 731050). Cells were set to a cell density of 0.33 x $10^6$ cells/mL and 150 μL of this cell suspension were transferred to each well of a sterile white 96-well flat bottom tissue culture plate with lid (greiner bio one, Cat. No. 655083) and as a control to normal 96 well flat bottom tissue culture plate (TPP Cat. No. 92096) to test survival and cell density the next day. Cells were incubated at 37°C and 5 % $CO_2$ overnight. The next day 50 μL of medium containing different concentrations of recombinant human tumor necrosis factor alpha (rhTNF α, PeproTech, Cat.-No. 300 01A) were added to each well of a 96-well plate resulting in final concentration of rhTNF α of 100, 50, 25, 12.5, 6.25 and 0 ng/well. Cells were incubated for 6 hours at 37 °C and 5 % $CO_2$ and then washed three times with 200 μL/well DPBS. Reporter Lysis Buffer (Promega, Cat-No: E3971) was added to each well (30 μl) and the plates were stored over night at -20 °C. The next day frozen cell plates and Detection Buffer (Luciferase 1000 Assay System, Promega, Cat. No. E4550) were thawed to room temperature. 100 uL of detection buffer were added to each well and the plate was measured as fast as possible using a SpectraMax M5/M5e microplate reader and the SoftMax Pro Software (Molecular Devices). Measured URLs above control (no rhTNF α added) were taken as luciferase activity. The NFκB-luc-4-1BB-HeLa clone 26 was chosen for further use exhibiting the highest luciferase activity and a considerable level of 4-1BB-expression.

**6.2 NF-κB activation of HeLa reporter cells expressing human 4-1BB co-cultured with PD1-expressing CHO-k1-huPD1 clone 5 cells**

NFκB-luc-4-1BB-HeLa clone 26 cells were washed with DPBS (Gibco by Life Technologies, Cat. No. 14190 326) and treated with 0.05 % Trypsin EDTA (Gibco by Life Technologies Cat. No. 25300 054) for 5 min at 37 °C. Cells were harvested and resuspended in DMEM medium (Gibco by Life Technologies Cat. No. 42430 025) supplied with 10 % Fetal Calf Serum (FBS, US-origin, PAN biotech, P30-2009, Lot P150307GI, gamma irradiated mycoplasma free, heat inactivated 35 min 56 °C) and 1 % GlutaMAX-1 (GIBCO by Life Technologies, Cat. No. 35050 038). Cells were counted using Cell Counter Vi-cell xr 2.03 (Beckman Coulter, Cat. No. 731050) and set to a cell density of 0.2 x $10^6$ cells/mL. 100 μL (2 x $10^4$ cells) of this cell suspension were transferred to each well of a sterile white 96-well flat bottom tissue culture plate with lid (greiner bio one, Cat. No. 655083) and the plates were incubated at 37 °C and 5 % $CO_2$ overnight. The next day 50 μL of medium containing different titrated split trimeric 4-1BB ligand containing fusion molecules construct 7.1., construct 7.3, construct 7.5 and construct 7.6 or the fitting controls control B, control C, control D, control F and control M.For PD1-binding-mediated crosslinking the PD1-expressing CHO-k1-huPD1 clone 5 cells were used.

Adherent CHO-k1-huPD1 clone 5 cells were washed with DPBS (Gibco by Life Technologies, Cat. No. 14190 326) and treated with enzyme-free, PBS-based Cell Dissociation Buffer (Gibco by Life Technologies, Cat.-No. 13151-014) for 10 minutes at 37 °C. Afterwards cell were harvested and counted using Cell Counter Vi-cell xr 2.03. Cells were resuspended in DEM supplied with 10% FBS and 1% L-GlutaMAX-I to 2 x $10^6$ cells/mL and 50 μL were added/well. After adding crosslinking PD1-expressing CHO-k1-huPD1 clone 5 cells, plates were incubated for 6 hours at 37 °C and 5 % $CO_2$. White flat bottom 96-well plates were washed three times with 200 μL/well DPBS. 40 μl fresh prepared Reporter Lysis Buffer (Promega, Cat-No: E3971) were added to each well and the plate were stored over night at -20 °C. The next day frozen plates and detection buffer (Luciferase 1000 Assay System, Promega, Cat. No. E4550) were thawed to room temperature. 100 uL of detection buffer were added to each well and plates were measured as fast as possible using the SpectraMax M5/M5e microplate reader and SoftMax Pro Software (Molecular Devices) with following settings: for luciferase (RLUs), 500ms integration time, no filter, collecting all wave length and top reading.

PD1-targeted 4-1BB ligand trimer-containing Fc fusion antigen binding molecule (PD1 split 4-1BBL trimer) triggered activation of the NFκB signaling pathway in the reporter cell line in the presence of PD1-expressing tumor cells. In contrast, the untargeted variant of the same molecule failed to trigger such an effect at any of the tested concentrations **(Figures 17C and 17D).** This activity of targeted 4-1BBL was strictly dependent on the expression of PD1 at the cell surface of tumor cells as no NF-kB activation could be detected upon culturing of the NF-kB reporter cell line with PD1-negative tumor cells even in the presence of PD1-targeted 4-1BB ligand trimer-containing Fc fusion antigen binding molecule **(Figures 17A and 17B).** The activities as measured for Constructs 7.1, 7.3, 7.5 and 7.6 are shown. The $EC_{50}$ values as measured in the presence of PD1 expressing tumor cells are given in Table 29 below.

**Table 29: $EC_{50}$ values of NFκB-activation-induced Luciferase activity-curves**

| | Constuct 7.1 | Construct 7.3 | Construct 7.5 | Construct 7.6 |
|---|---|---|---|---|
| $EC_{50}$ [nM] with CHO-k1-huPD1 clone 5 | 0.004 | 0.004 | 0.006 | 0.007 |

**Citations:**

Ascierto, P. A., E. Simeone, M. Sznol, Y. X. Fu, and I. Melero (2010), Clinical experiences with anti-CD137 and anti-PD1 therapeutic antibodies. Semin Oncol 37:508-516.

Aggarwal B.B. (2003), Signalling pathways of the TNF superfamily: a double-edged sword. Nat. Rev. Immunol. 3(9),745-56.

Banner D. et al (1993), Crystal structure of the soluble human 55 kd TNF receptor-human TNF beta complex: implications for TNF receptor activation. Cell 73, 431-445.

Bodmer J., Schneider P. and Tschopp, J. (2002), The molecular architecture of the TNF superfamily. Trends in Biochemical Sciences 27(1), 19-26.

Broll, K., Richter, G., Pauly, S., Hofstaedter, F., and Schwarz, H. (2001). CD137 expression in tumor vessel walls. High correlation with malignant tumors. Am J Clin Pathol 115, 543-549.

Buechele, C., Baessler, T., Schmiedel, B.J., Schumacher, C.E., Grosse-Hovest, L., Rittig, K., and Salih, H.R. (2012). 4-1BB ligand modulates direct and Rituximab-induced NK-cell reactivity in chronic lymphocytic leukemia. Eur J Immunol 42, 737-748.

Chen S., Lee L., Fisher T., Jessen B., Elliott M., Evering W., Logronio K., Tu K.H., Tsaparikos K., Li X., Wang H., Ying C., Xiong M.., Van Arsdale T., and. Lin J.C. (2015), Combination of 4-1BB Agonist and PD-1 Antagonist Promotes Antitumor Effector/Memory CD8 T Cells in a Poorly Immunogenic Tumor Model. Cancer Immunology Research 3(2), 149-160.

Choi, B.K., Kim, Y.H., Kwon, P.M., Lee, S.C., Kang, S.W., Kim, M.S., Lee, M.J., and Kwon, B.S. (2009). 4-1BB functions as a survival factor in dendritic cells. J Immunol 182, 4107-4115.

Cuadros, C., Dominguez, A.L., Lollini, P.L., Croft, M., Mittler, R.S., Borgstrom, P., and Lustgarten, J. (2005). Vaccination with dendritic cells pulsed with apoptotic tumors in combination with anti-OX40 and anti-4-1BB monoclonal antibodies induces T cell-mediated protective immunity in Her-2/neu transgenic mice. Int J Cancer 116, 934-943.

Curran, M.A., Kim, M., Montalvo, W., Al-Shamkhani, A., and Allison, J.P. (2011). Combination CTLA-4 blockade and 4-1BB activation enhances tumor rejection by increasing T-cell infiltration, proliferation, and cytokine production. PLoS One 6, e19499.

Diehl, L., van Mierlo, G.J., den Boer, A.T., van der Voort, E., Fransen, M., van Bostelen, L., Krimpenfort, P., Melief, C.J., Mittler, R., Toes, R.E., and Offringa, R. (2002). In vivo triggering through 4-1BB enables Th-independent priming of CTL in the presence of an intact CD28 costimulatory pathway. J Immunol 168, 3755-3762.

Dubrot, J., Milheiro, F., Alfaro, C., Palazon, A., Martinez-Forero, I., Perez-Gracia, J.L., Morales-Kastresana, A., Romero-Trevejo, J.L., Ochoa, M.C., Hervas-Stubbs, S., et al. (2010). Treatment with anti-CD 137 mAbs causes intense accumulations of liver T cells without selective antitumor immunotherapeutic effects in this organ. Cancer Immunol Immunother 59, 1223-1233.

Futagawa, T., Akiba, H., Kodama, T., Takeda, K., Hosoda, Y., Yagita, H., and Okumura, K. (2002). Expression and function of 4-1BB and 4-1BB ligand on murine dendritic cells. Int Immunol 14, 275-286.

Guo, Z., Cheng, D., Xia, Z., Luan, M., Wu, L., Wang, G., and Zhang, S. (2013). Combined TIM-3 blockade and CD 137 activation affords the long-term protection in a murine model of ovarian cancer. J Transl Med 11, 215.

Heinisch, I.V., Daigle, I., Knopfli, B., and Simon, H.U. (2000). CD137 activation abrogates granulocyte-macrophage colony-stimulating factor-mediated anti-apoptosis in neutrophils. Eur J Immunol 30, 3441-3446.

Hornig, N., Kermer, V., Frey, K., Diebolder, P., Kontermann, R.E., Mueller, D. (2012), Combination of a bispecific antibody and costimulatory antibody-ligand fusion proteins for targeted cancer immunotherapy. J. Immunother. 35, 418-429.

Ju, S.A., Cheon, S.H., Park, S.M., Tam, N.Q., Kim, Y.M., An, W.G., and Kim, B.S. (2008). Eradication of established renal cell carcinoma by a combination of 5-fluorouracil and anti-4-1BB monoclonal antibody in mice. Int J Cancer 122, 2784-2790.

Kienzle, G., and von Kempis, J. (2000). CD137 (ILA/4-1BB), expressed by primary human monocytes, induces monocyte activation and apoptosis of B lymphocytes. Int Immunol 12, 73-82.

Kim, D.H., Chang, W.S., Lee, Y.S., Lee, K.A., Kim, Y.K., Kwon, B.S., and Kang, C.Y. (2008). 4-1BB engagement costimulates NKT cell activation and exacerbates NKT cell ligand-induced airway hyperresponsiveness and inflammation. J Immunol 180, 2062-2068.

Kim, Y.H., Choi, B.K., Oh, H.S., Kang, W.J., Mittler, R.S., and Kwon, B.S. (2009). Mechanisms involved in synergistic anticancer effects of anti-4-1BB and cyclophosphamide therapy. Mol Cancer Ther 8, 469-478.

Kwon, B.S., and Weissman, S.M. (1989). cDNA sequences of two inducible T-cell genes. Proc Natl Acad Sci U S A 86, 1963-1967.

Lee, H., Park, H.J., Sohn, H.J., Kim, J.M., and Kim, S.J. (2011). Combinatorial therapy for liver metastatic colon cancer: dendritic cell vaccine and low-dose agonistic anti-4-1BB antibody costimulatory signal. J Surg Res 169, e43-50.

Levitsky, V., de Campos-Lima, P.O., Frisan, T., and Masucci, M.G. (1998). The clonal composition of a peptide-specific oligoclonal CTL repertoire selected in response to persistent EBV infection is stable over time. J Immunol 161, 594-601.

Li, F., and Ravetch, J.V. (2011). Inhibitory Fcgamma receptor engagement drives adjuvant and anti-tumor activities of agonistic CD40 antibodies. Science 333, 1030-1034.

Lin, W., Voskens, C.J., Zhang, X., Schindler, D.G., Wood, A., Burch, E., Wei, Y., Chen, L., Tian, G., Tamada, K., et al. (2008). Fc-dependent expression of CD137 on human NK cells: insights into "agonistic" effects of anti-CD 137 monoclonal antibodies. Blood 112, 699-707.

Melero, I., Johnston, J.V., Shufford, W.W., Mittler, R.S., and Chen, L. (1998). NK1.1 cells express 4-1BB (CDw137) costimulatory molecule and are required for tumor immunity elicited by anti-4-1BB monoclonal antibodies. Cell Immunol 190, 167-172.

Melero, I., Shuford, W.W., Newby, S.A., Aruffo, A., Ledbetter, J.A., Hellstrom, K.E., Mittler, R.S., and Chen, L. (1997). Monoclonal antibodies against the 4-1BB T-cell activation molecule eradicate established tumors. Nat Med 3, 682-685.

Merchant, A.M., Zhu, Z., Yuan, J.Q., Goddard, A., Adams, C.W., Presta, L.G., and Carter, P. (1998). An efficient route to human bispecific IgG. Nat Biotechnol 16, 677-681.

Morales-Kastresana, A., Sanmamed, M.F., Rodriguez, I., Palazon, A., Martinez-Forero, I., Labiano, S., Hervas-Stubbs, S., Sangro, B., Ochoa, C., Rouzaut, A., et al. (2013). Combined immunostimulatory monoclonal antibodies extend survival in an aggressive transgenic hepatocellular carcinoma mouse model. Clin Cancer Res 19, 6151-6162.

Mueller, D., Frey, K., Kontermann, R.E. (2008), A novel antibody-4-1BB1 fusion protein for targeted costimulation in cancer immunotherapy, J. Immunother. 31, 714-722.

Murillo, O., Dubrot, J., Palazon, A., Arina, A., Azpilikueta, A., Alfaro, C., Solano, S., Ochoa, M.C., Berasain, C., Gabari, I., et al. (2009). In vivo depletion of DC impairs the anti-tumor effect of agonistic anti-CD137 mAb. Eur J Immunol 39, 2424-2436.

Narazaki, H., Zhu, Y., Luo, L., Zhu, G., and Chen, L. (2010). CD137 agonist antibody prevents cancer recurrence: contribution of CD 137 on both hematopoietic and nonhematopoietic cells. Blood 115, 1941-1948.

Nishimoto, H., Lee, S.W., Hong, H., Potter, K.G., Maeda-Yamamoto, M., Kinoshita, T., Kawakami, Y., Mittler, R.S., Kwon, B.S., Ware, C.F., et al. (2005). Costimulation of mast cells by 4-1BB, a member of the tumor necrosis factor receptor superfamily, with the high-affinity IgE receptor. Blood 106, 4241-4248.

Olofsson, P.S., Soderstrom, L.A., Wagsater, D., Sheikine, Y., Ocaya, P., Lang, F., Rabu, C., Chen, L., Rudling, M., Aukrust, P., et al. (2008). CD137 is expressed in human atherosclerosis and promotes development of plaque inflammation in hypercholesterolemic mice. Circulation 117, 1292-1301.

Palazon, A., Teijeira, A., Martinez-Forero, I., Hervas-Stubbs, S., Roncal, C., Penuelas, I., Dubrot, J., Morales-Kastresana, A., Perez-Gracia, J.L., Ochoa, M.C., et al. (2011). Agonist anti-CD137 mAb act on tumor endothelial cells to enhance recruitment of activated T lymphocytes. Cancer Res 71, 801-811.

Schwarz, H., Valbracht, J., Tuckwell, J., von Kempis, J., and Lotz, M. (1995). ILA, the human 4-1BB homologue, is inducible in lymphoid and other cell lineages. Blood 85, 1043-1052.

Shao, Z., and Schwarz, H. (2011). CD137 ligand, a member of the tumor necrosis factor family, regulates immune responses via reverse signal transduction. J Leukoc Biol 89, 21-29.

Shi, W., and Siemann, D.W. (2006). Augmented antitumor effects of radiation therapy by 4-1BB antibody (BMS-469492) treatment. Anticancer Res 26, 3445-3453.

Simeone, E., and Ascierto, P.A. (2012). Immunomodulating antibodies in the treatment of metastatic melanoma: the experience with anti-CTLA-4, anti-CD137, and anti-PD1. J Immunotoxicol 9, 241-247.

Snell, L.M., Lin, G.H., McPherson, A.J., Moraes, T.J., and Watts, T.H. (2011). T-cell intrinsic effects of GITR and 4-1BB during viral infection and cancer immunotherapy. Immunol Rev 244, 197-217.

Stagg, J., Loi, S., Divisekera, U., Ngiow, S.F., Duret, H., Yagita, H., Teng, M.W., and Smyth, M.J. (2011). Anti-ErbB-2 mAb therapy requires type I and II interferons and synergizes with anti-PD-1 or anti-CD137 mAb therapy. Proc Natl Acad Sci U S A 108, 7142-7147.

Teng, M.W., Sharkey, J., McLaughlin, N.M., Exley, M.A., and Smyth, M.J. (2009). CD1d-based combination therapy eradicates established tumors in mice. J Immunol 183, 1911-1920.

von Kempis, J., Schwarz, H., and Lotz, M. (1997). Differentiation-dependent and stimulus-specific expression of ILA, the human 4-1BB-homologue, in cells of mesenchymal origin. Osteoarthritis Cartilage 5, 394-406.

Wei, H., Zhao, L., Li, W., Fan, K., Qian, W., Hou, S., Wang, H., Dai, M., Hellstrom, I., Hellstrom, K.E., and Guo, Y. (2013). Combinatorial PD-1 blockade and CD 137 activation has therapeutic efficacy in murine cancer models and synergizes with cisplatin. PLoS One 8, e84927.

Wei H., Zhao L., Hellstrom I., Hellstrom K.E. and Guo Y. (2014). Dual targeting of CD137 costimulatory and PD-1 co-inhibitory molecules for ovarian cancer immunotherapy, OncoImmunology, 3:4, e28248, DOI: 10.4161/onci.28248.

Wilcox, R.A., Chapoval, A.I., Gorski, K.S., Otsuji, M., Shin, T., Flies, D.B., Tamada, K., Mittler, R.S., Tsuchiya, H., Pardoll, D.M., and Chen, L. (2002). Cutting edge: Expression of functional CD137 receptor by dendritic cells. J Immunol 168, 4262-4267.

Wilcox, R.A., Tamada, K., Flies, D.B., Zhu, G., Chapoval, A.I., Blazar, B.R., Kast, W.M., and Chen, L. (2004). Ligation of CD137 receptor prevents and reverses established anergy of CD8+ cytolytic T lymphocytes in vivo. Blood 103, 177-184.

Zhang, N., Sadun, R.E., Arias, R.S., Flanagan, M.L., Sachsman, S.M., Nien, Y, Khawli, L.A., Hu, P., Epstein, A.L. (2007). Targeted and untargeted CD137L fusion proteins for the immunotherapy of experimental solid tumors. Clin. Cancer Res. 13, 2758-2767.

Zhang, X., Voskens, C.J., Sallin, M., Maniar, A., Montes, C.L., Zhang, Y., Lin, W., Li, G., Burch, E., Tan, M., et al. (2010). CD137 promotes proliferation and survival of human B cells. J Immunol 184, 787-795.

## Claims

1. A TNF family ligand trimer-containing antigen binding molecule comprising

   (a) at least one moiety capable of specific binding to PD1 and
   (b) a first and a second polypeptide that are linked to each other by a disulfide bond,

   wherein the antigen binding molecule is **characterized in that** the first polypeptide comprises two ectodomains of a TNF ligand family member or a fragment thereof that are connected to each other by a peptide linker and **in that** the second polypeptide comprises only one ectodomain of said TNF ligand family member or a fragment thereof.

2. The TNF family ligand trimer-containing antigen binding molecule of claim 1, further comprising

   (c) an Fc domain composed of a first and a second subunit capable of stable association.

3. The TNF family ligand trimer-containing antigen binding molecule of claims 1 or 2, comprising

   (a) at least one moiety capable of specific binding to PD1 and
   (b) a first and a second polypeptide that are linked to each other by a disulfide bond,

   wherein the antigen binding molecule is **characterized in that**

   (i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to the CH1 or CL domain by a peptide linker and wherein the second polypeptide comprises one ectodomain of said TNF ligand family member or a fragment thereof connected via a peptide linker to the CL or CH1 domain of said polypeptide, or
(ii) the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises only one ectodomain of said TNF ligand family member or a fragment thereof connected via a peptide linker to the C-terminus of the CH3 domain of said polypeptide, or
(iii) the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of a TNF ligand family member or fragments thereof that are connected to each other and to to VH or VL by a peptide linker and wherein the second polypeptide comprises one ectodomain of said TNF ligand family member or a fragment thereof connected via a peptide linker to VL or VH of said polypeptide.

**4.** The TNF family ligand trimer-containing antigen binding molecule of any one of claims 1 to 3, wherein the TNF ligand family member is selected from 4-1BBL and OX40L.

**5.** The TNF family ligand trimer-containing antigen binding molecule of any one of claims 1 to 4, wherein the TNF ligand family member is 4-1BBL.

**6.** The TNF family ligand trimer-containing antigen binding molecule of any one of claims 1 to 5, wherein the ectodomain of a TNF ligand family member comprises the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO:5.

**7.** The TNF family ligand trimer-containing antigen binding molecule of any one of claims 1 to 6, comprising

(a) at least one moiety capable of specific binding to PD1, and
(b) a first polypeptide containing a CH1 or CL domain and a second polypeptide containing a CL or CH1 domain, respectively, wherein the second polypeptide is linked to the first polypeptide by a disulfide bond between the CH1 and CL domain,

and wherein the antigen binding molecule is **characterized in that** the first polypeptide comprises two ectodomains of a TNF ligand family member or fragments thereof that are connected to each other and to the CH1 or CL domain by a peptide linker and **in that** the second polypeptide comprises only one ectodomain of said TNF ligand family member or a fragment thereof connected via a peptide linker to the CL or CH1 domain of said polypeptide.

**8.** The TNF family ligand trimer-containing antigen binding molecule of any one of claims 1 to 7, wherein the moiety capable of specific binding to PD1 is a Fab molecule capable of specific binding to PD1.

**9.** The TNF family ligand trimer-containing antigen binding molecule of any one of claims 1 to 8, wherein the moiety capable of specific binding to PD1 comprises

(a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO: 15, and a VL domain comprising (iv) HVR-L1 comprising the amino acid sequence of SEQ ID NO:16 (v) HVR-L2 comprising the amino acid sequence of SEQ ID NO: 17, and (vi) HVR-L3 comprising the amino acid sequence of SEQ ID NO:18,
(b) a VH domain comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28, and a VL domain comprising (iv) HVR-L1 comprising the amino acid sequence of SEQ ID NO:29 (v) HVR-L2 comprising the amino acid sequence of SEQ ID NO:30, and (vi) HVR-L3 comprising the amino acid sequence of SEQ ID NO:31,
(c) a VH domain comprising (i) HVR-H1 comprising the amino acid sequence of SEQ ID NO:34, (ii) HVR-H2 comprising the amino acid sequence of SEQ ID NO:35, and (iii) HVR-H3 comprising the amino acid sequence of SEQ ID NO:36, and a VL domain comprising (iv) HVR-L1 comprising the amino acid sequence of SEQ ID NO:37 (v) HVR-L2 comprising the amino acid sequence of SEQ ID NO:38, and (vi) HVR-L3 comprising the amino acid sequence of SEQ ID NO:39,
(d) a VH domain comprising (i) HVR-H1 comprising the amino acid sequence of SEQ ID NO:42, (ii) HVR-H2 comprising the amino acid sequence of SEQ ID NO:43, and (iii) HVR-H3 comprising the amino acid sequence of SEQ ID NO:44, and a VL domain comprising (iv) HVR-L1 comprising the amino acid sequence of SEQ ID NO:45 (v) HVR-L2 comprising the amino acid sequence of SEQ ID NO:46, and (vi) HVR-L3 comprising the amino acid sequence of SEQ ID NO:47,
(e) a VH domain comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52, and a VL domain comprising (iv) HVR-L1 comprising the amino acid sequence of SEQ ID NO:53 (v) HVR-L2 comprising the amino acid sequence of SEQ ID NO:54, and (vi) HVR-L3 comprising the amino acid sequence of SEQ ID NO:55,
(f) a VH domain comprising (i) HVR-H1 comprising the amino acid sequence of SEQ ID NO:58, (ii) HVR-H2 comprising the amino acid sequence of SEQ ID NO:59, and (iii) HVR-H3 comprising the amino acid sequence of SEQ ID NO:60, and a VL domain comprising (iv) HVR-L1 comprising the amino acid sequence of SEQ ID NO:61 (v) HVR-L2 comprising the amino acid sequence of SEQ ID NO:62, and (vi) HVR-L3 comprising the amino acid sequence of SEQ ID NO:63, or
(g) a VH domain comprising (i) HVR-H1 comprising the amino acid sequence of SEQ ID NO:66, (ii) HVR-H2 comprising the amino acid sequence of SEQ ID NO:67, and (iii) HVR-H3 comprising the amino acid sequence of SEQ ID NO:68, and a VL domain comprising (iv) HVR-L1 comprising the amino acid sequence of SEQ ID NO:69 (v) HVR-L2 comprising the amino acid sequence of SEQ ID NO:70, and (vi) HVR-L3 comprising the amino acid sequence of SEQ ID NO:71.

**10.** The TNF family ligand trimer-containing antigen binding molecule of any one of claims 1 to 9, wherein the moiety capable of specific binding to PD1 comprises

(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

**11.** The TNF family ligand trimer-containing antigen binding molecule of any one of claims 1 to 10, wherein the antigen binding molecule comprises
a first heavy chain and a first light chain, both comprising a Fab molecule capable of specific binding to PD1,
a first peptide comprising two ectodomains of a TNF ligand family member or fragments thereof connected to each other by a first peptide linker fused at its C-terminus by a second peptide linker to a second heavy or light chain,
and a second peptide comprising one ectodomain of said TNF ligand family member fused at its C-terminus by a third peptide linker to a second light or heavy chain, respectively.

**12.** The TNF family ligand trimer-containing antigen binding molecule of any one of claims 1 to 10, wherein the first peptide comprising two ectodomains of a TNF ligand family member or fragments thereof connected to each other by a first peptide linker is fused at its C-terminus by a second peptide linker to a CL domain that is part of a heavy chain, and the second peptide comprising one ectodomain of said TNF ligand family member or a fragment thereof is fused at its C-terminus by a third peptide linker to a CH1 domain that is part of a light chain.

**13.** The TNF family ligand trimer-containing antigen binding molecule of any one of claims 1 to 10, comprising

(a) at least one Fab domain capable of specific binding to PD1, and
(b) a first and a second polypeptide that are linked to each other by a disulfide bond,

wherein the antigen binding molecule is **characterized in that** the first polypeptide contains a CH3 domain and the second polypeptide contains a CH3 domain, respectively, and wherein the first polypeptide comprises two ectodomains of a TNF ligand family member or fragments thereof that are connected to each other and to the C-terminus of the CH3 domain by a peptide linker and wherein the second polypeptide comprises one ectodomain of said TNF ligand family member or a fragment thereof connected via a peptide linker to C-terminus of the CH3 domain of said polypeptide.

**14.** The TNF family ligand trimer-containing antigen binding molecule of any one of claims 1 to 10, wherein the antigen binding molecule comprises

(i) a fusion polypeptide comprising a first subunit of a Fc domain and two ectodomains of a TNF ligand family member or fragments thereof that are connected to each other and to the C-terminus of the CH3 domain by a peptide linker, (ii) a heavy chain comprising the VH domain of the Fab domain capable of specific binding to PD1, a second subunit of the Fc domain and one ectodomain of said TNF ligand family member or a fragment thereof connected via a peptide linker to C-terminus of the CH3 domain, and (iii) a light chain comprising the VL domain of the Fab domain capable of specific binding to PD1, or
(i) a fusion polypeptide comprising a first subunit of a Fc domain and one ectodomain of a TNF ligand family member or a fragment thereof that is connected to the C-terminus of the CH3 domain by a peptide linker, (ii) a heavy chain comprising the VH domain of the Fab domain capable of specific binding to PD1, a second subunit of the Fc domain and two ectodomains of said TNF ligand family member or fragments thereof that are connected to each other and to the C-terminus of the CH3 domain via a peptide linker, and (iii) a light chain comprising the VL domain of the Fab domain capable of specific binding to PD1.

**15.** The TNF family ligand trimer-containing antigen binding molecule of any one of claims 1 to 14 for use in the treatment of cancer.

Fig. 1

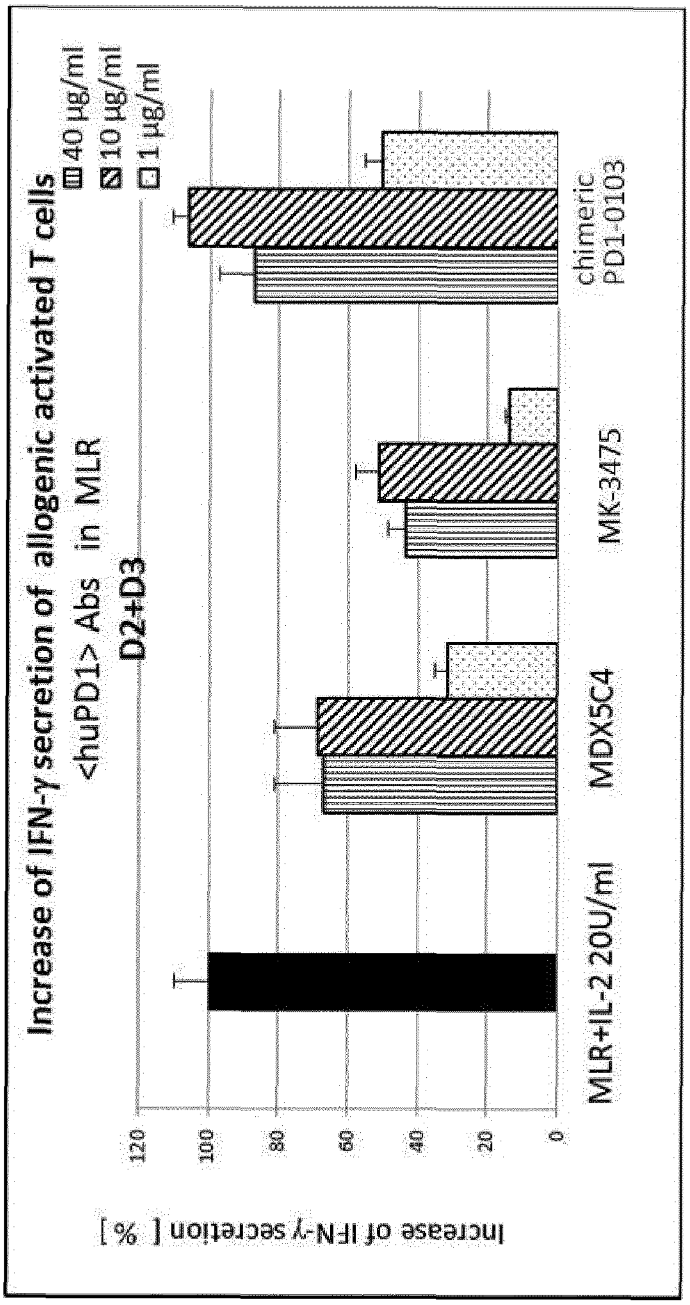
Increase of IFN-γ secretion of allogenic activated T cells
<huPD1> Abs in MLR
D2+D3
40 µg/ml
10 µg/ml
1 µg/ml
Increase of IFN-γ secretion [ %]
120
100
80
60
40
20
0
MLR+IL-2 20U/ml
MDX5C4
MK-3475
chimeric PD1-0103

Fig. 2

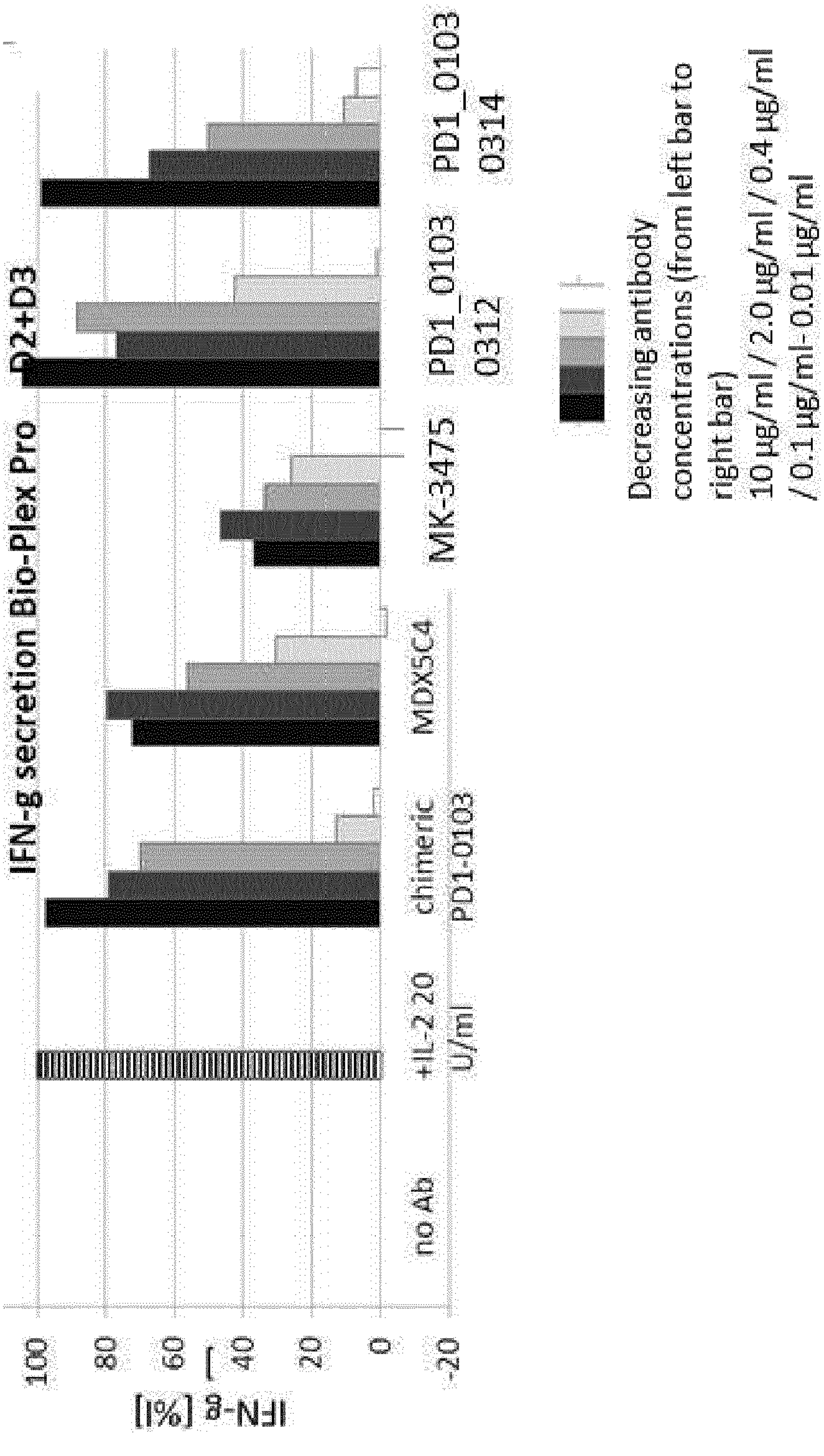
IFN-g secretion Bio-Plex Pro
D2+D3
IFN-g [%]
100
80
60
40
20
0
-20
no Ab
+IL-2 20 U/ml
chimeric PD1-0103
MDX5C4
MK-3475
PD1_0103 0312
PD1_0103 0314
Decreasing antibody concentrations (from left bar to right bar)
10 µg/ml / 2.0 µg/ml / 0.4 µg/ml / 0.1 µg/ml- 0.01 µg/ml

Fig. 3

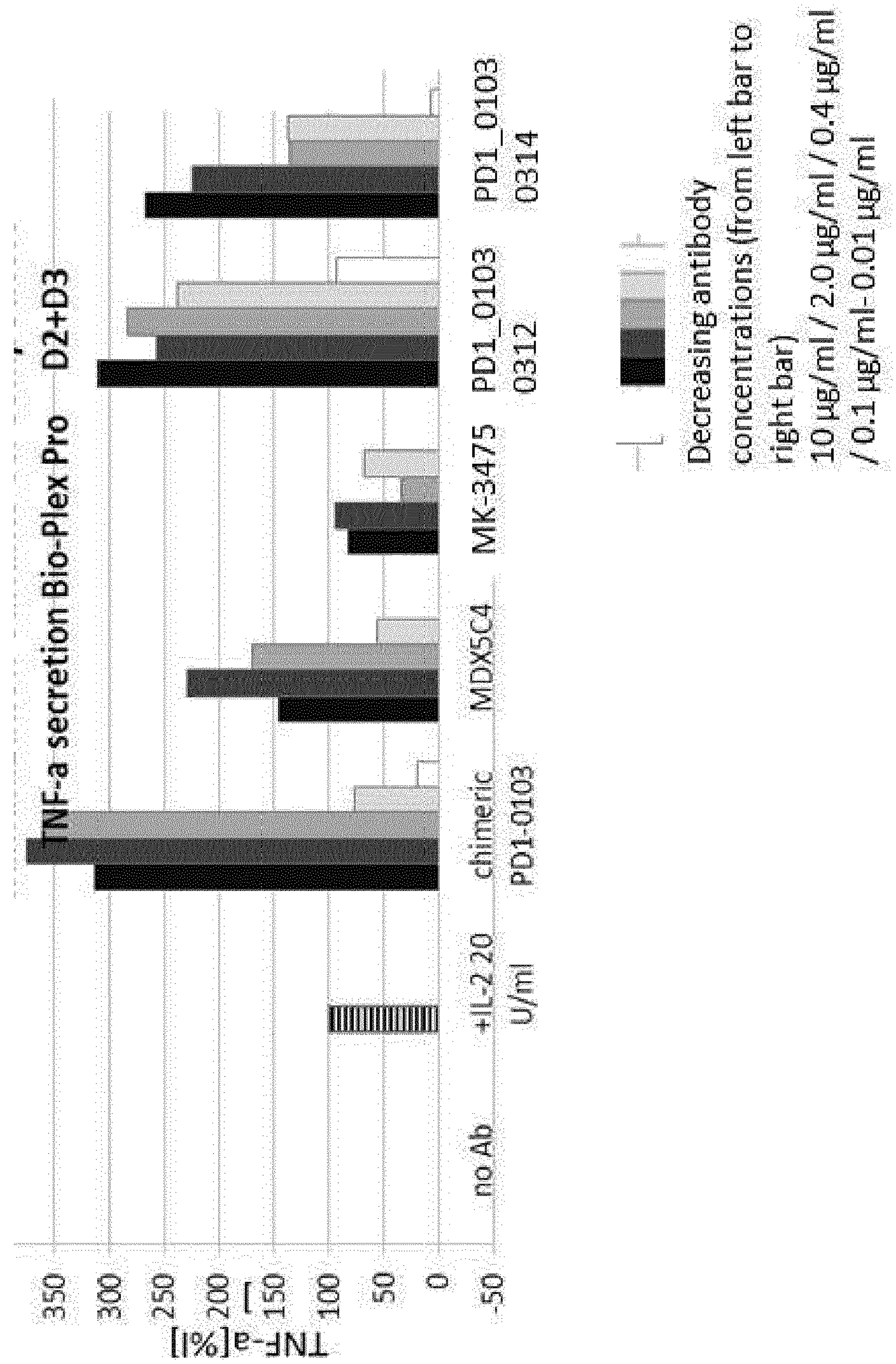
TNF-a secretion Bio-Plex Pro D2+D3
TNF-a[%l]
350
300
250
200
150
100
50
0
-50
no Ab
+IL-2 20 U/ml
chimeric PD1-0103
MDX5C4
MK-3475
PD1_0103_0312
PD1_0103_0314
Decreasing antibody concentrations (from left bar to right bar)
10 µg/ml / 2.0 µg/ml / 0.4 µg/ml / 0.1 µg/ml- 0.01 µg/ml

Fig. 4A

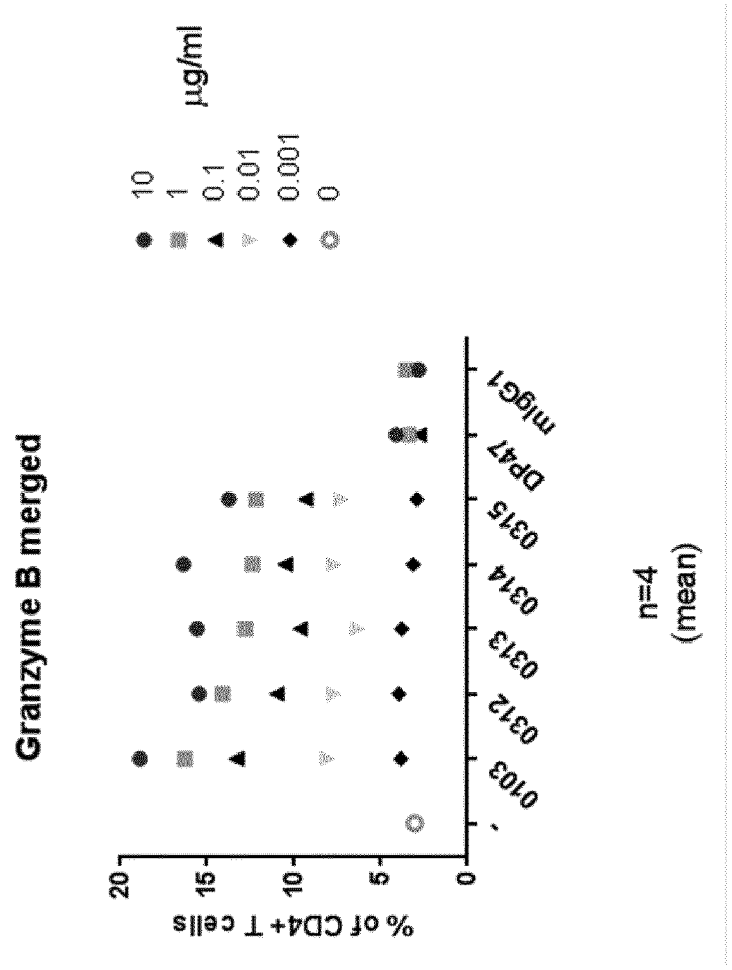
Granzyme B merged
% of CD4+ T cells
20
15
10
5
0
-
0103
0312
0313
0314
0315
DP47
mIgG1
10
1
0.1
0.01
0.001
0
µg/ml
n=4
(mean)

Fig. 4B

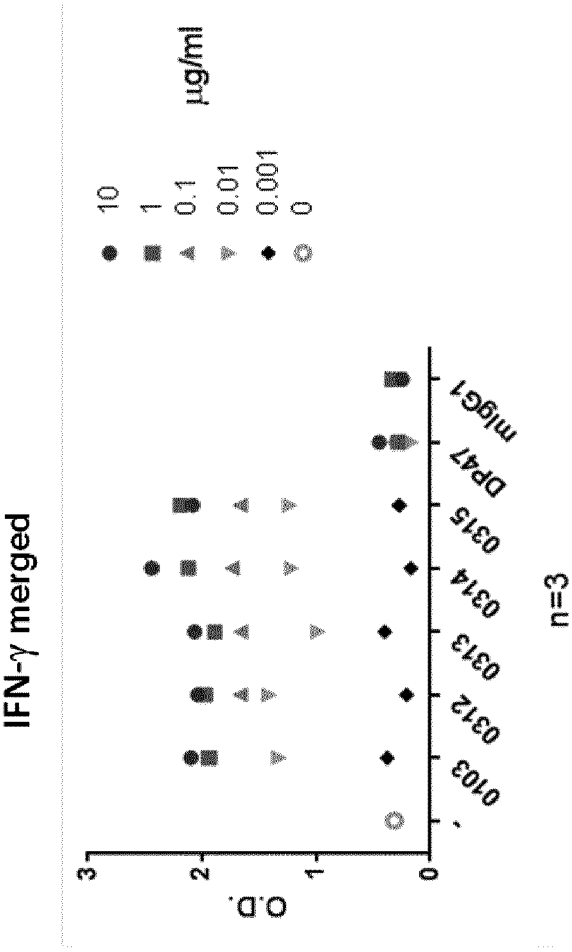
IFN-γ merged
O.D.
3
2
1
0
-
0103
0312
0313
0314
0315
DP47
mIgG1
n=3
(mean)
10
1
0.1
0.01
0.001
0
µg/ml

# Fig. 5

5A

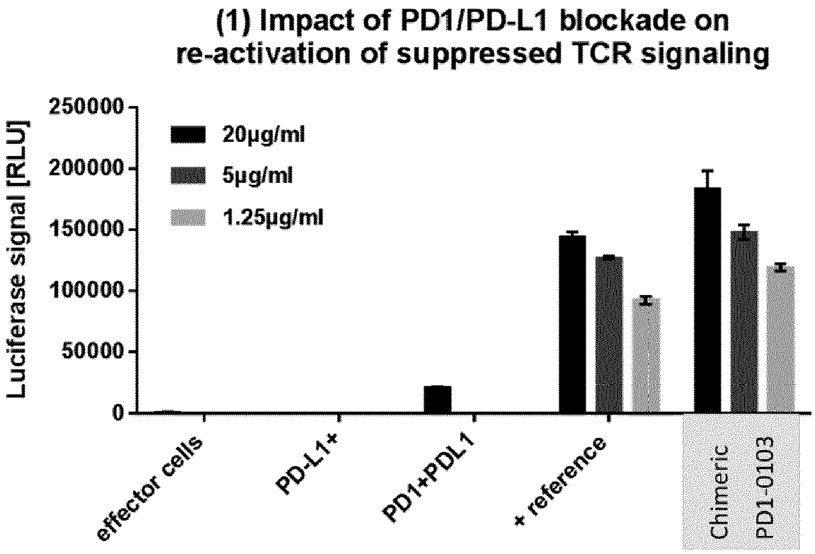
(1) Impact of PD1/PD-L1 blockade on
re-activation of suppressed TCR signaling
Luciferase signal [RLU]
250000
200000
150000
100000
50000
0
20µg/ml
5µg/ml
1.25µg/ml
effector cells
PD-L1+
PD1+PDL1
+ reference
Chimeric
PD1-0103

5B

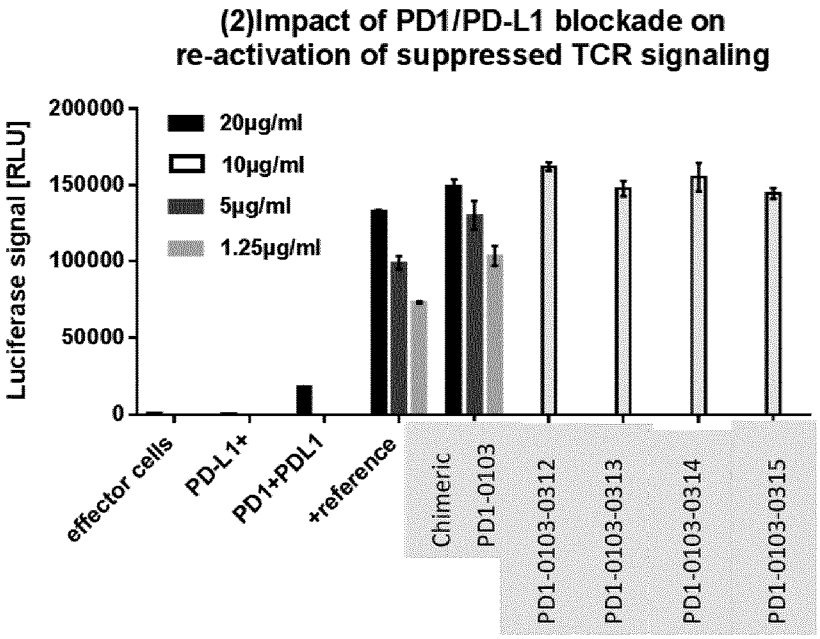
(2)Impact of PD1/PD-L1 blockade on
re-activation of suppressed TCR signaling
Luciferase signal [RLU]
200000
150000
100000
50000
0
20µg/ml
10µg/ml
5µg/ml
1.25µg/ml
effector cells
PD-L1+
PD1+PDL1
+reference
Chimeric
PD1-0103
PD1-0103-0312
PD1-0103-0313
PD1-0103-0314
PD1-0103-0315

Fig. 6

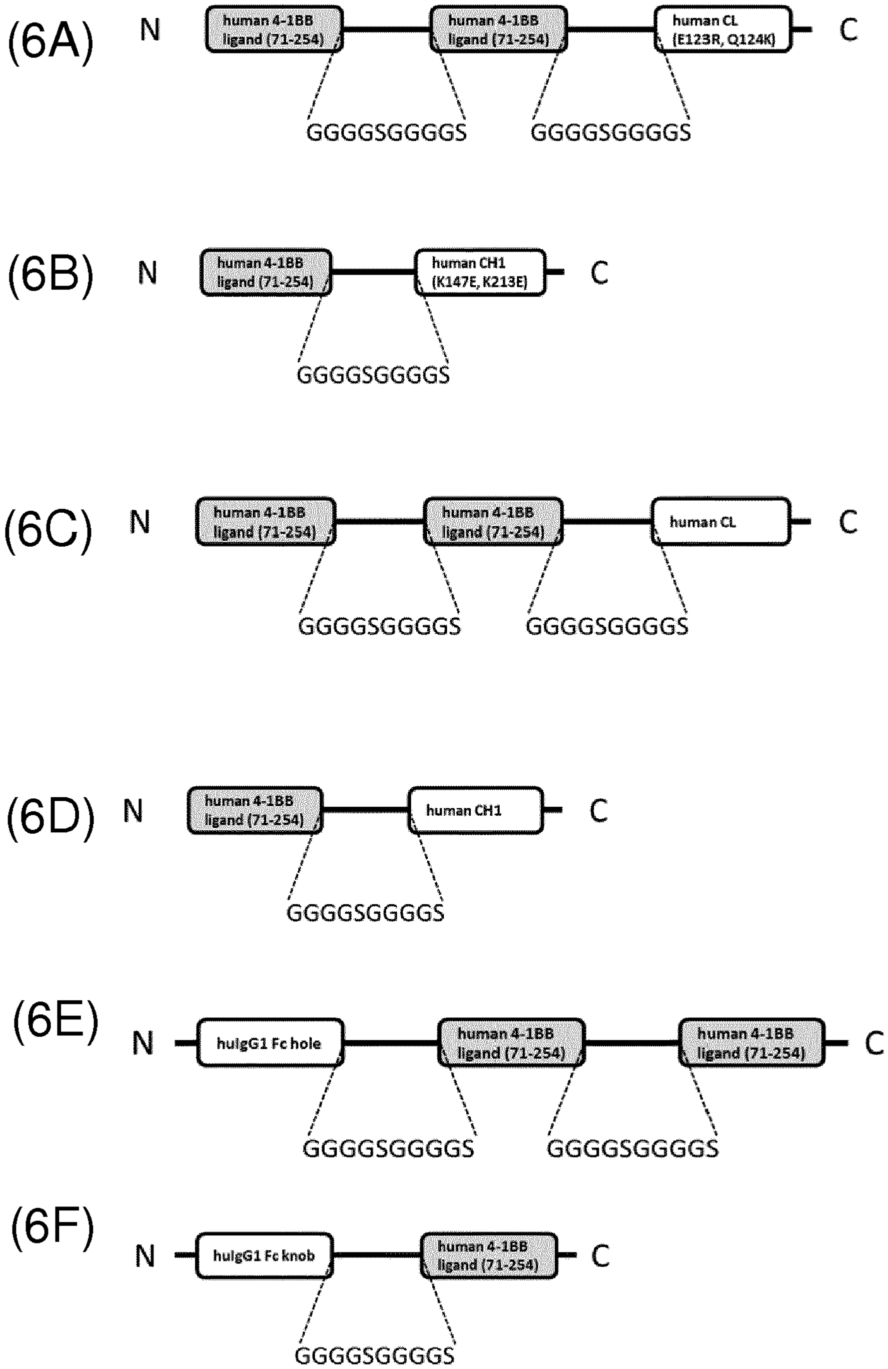
(6A)
N
human 4-1BB ligand (71-254)
human 4-1BB ligand (71-254)
human CL (E123R, Q124K)
C
GGGGSGGGGS
GGGGSGGGGS
(6B)
N
human 4-1BB ligand (71-254)
human CH1 (K147E, K213E)
C
GGGGSGGGGS
(6C)
N
human 4-1BB ligand (71-254)
human 4-1BB ligand (71-254)
human CL
C
GGGGSGGGGS
GGGGSGGGGS
(6D)
N
human 4-1BB ligand (71-254)
human CH1
C
GGGGSGGGGS
(6E)
N
huIgG1 Fc hole
human 4-1BB ligand (71-254)
human 4-1BB ligand (71-254)
C
GGGGSGGGGS
GGGGSGGGGS
(6F)
N
huIgG1 Fc knob
human 4-1BB ligand (71-254)
C
GGGGSGGGGS

Fig. 7

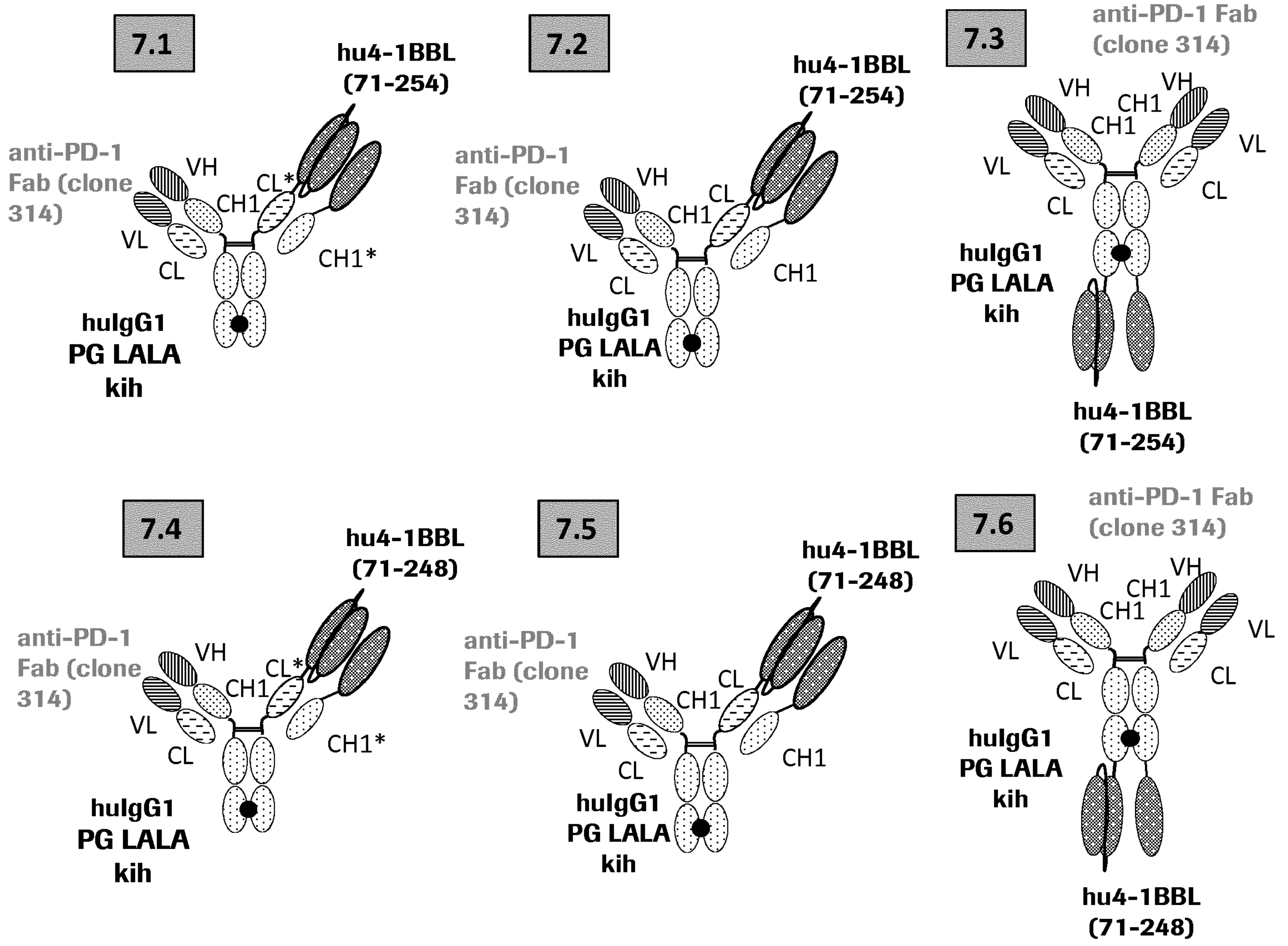
7.1
anti-PD-1 Fab (clone 314)
VH
CH1
CL*
CH1*
VL
CL
hu4-1BBL (71-254)
huIgG1 PG LALA kih
7.2
anti-PD-1 Fab (clone 314)
VH
CH1
CL
CH1
VL
CL
hu4-1BBL (71-254)
huIgG1 PG LALA kih
7.3
anti-PD-1 Fab (clone 314)
VH
CH1
CH1
VH
VL
VL
CL
CL
huIgG1 PG LALA kih
hu4-1BBL (71-254)
7.4
anti-PD-1 Fab (clone 314)
VH
CH1
CL*
CH1*
VL
CL
hu4-1BBL (71-248)
huIgG1 PG LALA kih
7.5
anti-PD-1 Fab (clone 314)
VH
CH1
CL
CH1
VL
CL
hu4-1BBL (71-248)
huIgG1 PG LALA kih
7.6
anti-PD-1 Fab (clone 314)
VH
CH1
CH1
VH
VL
VL
CL
CL
huIgG1 PG LALA kih
hu4-1BBL (71-248)

Fig. 8

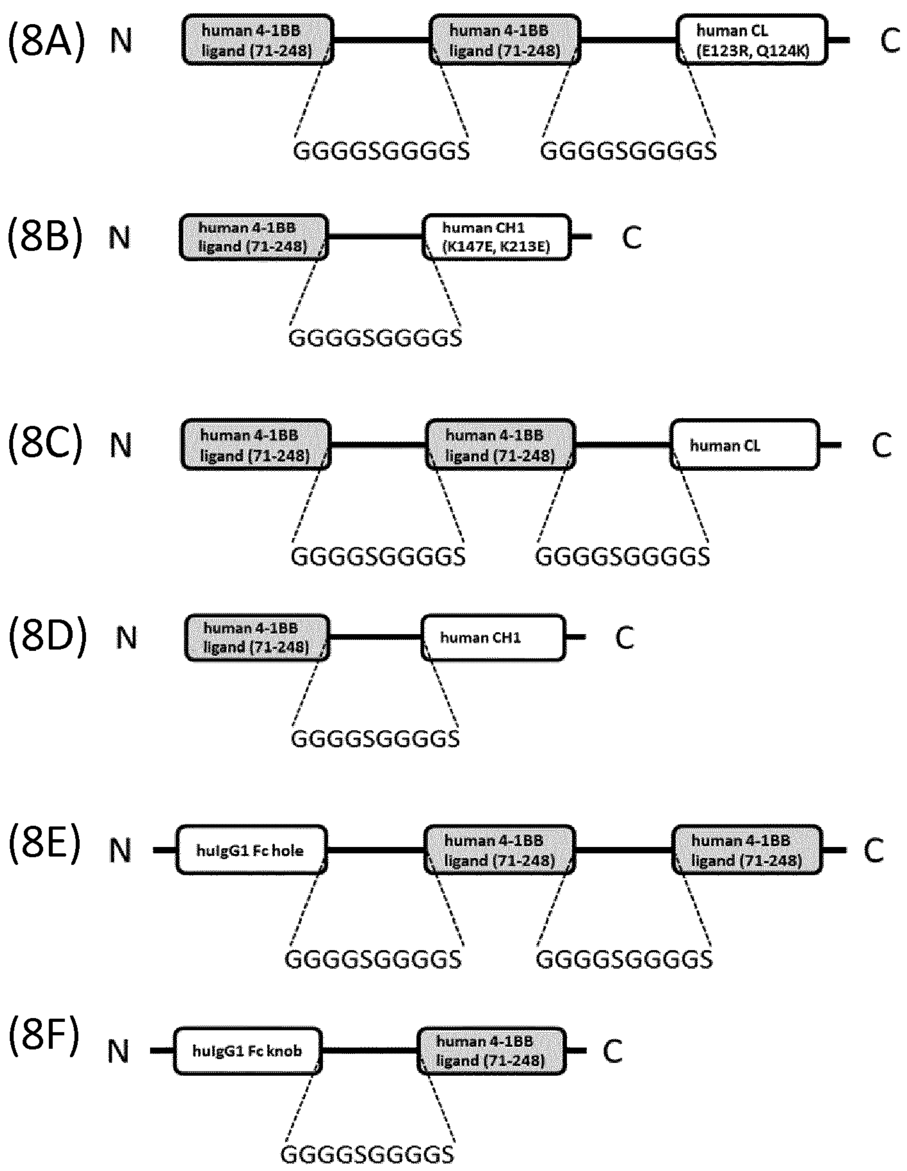
(8A) N
human 4-1BB ligand (71-248)
human 4-1BB ligand (71-248)
human CL (E123R, Q124K)
C
GGGGSGGGGS
GGGGSGGGGS
(8B) N
human 4-1BB ligand (71-248)
human CH1 (K147E, K213E)
C
GGGGSGGGGS
(8C) N
human 4-1BB ligand (71-248)
human 4-1BB ligand (71-248)
human CL
C
GGGGSGGGGS
GGGGSGGGGS
(8D) N
human 4-1BB ligand (71-248)
human CH1
C
GGGGSGGGGS
(8E) N
huIgG1 Fc hole
human 4-1BB ligand (71-248)
human 4-1BB ligand (71-248)
C
GGGGSGGGGS
GGGGSGGGGS
(8F) N
huIgG1 Fc knob
human 4-1BB ligand (71-248)
C
GGGGSGGGGS

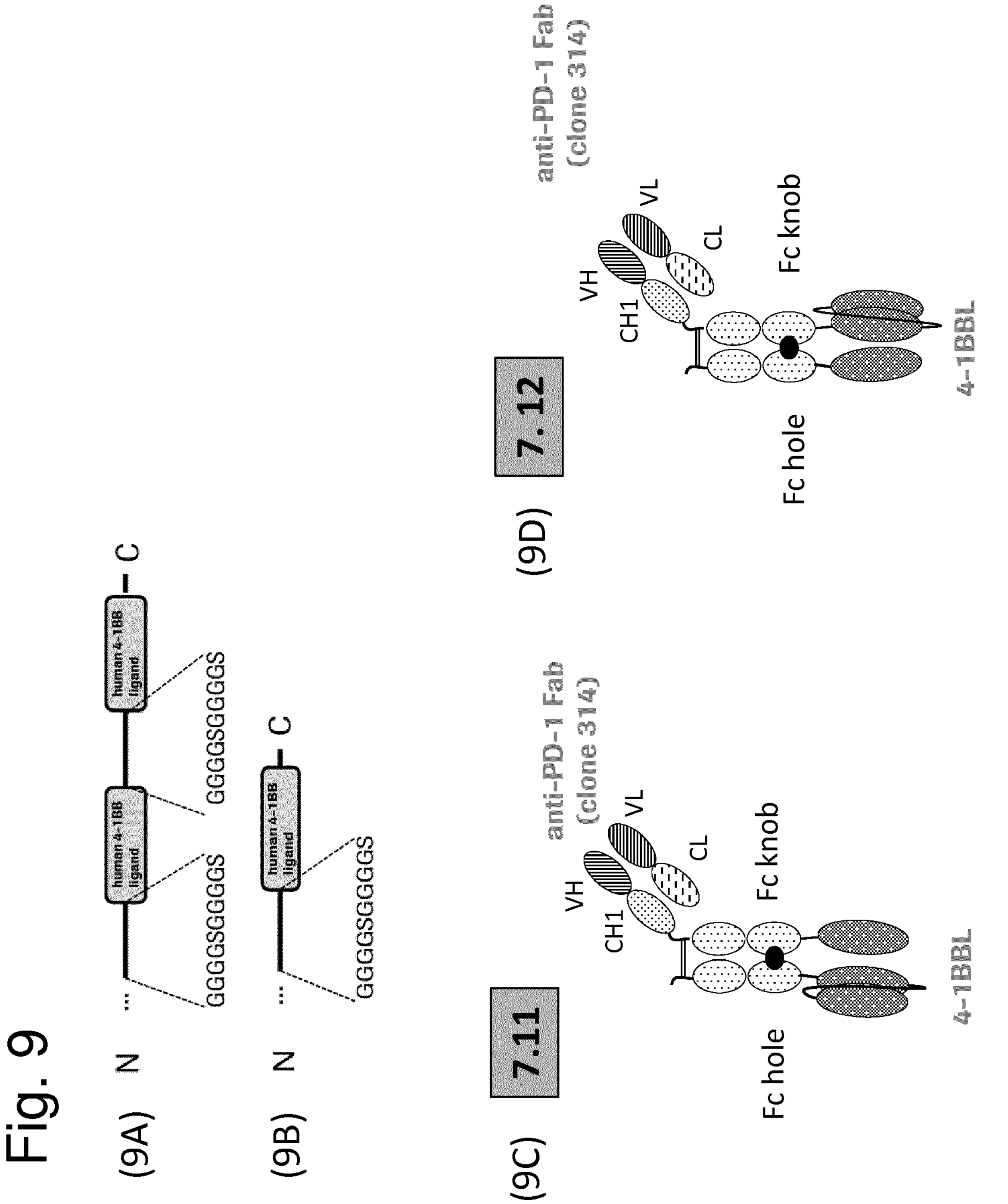
Fig. 9
(9A)
N
...
human 4-1BB ligand
human 4-1BB ligand
C
GGGGSGGGGS
GGGGSGGGGS
GGGGSGGGGS
(9B)
N
...
human 4-1BB ligand
C
GGGGSGGGGS
GGGGSGGGGS
(9C)
7.11
anti-PD-1 Fab (clone 314)
VH
VL
CH1
CL
Fc knob
Fc hole
4-1BBL
(9D)
7. 12
anti-PD-1 Fab (clone 314)
VH
VL
CH1
CL
Fc knob
Fc hole
4-1BBL

Fig. 10

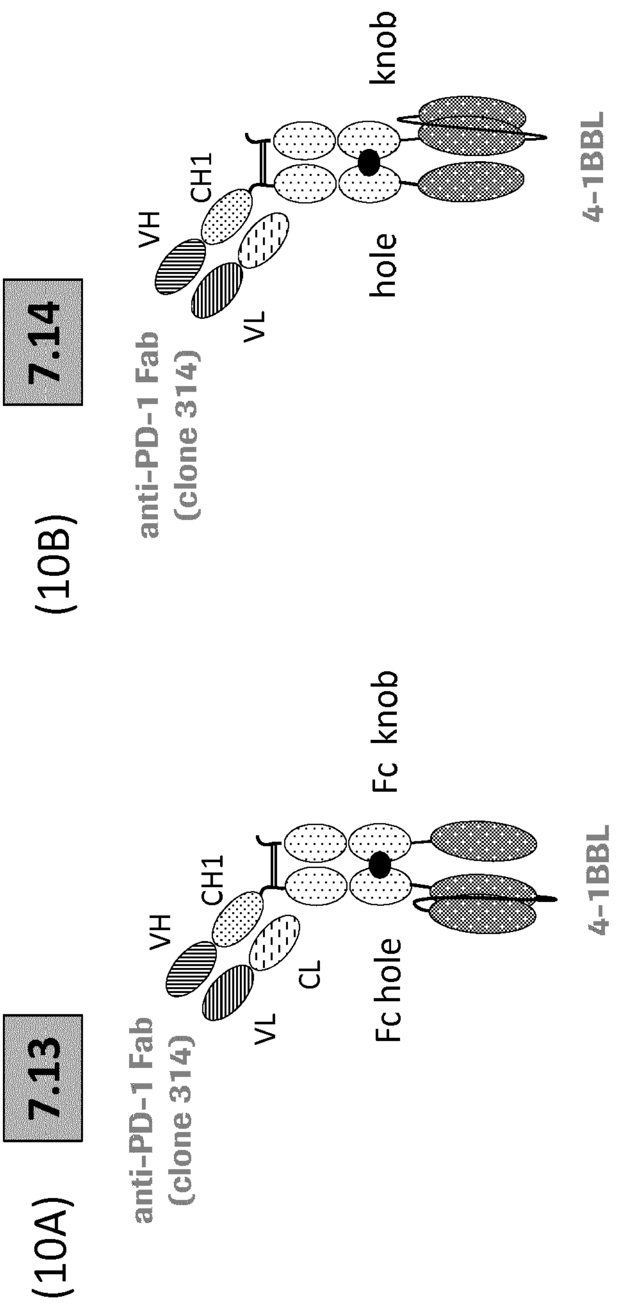
(10A)
7.13
anti-PD-1 Fab
(clone 314)
VH
CH1
VL
CL
Fc hole
Fc knob
4-1BBL
(10B)
7.14
anti-PD-1 Fab
(clone 314)
VH
CH1
VL
hole
knob
4-1BBL

Fig. 11

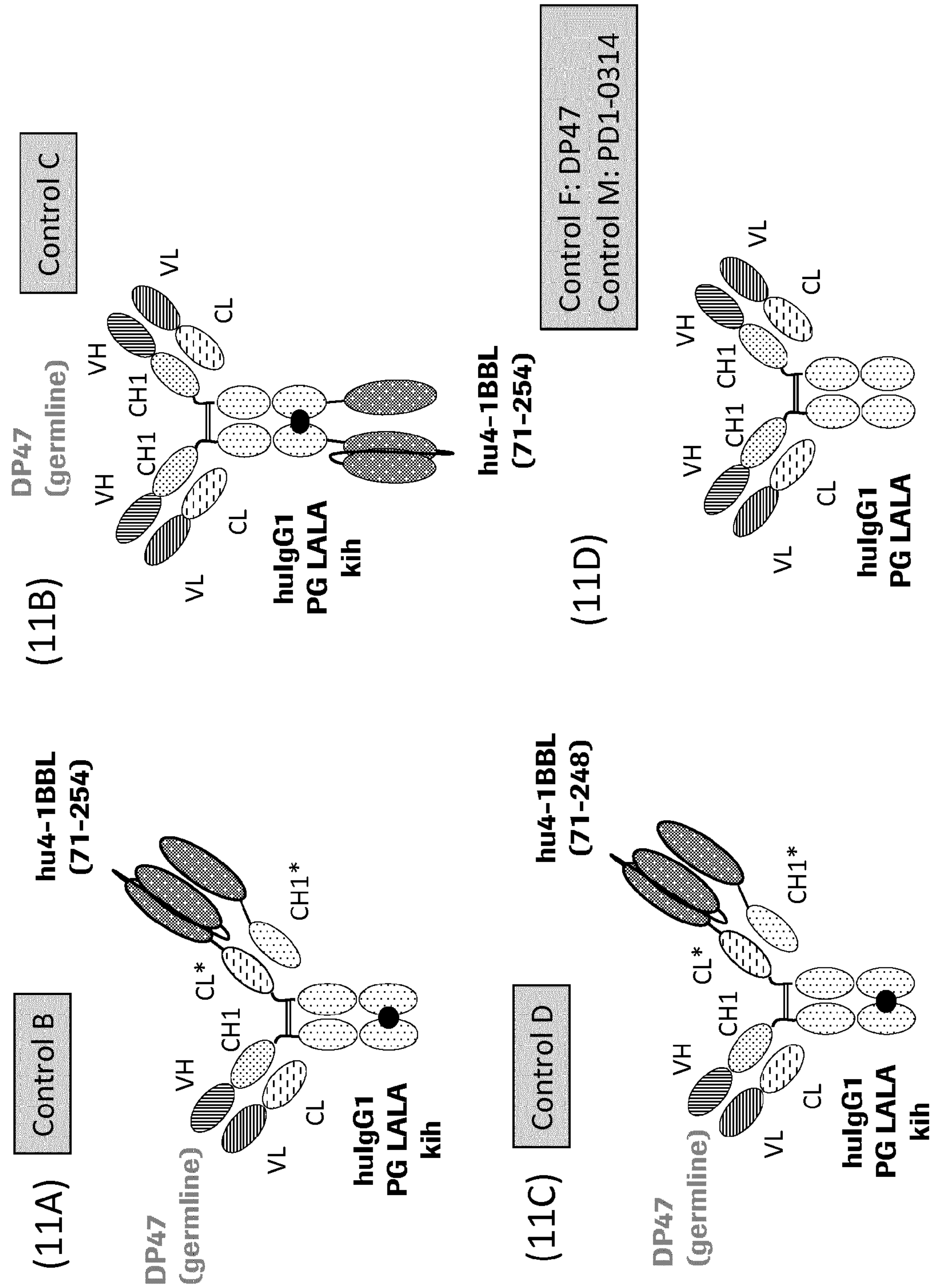
(11A)
Control B
DP47 (germline)
VH
VL
CH1
CL
CL*
CH1*
hu4-1BBL (71-254)
huIgG1 PG LALA kih
(11B)
Control C
DP47 (germline)
VH
VL
CH1
CL
hu4-1BBL (71-254)
huIgG1 PG LALA kih
(11C)
Control D
DP47 (germline)
VH
VL
CH1
CL
CL*
CH1*
hu4-1BBL (71-248)
huIgG1 PG LALA kih
(11D)
Control F: DP47
Control M: PD1-0314
VH
VL
CH1
CL
huIgG1 PG LALA

Fig. 12

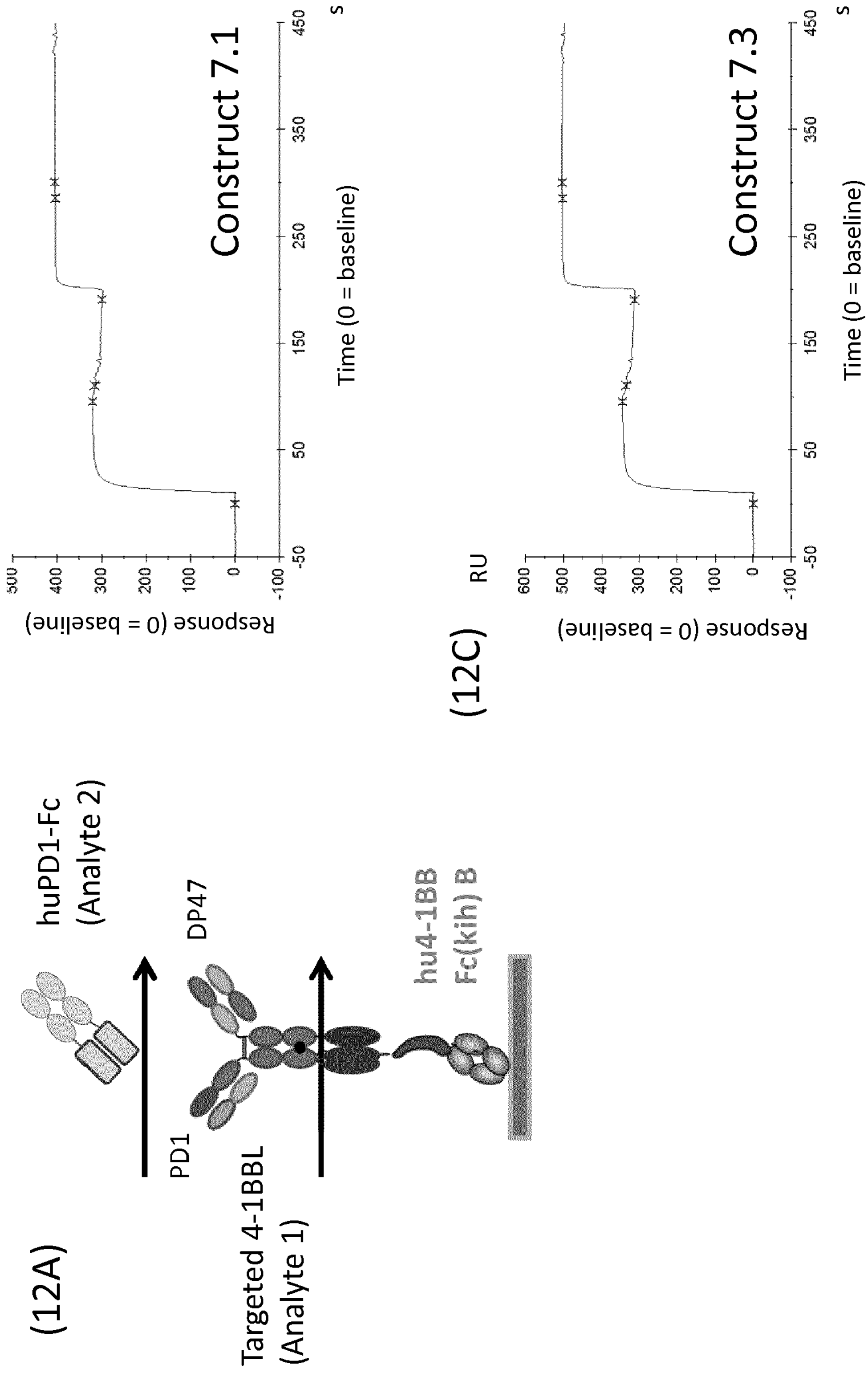
(12A)
Targeted 4-1BBL
(Analyte 1)
PD1
DP47
huPD1-Fc
(Analyte 2)
hu4-1BB
Fc(kih) B
(12B)
Response (0 = baseline)
RU
Time (0 = baseline)
s
Construct 7.1
(12C)
Response (0 = baseline)
RU
Time (0 = baseline)
s
Construct 7.3

Fig. 12

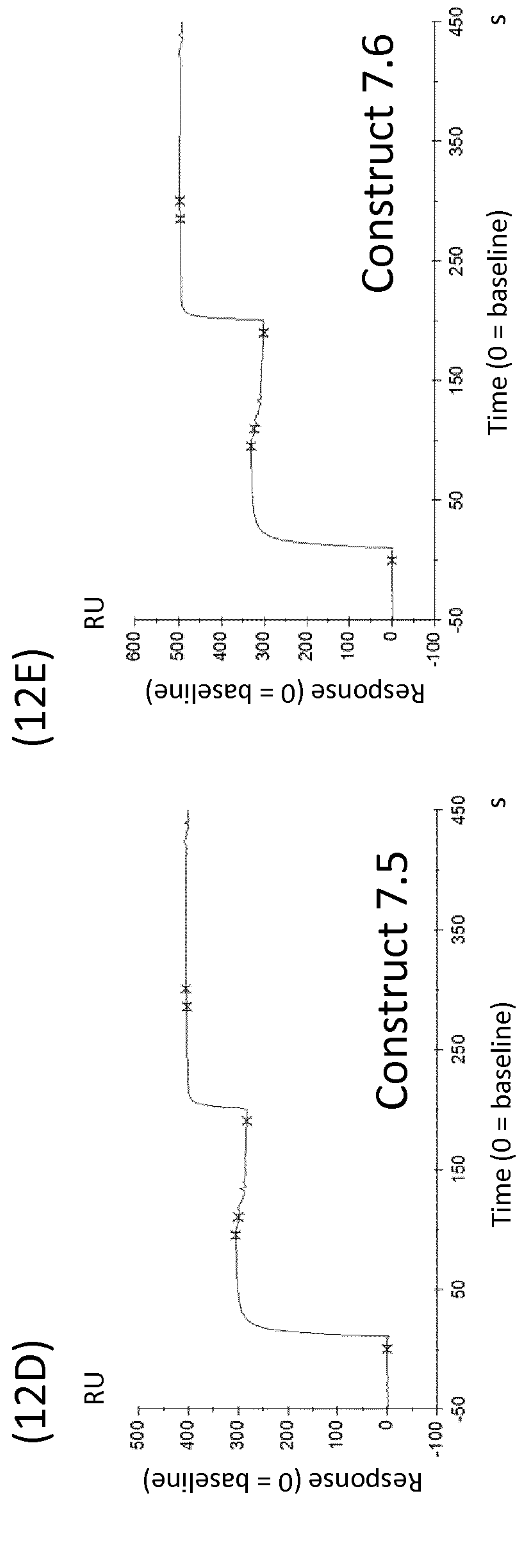
(12D)
RU
Response (0 = baseline)
500
400
300
200
100
0
-100
-50
50
150
250
350
450
s
Time (0 = baseline)
Construct 7.5
(12E)
RU
Response (0 = baseline)
600
500
400
300
200
100
0
-100
-50
50
150
250
350
450
s
Time (0 = baseline)
Construct 7.6

Fig. 13

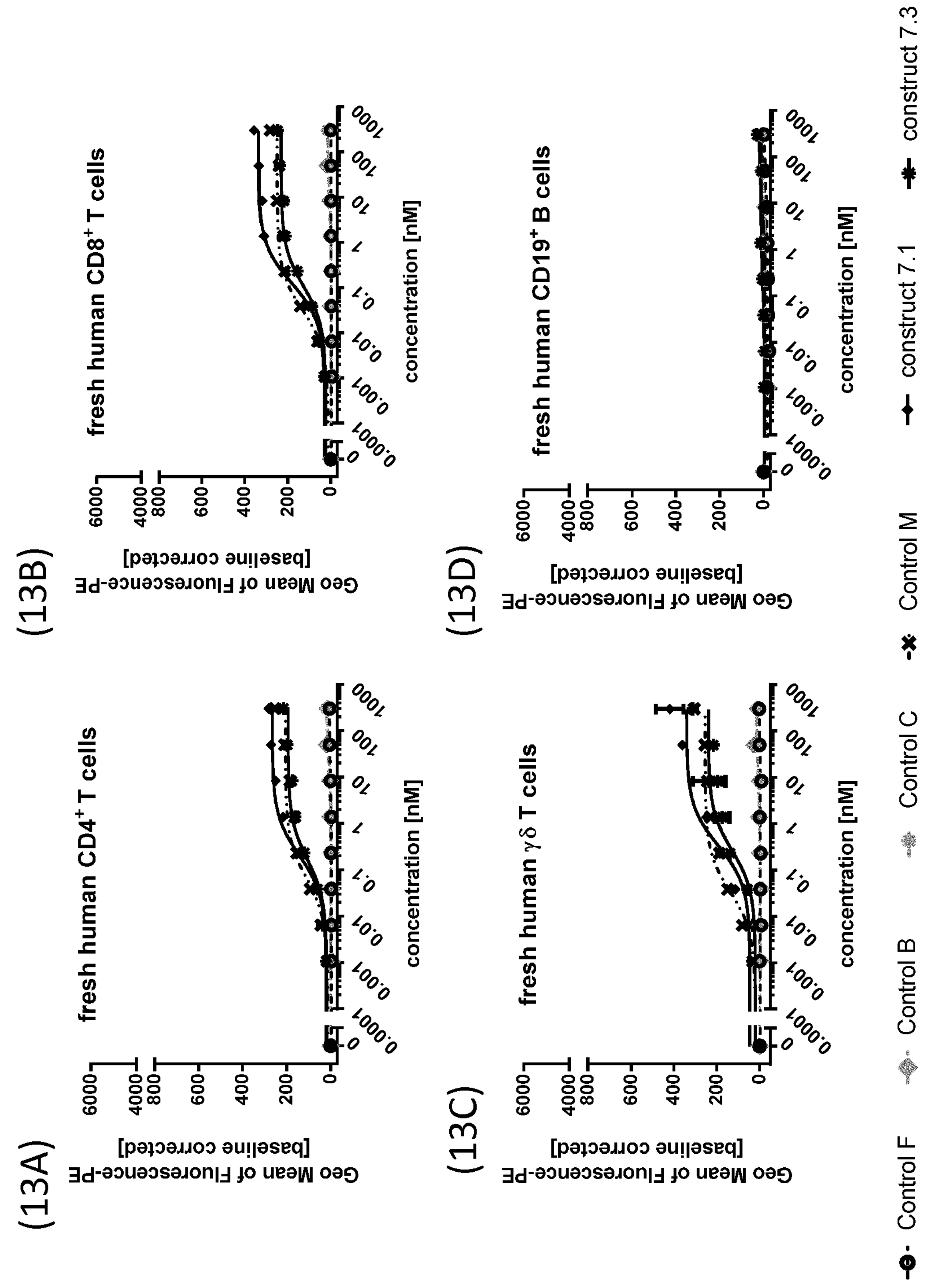
(13A)
fresh human CD4⁺ T cells
(13B)
fresh human CD8⁺ T cells
(13C)
fresh human γδ T cells
(13D)
fresh human CD19⁺ B cells
Geo Mean of Fluorescence-PE [baseline corrected]
6000
4000
800
600
400
200
0
concentration [nM]
0
0.0001
0.001
0.01
0.1
1
10
100
1000
Control F
Control B
Control C
Control M
construct 7.1
construct 7.3

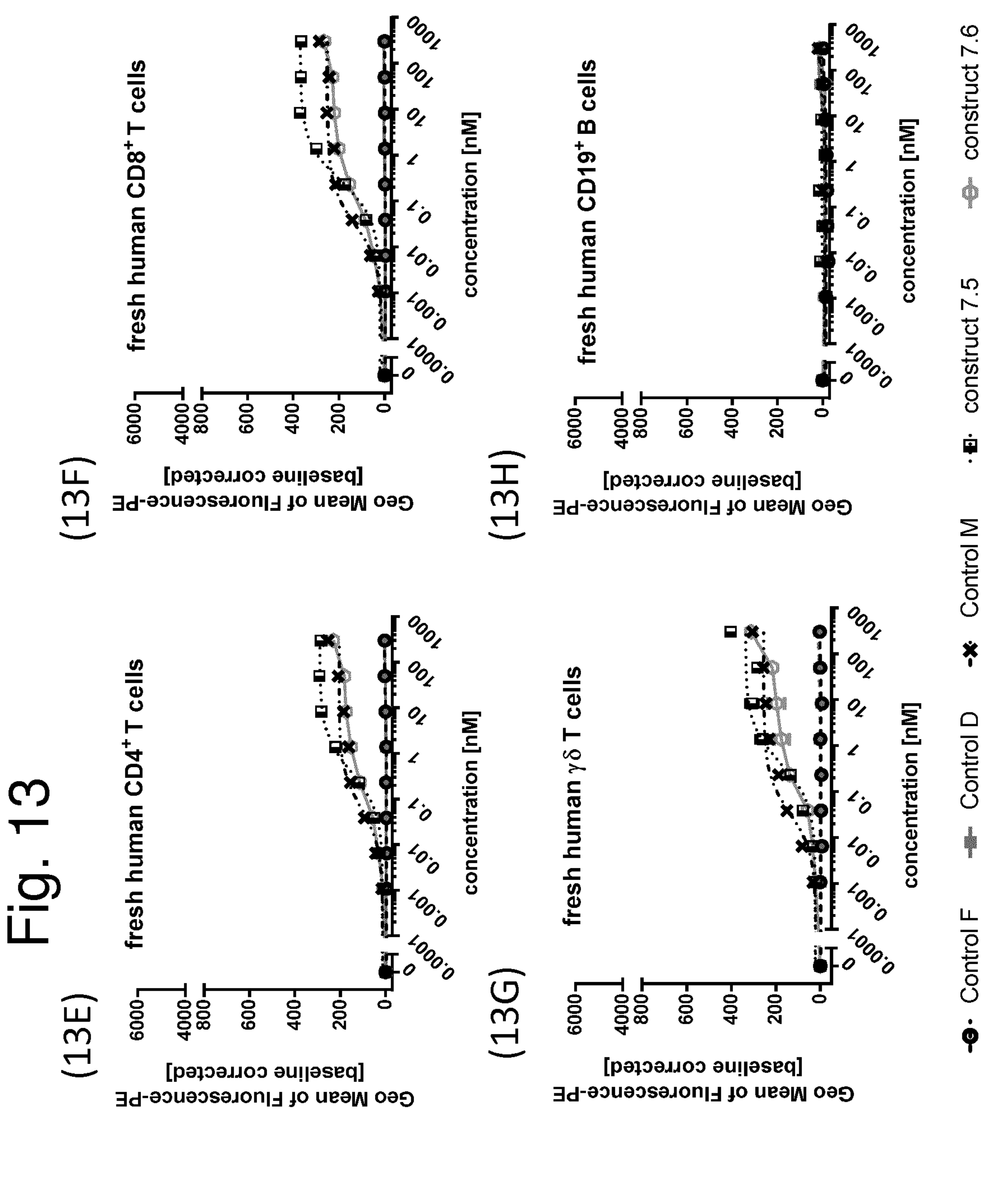
(13E)
fresh human CD4⁺ T cells
(13F)
fresh human CD8⁺ T cells
(13G)
fresh human γδ T cells
(13H)
fresh human CD19⁺ B cells
Geo Mean of Fluorescence-PE [baseline corrected]
6000
4000
800
600
400
200
0
concentration [nM]
0
0.0001
0.001
0.01
0.1
1
10
100
1000
Control F
Control D
Control M
construct 7.5
construct 7.6

Fig. 13

Fig. 14

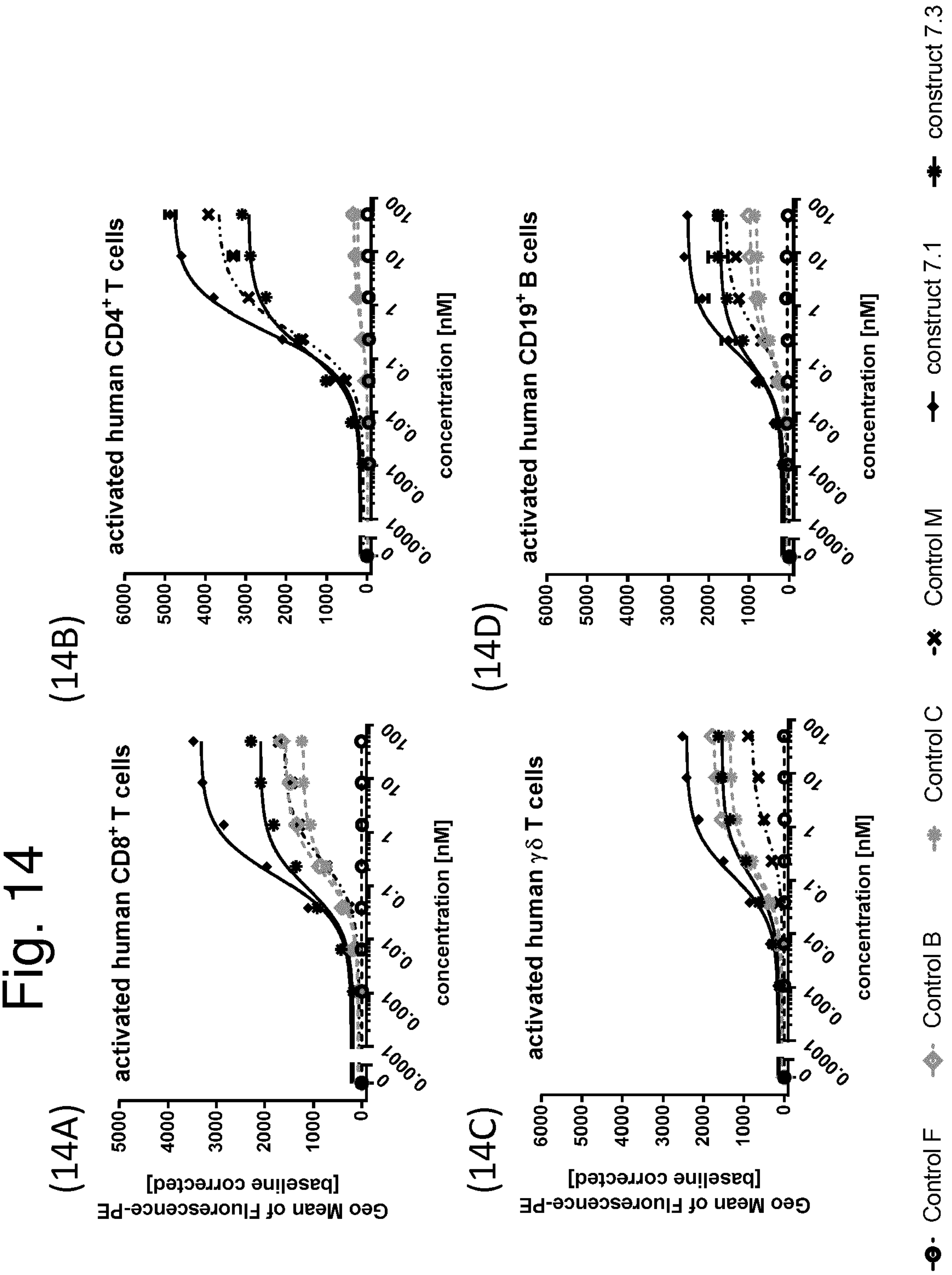
(14A)
activated human CD8+ T cells
Geo Mean of Fluorescence-PE [baseline corrected]
5000
4000
3000
2000
1000
0
0
0.0001
0.001
0.01
0.1
1
10
100
concentration [nM]
(14B)
activated human CD4+ T cells
6000
5000
4000
3000
2000
1000
0
concentration [nM]
(14C)
activated human γδ T cells
Geo Mean of Fluorescence-PE [baseline corrected]
6000
5000
4000
3000
2000
1000
0
concentration [nM]
(14D)
activated human CD19+ B cells
6000
5000
4000
3000
2000
1000
0
concentration [nM]
Control F
Control B
Control C
Control M
construct 7.1
construct 7.3

Fig. 14

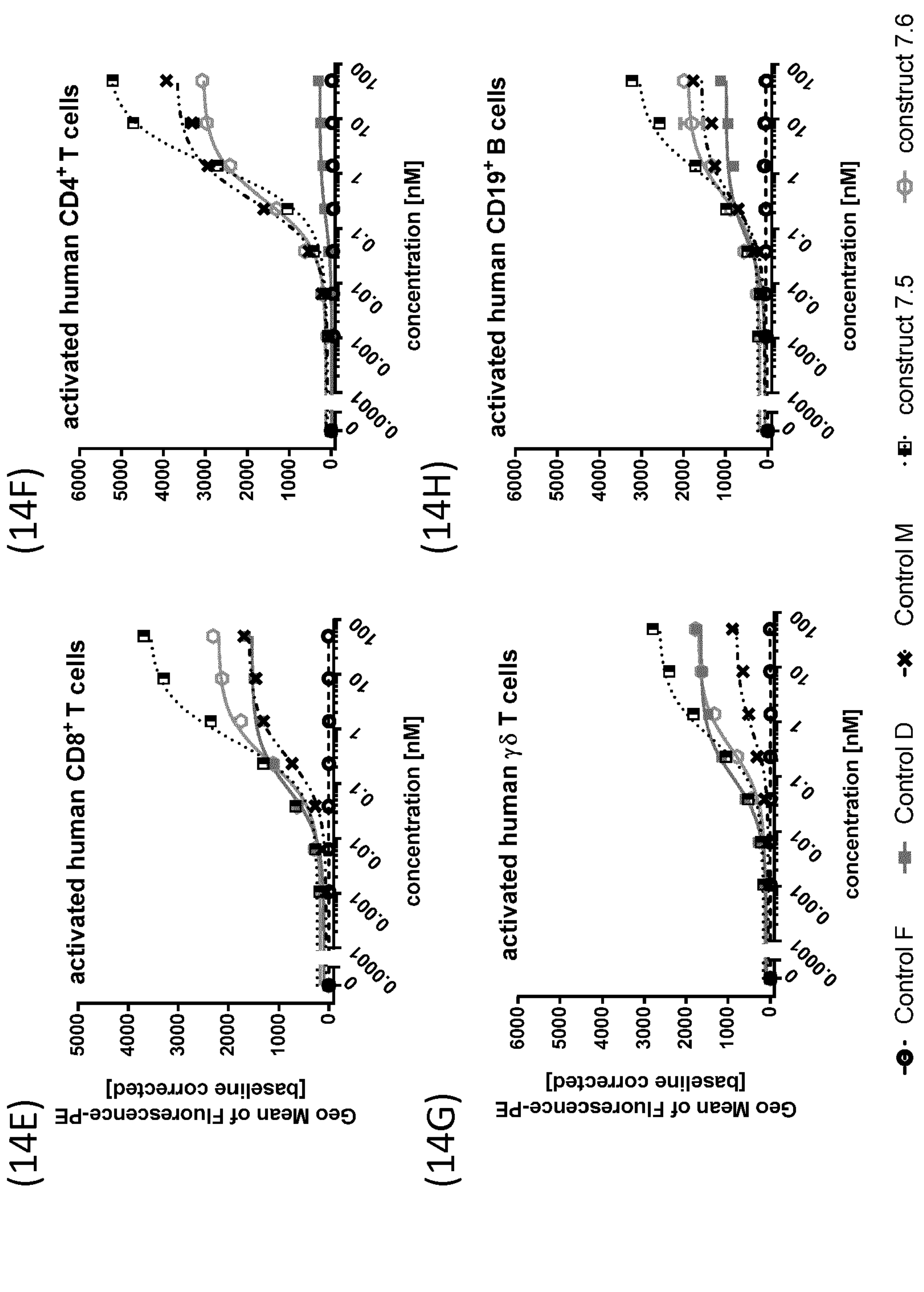

(14E)
activated human CD8⁺ T cells
(14F)
activated human CD4⁺ T cells
(14G)
activated human γδ T cells
(14H)
activated human CD19⁺ B cells
Geo Mean of Fluorescence-PE [baseline corrected]
concentration [nM]
0
0.0001
0.001
0.01
0.1
1
10
100
0
1000
2000
3000
4000
5000
6000
Control F
Control D
Control M
construct 7.5
construct 7.6

Fig. 15

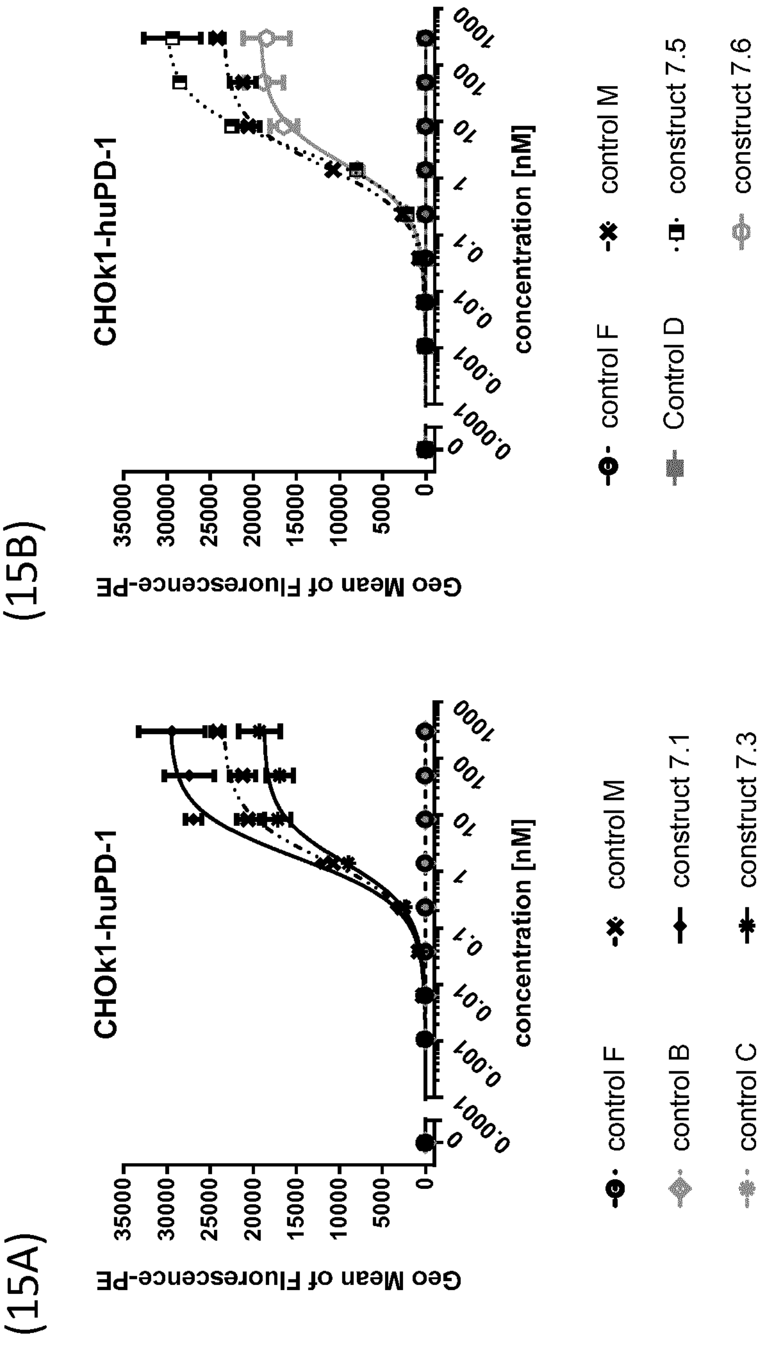

(15A)
CHOk1-huPD-1
Geo Mean of Fluorescence-PE
35000
30000
25000
20000
15000
10000
5000
0
0
0.0001
0.001
0.01
0.1
1
10
100
1000
concentration [nM]
control F
control M
control B
construct 7.1
control C
construct 7.3
(15B)
CHOk1-huPD-1
Geo Mean of Fluorescence-PE
35000
30000
25000
20000
15000
10000
5000
0
0
0.0001
0.001
0.01
0.1
1
10
100
1000
concentration [nM]
control F
control M
Control D
construct 7.5
construct 7.6

Fig. 16

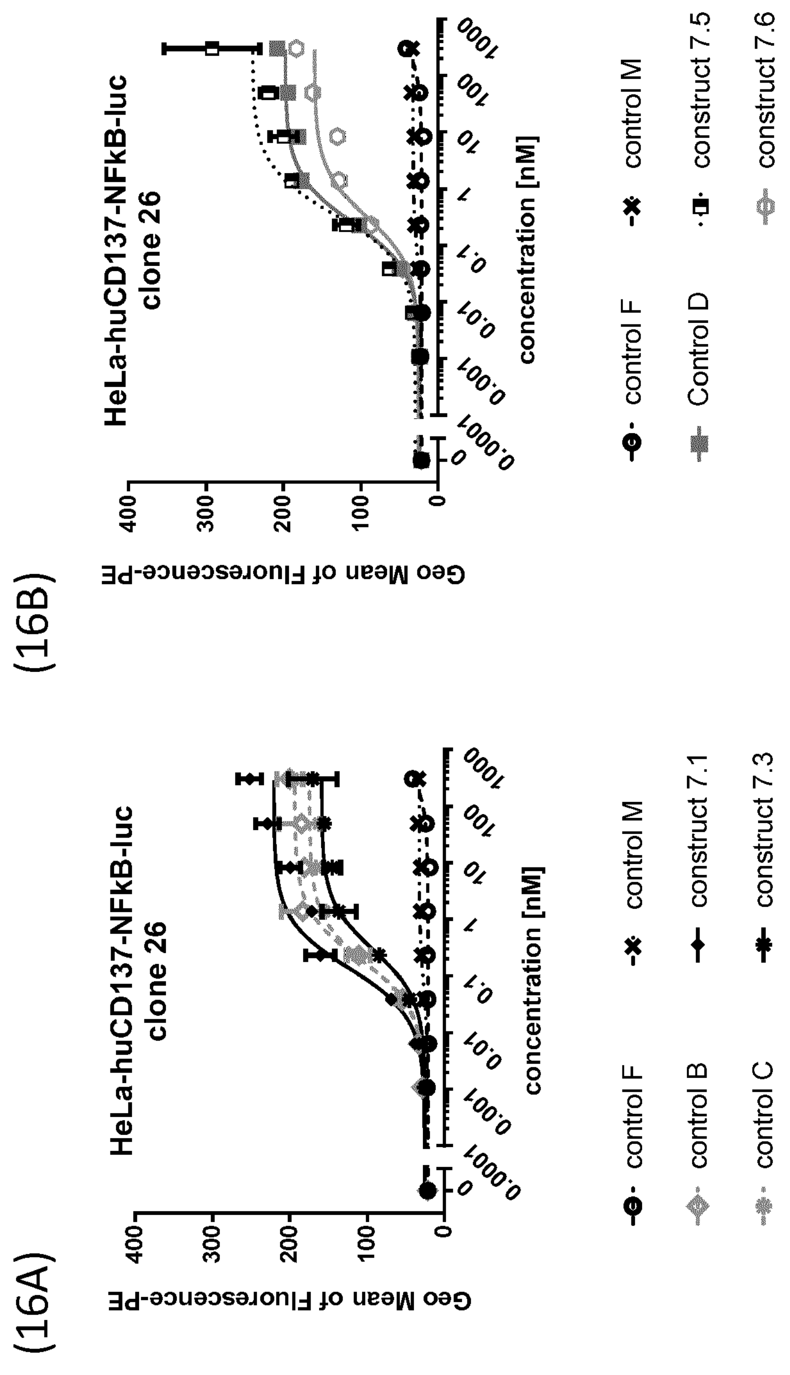
(16A)
HeLa-huCD137-NFkB-luc
clone 26
Geo Mean of Fluorescence-PE
concentration [nM]
0
0.0001
0.001
0.01
0.1
1
10
100
1000
400
300
200
100
control F
control M
control B
construct 7.1
control C
construct 7.3
(16B)
HeLa-huCD137-NFkB-luc
clone 26
Geo Mean of Fluorescence-PE
concentration [nM]
control F
control M
Control D
construct 7.5
construct 7.6

Fig. 17

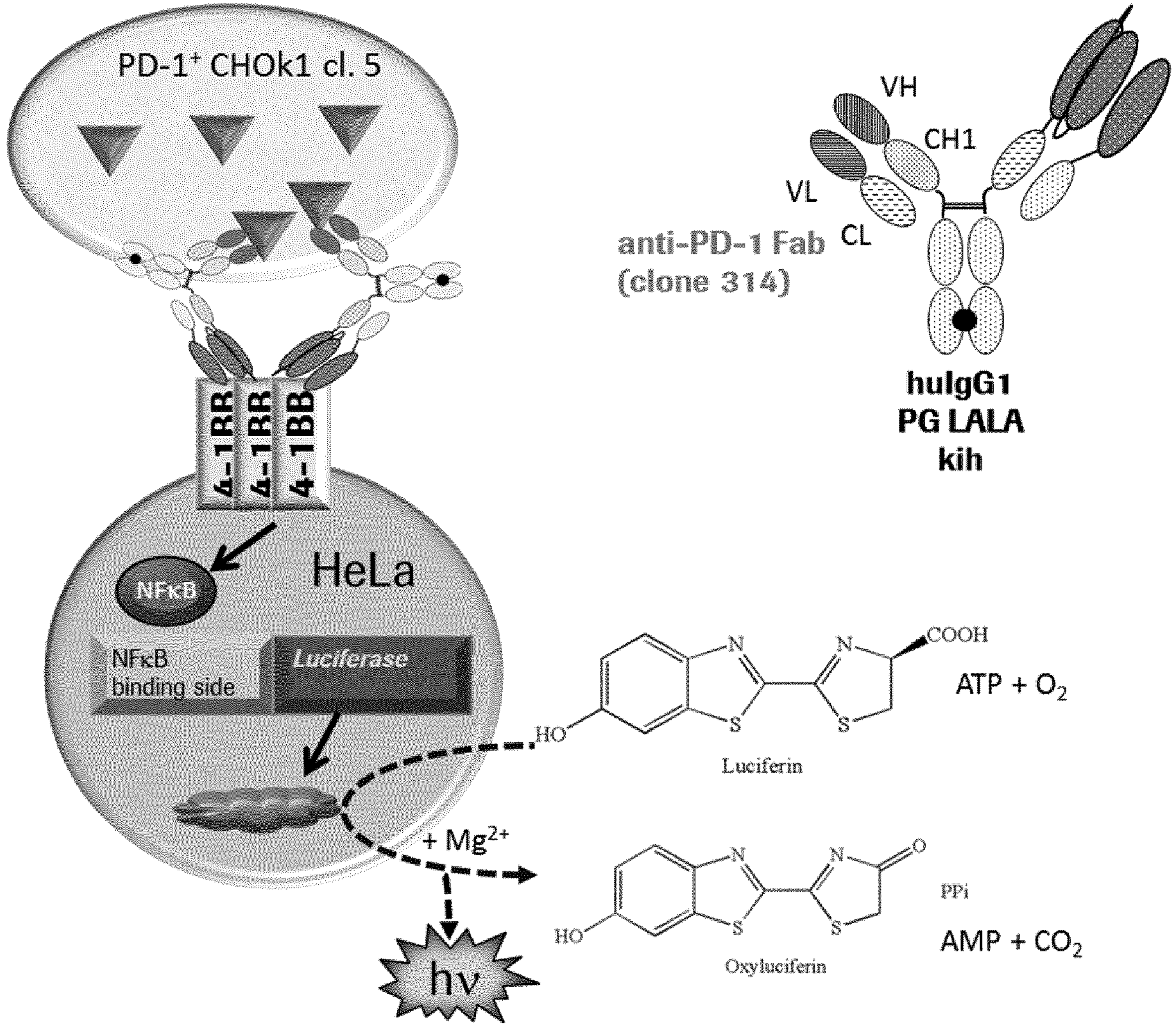
PD-1+ CHOk1 cl. 5
4-1BB
4-1BB
4-1BB
HeLa
NFκB
NFκB binding side
Luciferase
+ Mg2+
hν
Luciferin
ATP + O2
Oxyluciferin
PPi
AMP + CO2
VH
CH1
VL
CL
anti-PD-1 Fab (clone 314)
huIgG1
PG LALA
kih

Fig. 18

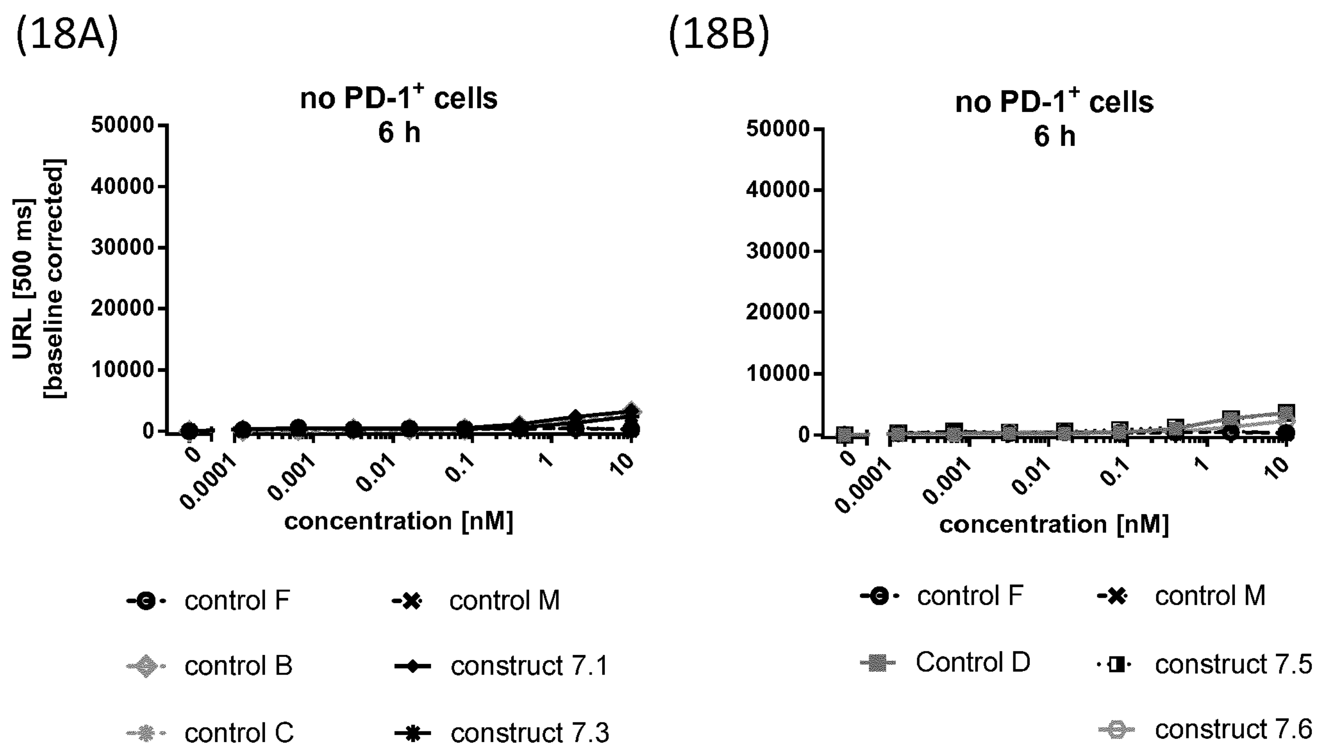
(18A)
no PD-1+ cells
6 h
URL [500 ms]
[baseline corrected]
50000
40000
30000
20000
10000
0
0
0.0001
0.001
0.01
0.1
1
10
concentration [nM]
control F
control M
control B
construct 7.1
control C
construct 7.3
(18B)
no PD-1+ cells
6 h
50000
40000
30000
20000
10000
0
0
0.0001
0.001
0.01
0.1
1
10
concentration [nM]
control F
control M
Control D
construct 7.5
construct 7.6

Fig. 18

(18C)

(18D)

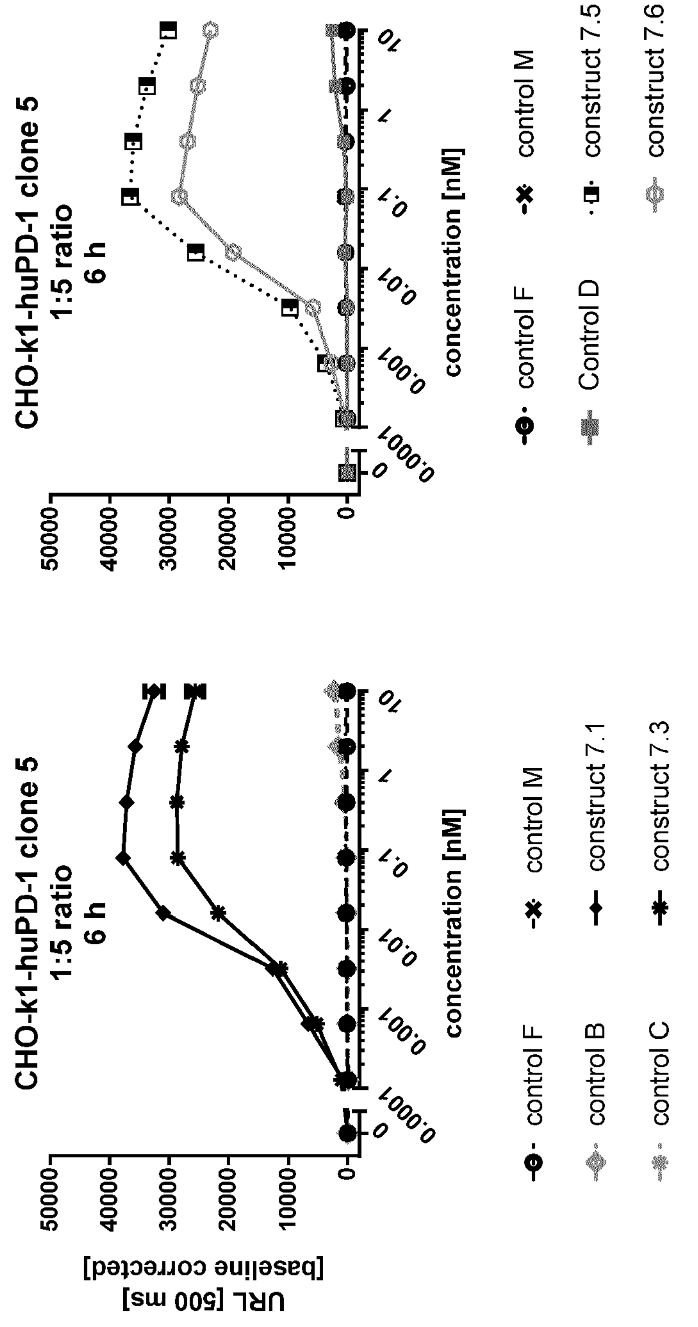

Europäisches Patentamt
European Patent Office
Office européen des brevets

# EUROPEAN SEARCH REPORT

Application Number
EP 16 16 9487

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | S. CHEN ET AL: "Combination of 4-1BB Agonist and PD-1 Antagonist Promotes Antitumor Effector/Memory CD8 T Cells in a Poorly Immunogenic Tumor Model", CANCER IMMUNOLOGY RESEARCH, vol. 3, no. 2, 11 November 2014 (2014-11-11), pages 149-160, XP055293412, US ISSN: 2326-6066, DOI: 10.1158/2326-6066.CIR-14-0118 * abstract * | 1-15 | INV. C07K14/705 A61K39/395 C07K16/28 C12N15/62 |
| Y | SHINDO Y ET AL: "Combination immunotherapy with 4-1BB activation and PD-1 blockade enhances antitumor efficacy in a mouse model of subcutaneous tumor", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 35, no. 1, 1 January 2015 (2015-01-01), pages 129-136, XP002746546, ISSN: 0250-7005 * abstract * | 1-15 | TECHNICAL FIELDS SEARCHED (IPC) A61K C07K C12N |
| Y,D | HORNIG NORA ET AL: "Combination of a Bispecific Antibody and Costimulatory Antibody-Ligand Fusion Proteins for Targeted Cancer Immunotherapy", JOURNAL OF IMMUNOTHERAPY, LIPPINCOTT WILLIAMS & WILKINGS INC, US, vol. 35, no. 5, 1 June 2012 (2012-06-01), pages 418-429, XP009163394, ISSN: 1524-9557, DOI: 10.1097/CJI.0B013E3182594387 * abstract * | 1-15 | |

-/--

The present search report has been drawn up for all claims

1

| Place of search | Date of completion of the search | Examiner |
|---|---|---|
| Munich | 5 August 2016 | Pilat, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

## EUROPEAN SEARCH REPORT

Application Number
EP 16 16 9487

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NORA HORNIG ET AL: "Evaluating combinations of costimulatory antibody-ligand fusion proteins for targeted cancer immunotherapy", CANCER IMMUNOLOGY, IMMUNOTHERAPY, vol. 62, no. 8, 1 August 2013 (2013-08-01), pages 1369-1380, XP055184710, ISSN: 0340-7004, DOI: 10.1007/s00262-013-1441-7 * abstract * ----- | 1-15 | |
| A | WO 2013/164694 A1 (BIOCON LTD [IN]) 7 November 2013 (2013-11-07) * abstract; table 1 * ----- | 1-15 | |
| E | WO 2016/075278 A1 (HOFFMANN LA ROCHE [CH]; HOFFMANN LA ROCHE [US]) 19 May 2016 (2016-05-19) * the whole document * ----- | 1-15 | |
| A,D | WO 2010/010051 A1 (APOGENIX GMBH [DE]; HILL OLIVER [DE]; GIEFFERS CHRISTIAN [DE]; THIEMAN) 28 January 2010 (2010-01-28) * abstract; figures 13,14,16 * * page 9, last paragraph - page 10, paragraph 1 * ----- | 1-15 | TECHNICAL FIELDS SEARCHED (IPC) |
| A | WO 2015/095423 A2 (GENENTECH INC [US]; HOFFMANN LA ROCHE [CH]) 25 June 2015 (2015-06-25) * abstract; claims * ----- | 1-15 | |

1

The present search report has been drawn up for all claims

| Place of search | Date of completion of the search | Examiner |
|---|---|---|
| Munich | 5 August 2016 | Pilat, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 16 16 9487

This annex lists the patent family members relating to the patent documents cited in the above-mentioned European search report. The members are as contained in the European Patent Office EDP file on
The European Patent Office is in no way liable for these particulars which are merely given for the purpose of information.

05-08-2016

| Patent document cited in search report | Publication date | Patent family member(s) | Publication date |
|---|---|---|---|
| WO 2013164694 A1 | 07-11-2013 | AU 2013255542 A1 | 20-11-2014 |
| | | CA 2871706 A1 | 07-11-2013 |
| | | CN 104334573 A | 04-02-2015 |
| | | EP 2844667 A1 | 11-03-2015 |
| | | HK 1202295 A1 | 25-09-2015 |
| | | JP 2015516815 A | 18-06-2015 |
| | | NZ 701821 A | 24-06-2016 |
| | | RU 2014145158 A | 20-06-2016 |
| | | US 2013287802 A1 | 31-10-2013 |
| | | US 2014356362 A1 | 04-12-2014 |
| | | US 2016168260 A1 | 16-06-2016 |
| | | WO 2013164694 A1 | 07-11-2013 |
| WO 2016075278 A1 | 19-05-2016 | US 2016200833 A1 | 14-07-2016 |
| | | WO 2016075278 A1 | 19-05-2016 |
| WO 2010010051 A1 | 28-01-2010 | CA 2731388 A1 | 28-01-2010 |
| | | CA 2910512 A1 | 28-01-2010 |
| | | DK 2310509 T3 | 27-04-2015 |
| | | DK 2604693 T3 | 30-05-2016 |
| | | EP 2310509 A1 | 20-04-2011 |
| | | EP 2604693 A2 | 19-06-2013 |
| | | EP 2806029 A1 | 26-11-2014 |
| | | ES 2538122 T3 | 17-06-2015 |
| | | ES 2571879 T3 | 27-05-2016 |
| | | HR P20150425 T1 | 05-06-2015 |
| | | JP 5674155 B2 | 25-02-2015 |
| | | JP 2011528562 A | 24-11-2011 |
| | | JP 2014230545 A | 11-12-2014 |
| | | JP 2014230546 A | 11-12-2014 |
| | | SI 2310509 T1 | 31-07-2015 |
| | | SM T201500135 B | 09-07-2015 |
| | | US 2011162095 A1 | 30-06-2011 |
| | | US 2014056843 A1 | 27-02-2014 |
| | | US 2015110734 A1 | 23-04-2015 |
| | | US 2015125419 A1 | 07-05-2015 |
| | | WO 2010010051 A1 | 28-01-2010 |
| WO 2015095423 A2 | 25-06-2015 | AU 2014364606 A1 | 07-07-2016 |
| | | CA 2934028 A1 | 25-06-2015 |
| | | SG 11201604979W A | 28-07-2016 |
| | | US 2015190506 A1 | 09-07-2015 |
| | | WO 2015095423 A2 | 25-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010010051 A
- WO 2003042402 A
- WO 2004004771 A
- WO 2004056875 A
- WO 2004072286 A
- WO 2004087196 A
- WO 2006121168 A
- WO 2006133396 A
- WO 2007005874 A
- WO 2008083174 A
- WO 2008156712 A
- WO 2009024531 A
- WO 2009014708 A
- WO 2009101611 A
- WO 2009114335 A
- WO 2009154335 A
- WO 2010027828 A
- WO 2010027423 A
- WO 2010029434 A
- WO 2010029435 A
- WO 2010036959 A
- WO 2010063011 A
- WO 2010089411 A
- WO 2011066342 A
- WO 2011110604 A
- WO 2011110621 A
- WO 2012145493 A
- WO 2013014668 A
- WO 2014179664 A
- WO 2015112900 A
- WO 2002020565 A
- WO 9316185 A
- US 5571894 A
- US 5587458 A
- US 5869046 A
- EP 404097 A
- WO 199301161 A
- US 6248516 B1
- US 7166697 B1
- US 7250297 B1
- US 20070224633 A
- EP 1641818 A1
- US 20040132028 A1
- US 20080139791 A
- WO 2005056764 A
- US 6818418 B1
- WO 2008098796 A
- US 5821333 A
- US 5731168 A
- US 7695936 B
- US 20030157108 A, Presta, L.
- US 20040093621 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 7521541 B
- WO 2012130831 A1
- US 6737056 B
- US 7332581 B
- WO 2012130831 A
- US 5500362 A
- US 5821337 A
- WO 2006029879 A
- WO 2005100402 A
- WO 2009089004 A
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- WO 2012020006 A
- US 20040132066 A
- US 20050260186 A
- US 20060104968 A
- US 6267958 B
- US 6171586 B
- WO 2006044908 A
- WO 2015150447 A


### Non-patent literature cited in the description


- **BANNER et al.** *Cell,* 1993, vol. 73, 431-445

- **OKAZAKI et al.** *Curr. Opin. Immunol.,* 2002, vol. 14, 391779-82
- **BENNETT et al.** *J Immunol,* 2003, vol. 170, 711-8
- **SHERIDAN.** *Nature Biotechnology,* 2012, vol. 30, 729-730
- **KOYAMA et al.** *Nature Communications,* 2016
- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **HOUSTON, J.S.** *Methods in Enzymol.,* 1991, vol. 203, 46-96
- **GEBAUER ; SKERRA.** Engineered protein scaffolds as next-generation antibody therapeutics. *Curr Opin Chem Biol,* 2009, vol. 13, 245-255
- **STUMPP et al.** Darpins: A new generation of protein therapeutics. *Drug Discovery Today,* 2008, vol. 13, 695-701
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- **LILJEBLAD et al.** *Glyco J,* 2000, vol. 17, 323-329
- **HEELEY.** *Endocr Res,* 2002, vol. 28, 217-229
- **ISHIDA et al.** *EMBO J.,* 1992, vol. 11, 3887-3895
- **AGATA et al.** *Int. Immunology,* 1996, vol. 8, 765-772
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ALMAGRO ; FRANSSON.** *Front. Biosci.,* 2008, vol. 13, 1619-1633
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **BOWIE, J. U. et al.** *Science,* 1990, vol. 247, 1306-10
- **CUNNINGHAM ; WELLS.** *Science,* 1989, vol. 244, 1081-1085
- **WRIGHT et al.** *TIBTECH,* 1997, vol. 15, 26-32
- **KAM, N.W. et al.** *Proc. Natl. Acad. Sci. USA,* 2005, vol. 102, 11600-11605
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202, 163
- **CRAGG et al.** *Blood,* 2003, vol. 101, 1045-1052
- **CRAGG ; GLENNIE.** *Blood,* 2004, vol. 103, 2738-2743
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525

- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** *Methods in Molecular Biology,* vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- Remington's Pharmaceutical Sciences. Mack Printing Company, 1990
- **FINGL et al.** The Pharmacological Basis of Therapeutics. 1975, 1
- **KABAT, E.A. et al.** Sequences of Proteins of Immunological Interest. Public Health Service, National Institutes of Health, 1991
- **SAMBROOK et al.** Molecular cloning: A laboratory manual. Cold Spring Harbor Laboratory Press, Cold Spring Harbor, 1989
- **KABAT, E.A. et al.** Sequences of Proteins of Immunological Interest. NIH Publication, 1991
- Current Protocols in Cell Biology. John Wiley & Sons, Inc, 2000
- **ASCIERTO, P. A. ; E. SIMEONE ; M. SZNOL ; Y. X. FU ; I. MELERO.** Clinical experiences with anti-CD137 and anti-PD1 therapeutic antibodies. *Semin Oncol,* 2010, vol. 37, 508-516
- **AGGARWAL B.B.** Signalling pathways of the TNF superfamily: a double-edged sword. *Nat. Rev. Immunol.,* 2003, vol. 3 (9), 745-56
- **BANNER D. et al.** Crystal structure of the soluble human 55 kd TNF receptor-human TNF beta complex: implications for TNF receptor activation. *Cell,* 1993, vol. 73, 431-445
- **BODMER J. ; SCHNEIDER P. ; TSCHOPP, J.** The molecular architecture of the TNF superfamily. *Trends in Biochemical Sciences,* 2002, vol. 27 (1), 19-26
- **BROLL, K. ; RICHTER, G. ; PAULY, S. ; HOFSTAEDTER, F. ; SCHWARZ, H.** CD137 expression in tumor vessel walls. High correlation with malignant tumors. *Am J Clin Pathol,* 2001, vol. 115, 543-549
- **BUECHELE, C. ; BAESSLER, T. ; SCHMIEDEL, B.J. ; SCHUMACHER, C.E. ; GROSSE-HOVEST, L. ; RITTIG, K. ; SALIH, H.R.** 4-1BB ligand modulates direct and Rituximab-induced NK-cell reactivity in chronic lymphocytic leukemia. *Eur J Immunol,* 2012, vol. 42, 737-748
- **CHEN S. ; LEE L. ; FISHER T. ; JESSEN B. ; ELLIOTT M. ; EVERING W. ; LOGRONIO K. ; TU K.H. ; TSAPARIKOS K. ; LI X.** Combination of 4-1BB Agonist and PD-1 Antagonist Promotes Antitumor Effector/Memory CD8 T Cells in a Poorly Immunogenic Tumor Model. *Cancer Immunology Research,* 2015, vol. 3 (2), 149-160
- **CHOI, B.K. ; KIM, Y.H. ; KWON, P.M. ; LEE, S.C. ; KANG, S.W. ; KIM, M.S. ; LEE, M.J. ; KWON, B.S.** 4-1BB functions as a survival factor in dendritic cells. *J Immunol,* 2009, vol. 182, 4107-4115
- **CUADROS, C. ; DOMINGUEZ, A.L. ; LOLLINI, P.L. ; CROFT, M. ; MITTLER, R.S. ; BORGSTROM, P. ; LUSTGARTEN, J.** Vaccination with dendritic cells pulsed with apoptotic tumors in combination with anti-OX40 and anti-4-1BB monoclonal antibodies induces T cell-mediated protective immunity in Her-2/neu transgenic mice. *Int J Cancer,* 2005, vol. 116, 934-943
- **CURRAN, M.A. ; KIM, M. ; MONTALVO, W. ; AL-SHAMKHANI, A. ; ALLISON, J.P.** Combination CTLA-4 blockade and 4-1BB activation enhances tumor rejection by increasing T-cell infiltration, proliferation, and cytokine production. *PLoS One,* 2011, vol. 6, e19499
- **DIEHL, L. ; VAN MIERLO, G.J. ; DEN BOER, A.T. ; VAN DER VOORT, E. ; FRANSEN, M. ; VAN BOSTELEN, L. ; KRIMPENFORT, P. ; MELIEF, C.J. ; MITTLER, R. ; TOES, R.E.** In vivo triggering through 4-1BB enables Th-independent priming of CTL in the presence of an intact CD28 costimulatory pathway. *J Immunol,* 2002, vol. 168, 3755-3762

- **DUBROT, J. ; MILHEIRO, F. ; ALFARO, C. ; PALAZON, A. ; MARTINEZ-FORERO, I. ; PEREZ-GRACIA, J.L. ; MORALES-KASTRESANA, A. ; ROMERO-TREVEJO, J.L. ; OCHOA, M.C. ; HERVAS-STUBBS, S. et al.** Treatment with anti-CD 137 mAbs causes intense accumulations of liver T cells without selective antitumor immunotherapeutic effects in this organ. *Cancer Immunol Immunother,* 2010, vol. 59, 1223-1233
- **FUTAGAWA, T. ; AKIBA, H. ; KODAMA, T. ; TAKEDA, K. ; HOSODA, Y. ; YAGITA, H. ; OKUMURA, K.** Expression and function of 4-1BB and 4-1BB ligand on murine dendritic cells. *Int Immunol,* 2002, vol. 14, 275-286
- **GUO, Z. ; CHENG, D. ; XIA, Z. ; LUAN, M. ; WU, L. ; WANG, G. ; ZHANG, S.** Combined TIM-3 blockade and CD 137 activation affords the long-term protection in a murine model of ovarian cancer. *J Transl Med,* 2013, vol. 11, 215
- **HEINISCH, I.V. ; DAIGLE, I. ; KNOPFLI, B. ; SIMON, H.U.** CD137 activation abrogates granulocyte-macrophage colony-stimulating factor-mediated anti-apoptosis in neutrophils. *Eur J Immunol,* 2000, vol. 30, 3441-3446
- **HORNIG, N. ; KERMER, V. ; FREY, K. ; DIEBOLDER, P. ; KONTERMANN, R.E. ; MUELLER, D.** Combination of a bispecific antibody and costimulatory antibody-ligand fusion proteins for targeted cancer immunotherapy. *J. Immunother.,* 2012, vol. 35, 418-429
- **JU, S.A. ; CHEON, S.H. ; PARK, S.M. ; TAM, N.Q. ; KIM, Y.M. ; AN, W.G. ; KIM, B.S.** Eradication of established renal cell carcinoma by a combination of 5-fluorouracil and anti-4-1BB monoclonal antibody in mice. *Int J Cancer,* 2008, vol. 122, 2784-2790
- **KIENZLE, G. ; VON KEMPIS, J.** CD137 (ILA/4-1BB), expressed by primary human monocytes, induces monocyte activation and apoptosis of B lymphocytes. *Int Immunol,* 2000, vol. 12, 73-82
- **KIM, D.H. ; CHANG, W.S. ; LEE, Y.S. ; LEE, K.A. ; KIM, Y.K. ; KWON, B.S. ; KANG, C.Y.** 4-1BB engagement costimulates NKT cell activation and exacerbates NKT cell ligand-induced airway hyperresponsiveness and inflammation. *J Immunol,* 2008, vol. 180, 2062-2068
- **KIM, Y.H. ; CHOI, B.K. ; OH, H.S. ; KANG, W.J. ; MITTLER, R.S. ; KWON, B.S.** Mechanisms involved in synergistic anticancer effects of anti-4-1BB and cyclophosphamide therapy. *Mol Cancer Ther,* 2009, vol. 8, 469-478
- **KWON, B.S. ; WEISSMAN, S.M.** cDNA sequences of two inducible T-cell genes. *Proc Natl Acad Sci U S A,* 1989, vol. 86, 1963-1967
- **LEE, H. ; PARK, H.J. ; SOHN, H.J. ; KIM, J.M. ; KIM, S.J.** Combinatorial therapy for liver metastatic colon cancer: dendritic cell vaccine and low-dose agonistic anti-4-1BB antibody costimulatory signal. *J Surg Res,* 2011, vol. 169, e43-50
- **LEVITSKY, V. ; DE CAMPOS-LIMA, P.O. ; FRISAN, T. ; MASUCCI, M.G.** The clonal composition of a peptide-specific oligoclonal CTL repertoire selected in response to persistent EBV infection is stable over time. *J Immunol,* 1998, vol. 161, 594-601
- **LI, F. ; RAVETCH, J.V.** Inhibitory Fcgamma receptor engagement drives adjuvant and anti-tumor activities of agonistic CD40 antibodies. *Science,* 2011, vol. 333, 1030-1034
- **LIN, W. ; VOSKENS, C.J. ; ZHANG, X. ; SCHINDLER, D.G. ; WOOD, A. ; BURCH, E. ; WEI, Y. ; CHEN, L. ; TIAN, G. ; TAMADA, K. et al.** Fc-dependent expression of CD137 on human NK cells: insights into ''agonistic'' effects of anti-CD 137 monoclonal antibodies. *Blood,* 2008, vol. 112, 699-707
- **MELERO, I. ; JOHNSTON, J.V. ; SHUFFORD, W.W. ; MITTLER, R.S. ; CHEN, L.** NK1.1 cells express 4-1BB (CDw137) costimulatory molecule and are required for tumor immunity elicited by anti-4-1BB monoclonal antibodies. *Cell Immunol,* 1998, vol. 190, 167-172
- **MELERO, I. ; SHUFORD, W.W. ; NEWBY, S.A. ; ARUFFO, A. ; LEDBETTER, J.A. ; HELLSTROM, K.E. ; MITTLER, R.S. ; CHEN, L.** Monoclonal antibodies against the 4-1BB T-cell activation molecule eradicate established tumors. *Nat Med,* 1997, vol. 3, 682-685
- **MERCHANT, A.M. ; ZHU, Z. ; YUAN, J.Q. ; GODDARD, A. ; ADAMS, C.W. ; PRESTA, L.G. ; CARTER, P.** An efficient route to human bispecific IgG. *Nat Biotechnol,* 1998, vol. 16, 677-681
- **MORALES-KASTRESANA, A. ; SANMAMED, M.F. ; RODRIGUEZ, I. ; PALAZON, A. ; MARTINEZ-FORERO, I. ; LABIANO, S. ; HERVAS-STUBBS, S. ; SANGRO, B. ; OCHOA, C. ; ROUZAUT, A. et al.** Combined immunostimulatory monoclonal antibodies extend survival in an aggressive transgenic hepatocellular carcinoma mouse model. *Clin Cancer Res,* 2013, vol. 19, 6151-6162
- **MUELLER, D. ; FREY, K. ; KONTERMANN, R.E.** A novel antibody-4-1BB1 fusion protein for targeted costimulation in cancer immunotherapy. *J. Immunother.,* 2008, vol. 31, 714-722
- **MURILLO, O. ; DUBROT, J. ; PALAZON, A. ; ARINA, A. ; AZPILIKUETA, A. ; ALFARO, C. ; SOLANO, S. ; OCHOA, M.C. ; BERASAIN, C. ; GABARI, I. et al.** In vivo depletion of DC impairs the anti-tumor effect of agonistic anti-CD137 mAb. *Eur J Immunol,* 2009, vol. 39, 2424-2436
- **NARAZAKI, H. ; ZHU, Y. ; LUO, L. ; ZHU, G. ; CHEN, L.** CD137 agonist antibody prevents cancer recurrence: contribution of CD 137 on both hematopoietic and nonhematopoietic cells. *Blood,* 2010, vol. 115, 1941-1948

- **NISHIMOTO, H. ; LEE, S.W. ; HONG, H. ; POTTER, K.G. ; MAEDA-YAMAMOTO, M. ; KINOSHITA, T. ; KAWAKAMI, Y. ; MITTLER, R.S. ; KWON, B.S. ; WARE, C.F. et al.** Costimulation of mast cells by 4-1BB, a member of the tumor necrosis factor receptor superfamily, with the high-affinity IgE receptor. *Blood,* 2005, vol. 106, 4241-4248
- **OLOFSSON, P.S. ; SODERSTROM, L.A. ; WAGSATER, D. ; SHEIKINE, Y. ; OCAYA, P. ; LANG, F. ; RABU, C. ; CHEN, L. ; RUDLING, M. ; AUKRUST, P. et al.** CD137 is expressed in human atherosclerosis and promotes development of plaque inflammation in hypercholesterolemic mice. *Circulation,* 2008, vol. 117, 1292-1301
- **PALAZON, A. ; TEIJEIRA, A. ; MARTINEZ-FORERO, I. ; HERVAS-STUBBS, S. ; RONCAL, C. ; PENUELAS, I. ; DUBROT, J. ; MORALES-KASTRESANA, A. ; PEREZ-GRACIA, J.L. ; OCHOA, M.C. et al.** Agonist anti-CD137 mAb act on tumor endothelial cells to enhance recruitment of activated T lymphocytes. *Cancer Res,* 2011, vol. 71, 801-811
- **SCHWARZ, H. ; VALBRACHT, J. ; TUCKWELL, J. ; VON KEMPIS, J. ; LOTZ, M.** ILA, the human 4-1BB homologue, is inducible in lymphoid and other cell lineages. *Blood,* 1995, vol. 85, 1043-1052
- **SHAO, Z. ; SCHWARZ, H.** CD137 ligand, a member of the tumor necrosis factor family, regulates immune responses via reverse signal transduction. *J Leukoc Biol,* 2011, vol. 89, 21-29
- **SHI, W. ; SIEMANN, D.W.** Augmented antitumor effects of radiation therapy by 4-1BB antibody (BMS-469492) treatment. *Anticancer Res,* 2006, vol. 26, 3445-3453
- **SIMEONE, E. ; ASCIERTO, P.A.** Immunomodulating antibodies in the treatment of metastatic melanoma: the experience with anti-CTLA-4, anti-CD137, and anti-PD1. *J Immunotoxicol,* 2012, vol. 9, 241-247
- **SNELL, L.M. ; LIN, G.H. ; MCPHERSON, A.J. ; MORAES, T.J. ; WATTS, T.H.** T-cell intrinsic effects of GITR and 4-1BB during viral infection and cancer immunotherapy. *Immunol Rev,* 2011, vol. 244, 197-217
- **STAGG, J. ; LOI, S. ; DIVISEKERA, U. ; NGIOW, S.F. ; DURET, H. ; YAGITA, H. ; TENG, M.W. ; SMYTH, M.J.** Anti-ErbB-2 mAb therapy requires type I and II interferons and synergizes with anti-PD-1 or anti-CD137 mAb therapy. *Proc Natl Acad Sci U S A,* 2011, vol. 108, 7142-7147
- **TENG, M.W. ; SHARKEY, J. ; MCLAUGHLIN, N.M. ; EXLEY, M.A. ; SMYTH, M.J.** CD1d-based combination therapy eradicates established tumors in mice. *J Immunol,* 2009, vol. 183, 1911-1920
- **VON KEMPIS, J. ; SCHWARZ, H. ; LOTZ, M.** Differentiation-dependent and stimulus-specific expression of ILA, the human 4-1BB-homologue, in cells of mesenchymal origin. *Osteoarthritis Cartilage,* 1997, vol. 5, 394-406
- **WEI, H. ; ZHAO, L. ; LI, W. ; FAN, K. ; QIAN, W. ; HOU, S. ; WANG, H. ; DAI, M. ; HELLSTROM, I. ; HELLSTROM, K.E.** Combinatorial PD-1 blockade and CD 137 activation has therapeutic efficacy in murine cancer models and synergizes with cisplatin. *PLoS One,* 2013, vol. 8, e84927
- **WEI H. ; ZHAO L. ; HELLSTROM I. ; HELLSTROM K.E. ; GUO Y.** Dual targeting of CD137 costimulatory and PD-1 co-inhibitory molecules for ovarian cancer immunotherapy. *OncoImmunology,* 2014, vol. 3 (4), e28248
- **WILCOX, R.A. ; CHAPOVAL, A.I. ; GORSKI, K.S. ; OTSUJI, M. ; SHIN, T. ; FLIES, D.B. ; TAMADA, K. ; MITTLER, R.S. ; TSUCHIYA, H. ; PARDOLL, D.M.** Cutting edge: Expression of functional CD137 receptor by dendritic cells. *J Immunol,* 2002, vol. 168, 4262-4267
- **WILCOX, R.A. ; TAMADA, K. ; FLIES, D.B. ; ZHU, G. ; CHAPOVAL, A.I. ; BLAZAR, B.R. ; KAST, W.M. ; CHEN, L.** Ligation of CD137 receptor prevents and reverses established anergy of CD8+ cytolytic T lymphocytes in vivo. *Blood,* 2004, vol. 103, 177-184
- **ZHANG, N. ; SADUN, R.E. ; ARIAS, R.S. ; FLANAGAN, M.L. ; SACHSMAN, S.M. ; NIEN, Y ; KHAWLI, L.A. ; HU, P. ; EPSTEIN, A.L.** Targeted and untargeted CD137L fusion proteins for the immunotherapy of experimental solid tumors. *Clin. Cancer Res.,* 2007, vol. 13, 2758-2767
- **ZHANG, X. ; VOSKENS, C.J. ; SALLIN, M. ; MANIAR, A. ; MONTES, C.L. ; ZHANG, Y. ; LIN, W. ; LI, G. ; BURCH, E. ; TAN, M. et al.** CD137 promotes proliferation and survival of human B cells. *J Immunol,* 2010, vol. 184, 787-795